# EUROPEAN PATENT APPLICATION

(11) **EP 4 497 751 A1**
(43) Date of publication of application: **29.01.2025**
(21) Application number: 23773876.0
(22) Date of filing: 21.03.2023
(51) Int. Cl.: C07D 471/00, C07D 471/02, C07D 471/04, A61K 31/435, A61K 31/4375, A61K 31/517, A61P 35/00

(54) **SUBSTITUTED BRIDGED RING INHIBITOR, PREPARATION METHOD THEREFOR AND APPLICATION THEREOF**

(30) Priority: 22.03.2022 CN 202210288030; 23.08.2022 CN 202211015208; 13.01.2023 CN 202310059893
(71) Applicant: Suzhou Zelgen Biopharmaceuticals Co., Ltd., Suzhou, Jiangsu 215300 (CN); Shanghai Zelgen Pharma.Tech Co., Ltd., Shanghai 201203 (CN)
(72) Inventor: LV, Binhua, Shanghai 201203 (CN); CUI, Dawei, Shanghai 201203 (CN); ZHANG, Qing, Shanghai 201203 (CN)
(74) Representative: dompatent von Kreisler Selting Werner - Partnerschaft von Patent- und Rechtsanwälten mbB
(86) International application number: PCT/CN2023/082908
(87) International publication number: WO 2023/179629

(57) **Abstract**

The present invention relates to a substituted bridged ring inhibitor, and to a preparation method therefor and application thereof. Specifically, the compound of the present invention has the structure shown in a formula (A0), and further disclosed in the present invention are a method for preparing the compound and an application of the compound as a **KRAS^{G12D}** inhibitor; the compound has a good selective inhibition effect on KRAS^{G12D}, and has better pharmacodynamic and pharmacokinetic performance and lower toxic side effects.

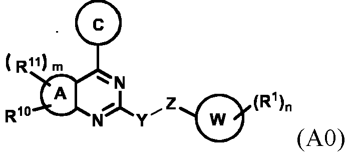

## Description

### TECHNICAL FIELD

The present invention belongs to the pharmaceutical field, specifically, relates to a substituted bridged ring inhibitor, preparation method therefor and application thereof.

### BACKGROUND

About a quarter of all human tumors are caused by RAS mutations, and nearly one million people lose their lives each year. In the RAS family, KRAS mutations account for 85% of all RAS mutations. KRAS mutations are found in nearly 90% of pancreatic cancer, 30-40% of colon cancer, and 15-20% of lung cancer (mainly non-small cell lung cancer). The mian mutations in KRAS mutations are G12C and G12D mutations, wherein G12C mutations mainly occur in HSCLC, while G12D mutations mainly occur in pancreatic cancer. So far, no drugs targeting KRAS^{G12D} mutations have been approved for marketing.

Currently, conventional treatments for pancreatic cancer clinically include gemcitabine monotherapy, gemcitabine combined with albumin-paclitaxel, and FOLFIRINOX regimen (oxaliplatin + irinotecan + 5-FU/LV) and the like. Among them, liposomal irinotecan is suitable for use in combination with fluorouracil and folinic acid to treat patients with advanced pancreatic cancer who has not responded well to gemcitabine chemotherapy (second-line therapy). But in general, there are currently limited effective treatments for pancreatic cancer, and the total survival time of patients does not exceed 1 year. Although drug exploration for patients with advanced pancreatic cancer is ongoing, research progress has been slow so far.

Because the KRAS^{G12D} target protein is pathologically associated with a variety of diseases, especially pancreatic cancer, new KRAS^{G12D} inhibitors are currently needed for clinical treatment. Highly selective and highly active KRAS^{G12D} inhibitors can more effectively treat diseases such as cancer caused by KRAS^{G12D} mutations, and have the potential to reduce off-target effects, therefore having a more urgent clinical need.

### SUMMARY OF THE INVENTION

The purpose of the present invention is to provide a novel class of compounds having selective inhibitory effects on KRAS^{G12D} and/or better pharmacodynamic properties and uses thereof.

In the first aspect of the present invention, provided is a compound of formula (A0), or a stereoisomer, a tautomer, a crystal form, a pharmaceutically acceptable salt, a hydrate, a solvate, or a prodrug thereof: 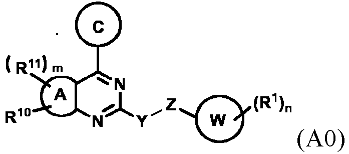
ring C is a group selected from the group consisting of: 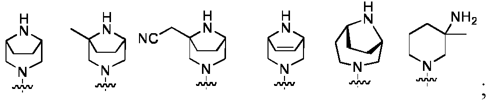
Y is selected from bond, O, NH, and N(C₁-C₃alkyl);
Z is substituted or unsubstituted C₁-C₆ alkylene; wherein said substituted refers to being substituted with one or more R;
W is selected from substituted or unsubstituted C₃-C₁₄cycloalkyl, and substituted or unsubstituted 4-14 membered saturated or unsaturated heterocyclyl; wherein said substituted refers to being substituted with one or more R; 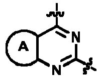 is a group selected from the group consisting of: 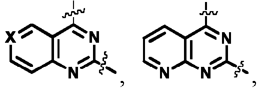 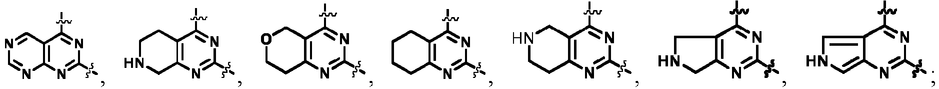 wherein, X is selected from N, CH, CD, CF, and C(CN);
R¹ is selected from -L₁-Q-L₂-L₃;
wherein,
L₁ is selected from substituted or unsubstituted C₁-C₆alkylene; wherein said substituted refers to being substituted with one or more R;
Q is selected from O, S, SO₂, NH, and N(C₁-C₃alkyl);
L₂ is selected from null, and substituted or unsubstituted C₁-C₆alkylene; wherein said substituted refers to being substituted with one or more R;
L₃ is a substituted or unsubstituted group selected from the group consisting of -C₁-C₆alkyl, -C₃-C₆cycloalkyl, -C₄-C₆heterocyclyl, -C₁-C₆alkylene(C₃-C₆cycloalkyl), -C₁-C₆alkylene(C₄-C₆heterocyclyl), -C₁-C₆alkylene(C₁-C₆alkoxy), -C₁-C₆alkylene(C₃-C₆cycloalkoxy), and -C₁-C₆alkylene(C₄-C₆heterocyclyloxy); wherein said substituted refers to being substituted with one or more R;
n is an integer of 0, 1, 2, 3, 4, 5 or 6; provided that when W is a monocyclic or bicyclic ring, n is not 0;
R¹⁰ is a substituted or unsubstituted group selected from the group consisting of C₆-C₁₄ aryl, and 5-14 membered heteroaryl; wherein said substituted refers to being substituted with one or more Ra;
R¹¹ is each independently a substituted or unsubstituted group selected from the group consisting of: H, deuterium, halogen, cyano, ester group, amino, amido, sulfonyl , ureido, C₁-C₆alkyl, C₁-C₆deuterated alkyl, C₁-C₆haloalkyl, C₃-C₆cycloalkyl, 4-6 membered heterocyclyl, C₁-C₆alkoxy, C₃-C₆cycloalkyloxy, and 4-6 membered heterocyclyloxy; wherein said substituted refers to being substituted with one or more R;
Ra is each independently a substituted or unsubstituted group selected from the group consisting of: H, D, halogen, CN, NH₂, OH, CONH₂, NHCO(C₁-C₆alkyl), NHCO(C₃-C₆cycloalkyl), SONH₂, SO₂NH₂, NHSO₂(C₁-C₆alkyl), NHSO₂(C₃-C₆cycloalkyl), C₁-C₆alkyl, C₁-C₆deuterated alkyl, C₁-C₆haloalkyl, C₁-C₆alkoxy, C₂-C₆alkenyl, C₂-C₆alkynyl, C₃-C₂₀cycloalkyl, 4-20 membered heterocyclyl, C₃-C₂₀cycloalkyloxy, 4-20 membered heterocyclyloxy; wherein said substituted refers to being substituted with one or more R;
m is an integer of 0, 1, 2, 3, 4, 5 or 6;
each R is the same or different, and is each independently selected from deuterium, C₁-C₁₈ alkyl, deuterated C₁-C₁₈ alkyl, halogenated C₁-C₁₈ alkyl, (C₃-C₁₈ cycloalkyl)C₁-C₁₈ alkyl, (4-20 membered heterocyclyl)C₁-C₁₈ alkyl, (C₁-C₁₈ alkoxy)C₁-C₁₈ alkyl, (C₃-C₁₈cycloalkyloxy)C₁-C₁₈ alkyl, (4-20 membered heterocyclyloxy)C₁-C₁₈ alkyl, ethenyl, ethynyl, (C₁-C₆ alkyl)ethenyl, deuterated (C₁-C₆ alkyl)ethenyl, halogenated (C₁-C₆ alkyl)ethenyl, (C₁-C₆ alkyl)ethynyl, deuterated (C₁-C₆ alkyl)ethynyl, halogenated (C₁-C₆ alkyl)ethynyl, (C₃-C₁₄ cycloalkyl)ethynyl, (4-14 membered heterocyclyl)ethynyl, C₁-C₁₈ alkoxy, deuterated C₁-C₁₈alkoxy, halogenated C₁-C₁₈alkoxy, 4-20 membered heterocyclylC(O), C₃-C₂₀ cycloalkyl, 4-20 membered heterocyclyl, C₆-C₁₄aryl, 5-14 membered heteroaryl, halogen, nitro, hydroxy, oxo, cyano, ester group, amino, amido, sulfonamido, sulfonyl, and ureido.

In another preferred embodiment, the compound, or a stereoisomer, a tautomer, a crystal form, a pharmaceutically acceptable salt, a hydrate, a solvate, or a prodrug thereof has a structure of formula (A): 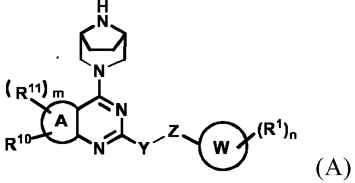
Y is selected from bond, O, NH, and N(C₁-C₃alkyl);
Z is substituted or unsubstituted C₁-C₆ alkylene; wherein said substituted refers to being substituted with one or more R;
W is selected from substituted or unsubstituted C₃-C₁₄cycloalkyl, and substituted or unsubstituted 4-14 membered saturated or unsaturated heterocyclyl; wherein said substituted refers to being substituted with one or more R; 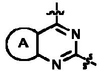
is a group selected from the group consisting of: 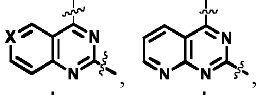 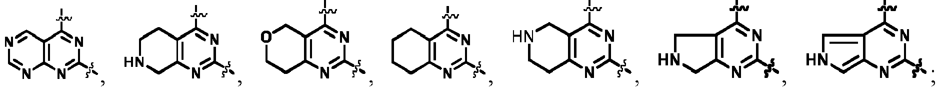 wherein X is selected from N, CH, CD, CF, and C(CN);
R¹ is selected from -L₁-Q-L₂-L₃;
wherein:
   L₁ is selected from substituted or unsubstituted C₁-C₆alkylene; wherein said substituted refers to being substituted with one or more R;
   Q is selected from O, S, SO₂, NH, and N(C₁-C₃alkyl);
   L₂ is selected from null, and substituted or unsubstituted C₁-C₆alkylene; wherein said substituted refers to being substituted with one or more R;
   L₃ is a substituted or unsubstituted group selected from the group consisting of -C₁-C₆alkyl, -C₃-C₆cycloalkyl, -C₄-C₆heterocyclyl, -C₁-C₆alkylene(C₃-C₆cycloalkyl), -C₁-C₆alkylene(C₄-C₆heterocyclyl), -C₁-C₆alkylene(C₁-C₆alkoxy), -C₁-C₆alkylene(C₃-C₆cycloalkoxy), and -C₁-C₆alkylene(C₄-C₆heterocyclyloxy); wherein said substituted refers to being substituted with one or more R;
n is an integer of 0, 1, 2, 3, 4, 5 or 6; provided that when W is a monocyclic or bicyclic ring, n is not 0;
R¹⁰ is a substituted or unsubstituted group selected from the group consisting of C₆-C₁₄ aryl, and 5-14 membered heteroaryl; wherein said substituted refers to being substituted with one or more Ra;
R¹¹ is each independently a substituted or unsubstituted group selected from the group consisting of: H, deuterium, halogen, cyano, ester group, amino, amido, sulfonyl , ureido, C₁-C₆alkyl, C₁-C₆deuterated alkyl, C₁-C₆haloalkyl, C₃-C₆cycloalkyl, 4-6 membered heterocyclyl, C₁-C₆alkoxy, C₃-C₆cycloalkoxy, and 4-6 membered heterocyclyloxy;
Ra is each independently a substituted or unsubstituted group selected from the group consisting of: H, D, halogen, CN, NH₂, OH, CONH₂, NHCO(C₁-C₆alkyl), NHCO(C₃-C₆cycloalkyl), SONH₂, SO₂NH₂, NHSO₂(C₁-C₆alkyl), NHSO₂(C₃-C₆cycloalkyl), C₁-C₆alkyl, C₁-C₆deuterated alkyl, C₁-C₆haloalkyl, C₁-C₆alkoxy, C₂-C₆alkenyl, C₂-C₆alkynyl, C₃-C₂₀cycloalkyl, 4-20 membered heterocyclyl, C₃-C₂₀cycloalkoxy, 4-20 membered heterocyclyloxy; wherein said substituted refers to being substituted with one or more R;
m is an integer of 0, 1, 2, 3, 4, 5 or 6;
each R is the same or different, and is each independently selected from deuterium, C₁-C₁₈ alkyl, deuterated C₁-C₁₈ alkyl, halogenated C₁-C₁₈ alkyl, (C₃-C₁₈cycloalkyl)C₁-C₁₈ alkyl, (4-20 membered heterocyclyl)C₁-C₁₈ alkyl, (C₁-C₁₈alkoxy)C₁-C₁₈ alkyl, (C₃-C₁₈cycloalkoxy)C₁-C₁₈ alkyl, (4-20 membered heterocyclyloxy)C₁-C₁₈ alkyl, ethenyl, ethynyl, (C₁-C₆alkyl)ethenyl, deuterated (C₁-C₆alkyl)ethenyl, halogenated (C₁-C₆alkyl)ethenyl, (C₁-C₆alkyl)ethynyl, deuterated (C₁-C₆alkyl)ethynyl, halogenated (C₁-C₆alkyl)ethynyl, (C₃-C₁₄cycloalkyl)ethynyl, (4-14 membered heterocyclyl)ethynyl, C₁-C₁₈alkoxy, deuterated C₁-C₁₈alkoxy, halogenated C₁-C₁₈alkoxy, 4-20 membered heterocyclylC(O), C₃-C₂₀cycloalkyl, 4-20 membered heterocyclyl, C₆-C₁₄aryl, 5-14 membered heteroaryl, halogen, nitro, hydroxy, oxo, cyano, ester group, amino, amido, sulfonamido, sulfonyl, and ureido.

In another preferred embodiment, Ra is each independently a substituted or unsubstituted group selected from the group consisting of: H, D, halogen, CN, OH, SONH₂, NHSO₂CH₃, C₁-C₆alkyl, C₁-C₆deuterated alkyl, C₁-C₆haloalkyl, C₁-C₆alkoxy, C₂-C₆alkenyl, C₂-C₆alkynyl, C₃-C₂₀cycloalkyl, and 4-20 membered heterocyclyl; wherein said substituted refers to being substituted with one or more R; R is defined as above.

In another preferred embodiment, the compound has a structure of formula A': 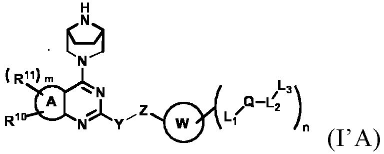 preferably, has a structure of formula A": 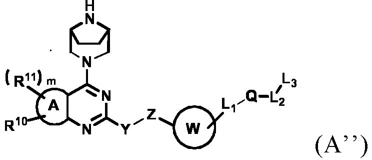 wherein, n, Y, Z, W, L₁, L₂, L₃, Q, R¹⁰, R¹¹, m and ring A are as defined above.

In another preferred embodiment, 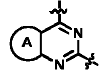 is a group selected from the group consisting of:

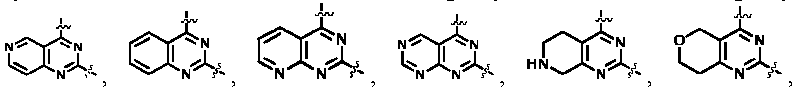

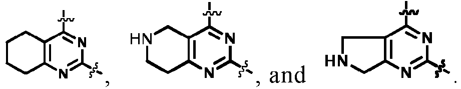

In another preferred embodiment, the compound has a structure of formula A''': 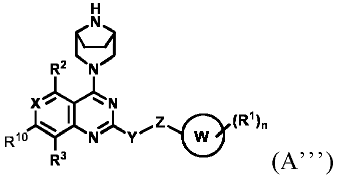
R² and R³ are the same or different, and are each independently a substituted or unsubstituted group selected from the group consisting of: H, D, halogen, CN, C ₁-C₆alkyl, C₁-C₆deuterated alkyl, C₁-C₆haloalkyl, and C₁-C₆alkoxy; wherein said substituted refers to being substituted with one or more R;
X, Y, Z, W, R¹, n and R¹⁰ are as defined above.

In another preferred embodiment, R³ is a substituted or unsubstituted group selected from the group consisting of: H, D, halogen, CN, C₁-C₆alkyl, C₁-C₆deuterated alkyl, C₁-C₆haloalkyl, C₁-C₆alkoxy, C₂-C₆alkenyl, C₂-C₆alkynyl, C₃-C₆cycloalkyl, C₃-C₆cycloalkoxy, 4-6 membered heterocyclyl, and 4-6 membered heterocyclyloxy; wherein said substituted refers to being substituted with one or more R.

In another preferred embodiment, R¹⁰ is selected from: is a substituted or unsubstituted group selected from the group consisting of: phenyl, naphthyl, 5-6membered monocyclic heteroaryl (such as pyridyl), 9-10 membered bicyclic heteroaryl(such as indazolyl, benzothiazole, benzothiophene, and benzofuran), wherein said substituted means being substituted with one or more groups selected from the group consisting of: halogen, hydroxy, cyano, NH₂, C₁-C₆ alkyl, halogenated C₁-C₆ alkyl, C₁-C₆alkoxy, halogenated C₁-C₆alkoxy, C₃-C₆cycloalkyl, 4-6membered heterocyclyl, C₂-C₆alkenyl, and C₂-C₆alkynyl: preferably, R¹⁰ is selected from

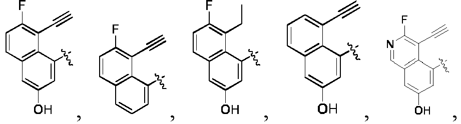

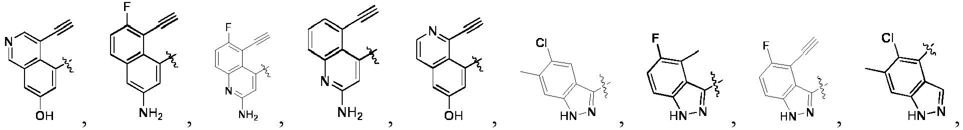

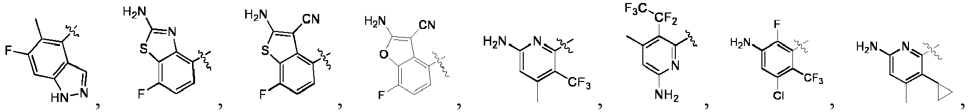

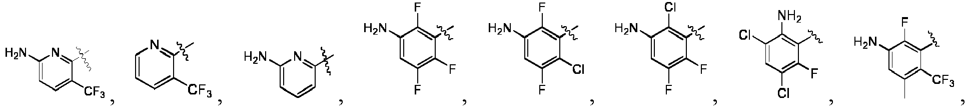

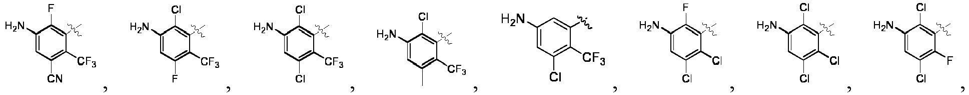

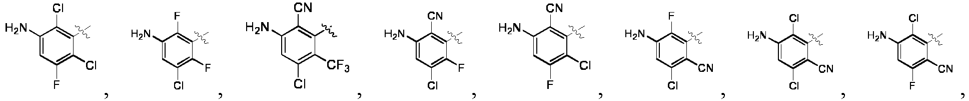

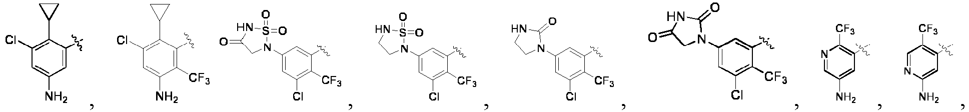

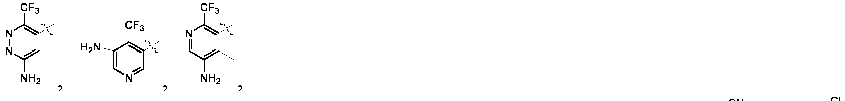

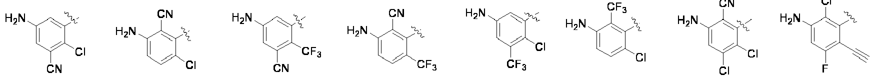

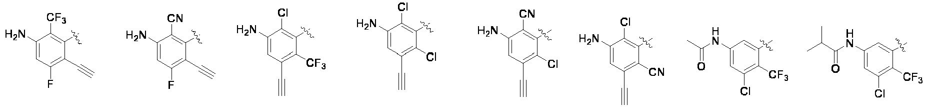

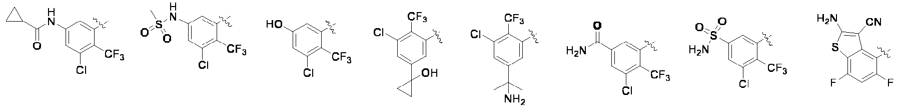

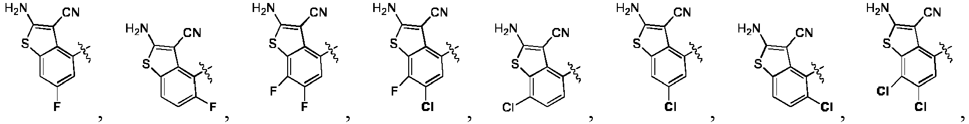

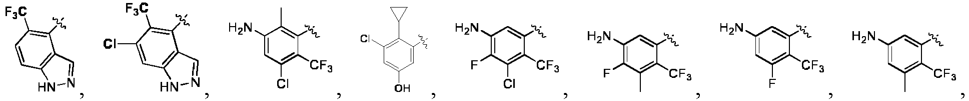

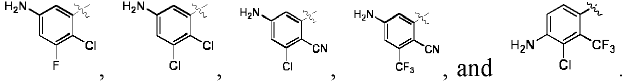

In another preferred embodiment, R⁸ is selected from NH₂, OH, SONH₂, and NHSO₂CH₃.

In another preferred embodiment, R¹⁰ is

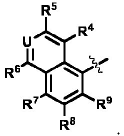

In another preferred embodiment, U is selected from N, CH, CD, and CF;
R⁴ is a substituted or unsubstituted group selected from the group consisting of: halogen, C₁-C₆alkyl, C₂-C₆alkenyl, and C₂-C₆alkynyl;
R⁵, R⁶, R⁷, and R⁹ are the same or different, and are each independently a substituted or unsubstituted group selected from the group consisting of: H, D, halogen, CN, C₁-C₆alkyl, C₁-C₆deuterated alkyl, C₁-C₆haloalkyl, and C₁-C₆alkoxy; wherein said substituted refers to being substituted with one or more R;
R⁸ is selected from: NH₂, OH, SONH₂, and NHSO₂CH₃.

In another preferred embodiment, R¹⁰ is 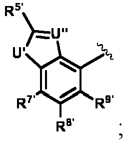 preferably, R¹⁰ is 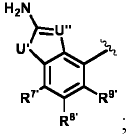 preferably, R¹⁰ is

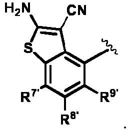

In another preferred embodiment, U' is selected from O, and S;
U" is selected from N, and C(CN);
R⁷ , R⁸, and R^{9'} are the same or different, and are each independently a substituted or unsubstituted group selected from the group consisting of: H, D, halogen, CN, C ₁-C₆alkyl, C₁-C₆deuterated alkyl, C₁-C₆haloalkyl, and C₁-C₆alkoxy; wherein said substituted refers to being substituted with one or more R;
R^{5'} is selected from H, D, halogen, CN, NH₂, OH, CONH₂, NHCO(C₁-C₆alkyl), NHCO(C₃-C₆cycloalkyl), SONH₂, SO₂NH₂, NHS0₂(C₁-C₆alkyl), NHSO₂(C₃-C₆cycloalkyl), C₁-C₆ alkyl, C₁-C₆deuterated alkyl, C₁-C₆haloalkyl, and C₁-C₆alkoxy.

In another preferred embodiment, R¹⁰ is 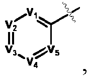 wherein, V₁, V₂, V₃, V₄, and V₅ are each independently selected from N, and CRᵥ; each Rᵥ is the same or different, and is each independently selected from H, C₁-C₃ alkyl, C₃-C₆cycloalkyl, 4-6membered heterocyclyl, C₁-C₃alkoxy, C₃-C₆cycloalkoxy, 4-6membered heterocyclyloxy, halogenated C₁-C₃ alkyl, halogenated C₃-C₆cycloalkyl, halogenated 4-6membered heterocyclyl, (HO)-C₁-C₃ alkyl, (HO)-C₃-C₆cycloalkyl, (NH₂)-C₁-C₃ alkyl, (NH₂)-C₃-C₆cycloalkyl, halogen, CN, -C=CH, OH, NH₂, CONH₂, NHCO(C₁-C₆alkyl), NHCO(C₃-C₆cycloalkyl), SO₂NH₂, NHSO₂(C₁-C₆alkyl), and NHSO₂(C₃-C₆cycloalkyl), wherein the heterocyclyl is optionally substituted with one or more oxo (=O).

In another preferred embodiment, R¹⁰ is 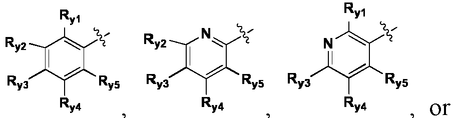 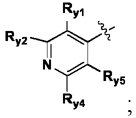 wherein Rᵥ₁, Rᵥ₂, Rᵥ₃, Rᵥ₄ and Rᵥ₅ are each independently selected from H, C₁-C₃ alkyl, C₃-C₆cycloalkyl, 4-6 membered heterocyclyl, C₁-C₃alkoxy, C₃-C₆cycloalkoxy, 4-6membered heterocyclyloxy, halogenated C₁-C₃alkyl(such as CF₃, and CF₂CF₃), halogenated C₃-C₆cycloalkyl, halogenated 4-6 membered heterocyclyl, (HO)-C₁-C₃ alkyl, (HO)-C₃-C₆cycloalkyl, (NH₂)-C₁-C₃ alkyl, (NH₂)-C₃-C₆cycloalkyl, halogen, CN, -C=CH, OH, NH₂, CONH₂, NHCO(C₁-C₆alkyl), NHCO(C₃-C₆cycloalkyl, SO₂NH₂, NHSO₂(C₁-C₆alkyl), NHSO₂(C₃-C₆cycloalkyl)H, C₁-C₃ alkyl, C₃-C₆cycloalkyl, 4-6membered heterocyclyl, C₁-C₃alkoxy, C₃-C₆cycloalkoxy, 4-6 membered heterocyclyloxy, halogenated C₁-C₃ alkyl, halogenated C₃-C₆cycloalkyl, halogenated 4-6 membered heterocyclyl, (HO)-C₁-C₃ alkyl, (HO)-C₃-C₆cycloalkyl, (NH₂)-C₁-C₃ alkyl, (NH₂)-C₃-C₆cycloalkyl, halogen, CN, -C=CH, OH, NH₂, CONH₂, NHCO(C₁-C₆alkyl), NHCO(C₃-C₆cycloalkyl), SO₂NH₂, NHS0₂(C₁-C₆alkyl), and NHSO₂(C₃-C₆cycloalkyl); the heterocyclyl is optionally substituted with one or more oxo (=O).

In another preferred embodiment, Rᵥ₅ is selected from halogenated C₁-C₃alkyl (such as CF₃, and CF₂CF₃).

In another preferred embodiment, Rᵥ₁ is selected from H.

In another preferred embodiment, Rᵥ₁ is selected from NH₂.

In another preferred embodiment, provided is a compoud of formula (IA) or formula (IB), or a stereoisomer, a tautomer, a crystal form, a pharmaceutically acceptable salt, a hydrate, a solvate, or a prodrug thereof: 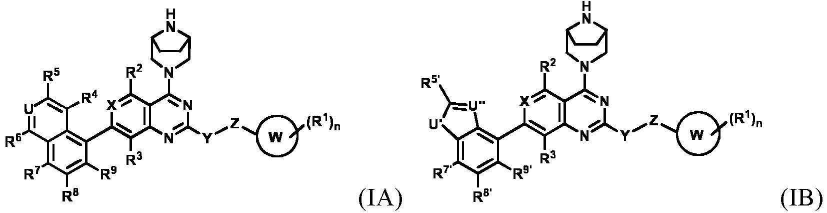 wherein,
U is selected from N, CH, CD, and CF;
U' is selected from O, and S;
U" is selected from N, and C(CN);
X is selected from N, CH, CD, CF, and C(CN);
Y is selected from bond, O, NH, and N(C₁-C₃alkyl);
Z is substituted or unsubstituted C₁-C₆ alkylene; wherein said substituted refers to being substituted with one or more R;
W is selected from substituted or unsubstituted C₃-C₁₄cycloalkyl, and substituted or unsubstituted 4-14 membered saturated or unsaturated heterocyclyl; wherein said substituted refers to being substituted with one or more R;
R¹ is selected from -L₁-Q-L₂-L₃; wherein:
   L₁ is selected from substituted or unsubstituted C₁-C₆alkylene; wherein said substituted refers to being substituted with one or more R;
   Q is selected from O, S, SO₂, NH, and N(C₁-C₃alkyl);
   L₂ is selected from null, and substituted or unsubstituted C₁-C₆alkylene; wherein said substituted refers to being substituted with one or more R;
   L₃ is a substituted or unsubstituted group selected from the group consisting of: -C₁-C₆alkyl, -C₃-C₆cycloalkyl, -C₄-C₆heterocyclyl, -C₁-C₆alkylene(C₃-C₆cycloalkyl), -C₁-C₆alkylene(C₄-C₆heterocyclyl), -C₁-C₆alkylene(C₁-C₆alkoxy), -C₁-C₆alkylene(C₃-C₆cycloalkoxy), and -C₁-C₆alkylene(C₄-C₆heterocyclyloxy); wherein said substituted refers to being substituted with one or more R;
   n is an integer of 1, 2, 3, 4, 5 or 6;
   R² and R³ are the same or different, and are each independently a substituted or unsubstituted group selected from the group consisting of: H, D, halogen, CN, C ₁-C₆alkyl, C₁-C₆deuterated alkyl, C₁-C₆haloalkyl, and C₁-C₆alkoxy; wherein said substituted refers to being substituted with one or more R;
   R⁴ is a substituted or unsubstituted group selected from the group consisting of: halogen, C₁-C₆alkyl, C₂-C₆alkenyl, and C₂-C₆alkynyl; wherein said substituted refers to being substituted with one or more R;
   R⁸ is selected from OH, SONH₂, and NHSO₂CH₃;
   R⁵, R⁶, R⁷, R⁹, R⁷ , R^{8'}, and R^{9'} are the same or different, and are each independently a substituted or unsubstituted group selected from the group consisting of: H, D, halogen, CN, C₁-C₆alkyl, C₁-C₆deuterated alkyl, C₁-C₆haloalkyl, and C₁-C₆alkoxy; wherein said substituted refers to being substituted with one or more R;
   R^{5'} is selected from H, D, halogen, CN, NH₂, OH, CONH₂, NHCO(C₁-C₆alkyl), NHCO(C₃-C₆cycloalkyl), SONH₂, SO₂NH₂, NHSO₂(C₁-C₆alkyl), NHSO₂(C₃-C₆cycloalkyl), C₁-C₆ alkyl, C₁-C₆deuterated alkyl, C₁-C₆haloalkyl, and C₁-C₆alkoxy;
   each R is the same or different, and is each independently selected from deuterium, C₁-C₁₈ alkyl, deuterated C₁-C₁₈ alkyl, halogenated C₁-C₁₈ alkyl, (C₃-C₁₈cycloalkyl)C₁-C₁₈ alkyl, (4-20 membered heterocyclyl)C₁-C₁₈ alkyl, (C₁-C₁₈alkoxy)C₁-C₁₈ alkyl, (C₃-C₁₈cycloalkoxy)C₁-C₁₈ alkyl, (4-20 membered heterocyclyloxy)C₁-C₁₈ alkyl, ethenyl, ethynyl, (C₁-C₆alkyl)ethenyl, deuterated (C₁-C₆alkyl)ethenyl, halogenated (C₁-C₆alkyl)ethenyl, (C₁-C₆alkyl)ethynyl, deuterated (C₁-C₆alkyl)ethynyl, halogenated (C₁-C₆alkyl)ethynyl, (C₃-C₁₄cycloalkyl)ethynyl, (4-14 membered heterocyclyl)ethynyl, C₁-C₁₈alkoxy, deuterated C₁-C₁₈alkoxy, halogenated C₁-C₁₈alkoxy, C₃-C₂₀cycloalkyl, 4-20 membered heterocyclyl, C₆-C₁₄aryl, 5-14 membered heteroaryl, halogen, nitro, hydroxy, oxo, cyano, ester group, amino, amido, sulfonamido, sulfonyl, and ureido.

In another preferred embodiment, each R is the same or different, and is each independently selected from deuterium, C₁-C₁₈ alkyl, deuterated C₁-C₁₈ alkyl, halogenated C₁-C₁₈ alkyl, (C₃-C₁₈cycloalkyl)C₁-C₁₈ alkyl, (4-20 membered heterocyclyl)C₁-C₁₈ alkyl, (C₁-C₁₈alkoxy)C₁-C₁₈ alkyl, (C₃-C₁₈cycloalkoxy)C₁-C₁₈ alkyl, (4-20 membered heterocyclyloxy)C₁-C₁₈ alkyl, ethenyl, ethynyl, (C₁-C₆alkyl)ethenyl, deuterated (C₁-C₆alkyl)ethenyl, halogenated (C₁-C₆alkyl)ethenyl, (C₁-C₆alkyl)ethynyl, deuterated (C₁-C₆alkyl)ethynyl, halogenated (C₁-C₆alkyl)ethynyl, (C₃-C₁₄cycloalkyl)ethynyl, (4-14 membered heterocyclyl)ethynyl, C₁-C₁₈alkoxy, deuterated C₁-C₁₈alkoxy, halogenated C₁-C₁₈alkoxy, C₃-C₂₀cycloalkyl, 4-20 membered heterocyclyl, C₆-C₁₄aryl, 5-14 membered heteroaryl, halogen, nitro, hydroxy, oxo, cyano, ester group, amino, amido, sulfonyl, and ureido.

In another preferred embodiment, each R is the same or different, and is each independently selected from deuterium, C₁-C₆ alkyl, deuterated C₁-C₆ alkyl, halogenated C₁-C₆ alkyl, (C₃-C₁₀cycloalkyl)C₁-C₆ alkyl, (4-10 membered heterocyclyl)C₁-C₆ alkyl, (C₁-C₆alkoxy)C₁-C₆ alkyl, (C₃-C₆cycloalkoxy)C₁-C₆ alkyl, (4-20 membered heterocyclyloxy)C₁-C₁₈ alkyl, ethenyl, ethynyl, (C₁-C₆alkyl)ethenyl, deuterated (C₁-C₆alkyl)ethenyl, halogenated (C₁-C₆alkyl)ethenyl, (C₁-C₆alkyl)ethynyl, deuterated (C₁-C₆alkyl)ethynyl, halogenated (C₁-C₆alkyl)ethynyl, (C₃-C₁₀cycloalkyl)ethynyl, (4-10 membered heterocyclyl)ethynyl, C₁-C₆alkoxy, deuterated C₁-C₆alkoxy, halogenated C₁-C₆alkoxy, C₃-C₁₀cycloalkyl, 4-10 membered heterocyclyl, C₆-C₁₀aryl, 5-10 membered heteroaryl, halogen, nitro, hydroxy, oxo, cyano, ester group, amino, amido, sulfonamido, sulfonyl, and ureido.

In another preferred embodiment, the compound of formula I has a structure of formula (I'A) or formula (I'B); 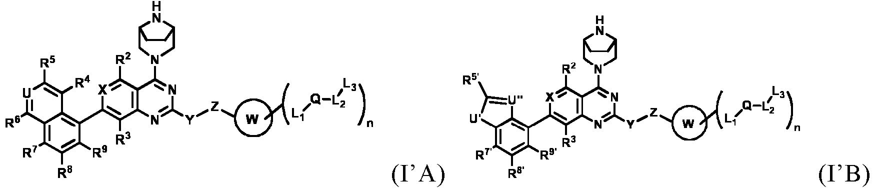 preferably, has a structure of formula (I''A) or formula (I"B) 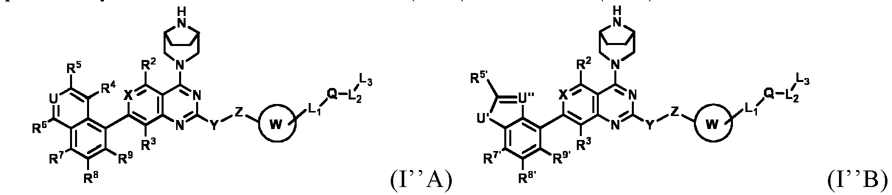 wherein, n, U, U', U", X, Y, Z, W, L₁, L₂, L₃, Q, R², R³, R⁴, R⁵, R⁶, R⁷, R⁸, R⁹, R^{5'}, R^{7'}, R^{8'}, and R^{9'} are defined as above.

In another preferred embodiment, the compound has a structure of formula (IIA) or formula (IIB): 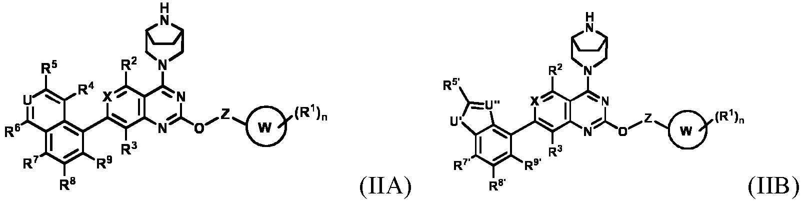 wherein, R¹, R², R³, R⁴, R⁵, R⁶, R⁷, R⁸, R⁹, R^{5'}, R^{7'}, R^{8'}, R^{9'}, U, U', U", X, Z, W and n are defined as above.

In another preferred embodiment, the compound has a structure of formula (IIIA) or formula (IIIB): 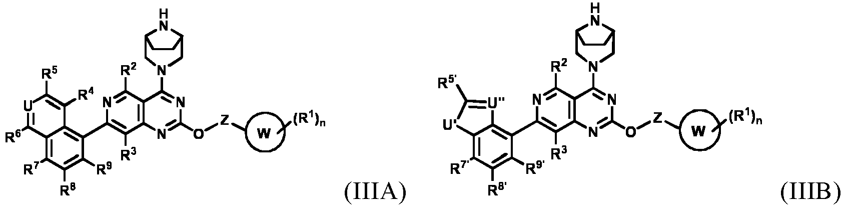 wherein R¹, R², R³, R⁴, R⁵, R⁶, R⁷, R⁸, R⁹, R^{5'}, R^{7'}, R^{8'}, R^{9'}, U, U', U", Z, W and n are defined as above.

In another preferred embodiment, R³ is selected from H, D, F, Cl, Br, CN, methyl, ethyl, propyl, isopropyl, deuterated methyl, CH₂F, CHF₂, CF₃, methoxy, ethoxy, propoxy, OCH₂F, OCHF₂, and OCF₃.

In another preferred embodiment, the compound has a structure of formula (IVA) or formula (IVB): 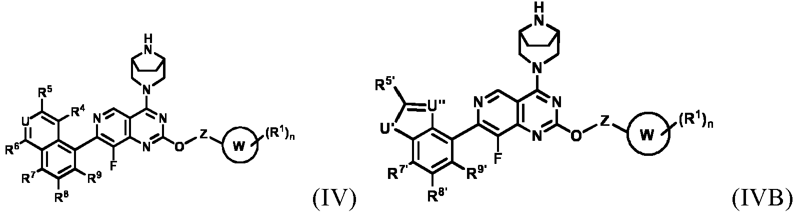 wherein R¹, R⁴, R⁵, R⁶, R⁷, R⁸, R⁹, R^{5'}, R^{7'}, R^{8'}, R^{9'}, U, U', U", Z, W and n are defined as above.

In another preferred embodiment, the compound has a structure of formula (VA) or formula (VB): 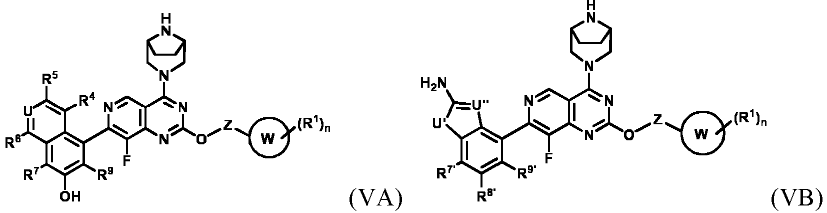 wherein R¹, R⁴, R⁵, R⁶, R⁷, R⁹, R^{7'}, R^{8'}, R^{9'}, U, U', U", Z, W and n are defined as above.

In another preferred embodiment, the compound has a structure of formula (VIA) or formula (VIB): 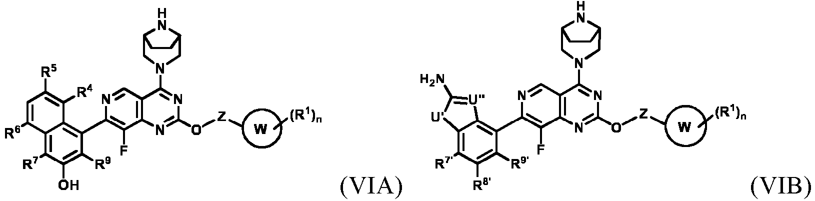 wherein R¹, R⁴, R⁵, R⁶, R⁷, R⁹, R⁷, R^{8'}, R^{9'}, U", Z, W and n are defined as above.

In another preferred embodiment, the compound has a structure of formula (VIIA) or formula (VIIB): 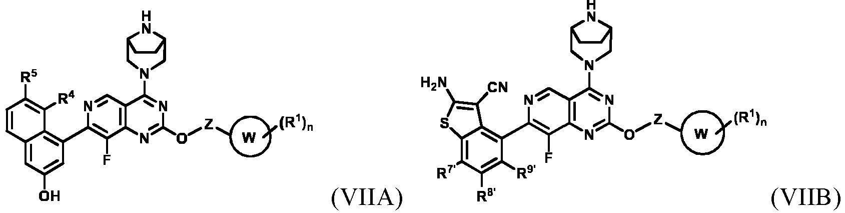 wherein R¹, R⁴, R⁵, R⁷ , R^{8'}, R^{9'}, Z, W and n are defined as above.

In another preferred embodiment, the compound has a structure of formula (VIIIA) or formula (VIIIB): 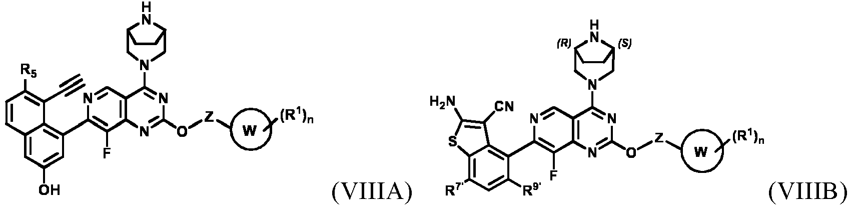 wherein R¹, R⁴, R⁵, R⁷ , R^{9'}, Z, W and n are defined as above.

In another preferred embodiment, W is a substituted or unsubstituted group selected from the group consisting of: C₃-C₆monocyclic cycloalkyl, C₇-C₁₀ bicyclic or C₇-C₁₁ tricyclic cycloalkyl, 4-6 membered substituted or unsubstituted monocyclic heterocyclyl, and 7-10 membered bicyclic or 7-11 membered tricyclic heterocyclyl; preferably, W is selected from substituted or unsubstituted 7-10 membered saturated or unsaturated bridged heterocyclyl, and substituted or unsubstituted 7-10 membered saturated or unsaturated fused heterocyclyl.

In another preferred embodiment, W is selected from: 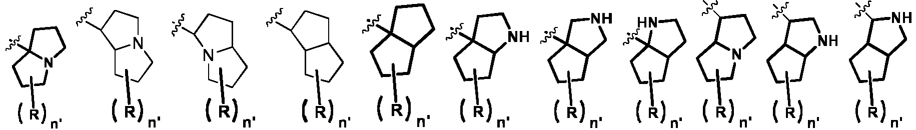 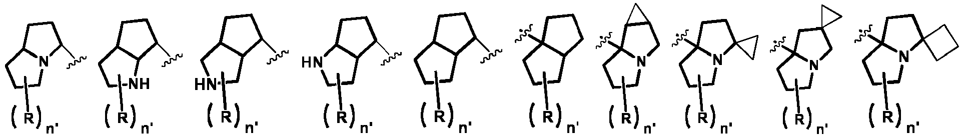 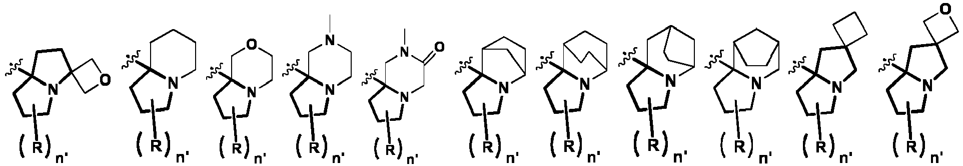 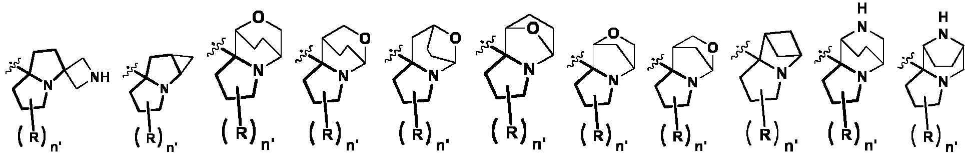 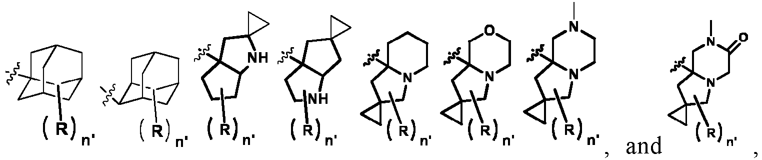 wherein n' is an integer of 0, 1, 2, 3, 4, 5 or 6; R is defined as above, R may be substituted on any ring of the polycyclic ring (such as bridged ring or spiro ring).

In another preferred embodiment, when W is selected from: 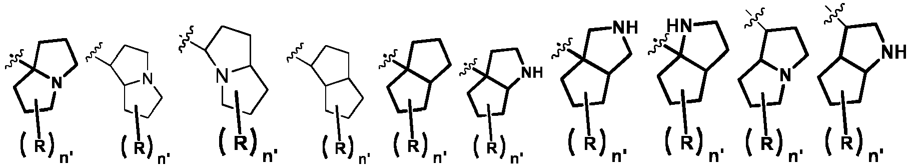 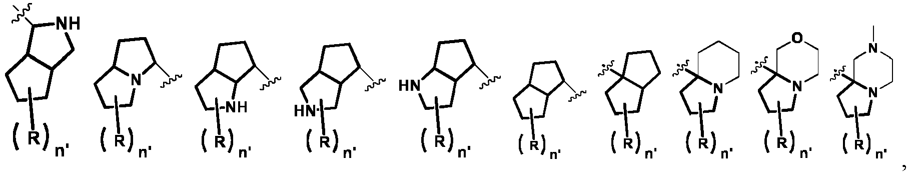 and 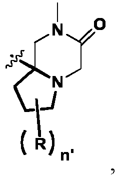 n' may be 1 or 2, more preferably, n' is 1, R is defined as above, R may be substituted on any ring of the polycyclic ring (such as bridged ring or spiro ring).

In another preferred embodiment, when W is selected from: 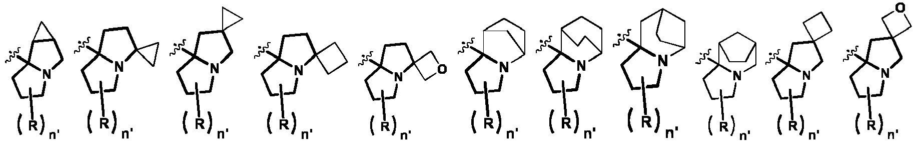 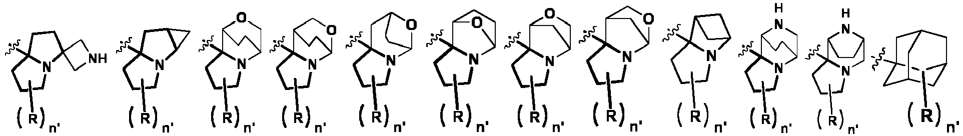 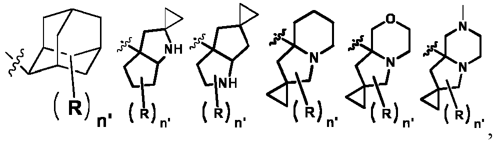 and 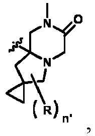 n' may be 0, 1 or 2, R is defined as above, R may be substituted on any ring of the polycyclic ring (such as bridged ring or spiro ring).

In another preferred embodiment, when W is selected from: 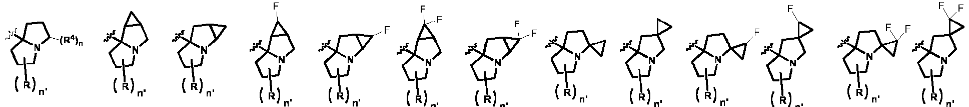 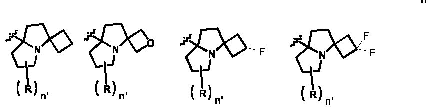 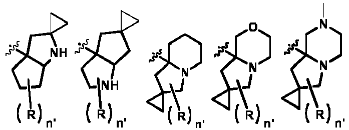 and 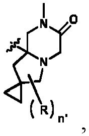 n' may be 0, 1 or 2, R is defined as above, R may be substituted on any ring of the polycyclic ring (such as bridged ring or spiro ring).

In another preferred embodiment, R is selected from: deuterium, C₁-C₆ alkyl, deuterated C₁-C₆ alkyl, halogenated C₁-C₆ alkyl, (C₃-C₆cycloalkyl)C₁-C₁₈ alkyl, (4-6 membered heterocyclyl)C₁-C₆ alkyl, (C₁-C₆alkoxy)C₁-C₆ alkyl, (C₃-C₆cycloalkoxy)C₁-C₆ alkyl, (4-6 membered heterocyclyloxy)C₁-C₆ alkyl, ethenyl, ethynyl, (C₁-C₆alkyl)ethenyl, deuterated (C₁-C₆alkyl)ethenyl, halogenated (C₁-C₆alkyl)ethenyl, (C₁-C₆alkyl)ethynyl, deuterated (C₁-C₆alkyl)ethynyl, halogenated (C₁-C₆alkyl)ethynyl, (C₃-C₆cycloalkyl)ethynyl, (4-6 membered heterocyclyl)ethynyl, C₁-C₆alkoxy, deuterated C₁-C₆alkoxy, halogenated C₁-C₆alkoxy, 4-6 membered heterocyclylC(O), C₃-C₆cycloalkyl, 4-6 membered heterocyclyl, C₆-C₁₀aryl, 5-10 membered heteroaryl (preferably 5-6 membered ), halogen, nitro, hydroxy, oxo, cyano, COOC₁-C₆ alkyl, NH₂, NHC₁-C₆ alkyl, N(C₁-C₆alkyl)₂, CONH₂, CONHC₁-C₆ alkyl, CON(C₁-C₆alkyl)₂, SO2C₁-C₆ alkyl, -NHCONH₂, -NHCO NHC₁-C₆ alkyl, and -NHCON(C₁-C₆alkyl)₂.

In another preferred embodiment, Z is substituted or unsubstituted C₁-C₃ alkylene, preferably is methylene, ethylene, or propylene.

In another preferred embodiment, Z is CD₂.

In another preferred embodiment, the compound has a structure of formula (IX): 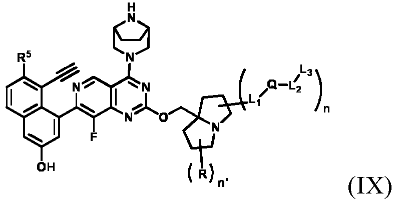
wherein n' is an integer of 0, 1, 2, 3, 4, 5, or 6;
R⁵, R, L₁, Q, L₂, L₃ and n are defined as above.

In another preferred embodiment, R⁴ is a substituted or unsubstituted group selected from the group consisting of: halogen, methyl, ethyl, propyl, ethenyl, propenyl, allyl, butenyl, ethynyl, propynyl, and butynyl.

In another preferred embodiment, 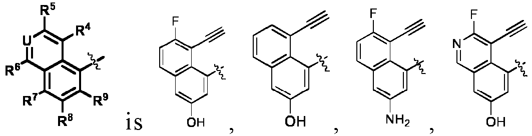 or

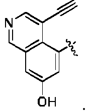

In another preferred embodiment, the compound has a structure of formula (X): 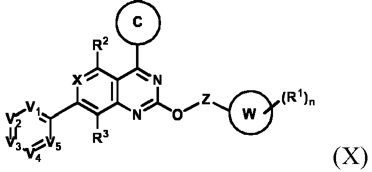
V₁, V₂, V₃, V₄, and V₅ are each independently selected from: N, and CRᵥ; Rᵥ is the same or different, and is each independently selected from: H, C₁-C₃ alkyl, C₃-C₆cycloalkyl, 4-6 membered heterocyclyl, C₁-C₃alkoxy, C₃-C₆cycloalkoxy, 4-6 membered heterocyclyloxy, halogenated C₁-C₃ alkyl, halogenated C₃-C₆cycloalkyl, halogenated 4-6membered heterocyclyl, (HO)-C₁-C₃ alkyl, (HO)-C₃-C₆cycloalkyl, (NH₂)-C₁-C₃ alkyl, (NH₂)-C₃-C₆cycloalkyl, halogen, CN, -C=CH, OH, NH₂, CONH₂, NHCO(C₁-C₆alkyl), NHCO(C₃-C₆cycloalkyl), SO₂NH₂, NHSO₂(C₁-C₆alkyl), and NHSO₂(C₃-C₆cycloalkyl), wherein the heterocyclyl is optionally substituted with one or more oxo (=O);
R² and R³ are the same or different, and are each independently a substituted or unsubstituted group selected from the group consisting of: H, D, halogen, CN, C ₁-C₆alkyl, C₁-C₆deuterated alkyl, C₁-C₆haloalkyl, and C₁-C₆alkoxy; wherein said substituted refers to being substituted with one or more R;
ring C, R, X, Z, W and n are defined as above.

In another preferred embodiment, the compound has a structure of formula (XI): 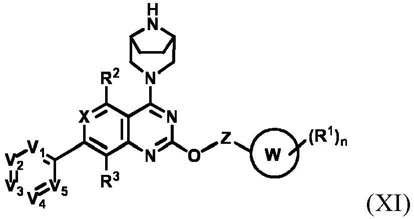
V₁, V₂, V₃, V₄, and V₅ are each independently selected from: N, and CRᵥ; each Rᵥ is the same or different, and is each independently selected from: H, C₁-C₃ alkyl, C₃-C₆cycloalkyl, 4-6 membered heterocyclyl, C₁-C₃alkoxy, C₃-C₆cycloalkoxy, 4-6 membered heterocyclyloxy, halogenated C₁-C₃ alkyl, halogenated C₃-C₆cycloalkyl, halogenated 4-6 membered heterocyclyl, (HO)-C₁-C₃ alkyl, (HO)-C₃-C₆cycloalkyl, (NH₂)-C₁-C₃ alkyl, (NH₂)-C₃-C₆cycloalkyl, halogen, CN, -C=CH, OH, NH₂, CONH₂, NHCO(C₁-C₆alkyl), NHCO(C₃-C₆cycloalkyl), SO₂NH₂, NHSO₂(C₁-C₆alkyl), and NHSO₂(C₃-C₆cycloalkyl), wherein the heterocyclyl is optionally substituted with one or more oxo (=O);
R² and R³ are the same or different, and are each independently a substituted or unsubstituted group selected from the group consisting of: H, D, halogen, CN, C ₁-C₆alkyl, C₁-C₆deuterated alkyl, C₁-C₆haloalkyl, and C₁-C₆alkoxy; wherein said substituted refers to being substituted with one or more R;
wherein R, R¹, X, Z, W and n are defined as above.

In another preferred embodiment, the compound has a structure of formula (XII): 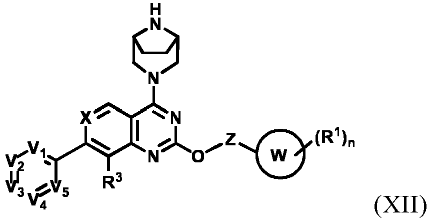
V₁, V₂, V₃, V₄, and V₅ are each independently selected from: N, and CRᵥ; each Rᵥ is the same or different, and is each independently selected from: H, C₁-C₃ alkyl, C₃-C₆cycloalkyl, 4-6 membered heterocyclyl, C₁-C₃alkoxy, C₃-C₆cycloalkoxy, 4-6 membered heterocyclyloxy, halogenated C₁-C₃ alkyl, halogenated C₃-C₆cycloalkyl, halogenated 4-6membered heterocyclyl, (HO)-C₁-C₃ alkyl, (HO)-C₃-C₆cycloalkyl, (NH₂)-C₁-C₃ alkyl, (NH₂)-C₃-C₆cycloalkyl, halogen, CN, -C=CH, OH, NH₂, CONH₂, NHCO(C₁-C₆alkyl), NHCO(C₃-C₆cycloalkyl), SO₂NH₂, NHS0₂(C₁-C₆alkyl), and NHSO₂(C₃-C₆cycloalkyl), wherein the heterocyclyl is optionally substituted with one or more oxo (=O);
R³ is a substituted or unsubstituted group selected from the group consisting of: H, D, halogen, CN, C₁-C₆alkyl, C₁-C₆deuterated alkyl, C₁-C₆haloalkyl, and C₁-C₆alkoxy; wherein said substituted refers to being substituted with one or more R;
wherein R, R¹, X, Z, W and n are defined as above.

In another preferred embodiment, the compound has a structure of formula (XIII): 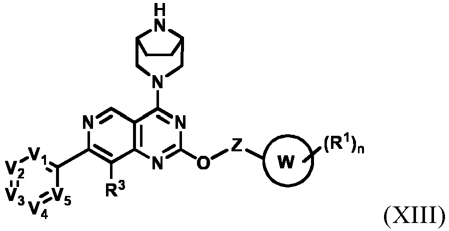
V₁, V₂, V₃, V₄, and V₅ are each independently selected from N, and CRᵥ; each Rᵥ is the same or different, and is each independently selected from: H, C₁-C₃ alkyl, C₃-C₆cycloalkyl, 4-6 membered heterocyclyl, C₁-C₃alkoxy, C₃-C₆cycloalkoxy, 4-6 membered heterocyclyloxy, halogenated C₁-C₃ alkyl, halogenated C₃-C₆cycloalkyl, halogenated 4-6 membered heterocyclyl, (HO)-C₁-C₃ alkyl, (HO)-C₃-C₆cycloalkyl, (NH₂)-C₁-C₃ alkyl, (NH₂)-C₃-C₆cycloalkyl, halogen, CN, -C=CH, OH, NH₂, CONH₂, NHCO(C₁-C₆alkyl), NHCO(C₃-C₆cycloalkyl), SO₂NH₂, NHSO₂(C₁-C₆alkyl), and NHSO₂(C₃-C₆cycloalkyl), wherein the heterocyclyl is optionally substituted with one or more oxo (=O);
R³ is a substituted or unsubstituted group selected from the group consisting of: H, D, halogen, CN, C₁-C₆alkyl, C₁-C₆deuterated alkyl, C₁-C₆haloalkyl, and C₁-C₆alkoxy; wherein said substituted refers to being substituted with one or more R;
wherein R, R¹, Z, W and n are defined as above.

In another preferred embodiment, Rᵥ is each independently selected from H, halogen, CN, -C=CH, and NH₂.

In another preferred embodiment, L₁ is selected from substituted or unsubstituted methylene and substituted or unsubstituted ethylene; Q is selected from O; L₂ is selected from null, and substituted or unsubstituted methylene; L₃ is a substituted or unsubstituted group selected from the group consisting of: -C₁-C₆ alkyl, -C₃-C₆cycloalkyl, -C₄-C₆ heterocyclyl, -C₁-C₆alkylene(C₃-C₆cycloalkyl), -C₁-C₆alkylene(C₄-C₆ heterocyclyl), -C₁-C₆alkylene(C₁-C₆alkoxy), -C₁-C₆alkylene(C₃-C₆cycloalkoxy), and -C₁-C₆alkylene(C₄-C₆ heterocyclyloxy); wherein said substituted refers to being substituted with one or more groups selected from the group consisting of: deuterium, C₁-C₆ alkyl, deuterated C₁-C₆ alkyl, halogenated C₁-C₆ alkyl, ethenyl, ethynyl, C₁-C₆alkoxy, deuterated C₁-C₆alkoxy, halogenated C₁-C₆alkoxy, C₃-C₆cycloalkyl, 4-6 membered heterocyclyl, C₆-C₁₄aryl, 5-14membered heteroaryl, halogen, nitro, hydroxy, oxo, cyano, ester group, amino, amido, sulfonyl and ureido.

In another preferred embodiment, -L₁-Q-L₂-L₃ is selected from:

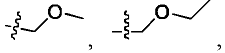

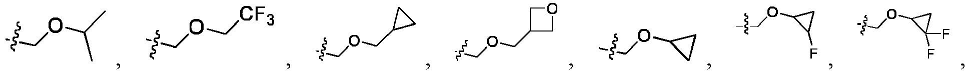

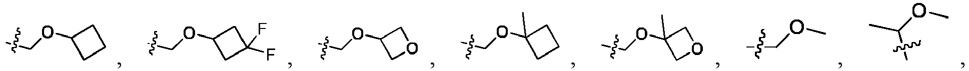

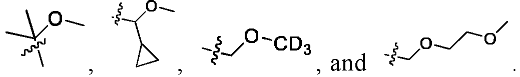

In another preferred embodiment, 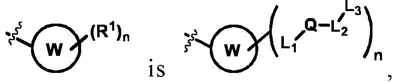 preferably, is

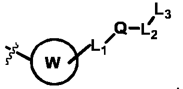

In another preferred embodiment, 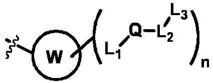 is 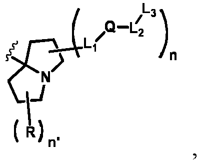 preferably, is

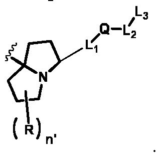

In another preferred embodiment, 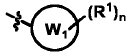 is selected from: 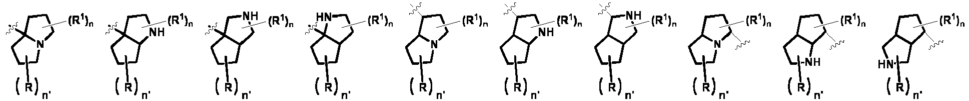 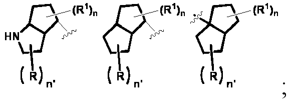 or is selected from: 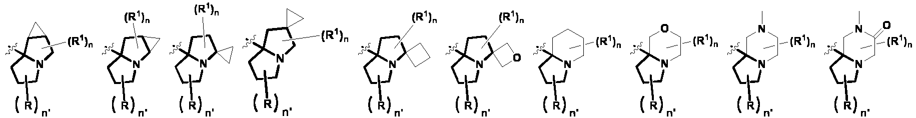 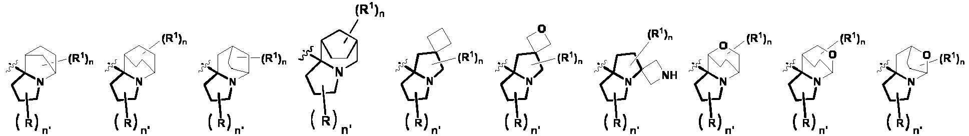 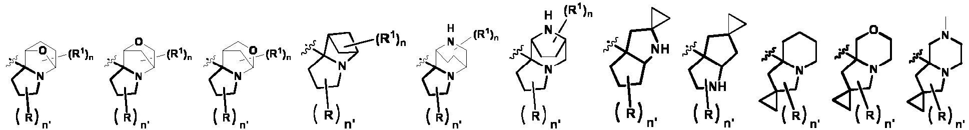 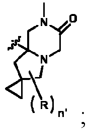 wherein n' is an integer of 0, 1, 2, 3, 4, 5, or 6; R, R¹ and n are defined as above, R, and R¹ may be substituted on any ring of the polycyclic ring (such as bridged ring or spiro ring);
or is selected from: 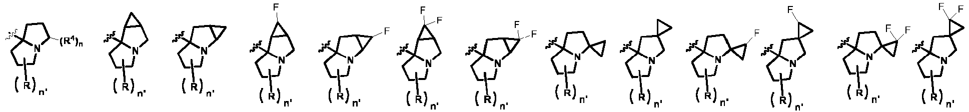 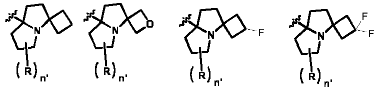 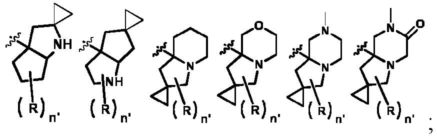 wherein n' is an integer of 0, 1, 2, 3, 4, 5, or 6; R is defined as above, R may be substituted on any ring of the polycyclic ring (such as bridged ring or spiro ring).

In another preferred embodiment, 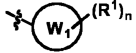 is selected from:

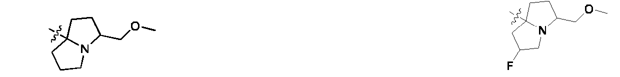

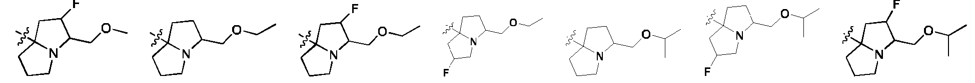

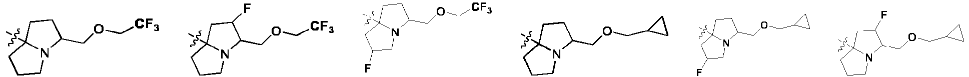

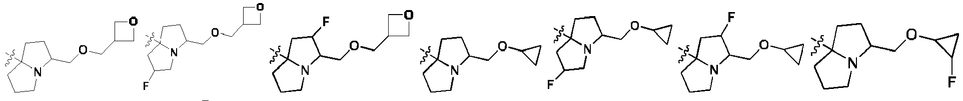

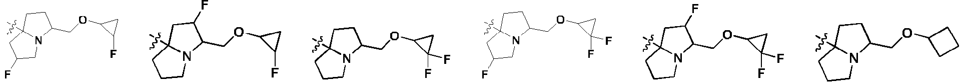

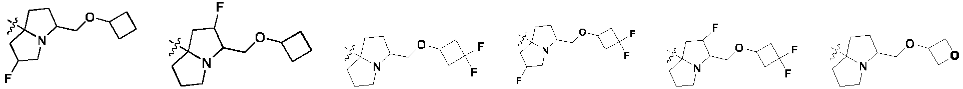

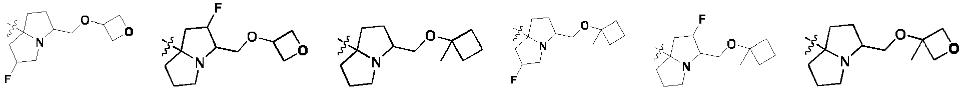

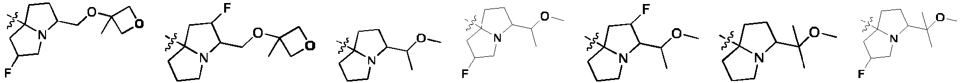

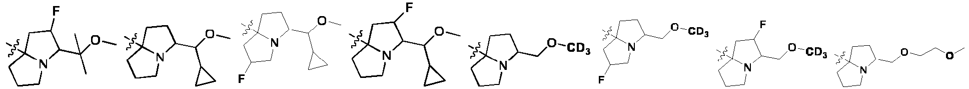

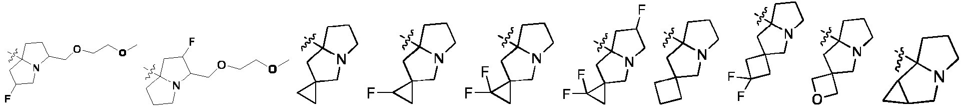

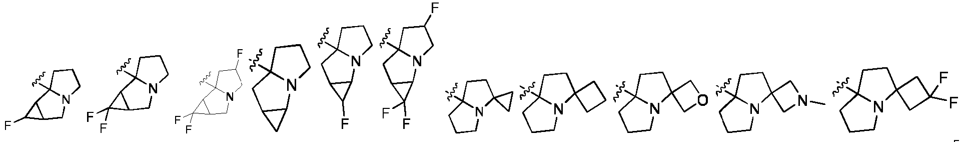

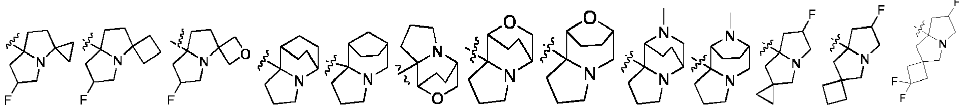

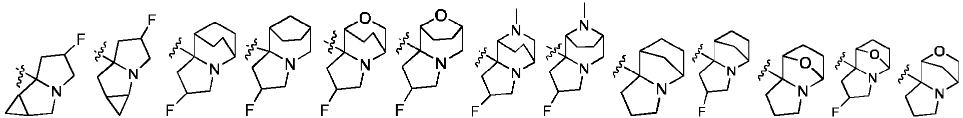

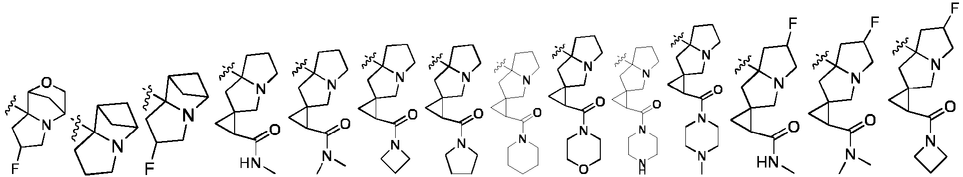

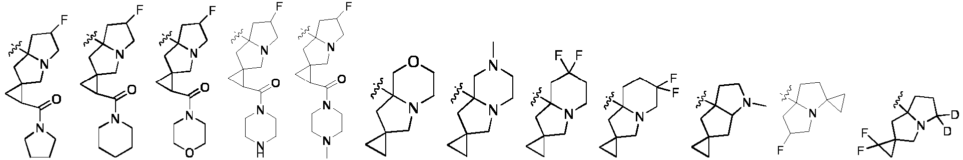

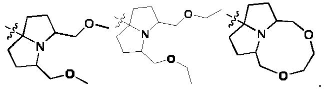

In another preferred embodiment, W is selected from

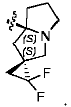

In another preferred embodiment, W is selected from

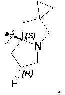

In another preferred embodiment, W is selected from

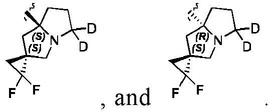

In another preferred embodiment, the prodrug of the compound has a structure of formula (XIV) below: 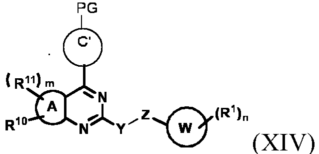
C' is selected from 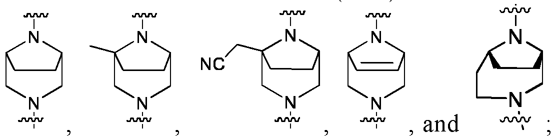 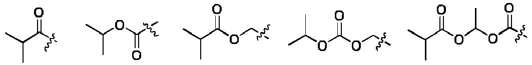
PG is selected from 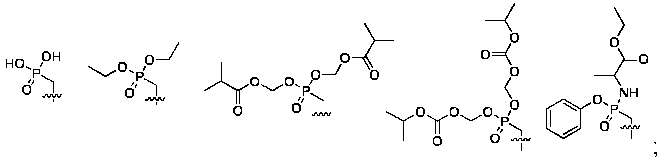
Y, Z, W, ring A, R¹, R¹⁰, R¹¹, m and n are defined as above.

In another preferred embodiment, m, ring A, n, U, X, Y, Z, W, L₁, L₂, L₃, Q, R², R³, R⁴, R⁵, R⁶, R⁷, R⁸, R⁹, R¹⁰ and R¹¹ have the corresponding groups in each specific compounds shown in examples.

In another preferred embodiment, V₁, V₂, V₃, V₄, V₅, ring C and PG have the corresponding groups in each specific compounds shown in examples.

In another preferred example, the compound is selected from the following group: 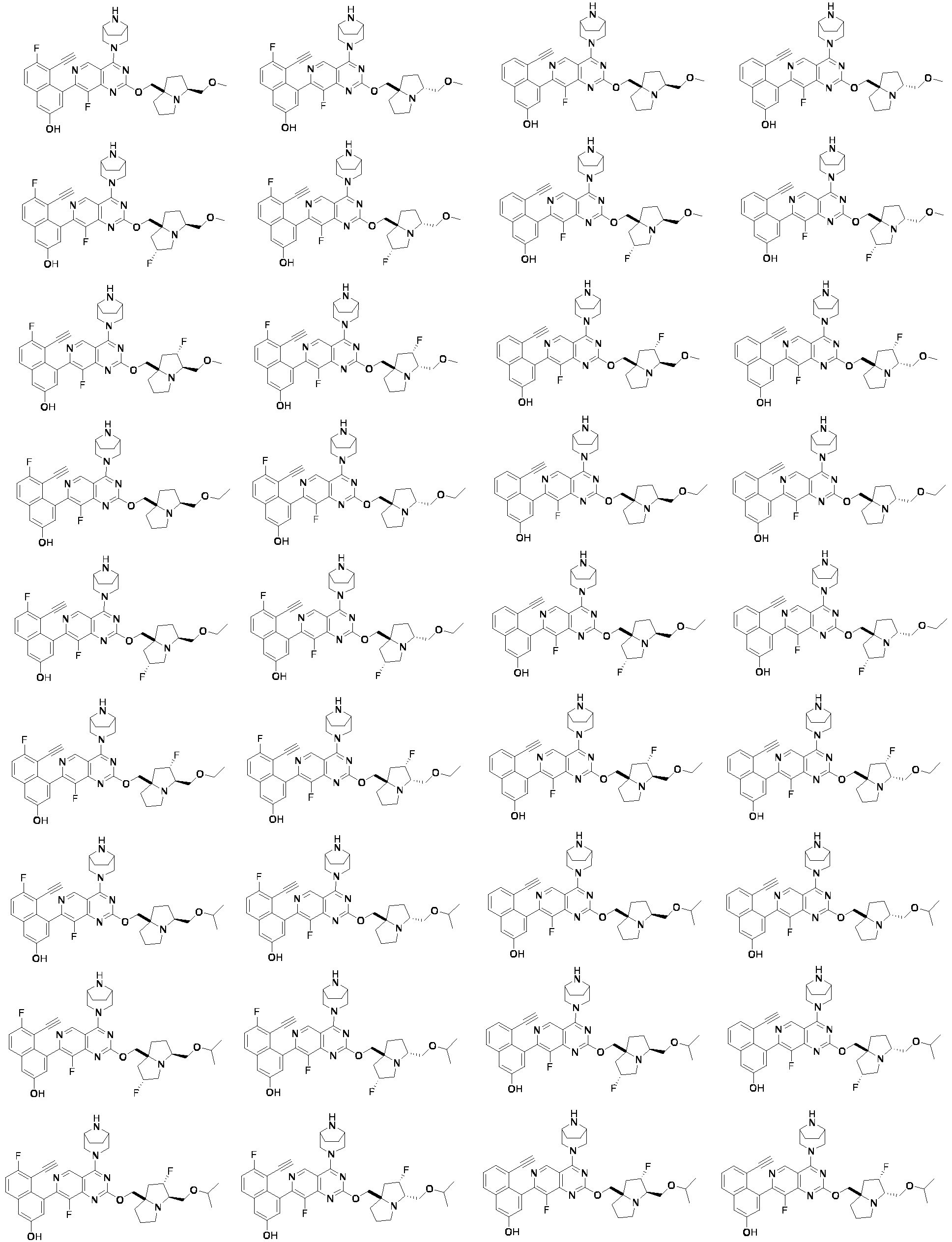 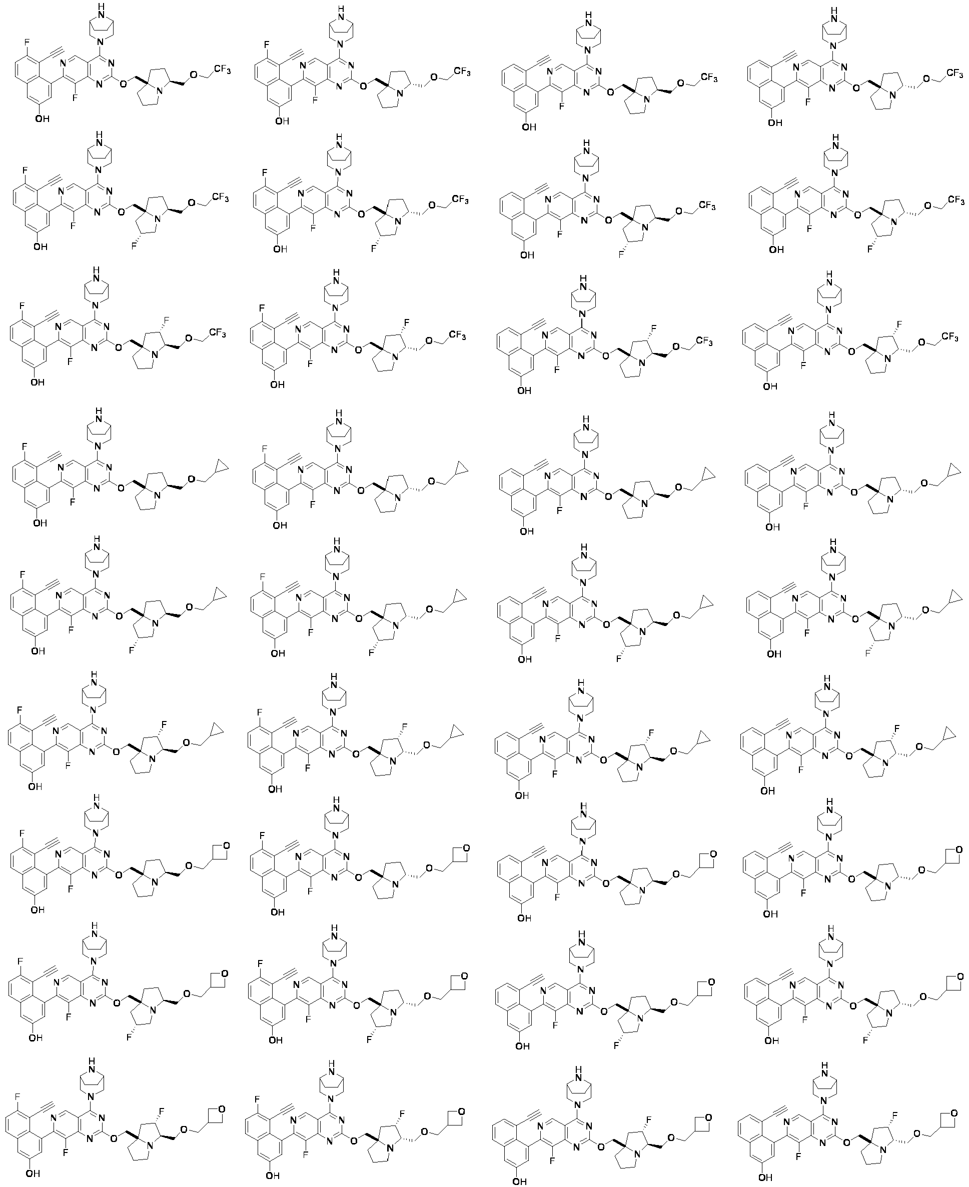 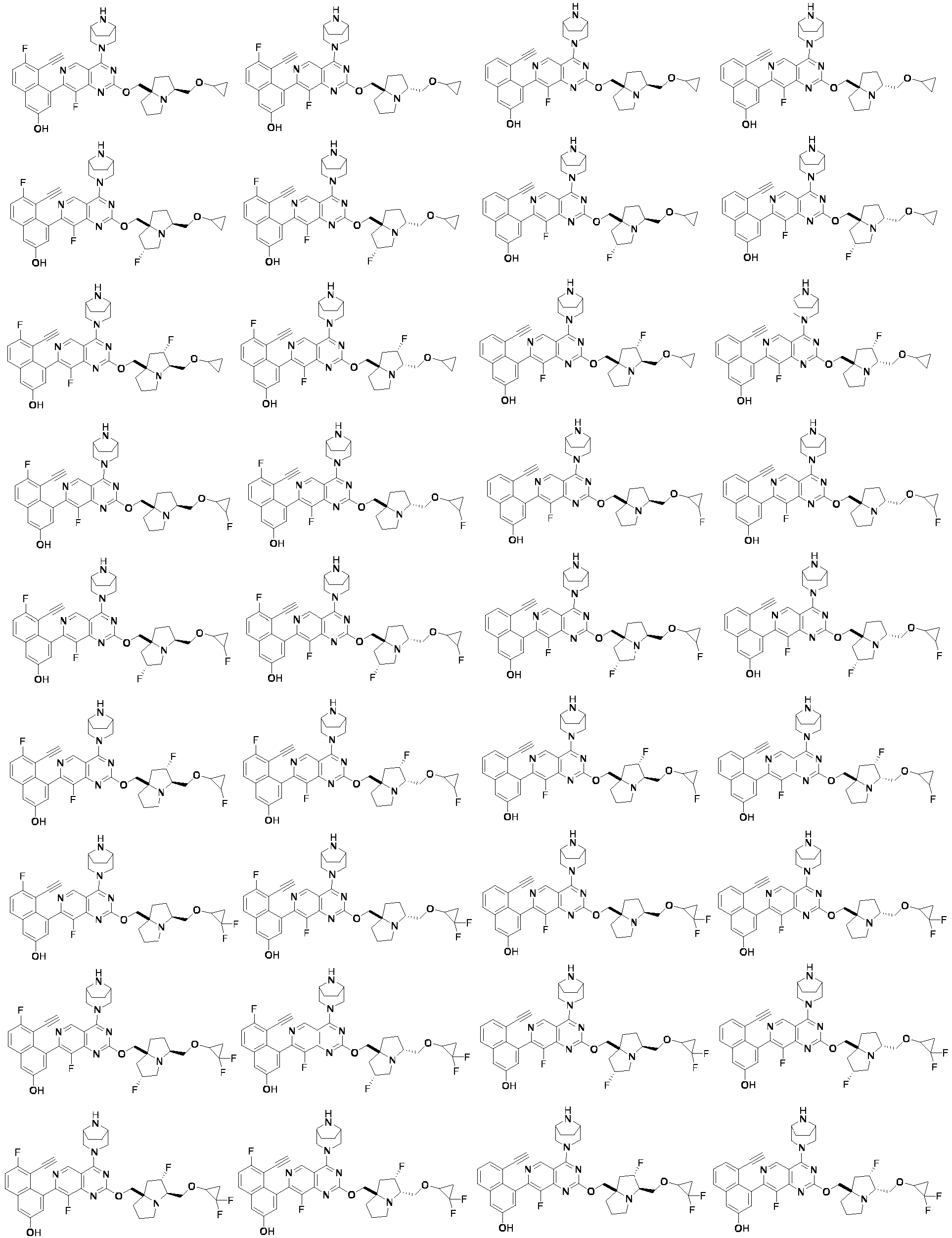 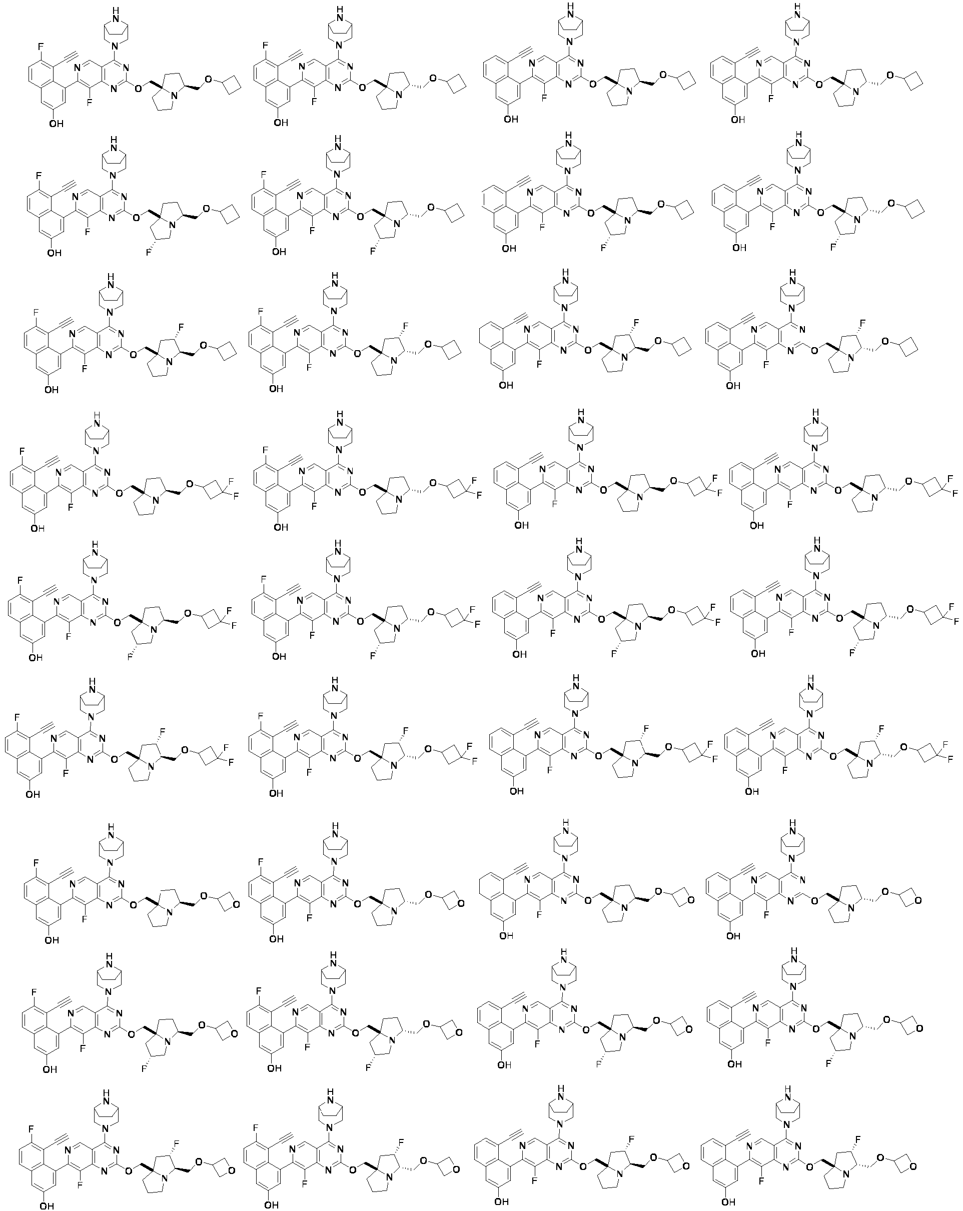 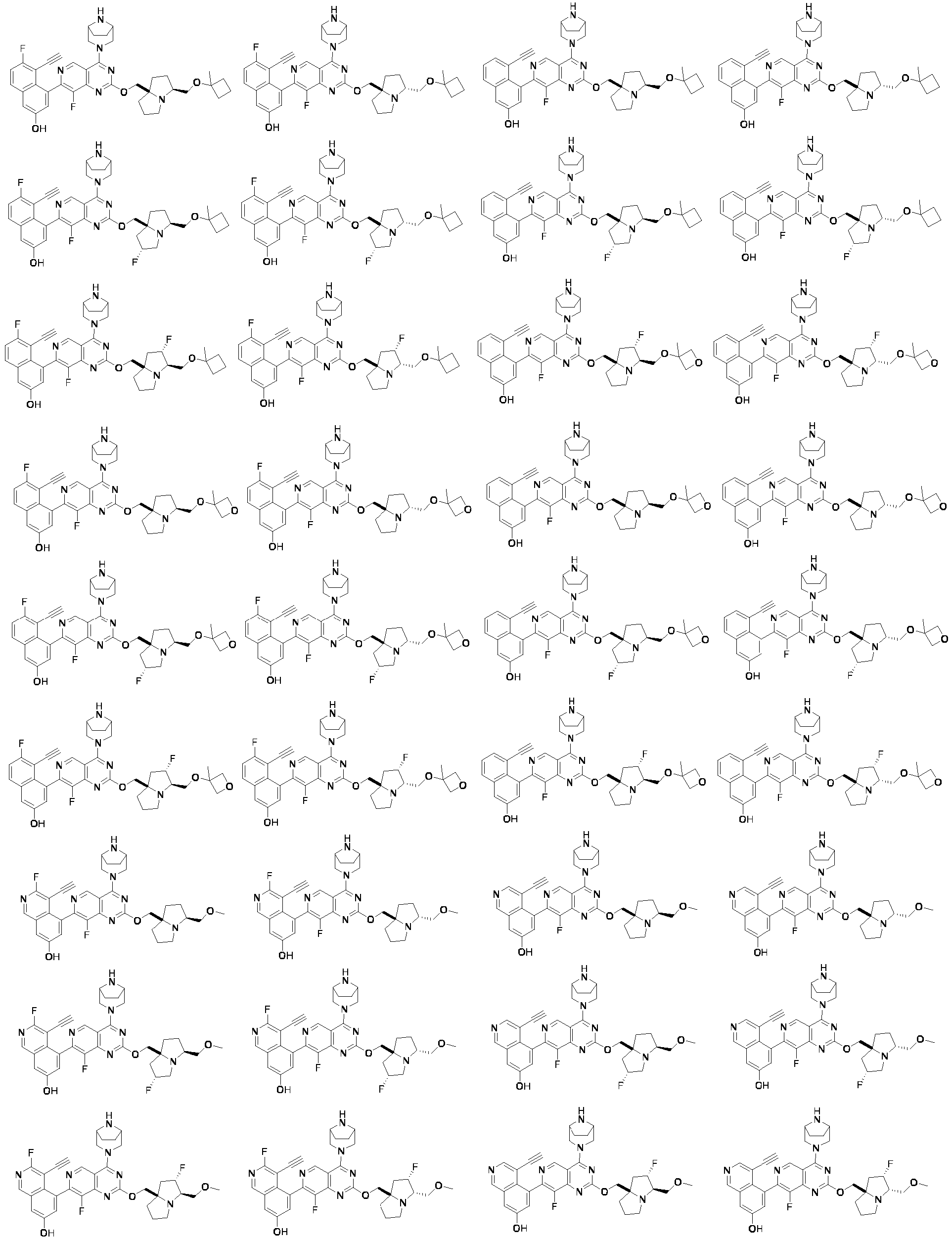 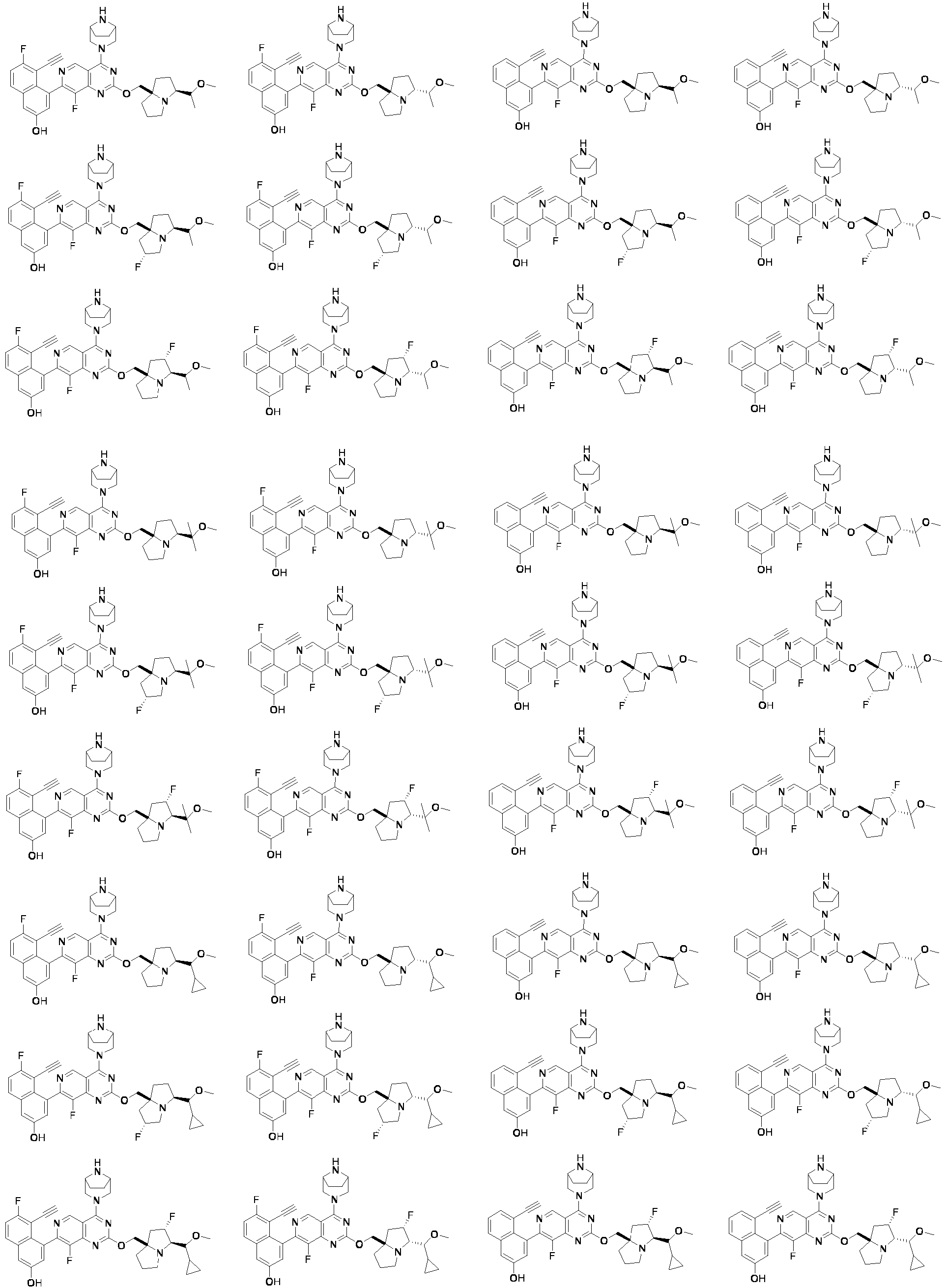 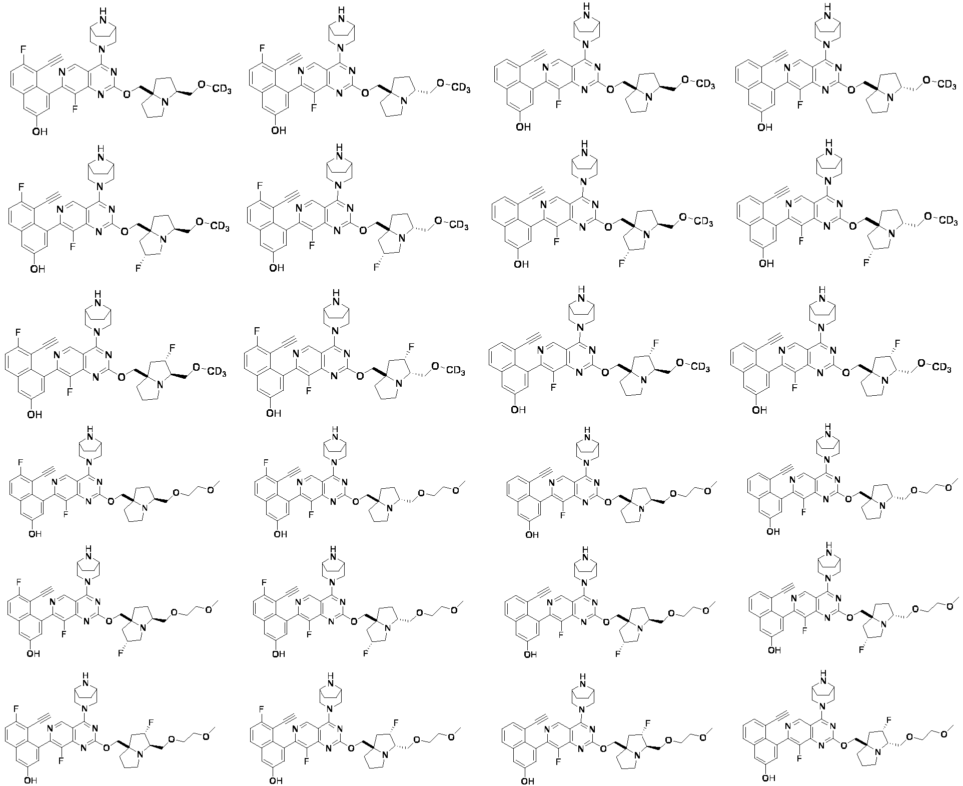 or is selected from 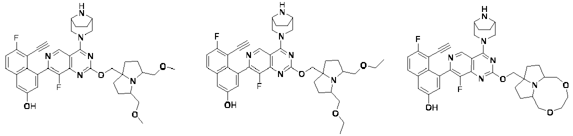 or is selected from 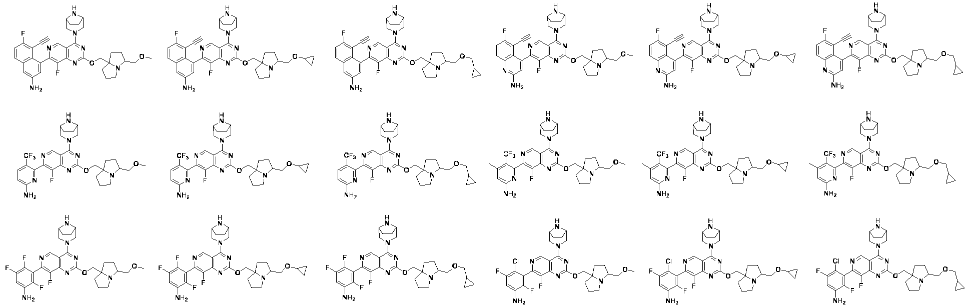 or is selected from 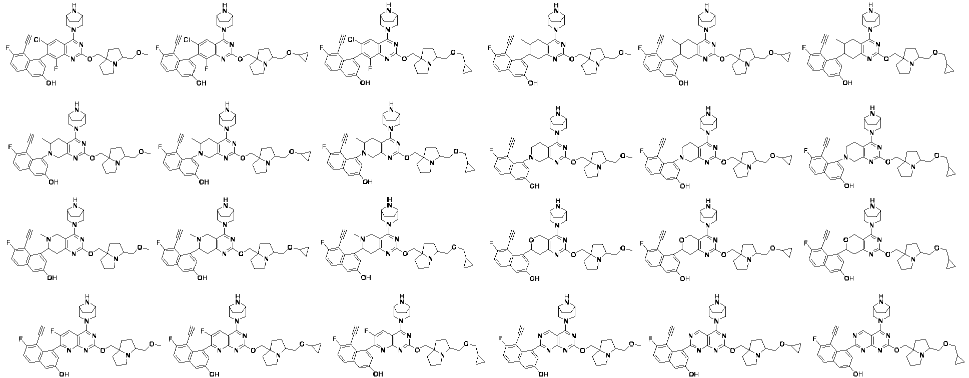 or is selected from 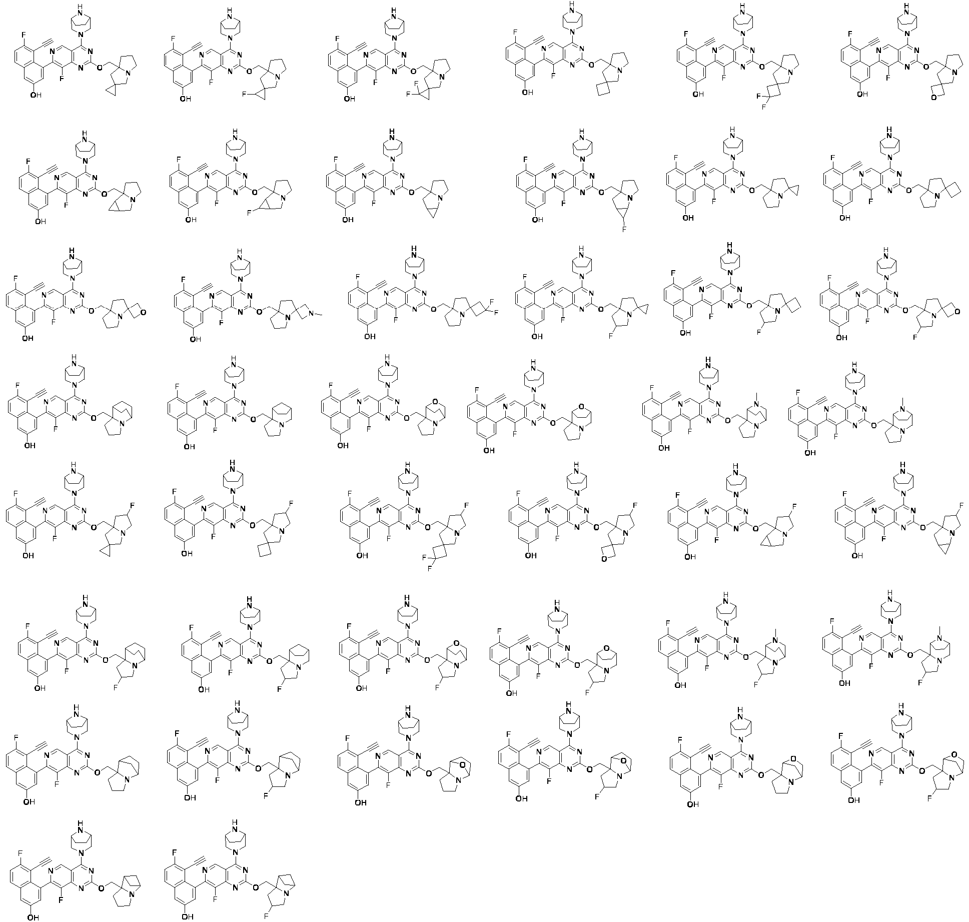 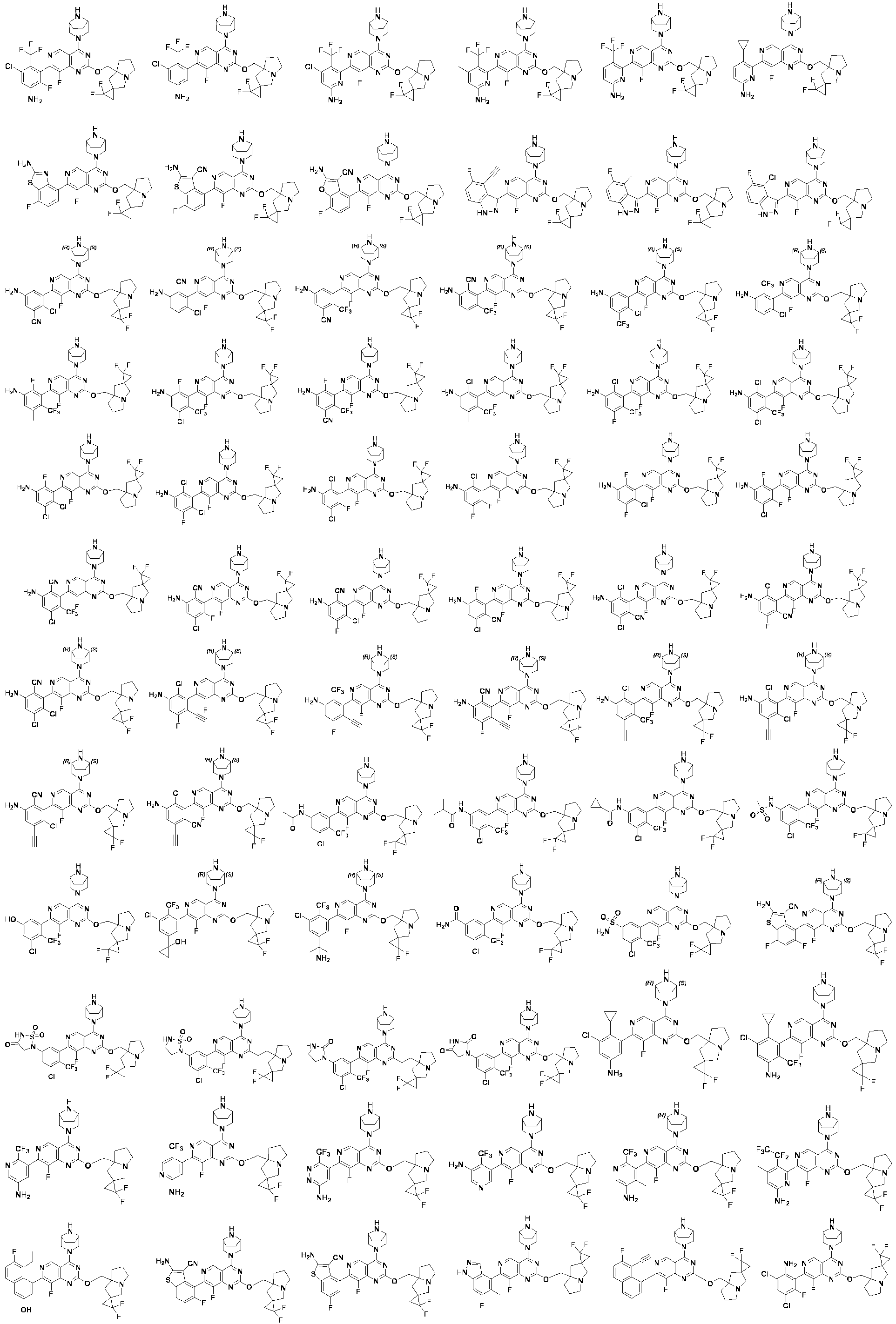 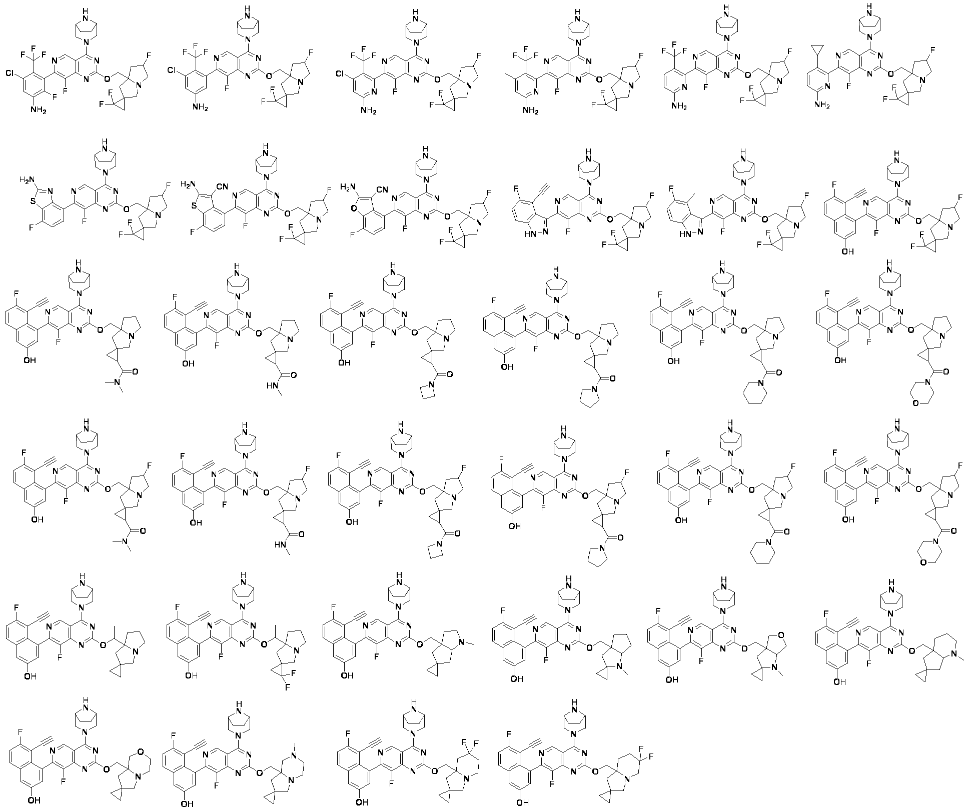 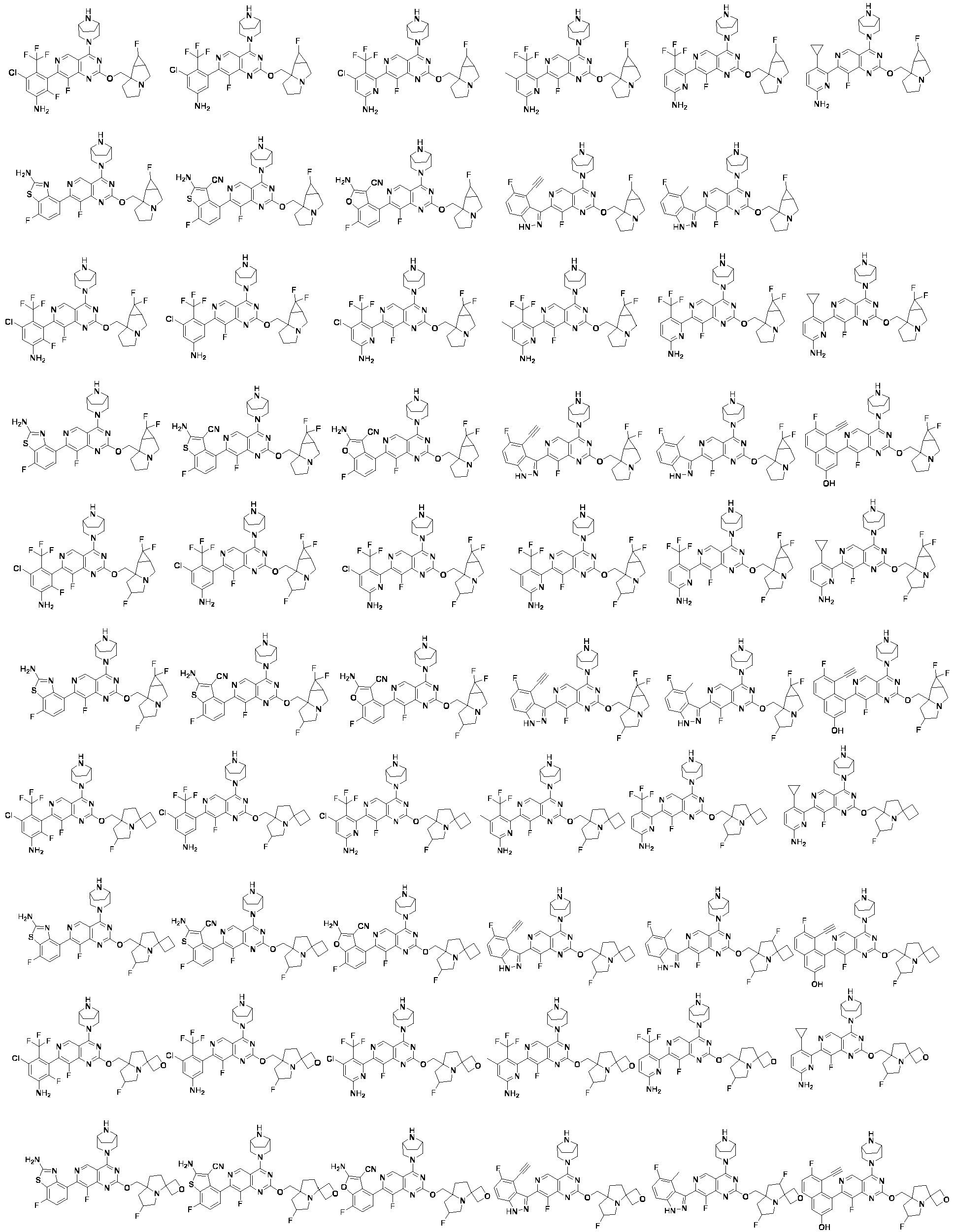 or is selected from 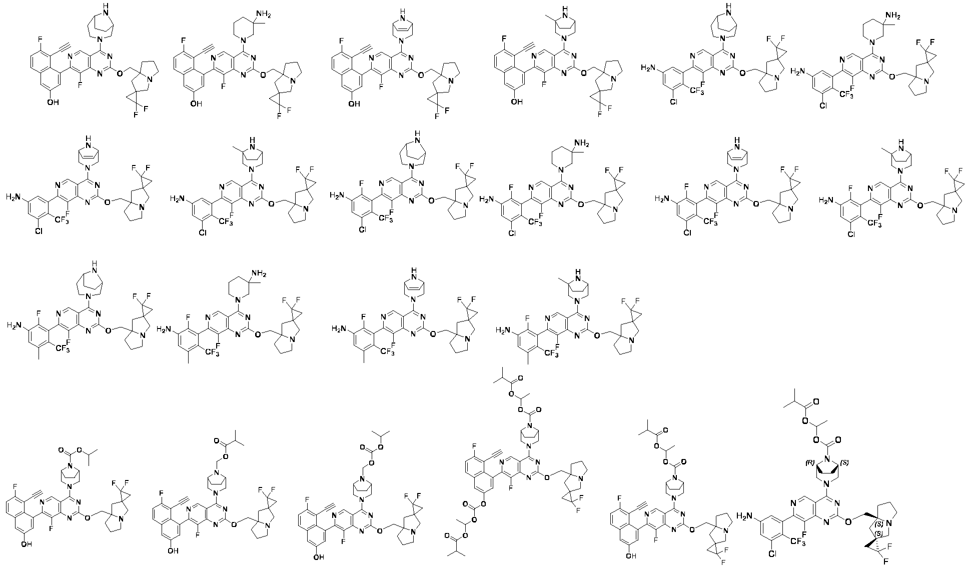 or is selected from 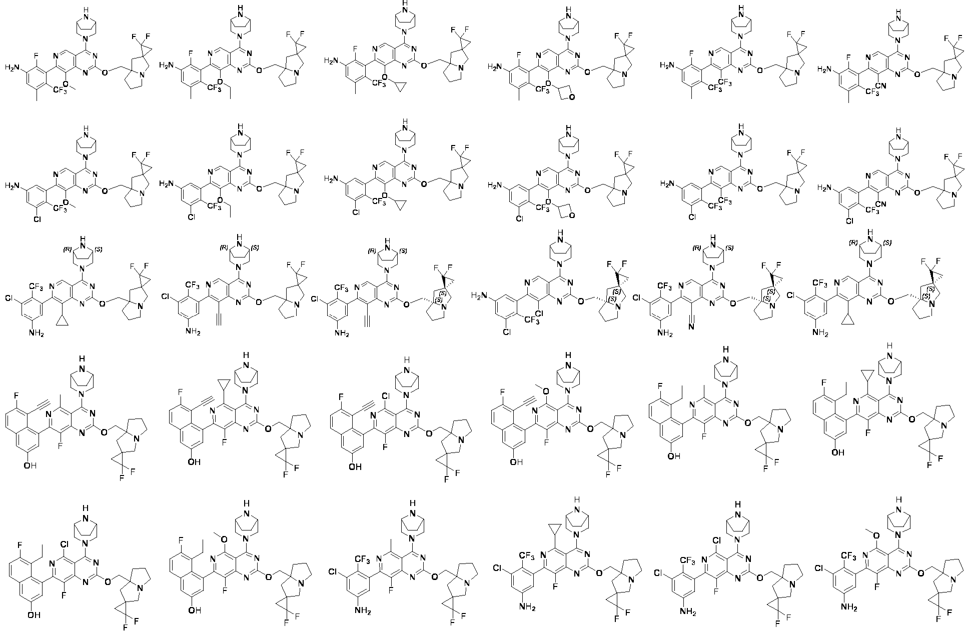 or is selected from:

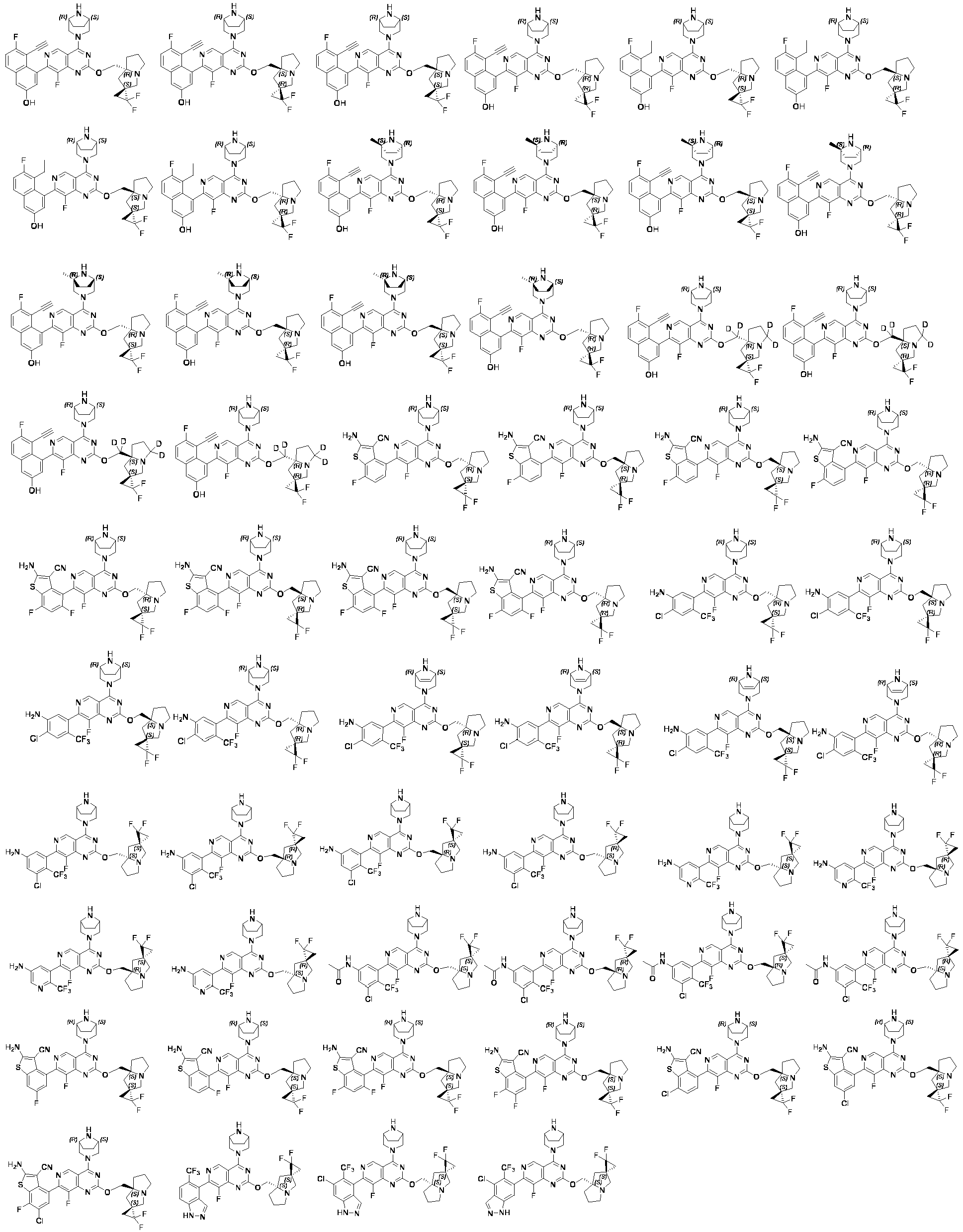

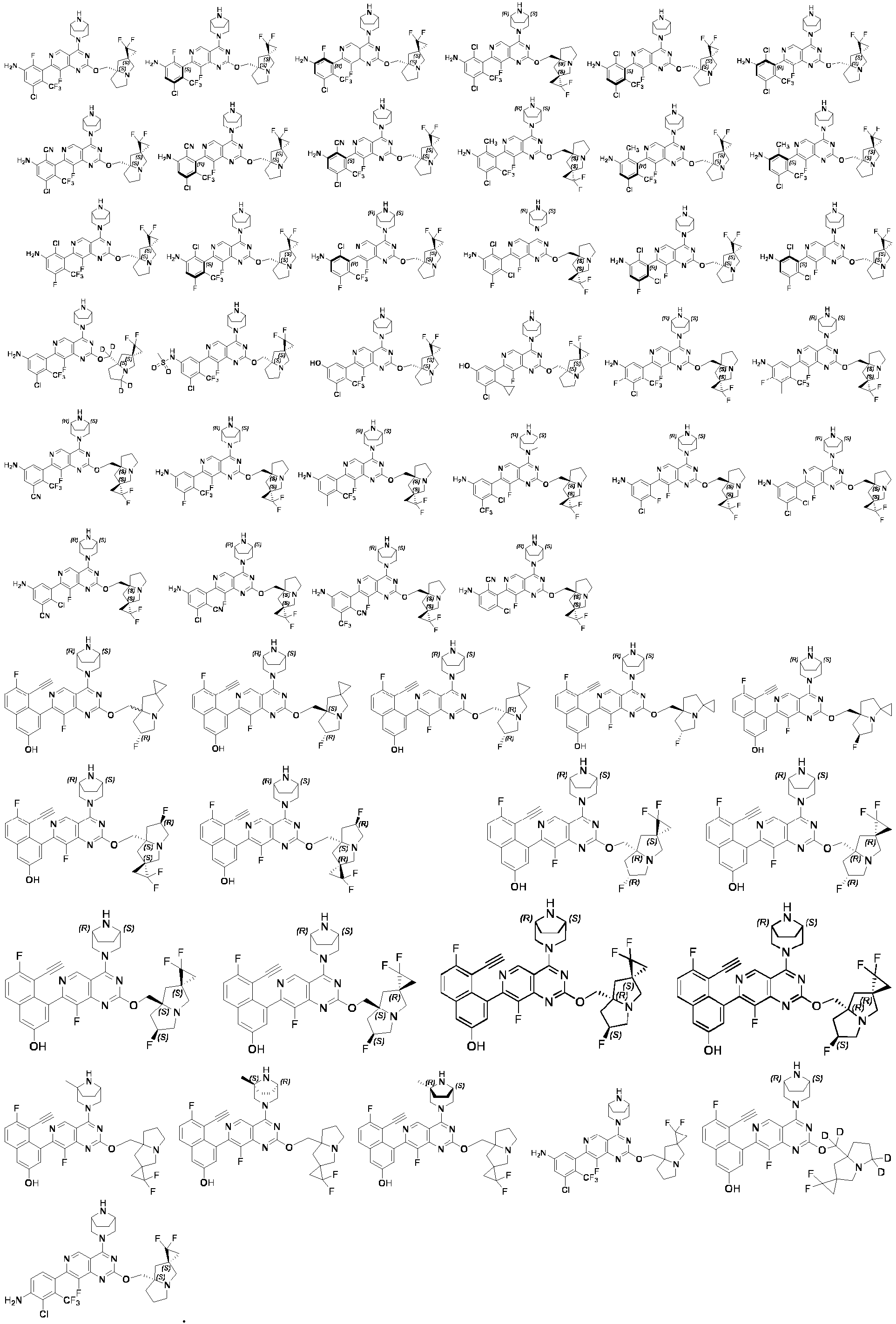

In another preferred embodiment, the compound is preferably the compound prepared in the examples.

In the second aspect of the present invention, provided is a method for preparing the compound of formula (A0), or the stereoisomer, the tautomer, the crystal form, the pharmaceutically acceptable salt, the hydrate, the solvate, or the prodrug thereof, wherein comprising steps of (taking formula IA as an example): 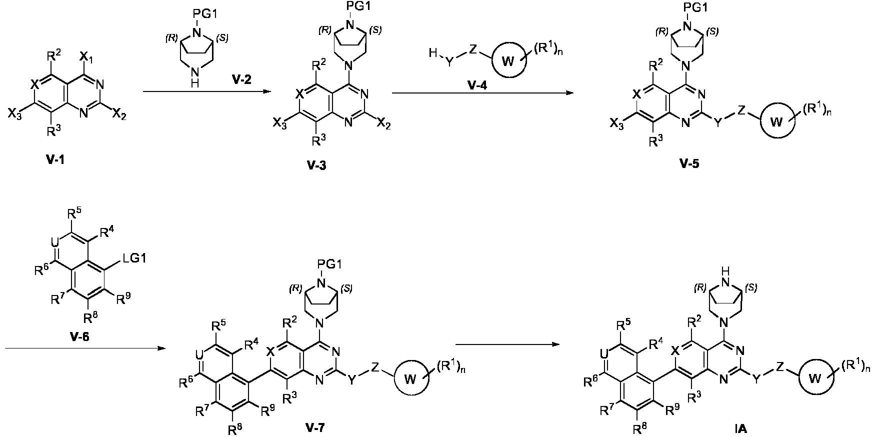
(i) in an inert solvent, in the presence of a base, with or without a Pd catalyst, with or without a condensing agent, reacting a compound of formula V-1 with a compound of formula V-2, thereby obtaining a compound of formula V-3;
(ii) in an inert solvent, in the presence of a base, with or without the presence of a Pd catalyst, reacting the compound of formula V-3 with a compound of formula V-4, thereby obtaining a compound of formula V-5;
(iii) in an inert solvent, in the presence of a base, in the presence of a Pd catalyst, reacting the compound of formula V-5 with a compound of formula V-6, thereby obtaining a compound of formula V-7;
(iv) under acid (such as TFA, HCl, etc.) condition or under Pd catalytic hydrogenation condition, removing the protective group PG1 from the compound of formula V-7, thereby obtaining a compound of formula (IA);
wherein,
X₁, X₂ and X₃ are each independently selected from OH, halogen, OTf, OTs and OMs;
PG1 is selected from Boc, Cbz, and Bn;
LG1 is selected from -B(OH)₂, -B(KBF₃), -Sn(ⁿBu)₃, 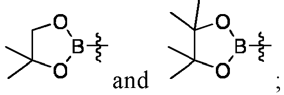
R¹, R², R³, R⁴, R⁵, R⁶, R⁷, R⁸, R⁹, U, X, Y, Z, W and n are defined as above.

In the third aspect of the present invention, provided is a pharmaceutical composition, comprising one or more of the compound according to the first aspect, or the stereoisomer, the tautomer, the crystal form, the pharmaceutically acceptable salt, the hydrate, the solvate, or the prodrug thereof; and pharmaceutically acceptable carriers.

In another preferred embodiment, the pharmaceutical composition further comprises a drug selected from the group consisting of: PD-1 inhibitor (such as nivolumab, pembrolizumab, pidilizumab, cemiplimab, JS-001, SHR-120, BGB-A317, IBI-308, GLS-010, GB-226, STW204, HX008, HLX10, BAT 1306, AK105, LZM 009, or biosimilars of the above drugs, and the like), PD-L1 inhibitor (such as durvalumab, atezolizumab, avelumab, CS1001, KN035, HLX20, SHR-1316, BGB-A333, JS003, CS1003, KL -A167, F 520, GR1405, MSB2311, or biosimilars of the above drugs, and the like), CD20 antibody (such as rituximab, obinutuzumab, ofatumumab, veltuzumab, tositumomab, 131I-tositumomab, ibritumomab, 90Y-ibritumomab, 90In-ibritumomab, ibritumomab tiuxetan, and the like), CD47 antibody (such as Hu5F9-G4, CC-90002, TTI-621, TTI-622, OSE-172, SRF-231, ALX-148, NI-1701, SHR-1603, IBI188, IMM01), ALK inhibitor (such as Ceritinib, Alectinib, Brigatinib, Lorlatinib, Alkotinib), PI3K inhibitor (such as Idelalisib, Duvelisib, Dactolisib, Taselisib, Bimiralisib, Omipalisib, Buparlisib, and the like), BTK inhibitor(such as Ibrutinib, Tirabrutinib, Acalabrutinib, Zanubrutinib, Vecabrutinib and the like), EGFR inhibitor(such as Afatinib, Gefitinib, Erlotinib , Lapatinib, Dacomitinib, Icotinib, Canertinib, Sapitinib, Naquotinib, Pyrotinib, Rociletinib, Osimertinib, and the like), VEGFR inhibitor (such as Sorafenib, Pazopanib, Regorafenib , Sitravatinib, Ningetinib, Cabozantinib, Sunitinib, Donafenib, and the like), HDAC inhibitor (such as Givinostat, Tucidinostat, Vorinostat, Fimepinostat, Droxinostat, Entinostat, Dacinostat, Quisinostat, Tacedinaline, and the like), CDK inhibitor (such as Palbociclib, Ribociclib, Abemaciclib, Milciclib, Trilaciclib, Lerociclib, and the like), MEK inhibitor(such as Selumetinib (AZD6244), Trametinib (GSK1120212), PD0325901, U0126, Pimasertib (AS-703026), PD184352 (CI-1040), and the like), mTOR inhibitor (such as Vistusertib, and the like), SHP2 inhibitor (such as RMC-4630, JAB-3068, TNO155, and the like), and combinations thereof.

In the fourth aspect of the present invention, provided is a use of the compound according to the first aspect, or the stereoisomer, the tautomer, the crystal form, the pharmaceutically acceptable salt, the hydrate, the solvate, or the prodrug thereof, or the pharmaceutical composition according to the third aspect in preparation of a drug for preventing and/or treating a disease associated with activity or expression of KRAS^{G12D}.

In another preferred embodiment, the disease is tumor or disorder.

In another preferred embodiment, the disease is selected from the group consisting of: lung cancer, breast cancer, prostate cancer, esophageal cancer, colorectal cancer, bone cancer, kidney cancer, stomach cancer, liver cancer, colon cancer, melanoma, lymphoma, blood cancer, brain tumor, myeloma, soft tissue sarcoma, pancreatic cancer, and skin cancer.

In the fourth aspect of the present invention, provided is a non-diagnostic and non-therapeutic method for inhibiting KRAS^{G12D}, comprising steps of administering to a subject in need thereof an effective amount of the compound according to the first aspect, or the stereoisomer, the tautomer, the crystal form, the pharmaceutically acceptable salt, the hydrate, the solvate, or the prodrug thereof, or administering the pharmaceutical composition as described above.

In another preferred embodiment, the subject is mammal; preferably, is human.

In the fifth aspect of the present invention, provided is an *in vitro* method for inhibiting KRAS^{G12D}, comprising steps of contacting the compound according to the first aspect, or the stereoisomer, the tautomer, the crystal form, the pharmaceutically acceptable salt, the hydrate, the solvate, or the prodrug thereof, or contacting the pharmaceutical composition as described above with protein or cells, thereby inhibiting the activity of KRAS^{G12D}.

In another preferred embodiment, the cells are selected from the group consisting of: macrophages, intestinal cells (including intestinal stem cells, intestinal epithelial cells), and a combination thereof.

In another preferred embodiment, the cells are from rodents (such as mice, rats) or primates (such as humans).

It should be understood that within the scope of the present invention, the above technical features of the present invention and the technical features specifically described in the following (eg, embodiments) can be combined with each other, thereby forming a new or preferred technical solution. Due to space limitations, it will not be repeated herein.

### EMBODIMENTS FOR CARRYING OUT THE INVENTION

After long and intensive research, a novel class of compounds with selective inhibitory effects on KRAS^{G12D} and/or better pharmacodynamic properties was prepared unexpectedly. The inventor has completed the present invention on this basis.

### TERMS

As used herein, unless otherwise specified, the terms used have a general meaning known to those skilled in the art.

The term "alkyl" refers to a straight or branched alkane group containing 1-20 carbon atoms, such as 1-18 carbon atoms, especially 1-18 carbon atoms, preferably containing 1-10 carbon atoms (C1-C10), more preferably containing 1-6 carbon atoms (C1-C6). Typical "alkyl" group includes methyl, ethyl, propyl, isopropyl, n-butyl, tert-butyl, isobutyl, 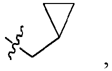 pentyl, isopentyl, heptyl, 4,4-dimethylpentyl, octyl, 2,2,4-trimethylpentyl, nonyl, decyl, undecyl, dodecyl and the like. In the present invention, the alkyl further includes substituted alkyl group. "Substituted alkyl" means that one or more positions in the alkyl are substituted, especially means 1 to 4 substituents, which can substitute at any position. Typical substitutions include, but are not limited to, one or more of the following groups: for example hydrogen, deuterium, halogens (such as monohalogen substituent or polyhalogen substituent, the latter may be such as trifluoromethyl or an alkyl group containing Cl₃), cyano, nitro, oxo (such as =O), trifluoromethyl, trifluoromethoxy, cycloalkyl, alkenyl, cycloalkenyl, alkynyl, heterocyclic ring, aromatic ring, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ; wherein Rₐ appearing herein can independently represent hydrogen, deuterium, C1-C6 alkyl, C3-C8cycloalkyl, C2-C6alkenyl, C3-C6cycloalkenyl, C2-C6alkynyl, 5-14memebered heterocyclic ring or C6-C14aromatic ring; R_{b}, R_{c} and R_{d} can independently represent hydrogen, deuterium, C1-C6alkyl, C3-C8cycloalkyl, 5-14membered heterocyclic ring or C6-C14aromatic ring; or R_{b} and R_{c} together with the N atom can form heterocyclic ring; Rₑ can independently represent hydrogen, C1-C6alkyl, C3-C8cycloalkyl, C2-C6alkenyl, C3-C6cycloalkenyl, C2-C6alkynyl, 5-14membered heterocyclic ring or C6-C14aromatic ring. The above typical substituents, such as alkyl, cycloalkyl, alkenyl, cycloalkenyl, alkynyl, heterocyclic ring or aromatic ring, may be optionally substituted.

The term "alkylene" refers to a group formed by removing a hydrogen atom from "an alkyl or substituted alkyl", such as methylene, ethylene, propylene, isopropylene (such as 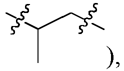 butylene (such as 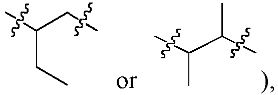 pentylene (such as 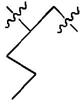 ), hexylene (such as 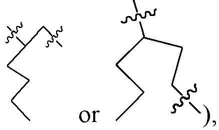 heptylene (such as 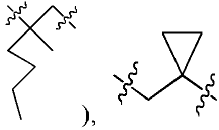 and the like. Additionally, the term also comprises a methylene group of an alkylene (such as C1-C18 alkylene) being replaced by a cycloalkylene (such as C3-C20 cycloalkylene), for example, "C1-C18 alkylene C3-C20 cycloalkylene" or "C3-C20 cycloalkylene C1-C18 alkylene".

The terms "C1-C18 alkylene C3-C20 cycloalkylene" or "C3-C20 cycloalkylene C1-C18 alkylene" have the same meaning and refer to a group formed by removing two hydrogen atoms from cycloalkylalkyl or alkylcycloalkyl, such as

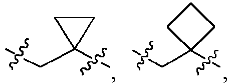

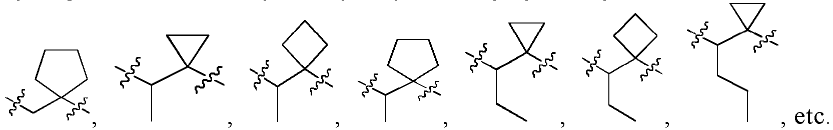

In the present invention, the term "alkenyl" refers to a straight or branched hydrocarbon group containing one or more double bonds and generally having a length of 2 to 20 carbon atoms. The alkenyl is preferably C2-C6 alkenyl, more preferably C2-C4 alkenyl. Alkenyl groups include, but are not limited to, for example, ethenyl, propenyl, butenyl, 1-methyl-2-buten-1-yl, and the like. In the present invention, the alkenyl conprises substituted alkenyl.

In the present invention, the term "alkynyl" refers to a straight or branched hydrocarbon group containing one or more triple bonds and generally having a length of 2 to 20 carbon atoms. The alkynyl group is preferably C2-C6 alkynyl, more preferably C2-C4 alkynyl. Alkynyl groups include, but are not limited to, ethynyl, propynyl, and the like. In the present invention, the alkynyl also comprises substituted alkynyl, and the substituent may be halogen, hydroxyl, cyano, nitro, and the like.

In the present invention, the term "cycloalkyl" refers to a fully saturated cyclic hydrocarbon compound group, including 1 to 4 rings, and each ring of which contains 3 to 8 carbon atoms. The term "C₃-C₂₀" refers to a cycloalkyl containing 3, 4, 5, 6, 7, 8, 9, 10, 11, 12, 13, 14, 15, 16, 17, 18, 19 or 20 carbon atoms. The cycloalkyl is preferably C₃-C₁₄ cycloalkyl, more preferably C₃-C₁₀ cycloalkyl, more preferably C₃-C₆ monocyclic cycloalkyl, C₇-C₁₀ bicyclic or tricyclic cycloalkyl. "Substituted cycloalkyl" means that one or more positions in the cycloalkyl are substituted, especially with 1 to 4 substituents, which can be substituted at any position. In the present invention, the "cycloalkyl" comprises substituted cycloalkyl. Typical substitutions include, but are not limited to, one or more of the following groups: for example hydrogen, deuterium, halogens (such as monohalogen substituent or polyhalogen substituent, the latter may be such as trifluoromethyl or an alkyl group containing Cl₃), cyano, nitro, oxo (such as =O), trifluoromethyl, trifluoromethoxy, cycloalkyl, alkenyl, cycloalkenyl, alkynyl, heterocyclic ring, aromatic ring, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ; wherein Rₐ appearing herein can independently represent hydrogen, deuterium, alkyl, cycloalkyl, alkenyl, cycloalkenyl, alkynyl, heterocyclic ring or aromatic ring; R_{b}, R_{c} and R_{d} can independently represent hydrogen, deuterium, alkyl, cycloalkyl, heterocyclic ring or aromatic ring; or R_{b} and R_{c} together with the N atom can form heterocyclic ring; Rₑ can independently represent hydrogen, deuterium, alkyl, cycloalkyl, alkenyl, cycloalkenyl, alkynyl, heterocyclic ring or aromatic ring. The above typical substituents may be optionally substituted. Typical substitutions also comprise spirocyclic, bridged or fused ring substituents, especially spirocyclic alkyl, spirocyclic alkenyl, spirocyclic heterocyclic ring (excluding heteroaromatic rings), bridged cycloalkyl, bridged cycloalkenyl, bridged heterocyclic ring (excluding heteroaromatic rings), fused cycloalkyl, fused cycloalkenyl, fused heterocyclic or fused aromatic ring groups. The above cycloalkyl, cycloalkenyl, heterocyclyl and heterocyclic aryl can be optionally substituted. Examples of cycloalkyl groups include, but are not limited to: cyclopropyl, cyclobutyl, cyclopentyl, cyclohexyl, adamantane, etc.

The terms "C3-C20 cycloalkylene" refers to a group formed by removing two hydrogen atoms from a cycloalkyl, such as 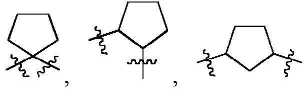 and the like.

In the present invention, the term "heterocyclyl" refers to a fully saturated or partially unsaturated cyclic group (including but not limited to 3-7 membered monocyclic ring, 4-7 membered monocyclic ring, 6-11 membered bicyclic ring or 8-16 membered tricyclic ring or polycyclic ring system), wherein at least one heteroatom present in the ring having at least one carbon atom. The term "4 -20 membered heterocycylyl" refers to heterocyclyl containing 4, 5, 6, 7, 8, 9, 10, 11, 12, 13, 14, 15, 16, 17, 18, 19 or 20 ring atoms. The "heterocyclyl" and the "saturated or unsaturated heterocyclyl" have the same meaning. The "heterocyclyl" is preferably 4-14 membered heterocyclyl (including but not limited to such as 4-6 membered monocyclic, 7-10 membered bicyclic or 8-14 membered tricyclic or polycyclic system), more preferably 4-12 membered heterocyclyl, more preferably 4-10 membered heterocyclyl, such as 4-6 membered monocyclic heterocyclyl, 7-10 membered bicyclic or tricyclic heterocyclyl, more preferably 4-8 membered heterocyclyl, more preferably 4-6 membered heterocyclyl. Each heterocyclic ring containing heteroatom s may have 1, 2, 3 or 4 heteroatoms selected from nitrogen atom, oxygen atom and sulfur atom, wherein the nitrogen atom or sulfur atom may be oxidized and the nitrogen atom may be quaternized. The heterocyclyl may be attached to the residue of any heteroatom or carbon atom of the ring or ring system molecule, preferably to the N or C atom of the ring or ring system molecule. Typical monocyclic heterocyclyl include, but are not limited to, azetidinyl, pyrrolidinyl, oxetanyl, pyrazolinyl, imidazolinyl, imidazolidinyl, oxazolidinyl, isoxazolidinyl, thiazolidinyl, isothiazolidinyl, tetrahydrofuranyl, piperidinyl, piperazinyl, 2-oxopiperazinyl, 2-oxopiperidinyl, 2-oxopyrrolidinyl, hexahydroazepanyl, 4-piperidinonyl, tetrahydropyranyl, morpholinyl, thiomorpholinyl, thiomorpholine sulfoxide group, thiomorpholine sulfone group, 1,3-dioxanyl group and tetrahydro-1,1-dioxythiophene, etc. The polycyclic heterocyclyl includes spirocyclic, fused and bridged heterocyclyl; wherein the spirocyclic, fused and bridged heterocyclyl are optionally connected to other groups through single bond, or further fused to other cycloalkyl, heterocyclyl, aryl and heteroaryl through any two or more atoms on the ring; the heterocyclic group may be substituted or unsubstituted, and when substituted, the substituent is preferably one or more of the following groups, independently selected from alkyl, deuterated alkyl, haloalkyl, alkoxy, haloalkoxy, alkenyl, alkynyl, alkylthio, alkylamino, halogen, amino, nitro, hydroxyl, thiol, cyano, cycloalkyl, heterocyclyl, aryl, heteroaryl, cycloalkylthio, oxo, carboxyl and carboxylate group.

The term "C4-C20 heterocyclylene" refers to a group formed by removing two hydrogen atoms from the heterocyclyl, such as 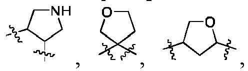 and the like.

In the present invention, the term "aryl" refers to an aromatic cyclic hydrocarbon group having 1 to 5 rings, especially refers to monocyclic or bicyclic group. Wherein, "C₆-C₁₄ aryl" refers to an aromatic cyclic hydrocarbon group containing 6, 7, 8, 9, 10, 11, 12, 13 or 14 ring carbon atoms. Aryl includes phenyl, biphenyl and naphthyl. Where there are two or more aromatic rings (bicyclic, etc.), the aromatic rings of the aryl can be connected by a single bond (such as biphenyl) or fused (such as naphthalene, anthracene, etc.). "Substituted aryl" means that one or more positions in the aryl are substituted, especially with 1 to 3 substituents, which can be substituted at any position. Typical substitutions include, but are not limited to, one or more of the following groups: for example hydrogen, deuterium, halogens (such as monohalogen substituent or polyhalogen substituent, the latter may be such as trifluoromethyl or an alkyl group containing Cl₃), cyano, nitro, oxo (such as =O), trifluoromethyl, trifluoromethoxy, cycloalkyl, alkenyl, cycloalkenyl, alkynyl, heterocyclic ring, aromatic ring, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ; wherein Rₐ appearing herein can independently represent hydrogen, deuterium, alkyl, cycloalkyl, alkenyl, cycloalkenyl, alkynyl, heterocyclic ring or aromatic ring; R_{b}, R_{c} and R_{d} can independently represent hydrogen, deuterium, alkyl, cycloalkyl, heterocyclic ring or aromatic ring; or R_{b} and R_{c} together with the N atom can form heterocyclic ring; Rₑ can independently represent hydrogen, deuterium, alkyl, cycloalkyl, alkenyl, cycloalkenyl, alkynyl, heterocyclic ring or aromatic ring. The above typical substituents may be optionally substituted. Typical substitutions also comprise fused ring substituents, especially fused cycloalkyl, fused cycloalkenyl, fused heterocyclic or fused aromatic ring groups. The above cycloalkyl, cycloalkenyl, heterocyclyl and heterocyclic aryl can be optionally substituted.

The term "heteroaryl" refers to an aromatic cyclic hydrocarbon group containing 1 to 4 heteroatoms selected from oxygen, nitrogen and sulfur. Wherein, "5-14 membered heteroaryl" refers to a heteroaromatic system containing 1-4 heteroatoms and 5-14 ring atoms. The heteroaryl is preferably 5 to 10 membered ring, more preferably 5 or 6 membered ring, such as pyrrolyl, pyrazolyl, imidazolyl, oxazolyl, isoxazolyl, thiazolyl, thiadiazolyl, isothiazolyl , furyl, pyridyl, pyrazinyl, pyrimidinyl, pyridazinyl, triazazinyl, triazolyl and tetrazolyl, etc. "heteroaryl" may be substituted or unsubstituted, and when substituted, the substituent is preferably one or more of the following groups, independently selected from alkyl, deuterated alkyl, haloalkyl, alkoxy, haloalkoxy, alkenyl, alkynyl, alkylthio, alkylamino, halogen, amino, nitro, hydroxyl, thiol, cyano, cycloalkyl, heterocyclyl, aryl, heteroaryl, cycloalkylthio, oxo, carboxyl and carboxylate group.

In the present invention, the term "alkoxy" refers to a straight or branched alkoxy group, including alkyl-O- and alkyl-O-alkyl, wherein "C₁-C₁₈ alkoxy" refers to a straight or branched alkoxy group having 1 to 18 carbon atoms, including C₁-C₁₈ alkyl-O- and -C₁-C₆ alkyl-O-C₁-C₆ alkyl, including but not limited to methoxy, ethoxy, propoxy, isopropoxy and butoxy, etc. Preferred is C₁-C₈ alkoxy, and more preferred is C₁-C₆ alkoxy group.

In the present invention, the term "cycloalkyloxy" refers to cycloalkyl-O-, wherein "C₃-C₂₀ cycloalkyloxy" refers to C₃-C₂₀ cycloalkyl-O-, wherein C₃-C₂₀ cycloalkyl is as defined above.

In the present invention, the term "heterocyclyloxy" refers to "heterocyclyl-O-, wherein "4-20 membered heterocyclyloxy" refers to 4-20 membered heterocyclyl-O-, wherein, 4-20 membered heterocyclyl is as defined above.

In the present invention, the term "C₁-C₁₈ alkyleneoxy" refers to a group obtained by removing a hydrogen atom from "C₁-C₁₈ alkoxy".

In the present invention, the term "halogen" or "halo" refers to chlorine, bromine, fluorine, or iodine.

In the present invention, the term "halogenated" means being substituted by halogen.

In the present invention, the term "deuterated" means being substituted by deuterium.

In the present invention, the term "hydroxyl" refers to a group with a structure of OH.

In the present invention, the term "nitro" refers to a group with a structure of NO₂.

In the present invention, the term "cyano" refers to a group with a structure of CN.

In the present invention, the term "ester group" refers to a group with a structure of -COOR, wherein R represents hydrogen, alkyl or substituted alkyl, cycloalkyl or substituted cycloalkyl, cycloalkenyl or subsitituted cycloalkenyl, aryl or substituted aryl, heterocyclyl or substituted heterocyclyl. The ester group is preferably -COO C₁-C₆ alkyl.

The term "amino" refers to a group with a structure of -NRR', wherein R and R' independently represent hydrogen, alkyl or substituted alkyl, cycloalkyl or substituted cycloalkyl, cycloalkenyl or subsitituted cycloalkenyl, aryl or substituted aryl, heterocyclyl or substituted heterocyclyl with the definitions as described above. R and R' may be the same or different in the dialkylamino segments. The amino is preferably NH₂, NHC₁-C₆alkyl, or N(C₁-C₆ alkyl)₂.

The term "amido" refers to a group with a structure of -CONRR' or ,-NRCOR' wherein R and R' independently represent hydrogen, alkyl or substituted alkyl, cycloalkyl or substituted cycloalkyl, cycloalkenyl or substituted cycloalkenyl, aryl or substituted aryl, heterocyclyl or substituted heterocyclyl with the definitions as described above. The amido is preferably CONH₂, NHCO(C₁-C₆alkyl), or NHCO(C₃-C₆ cycloalkyl).

The term "sulfonamido" refers to a group with a structure of -S₂ONRR' or -NRSO₂R', wherein R and R' each independently represent hydrogen, alkyl or substituted alkyl, cycloalkyl or substituted cycloalkyl, cycloalkenyl or subsitituted cycloalkenyl, aryl or substituted aryl, heterocyclyl or substituted heterocyclyl with the definitions as described above. The sulfonamido is preferably SO₂NH₂, NHSO₂(C₁-C₆alkyl), or NHSO₂(C₃-C₆ cycloalkyl).

The term "sulfonyl" refers to a group with a structure of -SO₂R, wherein R can independently represent hydrogen, alkyl or substituted alkyl, cycloalkyl or substituted cycloalkyl, cycloalkenyl or subsitituted cycloalkenyl, aryl or substituted aryl, heterocyclyl or substituted heterocyclyl with the definitions as described above.

The term "ureido" refers to a group with a structure of -NRCONR'R", wherein R, R' and R" independently represent hydrogen, alkyl or substituted alkyl, cycloalkyl or substituted cycloalkyl, cycloalkenyl or subsitituted cycloalkenyl, aryl or substituted aryl, heterocyclyl or substituted heterocyclyl with the definitions as described above. R, R' and R" may be the same or different in the dialkylamino segments.

The term "alkylaminoalkyl" refers to a group with a structure of -RNHR', wherein R and R' independently represent hydrogen, alkyl or substituted alkyl, cycloalkyl or substituted cycloalkyl, cycloalkenyl or subsitituted cycloalkenyl, aryl or substituted aryl, heterocyclyl or substituted heterocyclyl with the definitions as described above. R and R' may be the same or different.

The term "dialkylaminoalkyl" refers to a group with a structure of -RNHR'R", wherein R, R' and R" independently represent alkyl or substituted alkyl, cycloalkyl or substituted cycloalkyl, cycloalkenyl or subsitituted cycloalkenyl, aryl or substituted aryl, heterocyclyl or substituted heterocyclyl with the definitions as described above. R, R' and R" may be the same or different in the dialkylamino segments.

The term "heterocyclylalkyl" refers to a group with a structure of -RR', wherein R can independently represent alkyl or substituted alkyl, cycloalkyl or substituted cycloalkyl, cycloalkenyl or subsitituted cycloalkenyl, aryl or substituted aryl; R' represents heterocyclyl or substituted heterocyclyl.

In the present invention, the term "substituted" means that one or more hydrogen atoms on a specific group are replaced with a specific substituent. The specific substituents are the substituents described correspondingly in the foregoing, or the substituents appearing in the respective embodiments. Unless otherwise specified, a substituted group may have a substituent selected from a specific group at any substitutable position of the group, and the substituent may be the same or different at each position. Those skilled in the art will understand that the combinations of substituents contemplated by the present invention are those that are stable or chemically achievable. The substituents are, for example (but not limited to): halogen, hydroxyl, cyano, carboxyl (-COOH), C1-C6 alkyl, C2-C6 alkenyl, C2-C6 alkynyl, C3-C8 cycloalkyl, 3-12 membered heterocyclyl, aryl, heteroaryl, C1-C8 aldehyde group, C2-C10 acyl, C2-C10 ester group, amino, C₁-C₆ alkoxy, C1-C10 sulfonyl, C1-C6ureido and the like.

Unless otherwise stated, any heteroatom with insufficient valence is assumed to have sufficient hydrogen atoms to complete its valence.

When a substituent is a non-terminal substituent, it is a subunit of the corresponding group, for example, alkyl corresponds to alkylene, cycloalkyl corresponds to cycloalkylene, heterocyclyl corresponds to heterocyclylene, alkoxy corresponds to alkyleneoxy, and the like.

In the present invention, more refers to 2, 3, 4, or 5.

### Active Ingredients

As used herein, "the compound of the present invention" refers to a compound of formula I, and also includes a stereoisomer or an optical isomer, a pharmaceutically acceptable salt, a prodrug or a solvate of the compound of formula I.

The salts that may be formed by the compounds of the present invention also belong to the scope of the present invention. Unless otherwise specified, the compounds of the present invention are intended to comprise their salts. The term "salt" used herein refers to an acid or basic salt formed by an inorganic or organic acid or a base. In addition, when the compounds of the present invention contain a basic fragment, including but not limited to pyridine or imidazole, and when contain an acidic fragment, including but not limited to carboxylic acid, the zwitter-ions ("inner salts") that may be formed are included within the scope of the term "salt". Pharmaceutically acceptable (ie, non-toxic, physiologically acceptable) salts are preferred, although other salts are also useful, for example, in isolation or purification steps during preparation. The compounds of the present invention may form salts, for example, compound I reacts with a certain amount, such as an equivalent amount, of an acid or a base, and salts out in a medium, or is obtained by freeze-drying from an aqueous solution.

Basic fragments that contained in the compounds of the present invention, including but not limited to amine or pyridine or imidazole rings, may form salts with organic or inorganic acids. Typical acids from which salts may be formed include acetates (e.g., with acetic acid or trihaloacetic acid, such as trifluoroacetic acid), adipates, alginate, ascorbate, aspartate, benzoate, benzenesulfonate, bisulfate, borate, butyrate, citrate, camphorate, camphorsulfonate, cyclopentanepropionate, diglycolate, dodecylsulfate, ethane sulfonate, fumarate, glucoheptonate, glycerophosphate, hemisulfate, heptanoate, hexanoate, hydrochloride, hydrobromide, hydriodate, hydroxyethanesulfonate (e.g., 2-hydroxyethanesulfonate), lactate, maleate, methanesulfonate, naphthalenesulfonate (e.g., 2-naphthalenesulfonate), nicotinate, nitrate, oxalate, pectate, persulfate, phenpropionate (e.g., 3-phenylpropionate), phosphate, picrate, pivalate, propionate, salicylate, succinate, sulfate (e.g., formed with sulfuric acid), sulfonate, tartrate, thiocyanate, toluenesulfonate such as p-toluenesulfonate, dodecanoate, and the like.

Acidic fragments that may contained in the certain compounds of the present invention, including but not limited to carboxylic, may form salts with various organic or an inorganic base. Typical salts formed with bases include ammonium salts, alkali metal salts such as sodium, lithium, or potassium salts, alkaline earth metal salts such as calcium, or magnesium salts, and salts formed with organic bases (such as organic amines), such as benzathine, dicyclohexylamine, hydrabamine (salt with N,N-di(dehydroabietyl)ethylenediamine), N-methyl-D-glucamine, N-methyl-D-glucosamide, tert-butylamine, and salts formed with amino acids such as arginine, lysine, and the like. Basic nitrogen-containing groups can react with halide quaternary ammonium salts, such as small molecular alkyl halides (such as chlorides, bromides and iodides of methyl, ethyl, propyl and butyl), dialkyl sulfates (such as dimethyl sulfate, diethyl sulfate, dibutyl sulfate and dipentyl sulfate), long chain halides (such as chlorides, bromides and iodides of decyl, dodecyl, tetradecyl and tetradecyl), aralkyl halides (such as benzyl and phenyl bromides), etc.

Prodrugs and solvates of the compounds of the present invention are also within the scope of the present invention. The term "prodrug" herein refers to a compound that undergoes chemical transformation by metabolism or chemical processes to produce a compound, salt, or solvate of the present invention when used to treat a related disease. The compounds of the present invention comprise solvates, such as hydrates.

The compounds, salts or solvates of the present invention may exist in tautomeric forms (e.g., amides and imino ethers). All such tautomers are parts of the present invention.

All stereoisomers of the compounds (eg, those having asymmetric carbon atoms which may be present due to various substitutions), including enantiomeric and diastereomeric forms thereof, are all contemplated within the scope of the invention. Individual stereoisomers of the compounds of the present invention may not exist at the sam time with other isomers (for example, as a pure or substantially pure optical isomer having a particular activity), or may be a mixture, such as a racemate, or a mixture formed with all other stereoisomers or a portion thereof. The chiral centers of the present invention have either S or R configurations, as defined by the 1974 recommendations of the International Union of Pure and Applied Chemistry (IUPAC). The racemic forms can be resolved by physical methods such as fractional crystallization, or by derivatization into diastereoisomers and then fractional crystallization, or by separation by chiral column chromatography. Individual optical isomers can be obtained from the racemate by any suitable method, including but not limited to conventional methods, such as salt formation with an optically active acid followed by recrystallization.

The compounds of the present invention, which are obtained by sequential preparation, separation and purification, have a weight content equal to or greater than 90%, for example, equal to or greater than 95%, equal to or greater than 99% ("very pure" compounds), and are listed in the text description. The "very pure" compounds of the present invention herein are also intended to be a part of the present invention.

All configurational isomers of the compounds of the present invention, either in mixed, pure or very pure form, are contemplated within the scope. The definition of compounds of the present invention includes both cis (Z) and trans (E) isomers of olefins, as well as cis and trans isomers of carbocyclic and heterocyclic rings.

Throughout the specification, groups and substituents may be selected to provide stable fragments and compounds.

Specific functional groups and chemical term definitions are detailed below. For the purposes of this invention, chemical elements are as defined in Periodic Table of the Elements, CAS version, Handbook of Chemistry and Physics, 75th Ed. Definitions of specific functional groups are also described therein. In addition, basic principles of organic chemistry as well as specific functional groups and reactivity are described in "Organic Chemistry", Thomas Sorrell, University Science Books, Sausalito: 1999, which is incorporated by reference in its entirety.

Certain compounds of the present invention may exist in specific geometric isomer or stereoisomer forms. The present invention encompasses all compounds, including their cis and trans isomers, R and S enantiomers, diastereomers, (D) isomers, (L) isomers, racemic mixtures and other mixtures. Additionally, the asymmetric carbon atom may represent a substituent, such as an alkyl group. All isomers and mixtures thereof are encompassed in the present invention.

According to the present invention, the mixture of isomers may contain isomers at various ratios. For example, a mixture of only two isomers may have the following combinations: 50:50, 60:40, 70:30, 80:20, 90:10, 95:5, 96:4, 97:3, 98:2, 99:1, or 100:0, and all ratios of isomers are within the scope of the present invention. Similar ratios, as well as ratios of more complex mixtures of isomers that are readily understood by those skilled in the art are within the scope of the invention.

The present invention also includes isotopically labeled compounds, which is equivalent to the original compounds disclosed herein. In practice, however, it often occurs that one or more atoms are replaced by atoms having a different atomic mass or mass number. Examples of isotopes that may be listed as compounds of the present invention include hydrogen, carbon, nitrogen, oxygen, phosphorus, sulfur, fluorine and chlorine isotopes. such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively. The compounds, or enantiomers, diastereomers, isomers, or pharmaceutically acceptable salts or solvates of the present invention, wherein containing the isotopes or other isotopic atoms of the above-mentioned compounds are all within the scope of the present invention. Certain isotopically-labeled compounds of the present invention, for example, radioactive isotopes such as ³H and ¹⁴C, are also within it and are useful in the tissue distribution assays of drug and substrate. Tritium i.e., ³H, and carbon-14 i.e., ¹⁴C, are relatively easy to be prepared and detected and are the first choice among isotopes. In addition, substitution of heavier isotopes such as deuterium, also known as ²H, has advantages in certain therapies due to good metabolic stability, such as increased half-life or reduced dosage in the body. Therefore, in some cases, they can be given priority consideration. Isotope labeled compounds can be prepared using general methods by replacing non isotope reagents with readily available isotope labeled reagents, using the scheme disclosed in the example.

If a specific enantiomer of the compound of the present invention is to be designed for synthesis, it can be asymmetrically synthesized or derivatized using chiral excipients, thereby the resulting diastereoisomeric mixture is isolated, and then the chiral excipients is removed to obtain the pure enantiomer. In addition, if the molecule contains a basic functional group, such as an amino acid, or an acidic functional group, such as carboxyl group, a suitable optically active acid or base can be used to form a diastereoisomeric salt with it, which can then be separated by conventional means, such as separation crystallization or chromatography to obtain the pure enantiomer.

As described in this article, the compound of the present invention can be substituted by any number of substituents or functional groups to expand the inclusion range. In general, the term "substituted", whether preceding or following the term "optionally", refers to the replacement of a hydrogen radical with a substituent with a specified structure in the formula including substituents in the present invention. When a plurality of positions in a particular structure are replaced by a plurality of particular substituents, the substituents may be the same or different at each position. The term "substitution" used in this article includes all allowed organic compound substitutions. Broadly speaking, the allowed substituents include non-cyclic, cyclic, branched, non-branched, carbocyclic, and heterocyclic, aromatic, and non aromatic organic compounds. In the present invention, heteroatom nitrogen, for example, may have hydrogen substituents or any allowed organic compounds as described above to complement its valence. Furthermore, the present invention is not intended to limit in any way the permissible substitution of organic compounds. The present invention considers combinations of substituents and variable groups to be excellent in the treatment of diseases, such as infectious or proliferative diseases, in the form of stable compounds. The term "stable" herein refers to a compound with stability that can be detected for a sufficient period of time to maintain the structural integrity of the compound, preferably effective in a sufficient period of time, and is used herein for the foregoing purposes.

The metabolites of the compounds and pharmaceutically acceptable salts thereof involved in the present application, as well as prodrugs that can be in vivo converted into the structures of the compounds involved in the present application and pharmaceutically acceptable salts thereof, are also included in the claims of the present application. In the present invention, the term "prodrug" refers to a compound obtained by modifying the chemical structure of a drug with an easy-leaving group, which is inactive or less active in vitro, but releases the active drug through enzymatic(such as hydrolases, oxidases, etc.) or non enzymatic(such as pH, light, radiation, etc.) conversion in vivo and exerts its therapeutic effect. The prodrugs include, but are not limited to, carboxylates, phosphates, carbamates, carbonates, and the like. The structure of the easy-leaving group is (but not limited to:

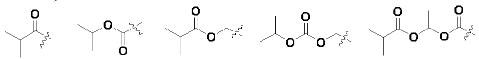

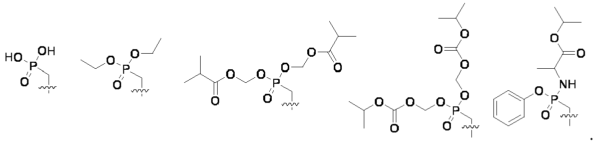

### Preparation method

The following provides a more specific description of the preparation methods for the compounds having a structure of formula (A0) of the present invention, but these specific methods do not pose any limitations to the present invention. The compound of the present invention can also be conveniently prepared by combining various synthesis methods described in this specification or known in the art, and such combinations can be easily carried out by those skilled in this field to which the present invention belongs.

Typically, the preparation process of the compound of the present invention is as follows, wherein the raw materials and reagents used can be purchased through commercial channels unless otherwise specified.

Preferably, the compound of the present invention are be prepared by the following methods (taking formula IA as an example): 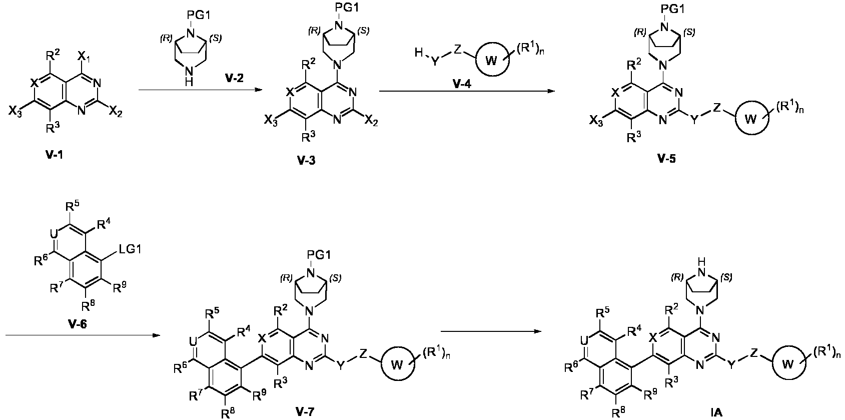
(i) in an inert solvent, in the presence of a base, with or without a Pd catalyst, with or without a condensing agent, reacting a compound of formula V-1 with a compound of formula V-2, thereby obtaining a compound of formula V-3;
(ii) in an inert solvent, in the presence of a base, with or without the presence of a Pd catalyst, reacting the compound of formula V-3 with a compound of formula V-4, thereby obtaining a compound of formula V-5;
(iii) in an inert solvent, in the presence of a base, in the presence of a Pd catalyst, reacting the compound of formula V-5 with a compound of formula V-6, thereby obtaining a compound of formula V-7;
(iv) under acid (such as TFA, HCl, etc) condition or under Pd catalytic hydrogenation condition, removing the protective group PG1 from the compound of formula V-7, thereby obtaining a compound of formula (IA);
wherein,
X₁, X₂ and X₃ are each independently selected from: OH, halogen, OTf, OTs and OMs;
PG1 is selected from: Boc, Cbz, and Bn;
LG1 is selected from -B(OH)₂, -B(KBF₃), -Sn(ⁿBu)₃, 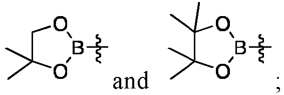
R¹, R², R³, R⁴, R⁵, R⁶, R⁷, R⁸, R⁹, U, X, Y, Z, W and n are defined as above.

### Pharmaceutical compositions and administration method

The pharmaceutical composition of the present invention is used for preventing and/or treating the following diseases: inflammation, cancer, cardiovascular disease, infection, immune disease, and metabolic disease.

The compound of general formula (A0) can be used in combination with other drugs known to treat or improve similar symptoms. When administered in combination, the administration method and dosage of the original drug can remain unchanged, and the compound of formula I can be taken simultaneously or subsequently. When the compound of formula (A0) is taken simultaneously with one or more other drugs, it is preferred to use a pharmaceutical composition containing one or more known drugs and the compound of formula I at the same time. Drug combination also includes taking the compound of formula (A0) and one or more other known drugs during overlapping time periods. When the compound of formula (A0) is used in combination with one or more other drugs, the dose of the compound of formula I or the known drug may be lower than the dose when used alone.

Drugs or active ingredients that can be used in combination with the compound of general formula (A0) include but are not limited to: PD-1 inhibitor (such as nivolumab, pembrolizumab, pidilizumab, cemiplimab, JS-001, SHR-120, BGB-A317, IBI-308, GLS-010, GB-226, STW204, HX008, HLX10, BAT 1306, AK105, LZM 009, or biosimilars of the above drugs, and the like), PD-L1 inhibitor (such as durvalumab, atezolizumab, avelumab, CS1001, KN035, HLX20, SHR-1316, BGB-A333, JS003, CS1003, KL -A167, F 520, GR1405, MSB2311, or biosimilars of the above drugs, and the like), CD20 antibody (such as rituximab, obinutuzumab, ofatumumab, veltuzumab, tositumomab, 1311-tositumomab, ibritumomab, 90Y-ibritumomab, 90In-ibritumomab, ibritumomab tiuxetan, and the like), CD47 antibody (such as Hu5F9-G4, CC-90002, TTI-621, TTI-622, OSE-172, SRF-231, ALX-148, NI-1701, SHR-1603, IBI188, IMM01), ALK inhibitor (such as Ceritinib, Alectinib, Brigatinib, Lorlatinib, Alkotinib), PI3K inhibitor (such as Idelalisib, Duvelisib, Dactolisib, Taselisib, Bimiralisib, Omipalisib, Buparlisib, and the like), BTK inhibitor(such as Ibrutinib, Tirabrutinib, Acalabrutinib, Zanubrutinib, Vecabrutinib and the like), EGFR inhibitor(such as Afatinib, Gefitinib, Erlotinib , Lapatinib, Dacomitinib, Icotinib, Canertinib, Sapitinib, Naquotinib, Pyrotinib, Rociletinib, Osimertinib, and the like), VEGFR inhibitor (such as Sorafenib, Pazopanib, Regorafenib , Sitravatinib, Ningetinib, Cabozantinib, Sunitinib, Donafenib, and the like), HDAC inhibitor (such as Givinostat, Tucidinostat, Vorinostat, Fimepinostat, Droxinostat, Entinostat, Dacinostat, Quisinostat, Tacedinaline, and the like), CDK inhibitor (such as Palbociclib, Ribociclib, Abemaciclib, Milciclib, Trilaciclib, Lerociclib, and the like), MEK inhibitor(such as Selumetinib (AZD6244), Trametinib (GSK1120212), PD0325901, U0126, Pimasertib (AS-703026), PD184352 (CI-1040), and the like), mTOR inhibitor (such as Vistusertib, and the like), SHP2 inhibitor (such as RMC-4630, JAB-3068, TNO155, and the like), or combinations thereof. The dosage forms of the pharmaceutical composition of the present invention include (but are not limited to): injection, tablet, capsule, aerosol, suppository, film, pill, external ointment, controlled release or sustained release or nano preparation.

The pharmaceutical composition of the invention comprises the compound of the present invention or the pharmaceutically acceptable salts thereof in a safe and effective dosage range, and pharmaceutically acceptable excipients or carriers. In which, "safe and effective amount" means that the amount of the compound is sufficient to significantly improve the condition without causing serious side effects. Generally, the pharmaceutical composition contains 1-2000 mg of the compound of the present invention/dose, more preferably, 10-1000 mg of the compound of the present invention/dose. Preferably, the "one dose" is one capsule or one pill.

"Pharmaceutically acceptable carrier" means one or more compatible solid or liquid fillers, or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agents, flavoring agents, stabilizers, antioxidants, preservatives, pyrogen-free water, etc..

There is no special limitation of administration mode for the compound or pharmaceutical compositions of the present invention, and the representative administration mode includes (but is not limited to): oral, intratumoral, rectal, parenteral (intravenous, intramuscular or subcutaneous), and topical administration.

Solid dosage forms for oral administration include capsules, tablets, pills, powders and granules. In these solid dosage forms, the active compounds are mixed with at least one conventional inert excipient (or carrier), such as sodium citrate or dicalcium phosphate, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents, such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants, such as talc, stearin calcium, magnesium stearate, solid polyethylene glycol, sodium lauryl sulfate, or the mixtures thereof. In capsules, tablets, and pills, the dosage form may also include buffer.

Solid dosage forms such as tablets, sugar pills, capsules, pills, and granules can be prepared using coating and shell materials, such as casings and other well-known materials in the art. They can contain an opaque agent. The active compounds or compounds in the compositions can be released in a delayed mode in a given portion of the digestive tract. Examples of embedding components that can be used are polymeric substances and waxes. If necessary, the active compound can also form microcapsules with one or more of the aforementioned excipients.

Liquid dosage forms for oral administration include pharmaceutically acceptable emulsions, solutions, suspensions, syrups or tinctures. In addition to the active compound, the liquid dosage form may contain inert diluents conventionally used in the art, such as water or other solvents, solubilizers and emulsifiers, such as ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances.

In addition to these inert diluents, the composition can also include additives such as wetting agents, emulsifiers and suspending agents, sweeteners, corrigents, and spices.

In addition to active compounds, suspensions can include suspending agents such as ethoxylated isooctadecanol, polyoxyethylene sorbitol and dehydrated sorbitol esters, microcrystalline cellulose, methanol aluminum and agar, or mixtures of these substances.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols, and their suitable mixtures.

The dosage forms of the compounds of the present invention used for local administration include ointments, powders, patches, sprays, and inhalants. The active ingredients are mixed under sterile conditions with physiologically acceptable carriers and any preservatives, buffers, or propellants that may be necessary.

The treatment method of the present invention can be administered alone or in combination with other treatment means or therapeutic drugs.

When the pharmaceutical composition is used, a safe and effective amount of the compound of the present invention is administered to a mammal (such as a human) in need of treatment, wherein the dosage at the time of administration is the pharmaceutically effective dosage, for people having a body weight of 60kg, the daily dose is usually 1 ~ 2000mg, preferably 50~1000mg. Of course, the specific dosage should also consider factors such as the route of administration and the patient's health status, which are within the skill range of a skilled physician.

The present invention further provides a preparation method for a pharmaceutical composition, comprising a step of: mixing pharmaceutically acceptable carriers with the compound of formula (A0) as described in the present invention, or the crystal form, the pharmaceutically acceptable salt, the hydrate or the solvate thereof, so as to form the pharmaceutical composition.

The present invention also provides a treatment method, comprising steps of administering the compound of the general formula (A0) of the present invention, or a crystal form, a pharmaceutically acceptable salt, a hydrate or a solvate thereof, or administering the pharmaceutical composition of the present invention to a subject in need of treatment, for selectively inhibiting KRAS^{G12D}.

### Compared with the prior art, the present invention has the following main advantages:

(1) The compounds have good selective inhibitory effect on KRAS^{G12D};
(2) The compound has better pharmacodynamics, pharmacokinetic properties and lower toxic side effects.

The present invention was further described hereafter in combination with specific embodiments. It should be understood that these examples are only used to illustrate the the invention and not to limit the scope of the invention. Experimental methods in which the specific conditions are not specified in the following examples are usually in accordance with the conventional conditions such as the conditions described in Sambrook et al., Molecular Cloning: Laboratory Manual (New York: Cold Spring Harbor Laboratory Press, 1989), or in accordance with the conditions recommended by the manufacturer. Unless indicated otherwise, percentage and parts are calculated by weight.

Unless otherwise defined, all professional and scientific terminology used in the text have the same meanings as known to the skilled in the art. In addition, any methods and materials similar or equal with the recorded content can apply to the methods of the invention. The method of the preferred embodiment described herein and the material are only for demonstration purposes.

The structures of the compounds of the present invention are determined by nuclear magnetic resonance (NMR) and liquid chromatography-mass spectrometry (LC-MS).

NMR was detected using a Bruker AVANCE-400 nuclear magnetic spectrometer. The detecting solvents included deuterated dimethyl sulfoxide (DMSO-d₆), deuterated acetone (CD₃COCD₃), deuterated chloroform (CDCl₃) and deuterated methanol (CD₃OD) and the like. Tetramethylsilane (TMS) was used as the internal standard. The chemical shift was measured in parts per million (ppm).

Liquid chromatography-mass spectrometry (LC-MS) was detected using a Waters SQD2 mass spectrometer. HPLC was detected using an Agilent 1100 high pressure chromatograph (Microsorb 5 micron C18 100 x 3.0 mm column).

The thin layer chromatography silica gel plate used was Qingdao GF254 silica gel plate, TLC used was 0.15-0.20 mm, and preparative thin layer chromatography used was 0.4 mm-0.5 mm. Column chromatography generally uses Qingdao silica gel 200-300 mesh silica gel as carriers.

The starting materials in the examples of the present invention are all known and commercially available, or can be synthesized using or according to literature data reported in the art.

Unless otherwise specified, all reactions of the present invention are carried out under the protection of dry inert gas (such as nitrogen or argon) with continuous magnetic stirring, and the reaction temperatures are all in degrees Celsius.

### Example

### Preparation of intermediate 1-1 (3-(methoxymethyl)tetrahydro-1H-pyrrolizin-7a(5H)-yl)methanol

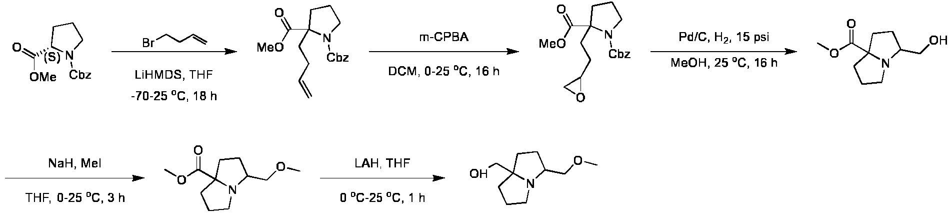

### Step 1: Preparation of 1-benzyl 2-methyl 2-(butyl-3-ene-1-yl)pyrrolin-1,2-diformate

Under nitrogen protection, a solution of 1-benzyl 2-methyl (S)-pyrrolin-1,2-diformate (50.0 g, 190 mmol, 1.00 eq) in THF (100 mL) was added dropwise to LiHMDS (1.00 M, 285 mL, 1.50 eq) at -70 °C. The resulting reaction solution was reacted at -70 °C for 2 h, and then added with 4-bromobut-1-ene (51.2 g, 380 mmol, 38.6 mL, 2.00 eq) dropwise. The resulting reaction solution was warmed to 25 °C and reacted for 16 h, then quenched with saturated NH₄Cl (200 mL) aqueous solution, and then extracted with EtOAc (100 mL *3). The combined organic phase was washed with saturated salt water (200 mL), then dried over anhydrous sodium sulfate, and filtered. The filtrate was concentrated under reduced pressure to obtain the target product (38.0 g, 120 mmol, 63.1% yield).

¹H NMR (400 MHz, CDCl₃) δ 7.33 (m, 5 H) 5.77 (m, 1 H) 5.07 (m, 4 H) 3.72 (m, 3 H) 3.48 (m, 2 H) 2.01 (m, 8 H)

### Step 2: Preparation of 1-benzyl 2-methyl 2-(2-(epoxypropan-2-yl)ethyl) pyrrolin-1,2-diformate

To a solution of 1-benzyl 2-methyl 2-(but-3-ene-1-yl)pyrrolin-1,2-diformate (37.5 g, 118 mmol, 1.00 eq) in DCM (650 mL) was added m-CPBA (60.0 g, 295 mmol, 85.0% purity, 2.50 eq) in batches at 0 °C. The resulting reaction solution was reacted at 25 °C for 16 h and then washed with saturated Na₂SO₃ (300 mL*3). The organic phase was dried over anhydrous sodium sulfate and then filtered. The filtrate was concentrated under reduced pressure, and the residue was separated by silica gel column chromatography to obtain the target product (29.0 g, 87.0 mmol, 73.6% yield).

¹H NMR (400 MHz, CDCl₃) δ 7.33 (br d, J=7.88 Hz, 5 H) 5.11 (m, 2 H) 3.70 (m, 3 H) 3.48 (m, 2 H) 2.73 (m, 2 H) 2.36 (m, 2 H) 1.98 (m, 5 H) 1.50 (m, 2 H)

### Step 3: Preparation of methyl 3-(hydroxymethyl)tetrahydro-1H-pyrrolizin-7a(5H)-formate

To a solution of 1-benzyl 2-methyl 2-(2-(epoxypropan-2-yl)ethyl)pyrrolin-1,2-diformate (28.0 g, 84.0 mmol, 1.00 eq) in MeOH (600 mL) was added Pd/C (6.00 g, 10.0% wt). Under hydrogen atmosphere, the reactants were reacted at 25 °C for 16 h and then filtered. The filtrate was concentrated under reduced pressure to obtain the target product (16.8 g), which was used directly in the next reaction without purification.

Methyl 3-(hydroxymethyl)tetrahydro-1H-pyrrolizin-7a(5H)-formate was subjected to chiral resolution to obtain methyl cis-3-(hydroxymethyl)tetrahydro-1H-pyrrolizin-7a(5H)-formate and methyl trans 3-(hydroxymethyl)tetrahydro-1H-pyrrolizin-7a(5H)-fromate.

### Step 4: Preparation of methyl 3-(methoxymethyl)tetrahydro-1H-pyrrolizin-7a(5H)-formate

Under nitrogen protection, to a solution of methyl 3-(hydroxymethyl)tetrahydro-1H-pyrrolizin-7a(5H)-formate (1.00 g, 5.02 mmol, 1.00 eq) in THF (20 mL) was added NaH (602 mg, 15.1 mmol, 60.0% wt, 3.00 *eq)* at 0 °C. The reaction solution was reacted at 25 °C for 0.5 h, and then added with Mel (1.42 g, 10.0 mmol, 625 uL, 2.00 eq). The resulting reaction solution was reacted at 25 °C for 3 h, then quenched with H₂O (10 mL) at 0 °C, and then extracted with EtOAc (30 mL *2). The combined organic phase was dried over anhydrous magnesium sulfate and then filtered. The filtrate was concentrated under reduced pressure, and the residue was separated by silica gel column chromatography to obtain the target product (1.0 g, 1.88 mmol, 37.5% yield).

¹H NMR (400 MHz, CDCl₃) δ 3.66 - 3.72 (m, 3H) 3.30 - 3.33 (m, 3 H) 2.22 - 2.43 (m, 1 H) 1.83 - 2.12 (m, 6 H) 1.56 - 1.72 (m, 1 H).

Methyl *cis*-3-(methoxymethyl)tetrahydro-1H-pyrrolizin-7a(SH)-formate and methyl trans3-3-(methoxymethyl)tetrahydro-1H-pyrrolizin-7a(5H)-formate were obtained by using methyl *cis*-3-(hydroxymethyl)tetrahydro-1H-pyrrolizin-7a(SH)-formate and methyl *trans* 3-(hydroxymethyl)tetrahydro-1H-pyrrolizin-7a(5H)-formate as starting materials, respectively.

### Step 5: Preparation of (3-(methoxymethyl)tetrahydro-1H-pyrrolizin-7a(5H)-yl)methanol

Under nitrogen protection, a solution of methyl 3-(methoxymethyl)tetrahydro-1H-pyrrolizin-7a(5H)-formate (1.00 g, 4.69 mmol, 1.00 eq) in THF (10 mL) was added with LiAlH₄ (356 mg, 9.38 mmol, 2.00 *eq)* at 0 °C. The reaction solution was reacted at 25 °C for 1 h, then quenched by adding H₂O (10 mL), 15% NaOH (10 mL) and H₂O (30 mL) sequentially at 0 °C, and then extracted with EtOAc (50 mL*2). The combined organic phase was dried over anhydrous magnesium sulfate and then filtered. The filtrate was concentrated under reduced pressure, and the residue was separated by silica gel column chromatography to obtain the target product (1.1g).

LC-MS: m/z 186 (M+H)⁺. ¹H NMR (400 MHz, CDCl₃) δ 4.39 (dd, *J*=10.54, 9.03 Hz, 1 H) 4.26 (br d, *J*=6.53 Hz, 1 H) 3.97 - 4.06 (m, 1 H) 3.80 - 3.96 (m, 2 H) 3.68 - 3.76 (m, 2 H) 3.50 (s, 3 H) 2.04 - 2.40 (m, 6 H) 1.78 - 1.93 (m, 2 H)

The following compounds were synthesized according to the same synthesis method for Intermediate 1-1 with different starting materials:

### Intermediate 1-1A and Intermediate 1-1B cis-(3-(methoxymethyl)tetrahydro-1H-pyrrolizin-7a(SH)-yl)methanol and trans-(3-(methoxymethyl)tetrahydro-1H-pyrrolizin-7a(5H)-yl)methanol

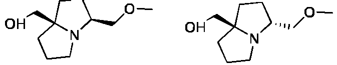

### Intermediate 1-1A

LC-MS: m/z 186 (M+H)⁺.

### Intermediate 1-1B

LC-MS: m/z 186 (M+H)⁺.

### Intermediate 1-2 (3-((ethoxy)methyl)tetrahydro-1H-pyrrolizin-7a(5H)-yl)methanol

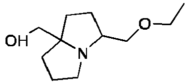

LC-MS: m/z 200 (M+H)⁺.

### Intermediate 1-3 (3-((isopropoxy)methyl)tetrahydro-1H-pyrrolizin-7a(5H)-yl)methanol

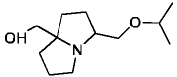

LC-MS: m/z 214 (M+H)⁺.

### Intermediate 1-4 (3-((cyclopropylmethoxy)methyl)tetrahydro-1H-pyrrolizin-7a(SH)-yl)methanol

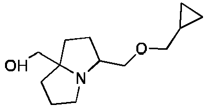

LC-MS: m/z 226 (M+H)⁺.

### Intermediate 1-5 (3-(cyclopropoxymethyl)tetrahydro-1H-pyrrolizin-7a(SH)-yl)methanol

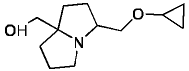

LC-MS: m/z 212 (M+H)⁺.

### Intermediate 1-6 ((2R)-2-fluoro-5-(methoxymethyl)tetrahydro-1H-pyrrolizin-7a(SH)-yl)methanol

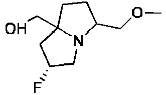

LC-MS: m/z 204 (M+H)⁺.

### Intermediate 1-7 ((2R)-2-fluoro-5-(cyclopropoxymethyl)tetrahydro-1H-pyrrolizin-7a(5H)-yl)methanol

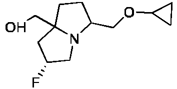

LC-MS: m/z 230 (M+H)⁺.

### Preparation of Intermediate 1-8 (dihydro-1'H,3'H-spiro[cyclopropan-1,2'-pyrrolizin]-7a'(5'H)-yl)methanol

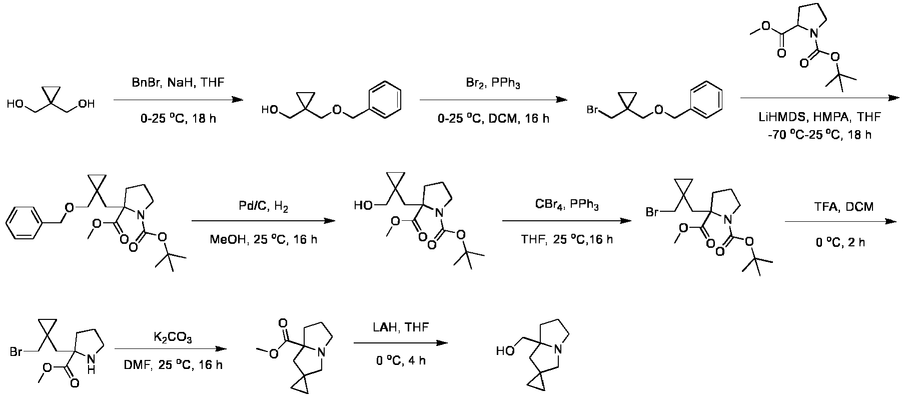

### Step 1: Preparation of (1-((benzyloxy)methyl)cyclopropyl)methanol

To a solution of cyclopropan-1,1-dimethanol (25.0 g, 245 mmol, 1.00 eq) in THF (500 mL) was added NaH (9.79 g, 245 mmol, 60.0% wt, 1.00 *eq*) at 0°C. The mixture was stirred at 25 °C for 30 min, and then BnBr (41.9 g, 245 mmol, 29.1 mL, 1.00 eq) was added. The resulting reaction solution was reacted at 25 °C for 18 h, then quenched with saturated NH₄Cl (50 mL) aqueous solution, and then extracted with EtOAc (50 mL *3). The combined organic phase was washed with saturated salt water (20 mL*3), then dried over anhydrous magnesium sulfate, and then filtered. The filtrate was concentrated under reduced pressure, and the residue was separated by silica gel column chromatography to obtain the target product (26.00 g, 108 mmol, 44.1% yield).

¹H NMR (400 MHz, CDCl₃) δ 7.35 (s, 5H), 4.56 (s, 2H), 3.58 (s, 2H), 3.47 (s, 2H), 0.64 - 0.44 (m, 4H)

### Step 2: Preparation of (((1-(bromomethyl)cyclopropyl)methoxy)methyl)benzene

To a solution of (1-((benzyloxy)methyl)cyclopropyl)methanol (26.0 g, 135 mmol, 1.00 eq) in DCM (300 mL) was added PPh₃ (39.0 g, 149 mmol, 1.10 eq) and NBS (26.5 g, 148 mmol, 1.10 eq) at 0°C. The reaction solution was reacted at 25 °C for 16 h, and then concentrated under reduced pressure. The residue was separated by silica gel column chromatography to obtain the target product (24.0 g, 84.7 mmol, 62.6% yield).

¹H NMR (400 MHz, CDCl₃) δ 7.27 (s, 5H), 4.47 (s, 2H), 3.49 (s, 2H), 3.38 (s, 2H), 0.54 - 0.27 (m, 4H)

### Step 3: Preparation of 1-(tert-butyl)2-methyl2-((1-((benzyloxy)methyl)cyclopropyl)methyl)pyrrolidin-1,2-dif ormate

To a solution of 1-(tert-butyl) 2-methylpyrrolidin-1,2-diformate (15.0 g, 65.4 mmol, 1.00 eq) in THF (150 mL) was added LiHMDS (1.00 M, 78.5 mL, 1.20 eq) dropwise at -70 °C. The resulting reaction solution was reacted at the same temperature for 2 h, and then (((1-(bromomethyl)cyclopropyl)methoxy)methyl)benzene (21.7 g, 85.1 mmol, 1.30 eq) and HMPA (44.5 g, 249 mmol, 43.7 mL, 3.80 eq) were added. The resulting reaction solution was reacted at 25 °C for 16 h, then quenched with water (100 mL), and then extracted with EtOAc (100 mL *2). The combined organic phase was washed with saturated salt water (50 mL*2), then dried over anhydrous sodium sulfate, and then filtered. The filtrate was concentrated under reduced pressure, and the residue was separated by silica gel column chromatography to obtain the target product (12.0 g, 26.8 mmol, 40.9% yield).

¹H NMR (400 MHz, CD₃OD) δ 7.46 - 7.21 (m, 5H), 4.68 - 4.35 (m, 2H), 3.79 - 3.56 (m, 4H), 3.45 - 3.26 (m, 3H), 2.59 - 2.38 (m, 2H), 2.09 - 1.82 (m, 4H), 1.47 - 1.37 (m, 9H), 0.54 - 0.42 (m, 4H).

### Step 4: Preparation of 1-(tert-butyl)2-methyl2-((1-(hydroxymethyl)cyclopropyl)methyl)pyrrolidin-1,2-difor mate

Under nitrogen protection, to a solution of 1-(tert-butyl)2-methyl 2-((1-((benzyloxy)methyl)cyclopropyl)methyl)pyrrolidin-1,2-diformate (11.0 g, 27.3 mmol, 1.00 eq) in MeOH (100 mL) was added Pd/C (10%wt, 1.50 g). The resulting reaction solution was reacted under hydrogen atmosphere (15 psi) at 25 °C for 16 h. The reaction solution was filtered. The filtrate was concentrated under reduced pressure, and the residue was separated by silica gel column chromatography to obtain the target product (10.0 g, 25.5 mmol, 93.6% yield).

¹H NMR (400 MHz, CD₃OD) δ 3.73 - 3.60 (m, 4H), 3.37 (s, 5H), 2.08 - 1.90 (m, 4H), 1.48 - 1.42 (m, 9H), 0.59 - 0.36 (m, 4H).

### Step 5: Preparation of 1-(tert-butyl) 2-methyl 2-((1-(bromomethyl)cyclopropyl)methyl)pyrrolidin-1,2-diformate

To a solution of 1-(tert-butyl) 2-methyl 2-((1-(hydroxymethyl)cyclopropyl)methyl)pyrrolidin-1,2-diformate (5.00 g, 16.0 mmol, 1.00 eq) in DCM (50 mL) was added CBr₄ (7.94 g, 23.9 mmol, 1.50 eq) and PPh₃ (6.11 g, 23.3 mmol, 1.46 eq). The resulting mixture was reacted at 25 °C for 16 h, and then concentrated under reduced pressure. The residue was separated by silica gel column chromatography to obtain the target product (0.50 g, 1.20 mmol, 7.50% yield).

¹H NMR (400 MHz, CDCl₃) δ 3.80 - 3.70 (m, 5H), 3.52 - 3.42 (m, 1H), 3.19 - 3.02 (m, 1H), 2.82 (dd, *J =* 7.0, 15.6 Hz, 1H), 2.45 - 2.20 (m, 1H), 2.13 - 1.89 (m, 4H), 1.49 - 1.44 (m, 9H), 1.00 - 0.51 (m, 4H).

### Step 6: Preparation of methyl 2-((1-(bromomethyl)cyclopropyl)methyl)pyrrolidin-2-formate

To a solution of 1-(tert-butyl) 2-methyl 2-((1-(bromomethyl)cyclopropyl)methyl)pyrrolidin-1,2-diformate (1.40 g, 3.72 mmol, 1.00 eq) in DCM (14 mL) was added TFA (64.7 g, 567.3 mmol, 42.0 mL, 152 eq). The reaction was carried out at 0 °C for 2 h, and then concentrated under reduced pressure to afford the target product (0.60 g, 1.74 mmol, 46.7% yield) , which was used directly in the next reaction without purification.

### Step 7: Preparation of methyl dihydro-1'H,3'H-spiro[cyclopropan-1,2'-pyrrolidin]-7a'(5'H)-formate

To a solution of methyl 2-((1-(bromomethyl)cyclopropyl)methyl)pyrrolidin-2-formate (0.60 g, 2.17 mmol, 1.00 eq) in DMF (10 mL) was added K₂CO₃ (1.20 g, 8.69 mmol, 4.00 eq). The resulting reaction solution was reacted at 25 °C for 16 h, and then concentrated under reduced pressure, and the residue was separated by silica gel column chromatography to obtain the target product (0.60 g, 1.84 mmol, 84.9% yield).

¹H NMR (400 MHz, CDCl₃) δ 3.75 (s, 3H), 3.31 - 3.18 (m, 1H), 3.01 (d, *J* = 10.0 Hz, 1H), 2.81 (s, 1H), 2.71 (d, *J* = 10.0 Hz, 1H), 2.28 - 2.22 (m, 2H), 1.94 - 1.84 (m, 4H), 0.64 - 0.45 (m, 4H).

### Step 8: Preparation of (dihydro-1'H,3'H-spiro[cyclopropan-1,2'-pyrrolizin]-7a'(5'H)-yl)methanol

Under nitrogen protection, to a solution of methyl dihydro-1'H,3'H-spiro[cyclopropan-1,2'-pyrrolidin]-7a'(5'H)-formate (0.60 g, 3.07 mmol, 1.00 eq) in THF (1 mL) was added LAH (174.9 mg, 4.61 mmol, 1.50 eq). The resulting reaction solution was reacted at 0 °C for 4 h, then quenched by adding water (0.3 mL), and then MgSO₄ (2 g) was added. The resulting reaction solution was stirred at room temperature for 30 min and then filtered. The filtrate was concentrated under reduced pressure to obtain the target product (0.200 g, 1.08 mmol, 35.0% yield).

LC-MS: m/z 168 (M+H)⁺. ¹H NMR (400 MHz, CDCl₃) δ 3.50 - 3.43 (m, 1H), 3.39 - 3.31 (m, 1H), 3.10 - 3.03 (m, 1H), 2.90 (d, *J =* 10.3 Hz, 1H), 2.80 (td, *J =* 6.3, 10.5 Hz, 1H), 2.69 (d, *J =* 10.5 Hz, 1H), 1.98 - 1.82 (m, 6H), 0.62 - 0.46 (m, 4H).

**The following compounds were synthesized according to the same synthesis method for Intermediate 1-8 with different starting materials:**

### Intermediate 1-9 ((6'R)-6'-fluorodihydro-1'H,3'H-spiro[cyclopropan-1,2'-pyrrolizin]-7a'(5'H)-yl)meth anol

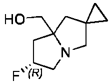

LC-MS: m/z 186 (M+H)⁺. ¹H NMR (400 MHz, CDCl₃) δ 7.00 (s, 1H), 5.44 - 4.99 (m, 1H), 3.60 - 3.25 (m, 3H), 3.23 - 2.95 (m, 3H), 2.88 - 2.51 (m, 1H), 2.36 - 2.03 (m, 4H), 1.96 - 1.57 (m, 1H), 1.43 - 1.25 (m, 1H), 0.67 - 0.38 (m, 4H)

### Intermediate 1-10 ((6'S)-6'-fluorodihydro-1'H,3'H-spiro[cyclopropan-1,2'-pyrrolizin]-7a'(5'H)-yl)metha nol

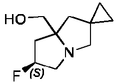

LC-MS: m/z 186 (M+H)⁺. ¹H NMR (400 MHz, CDCl₃) δ 5.15 - 5.43 (m, 1 H) 3.41 - 3.54 (m, 3 H) 3.00 - 3.14 (m, 3 H) 2.56 (d, *J* = 11.22 Hz, 1 H) 2.22 - 2.36 (m, 1 H) 2.00 - 2.17 (m, 1 H) 1.94 (d, *J* = 12.98 Hz, 1 H) 1.64 (d, *J* = 12.76 Hz, 1 H) 0.44 - 0.69 (m, 4 H).

### Synthetic route 1 for Intermediate 1-11A ((trans)-2,2-difluorodihydro-1'H,3'H-spiro[cyclopropan-1,2'-pyrrolizin]-7a'(5'H)-yl) methanol and 1-11B ((cis)-2,2-difluorodihydro-1'H,3'H-spiro[cyclopropan-1,2'-pyrrolizin]-7a'(5'H)-yl)met hanol

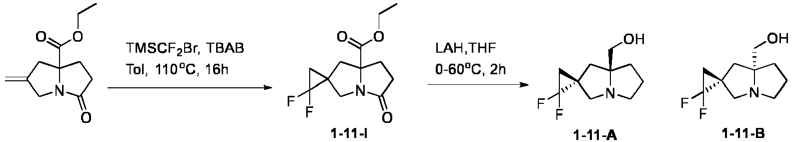

### Step 1: Preparation of ethyl 2,2-difluoro-5'-oxodihydro-1'H,3'H-spiro[cyclopropan-1,2'-pyrrolizin]-7a'(5'H)-form ate (Intermediate 1-11-I)

To a solution of ethyl 2-methylene-5-oxotetrahydro-1H-pyrrolizin-7a(5H)-formate (4.8 g, 22.9 mmol, 1.00 eq) in toluene (75 mL) was added TBAB (222 mg, 688 umol, 0.03 eq) and [bromo(difluoro)methyl]-trimethyl-silicon (14.0 g, 68.8 mmol, 3.00 eq). The reaction solution was reacted at 110 °C for 16 h, and then concentrated under reduced pressure. The residue was separated by silica gel column chromatography to obtain the target product (5.2g, 87.6% yield).

LC-MS: m/z 260 (M+H)⁺.

### Step 2: Preparation of ((trans)-2,2-difluorodihydro-1'H,3'H-spiro[cyclopropan-1,2'-pyrrolizin]-7a'(5'H)-yl) methanol and ((cis)-2,2-difluorodihydro-1'H,3'H-spiro[cyclopropan-1,2'-pyrrolizin]-7a'(5'H)-yl)met hanol (Intermediate 1-11A and 1-11B)

At 0°C, to a solution of ethyl 2,2-difluoro-5'-oxodihydro-1'H,3'H-spiro[cyclopropan-1,2'-pyrrolizin]-7a'(5'H)-formate (2.40 g, 9.26 mmol, 1.00 eq) in THF (40 mL) was added LAH (2.1 g, 57.6 mmol, 6.00 eq). The reaction solution was reacted at 60 °C for 2 h and then quenched with Na₂SO₄.10H₂O (20 g) at 0 °C and then filtered. The filter cake was washed with THF (40 mL). The filtrate was concentrated under reduced pressure to obtain Intermediate 1-11 ((2,2-difluorodihydro-1'H,3'H-spiro[cyclopropan-1,2'-pyrrolizin]-7a'(5'H)-yl)methanol ).

LC-MS: m/z 204 (M+H)⁺.

Intermediate 1-11 was separated by silica gel column chromatography (DCM: MeOH: NH₃.H₂O = 20:1:0.05) to obtain ((*trans*)-2,2-difluorodihydro-1'H,3'H-spiro[cyclopropan-1,2'-pyrrolizin]-7a'(5'H)-yl)metha nol and ((*cis*)-2,2-difluorodihydro-1'H,3'H-spiro[cyclopropan-1,2'-pyrrolizin]-7a'(5'H)-yl)methano l.

### Isomer 1-11A ((trans)-2,2-difluorodihydro-1'H,3'H-spiro[cyclopropan-1,2'-pyrrolizin]-7a'(5'H)-yl) methanol (0.50 g):

LC-MS: m/z 204 (M+H)⁺. ¹H NMR (400 MHz, CDCl₃) δ 3.44 - 3.35 (m, 2H), 3.19 - 3.13 (m, 2H), 2.83 (d, *J* = 12.1 Hz, 1H), 2.73 - 2.59 (m, 1H), 2.73 - 2.59 (m, 1H), 2.05 (dd, *J =* 6.3, 13.3 Hz, 1H), 1.93 - 1.84 (m, 3H), 1.83 - 1.74 (m, 1H), 1.74 - 1.63 (m, 1H), 1.36 (ddd, *J* = 4.0, 8.1, 12.1 Hz, 1H), 1.28 (ddd, *J* = 4.2, 8.1, 12.4 Hz, 1H).

### Isomer 1-11B ((cis)-2,2-difluorodihydro-1'H,3'H-spiro[cyclopropan-1,2'-pyrrolizin]-7a'(5'H)-yl)met hanol (0.64 g):

LC-MS: m/z 204 (M+H)⁺. ¹H NMR (400 MHz, CDCl₃) δ 3.32 (d, *J* = 2.0 Hz, 2H), 3.24 (d, *J* = 11.0 Hz, 1H), 3.12 - 3.02 (m, 1H), 2.83 - 2.71 (m, 2H), 2.05 (d, *J* = 13.2 Hz, 1H), 1.98 - 1.89 (m, 2H), 1.89 - 1.81 (m, 1H), 1.81 - 1.70 (m, 2H), 1.36 - 1.28 (m, 2H).

### Synthetic route 2 of Intermediate 1-11A ((trans)-2,2-difluorodihydro-1'H,3'H-spiro[cyclopropan-1,2'-pyrrolizin]-7a'(5'H)-yl) methanol and 1-11B ((cis)-2,2-difluorodihydro-1'H,3'H-spiro[cyclopropan-1,2'-pyrrolizin]-7a'(5'H)-yl)met hanol

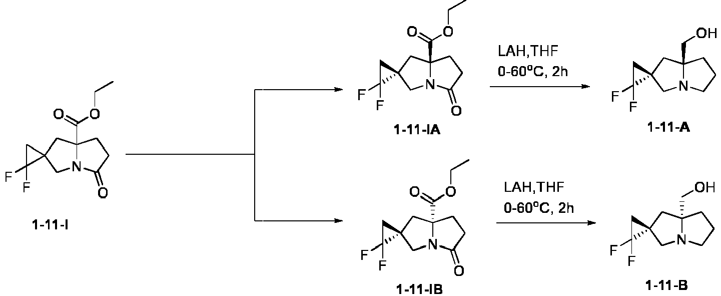

### Step 1: Preparation of ethyl (trans)-2,2-difluoro-5'-oxodihydro-1'H,3'H-spiro[cyclopropan-1,2'-pyrrolizin]-7a'(5' H)-formate and ethyl (cis)-2,2-difluoro-5'-oxodihydro-1'H,3'H-spiro[cyclopropan-1,2'-pyrrolizin]-7a'(5'H)-formate (Intermediate 1-11-IA and Intermediate 1-11-IB)

Intermediate 1-11-I (6 g) was separated by silica gel column chromatography (SiO2^{®}; 40 g SepaFlash^{®} Silica Flash Column, eluent: 0~50% THF/petroleum ether 40 mL/min) to obtain Intermediate 1-11-IA (2.80 g, 10.8 mmol, 47% yield) and Intermediate 1-11-IB (2.40 g, 9.26 mmol, 40% yield) in turn.

### Step 2: Preparation of ((trans)-2,2-difluorodihydro-1'H,3'H-spiro[cyclopropan-1,2'-pyrrolizin]-7a'(5'H)-yl) methanol and ((cis)-2,2-difluorodihydro-1'H,3'H-spiro[cyclopropan-1,2'-pyrrolizin]-7a'(5'H)-yl)met hanol (Intermediate 1-11-A and Intermediate 1-11-B)

Intermediate 1-11A was synthesized according to the same method as synthetic route 1 using Intermediate 1-11-IA.

Intermediate 1-11B was synthesized according to the same method as synthetic route 1 using Intermediate 1-11-IB.

### Isomer 1-11A was subjected to chiral resolution to obtain isomer 1-11A-1(((1S,7a'S)-2,2-difluorodihydro-1'H,3'H-spiro[cyclopropan-1,2'-pyrrolizin]-7 a'(5'H)-yl)methanol) and isomer 1-11A-2 (((1R,7a'R)-2,2-difluorodihydro-1'H,3'H-spiro [cyclopropan-1,2'-pyrrolizin]-7a'(5'H)-yl)methanol).

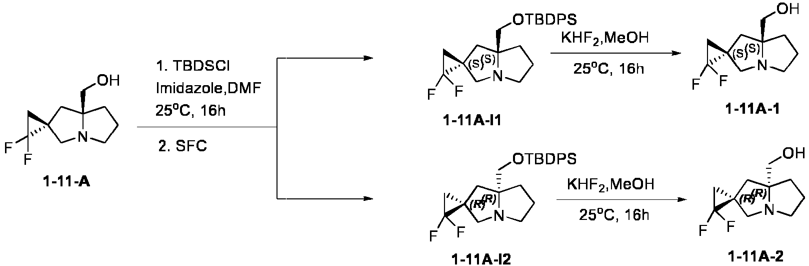

### Step 1: Preparation of Intermediate 1-11A-I1 and Intermediate 1-11A-I2

To a solution of Intermediate 1-11A (9.00 g, 44.3 mmol, 1.00 eq) in DMF (100 mL) was added imidazole (6.03 g, 88.6 mmol, 2.00 eq) and TBDPSCl (14.6 g, 53.1 mmol, 13.6 mL, 1.20 eq). The reaction solution was reacted at 25 °C for 16 h, and then concentrated under reduced pressure. The residue was subjected to silica gel column chromatography to obtain the target product Intermediate 1-11A-I (20.0 g, 41.7 mmol, 94.1% yield).

LC-MS: m/z 442 (M+H)⁺. ¹H NMR (400 MHz, CDCl₃) δ 7.77 - 7.62 (m, 4H), 7.53 - 7.34 (m, 6H), 3.51 (s, 2H), 3.14 - 3.02 (m, 2H), 2.79 (d, *J =* 12.0 Hz, 1H), 2.65 - 2.54 (m, 1H), 2.13 - 1.99 (m, 2H), 1.90 (d, *J =* 13.3 Hz, 1H), 1.86 - 1.77 (m, 1H), 1.72 - 1.61 (m, 2H), 1.23 - 1.16 (m, 2H), 1.10 (s, 9H).

Intermediate 1-11A-I was subjected to SFC chiral separation (column: DAICEL CHIRALPAK IC (250mm*50mm, 10um); mobile phase: [CO₂-EtOH (0.1%NH₃H₂O)]; 15% B isocratic elution mode) to obtain two isomers:
Intermediate 1-11A-I1 (4.50 g, 10.2 mmol, yield 45.0%): RT 1.931min. LC-MS: m/z 442 (M+H)⁺.
Intermediate 1-11A-I2 (4.20 g, 9.51 mmol, yield 42.0%): RT 2.412min. LC-MS: m/z 442 (M+H)⁺.

### Step 2: Preparation of Intermediate 1-11A-1 and Intermediate 1-11A-2

To a solution of Intermediate 1-11A-I1 (4.50 g, 10.2 mmol, 1.00 eq) in MeOH (50 mL) was added KHF₂ (15.9 g, 203 mmol, 6.72 mL, 20.0 eq). The reaction solution was reacted at 25 °C for 16 h, and then concentrated under reduced pressure. The residue was separated by silica gel column chromatography (SiO₂^{®}; 20 g SepaFlash^{®} Silica Flash Column, eluent: 0~25% MeOH/DCM@40 mL/min) to obtain the target product **1-11A-1** (1.71 g, 7.98 mmol, 78.3% yield). LC-MS: m/z 204 (M+H)⁺. ¹H NMR (400 MHz, CDCl₃) δ 3.40 - 3.16 (m, 2H), 3.03 (dd, *J* = 7.4, 12.1 Hz, 2H), 2.73 (d, *J =* 12.1 Hz, 1H), 2.66 - 2.43 (m, 1H), 2.00 - 1.86 (m, 1H), 1.84 - 1.73 (m, 3H), 1.72 - 1.54 (m, 2H), 1.33 - 1.10 (m, 2H).

### Intermediate 1-11A-2 (1.79 g, 8.38 mmol, 88.1% yield) was synthesized using the same method:

LC-MS: m/z 204 (M+H)⁺. ¹H NMR (400 MHz, CDCl₃) δ 3.35 - 3.20 (m, 2H), 3.10 - 2.96 (m, 2H), 2.73 (d, *J* = 12.1 Hz, 1H), 2.63 - 2.49 (m, 1H), 1.97 - 1.87 (m, 1H), 1.84 - 1.74 (m, 3H), 1.72 - 1.53 (m, 2H), 1.31 - 1.11 (m, 2H).

### Intermediate

### D-1-11A-1(((1S,7a'S)-2,2-difluorodihydro-1'H,3'H-spiro[cyclopropan-1,2'-pyrrolizin] -7a'(5'H)-yl-5',5'-d2)methan-d2-ol) and Intermediate D-1-11A-2(((1R,7a'R)-2,2-difluorodihydro-1'H,3'H-spiro[cyclopropan-1,2'-pyrrolizin]-7a'(5'H)-yl-5',5'-d2) methan-d2-ol) were synthesized according to the same method for Intermediate 1-11A-1 and Intermediate 1-11A-2.

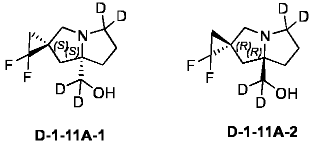

**Intermediate D-1-11A-1.** LC-MS: m/z 208 (M+H)⁺. ¹H NMR (400 MHz, CDCl₃) δ 2.81 - 3.08 (m, 2 H) 2.73 (d, *J*=12.13 Hz, 1 H) 1.92 (dd, *J*=13.26, 6.13 Hz, 1 H) 1.73 - 1.83 (m, 3 H) 1.52 - 1.71 (m, 2 H) 1.08 - 1.33 (m, 2 H).

**Intermediate D-1-11A-2.** LC-MS: m/z 208 (M+H)⁺. ¹H NMR (400 MHz, CDCl₃) δ 3.03 (dd, *J*= 12.13, 7.25 Hz, 2 H) 2.73 (d, *J*= 12.13 Hz, 1 H) 1.92 (dd, *J*= 13.26, 6.25 Hz, 1 H) 1.74 - 1.83 (m, 3 H) 1.53 - 1.70 (m, 2 H) 1.13 - 1.32 (m, 2 H).

### Preparation of Intermediate 1-12 (tetrahydrospiro[cyclopropan-1,3'-pyrrolizin]-7a'(5'H)-yl)methanol

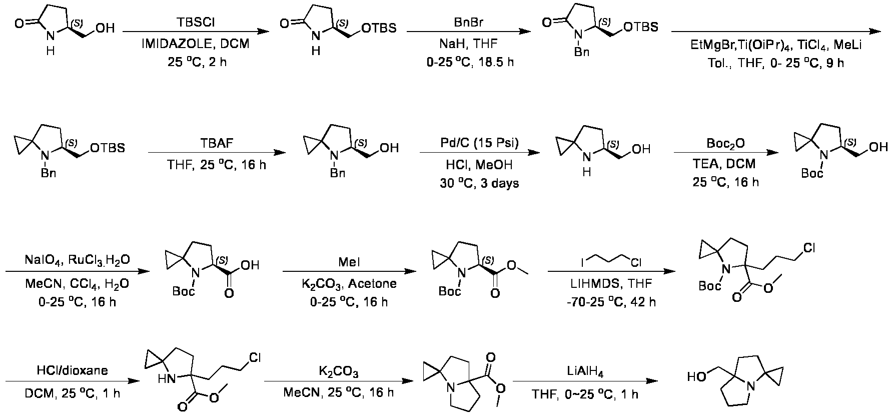

### Step 1: Preparation of (S)-5-(((tert-butylmethylsilyl)oxo)methyl)pyrrolin-2-one

To a solution of (S)-5-(hydroxymethyl)pyrrolin-2-one (50.0 g, 434 mmol, 1.00 eq) and imidazole (59.1 g, 868 mmol, 2.00 eq) in DCM (1000 mL) was added TBSCl (78.6 g, 521 mmol, 63.9 mL, 1.20 eq) in batches. The reaction solution was reacted at 25 °C for 2 h, and then added with H₂O (1000 mL) dropwise at 0 °C, and then diluted by adding DCM (1000 mL). The mixed liquid was washed with saturated salt water, then dried over anhydrous magnesium sulfate, and then filtered. The filtrate was concentrated under reduced pressure to obtain the target product, which was used directly in the next reaction without purification.

¹H NMR (400 MHz, CDCl₃) δ 6.43 (br s, 1 H) 3.71 (m, 1 H) 3.57 (m, 1 H) 3.44 (m, 1 H) 2.30 (m, 2 H) 2.13 (m, 1 H) 1.74 (m, 1 H) 0.85 (s, 9 H) 0.02 (m, 6 H).

### Step 2: Preparation of (S)-1-benzyl-5-(((tert-butylmethylsilyl)oxo)methyl)pyrrolin-2-one

Under nitrogen protection, to a solution of (S)-5-(((tert-butylmethylsilyl)oxo)methyl)pyrrolin-2-one (100 g, 436 mmol, 1.00 eq) in THF (1250 mL) was added NaH (72.7 g, 1.82 mol, 60.0%wt, 4.17 eq) in batches at 0 °C over 2 h. The mixture was reacted at 0 °C for 0.5h, and then BnBr (112 g, 654 mmol, 77.7 mL, 1.50 eq) was added. The resulting reaction solution was reacted at 25 °C for 16 h, then quenched by adding saturated NH₄Cl aqueous solution (1000 mL) at 0 to 10°C under nitrogen protection, and then extracted with EtOAc (1000 mL *2). The combined organic phase was washed with saturated salt water (1000 mL), then dried over anhydrous MgSO₄, and then filtered. The filtrate was concentrated under reduced pressure, and the residue was separated by silica gel column chromatography to obtain the target product (122 g, 382 mmol, 87.6% yield).

¹H NMR (400 MHz, CDCl₃) δ 7.29 (m, 5 H) 5.02 (d, *J*=14.97 Hz, 1 H) 4.06 (d, *J*=14.97 Hz, 1 H) 3.69 (m, 1 H) 3.54 (m, 2 H) 2.55 (m, 1 H) 2.38 (m, 1 H) 2.05 (m, 1 H) 1.91 (m, 1 H) 0.89 (s, 9 H) 0.04 (s, 6 H)

### Step 3: Preparation of (S)-4-benzyl-5-(((tert-butylmethylsilyl)oxo)methyl)-4-azaspiro[2.4]heptane

Under nitrogen protection, to a solution of TiCl₄ (49.9 g, 263 mmol, 0.750 eq) in toluene (263 mL) was added Ti(OiPr)₄ (224 g, 789 mmol, 2.25 eq) at 0 °C. The reaction solution was reacted at 25 °C for 2 h, and then added with MeLi (1.60 M, 657 mL, 3.00 eq) dropwise. The obtained compound was reacted at 25 °C for 1 h, and then added with a solution of (S)-1-benzyl-5-(((tert-butylmethylsilyl)oxo)methyl)pyrrolin-2-one (112 g, 351 mmol, 1.00 eq) in THF (560 mL), followed by the dropwise addition of EtMgBr (3.00 M, 400 mL, 3.42 eq) at 0 °C over 2 h. The resulting mixture was reacted at 25 °C for 2 h, and then the reaction was quenched by adding water (700 mL) dropwise at 0 °C and then filtered. The filter cake was washed with EtOAc (1000 mL). The combined organic phase was washed with saturated salt water (500 mL*2), then dried over anhydrous MgSO₄, and then filtered. The filtrate was concentrated under reduced pressure, and the residue was separated by silica gel column chromatography to obtain the target product (19.0 g, 57.3 mmol, 16.4% yield).

¹H NMR (400 MHz, CDCl₃) δ 7.29 (m, 5 H) 3.75 (d, *J*=13.82 Hz, 1 H) 3.54 (d, *J*=13.82 Hz, 1 H) 3.40 (m, 2 H) 3.03 (m, 1 H) 2.07 (m, 1 H) 1.95 (dt, *J=*11.80, 8.22 Hz, 1 H) 1.78 (m, 1 H) 1.55 (ddd, *J*=12.01, 8.04, 4.40 Hz, 1 H) 0.84 (s, 9 H) 0.79 (m, 1 H) 0.56 (m, 1 H) 0.48 (m, 1 H) 0.41 (m, 1 H) -0.06 (d, *J*=9.17 Hz, 6 H)

### Step 4: Preparation of (S)-(4-benzyl-4-azaspiro[2.4]heptan-5-yl)methanol

To a solution of (S)-4-benzyl-5-(((tert-butylmethylsilyl)oxo)methyl)-4-azaspiro[2.4]heptane (19.0 g, 57.3 mmol, 1.00 eq) in THF (300 mL) was added TBAF (1.00 M, 57.3 mL, 1.00 eq). The mixture was reacted at 25 °C for 16h, and then diluted by adding EtOAc (500 mL). The resulting mixture was wasded with water (100 mL) and saturated salt water (100 mL) sequentially, then dried over anhydrous MgSO₄, and then filtered. The filtrate was concentrated under reduced pressure, and the residue was separated by silica gel column chromatography to obtain the target product (5.30 g, 24.4 mmol, 42.6% yield).

¹H NMR (400 MHz, CDCl₃) δ 7.28 (m, 4 H) 7.20 (m, 1 H) 4.14 (br t, *J*=5.39 Hz, 1 H) 3.67 (d, *J*=14.09 Hz, 1 H) 3.50 (d, *J*=14.08 Hz, 1 H) 3.24 (m, 1 H) 3.14 (m, 1 H) 2.91 (m, 1 H) 1.96 (m, 2 H) 1.65 (m, 1 H) 1.49 (ddd, *J*=11.50, 8.20, 3.41 Hz, 1 H) 0.80 (m, 1 H) 0.45 (m, 2 H) 0.37 (m, 1 H).

### Step 5: Preparation of (S)-(4-azaspiro[2.4]heptan-5-yl)methanol hydrochloride

Under nitrogen protection, to a solution of (S)-(4-benzyl-4-azaspiro[2.4]heptan-5-yl)methanol (5.00 g, 23.0 mmol, 1.00 eq) in MeOH (50.0 mL) was added Pd/C (500 mg, 10.0%wt) and HCl (12.0 M, 8.00 mL, 4.17 eq). The mixture was reacted at 30 °C for 3 days under hydrogen atmosphere and then filtered. The filtrate was concentrated under reduced pressure to obtain the target product (4.50 g, crude), which was used directly in the next reaction without further purification.

¹H NMR (400 MHz, CDCl₃) δ 9.67 (br s, 1 H) 8.97 (br s, 1 H) 3.60 (m, 3 H) 2.07 (m, 1 H) 1.92 (m, 2 H) 1.79 (m, 1 H) 1.14 (m, 2 H) 0.77 (m, 2 H).

### Step 6: Preparation of tert-butyl (S)-5-(hydroxymethyl)-azaspiro[2.4]heptan-4-formate

To a solution of (S)-(4-azaspiro[2.4]heptan-5-yl)methanol hydrochloride (4.50 g, 27.5 mmol, 1.00 eq) in DCM (90.0 mL) was added TEA (3.34 g, 33.0 mmol, 4.59 mL, 1.20 eq) and Boc₂O (7.20 g, 33.0 mmol, 7.58 mL, 1.20 eq). The mixture was reacted at 25 °C for 16 h, then quenched by adding water (50 mL) at 0 °C and then extracted with DCM (50 mL*2). The combined organic phase was washed with saturated salt water (50 mL), then dried over anhydrous MgSO₄, and then filtered. The filtrate was concentrated under reduced pressure to obtain the target product (5.90 g, 26.0 mmol, 94.4% yield).

¹H NMR (400 MHz, CDCl₃) δ 3.85 (br d, *J*=5.94 Hz, 1 H) 3.52 (ddd, *J*=9.96, 5.56, 3.85 Hz, 1 H) 3.25 (ddd, *J*=10.12, 8.58, 5.94 Hz, 1 H) 2.14 (m, 1 H) 1.85 (m, 2 H) 1.50 (br s, 1 H) 1.35 (s, 9 H) 1.15 (m, 1 H) 0.84 (m, 1 H) 0.39 (m, 2H).

### Step 7: Preparation of (S)-4-(tert-butyloxycarbonyl)-4-azaspiro[2.4]heptan-5-formic acid

To a solution of NaIO₄ (11.7 g, 54.7 mmol, 3.03 mL, 2.54 eq) in H₂O (18.0 mL) was added a mixed solution of tert-butyl (S)-5-(hydroxymethyl)-azaspiro[2.4]heptan-4-formate (4.90 g, 21.6 mmol, 1.00 eq) in ACN (12.0 mL) and CCl₄ (12.0 mL) at 0 °C, followed by the addition of RuCl₃.H₂O (206 mg, 912 µmol, 0.0423 eq). The mixture was reacted at 25 °C for 16 h, then quenched by adding water (30 mL) at 0 °C and then extracted with DCM (50 mL*2). The combined organic phase was washed with saturated salt water (50 mL), then dried over anhydrous MgSO₄, and then filtered. The filtrate was concentrated under reduced pressure to obtain the target product (4.70 g, crude), which was used directly in the next reaction without further purification.

LC-MS: m/z 242 (M+H)⁺.

### Step 8: Preparation of methyl (S)-4-(tert-Butyloxycarbonyl)-4-azaspiro [2.4] heptan-5-formate

To a solution of (S)-4-(tert-butyloxycarbonyl)-4-azaspiro[2.4]heptan-5-formic acid (4.70 g, 19.5 mmol, 1.00 eq) in acetone (50.0 mL) was added Mel (5.53 g, 39.0 mmol, 2.43 mL, 2.00 eq) and K₂CO₃ (8.08 g, 58.4 mmol, 3.00 eq) at 0 °C. The mixture was reacted at 25 °C for 16 h, then quenched by adding water (30 mL) at 0 °C and then extracted with DCM (50 mL*2). The combined organic phase was washed with saturated salt water (50 mL), then dried over anhydrous MgSO₄, and then filtered. The filtrate was concentrated under reduced pressure, and the residue was separated by silica gel column chromatography to obtain the target product (1.10 g, 4.31 mmol, 22.1% yield).

¹H NMR (400 MHz, CDCl₃) δ 4.33 (br dd, *J*=8.44, 3.79 Hz, 1 H) 3.65 (m, 3 H) 2.20 (m, 1 H) 1.81 (m, 3 H) 1.39 (m, 1 H) 1.30 (br s, 9 H) 0.86 (m, 1 H) 0.48 (br s, 2 H)..

### Step 9: Preparation of 4-(tert-butyl) 5-methyl 5-(3-chloropropyl)-4-azaspiro[2.4]heptan-4,5-diformate

Under nitrogen protection, LiHMDS (1.00 M, 5.88 mL, 1.50 eq) was added to a solution of methyl (S)- 4-(tert-butyloxycarbonyl)-4-azaspiro[2.4]heptan-5- formate (1.00 g, 3.92 mmol, 1.00 eq) in THF (10.0 mL) at -70 °C. The reaction solution was reacted at -70 °C for 1 h, and then 1-chloro-3-iodo-propane (2.40 g, 11.8 mmol, 1.26 mL, 3.00 eq) was added. The resulting mixture was reacted at -70 °C for 1 h, and then reacted at 25 °C for 40 h. The mixture was quenched with saturated NH₄Cl (20 mL) at -0 °C, and then extracted with EtOAc (30 mL*2). The combined organic phase was washed with saturated salt water (50 mL), then dried over anhydrous MgSO₄, and then filtered. The filtrate was concentrated under reduced pressure, and the residue was separated by silica gel column chromatography to obtain the target product (767 mg, 2.31 mmol, 59.0% yield).

¹H NMR (400 MHz, CDCl₃) δ 3.73 (s, 3 H) 3.59 (br t, *J*=6.27 Hz, 2 H) 2.39 (m, 1 H) 2.07 (m, 4 H) 1.89 (m, 1 H) 1.74 (m, 2 H) 1.44 (m, 1 H) 1.39 (m, 9 H) 0.90 (br dd, *J*=14.97, 7.04 Hz, 1 H) 0.50 (m, 2 H).

### Step 10: Preparation of 5-methyl 5-(3-chloropropyl)-4-azaspiro[2.4]heptan-5-formate hydrochloride

To a solution of 4-(tert-butyl) 5-methyl 5-(3-chloropropyl)-4-azaspiro[2.4]heptan-4,5-diformate (700 mg, 2.11 mmol, 1.00 eq) in DCM (8.00 mL) was added HCl/dioxane (4.00 M, 4.00 mL, 7.58 eq). The mixture was reacted at 25 °C for 1 h and then concentrated under reduced pressure to afford the target product (698 mg, crude), which was used directly in the next reaction without further purification.

LC-MS: m/z 232 (M+H)⁺.

### Step 11: Preparation of methyl tetrahydrospiro[cyclopropan-1,3'-pyrrolizin]-7a'(5'H)-formate

To a solution of 5-methyl 5-(3-chloropropyl)-4-azaspiro[2.4]heptan-5-formate hydrochloride (698 mg, 2.60 mmol, 1.00 eq) in ACN (8.00 mL) was added K₂CO₃ (1.08 g, 7.81 mmol, 3.00 eq). The mixture was reacted at 25 °C for 16 h, and filtered, and the filtrate was concentrated under reduced pressure to afford the target product (412 mg, 2.11 mmol, 81.1% yield), which was used directly in the next reaction without further purification.

¹H NMR (400 MHz, CDCl₃) δ 3.74 (m, 3 H) 3.15 (dt, *J*=9.24, 4.62 Hz, 1 H) 2.73 (m, 1 H) 2.45 (m, 2 H) 2.13 (td, *J*=11.55, 7.92 Hz, 1 H) 1.94 (m, 1 H) 1.77 (m, 4 H) 1.31 (m, 2 H) 0.97 (m, 1 H) 0.66 (m, 2 H) 0.37 (m, 1 H)

### Step 12: Preparation of (tetrahydrospiro[cyclopropan-1,3'-pyrrolizin]-7a'(5'H)-yl)methanol

Under nitrogen protection, to a solution of methyl tetrahydrospiro[cyclopropan-1,3'-pyrrolizin]-7a'(5'H)-formate (412 mg, 1.78 mmol, 1.00 *eq*) in THF (4.00 mL) was added LiAlH₄ (135 mg, 3.56 mmol, 2.00 *eq*) at 0 °C. The reaction solution was reacted at 25 °C for 1 h, and then quenched by adding H₂O (0.04 mL), NaOH aqueous solution (15%wt, 0.04 mL) and H₂O (0.12 mL) sequentially. The mixture was dried over anhydrous magnesium sulfate and then filtered, and the filtrate was concentrated under reduced pressure to obtain the target product (263 mg, 1.57 mmol)

LC-MS: m/z 168 (M+H)⁺. ¹H NMR (400 MHz, CDCl₃) δ 3.40 (d, *J*=10.15 Hz, 1 H) 3.28 (d, *J*=10.15 Hz, 1 H) 2.99 (m, 1 H) 2.73 (m, 1 H) 2.19 (m, 1 H) 1.91 (m, 2 H) 1.69 (m, 4 H) 1.26 (m, 2 H) 0.86 (m, 1 H) 0.61 (dt, *J*=9.81, 5.73 Hz, 1 H) 0.50 (dt, *J*=10.18, 5.00 Hz, 1 H) 0.38 (ddd, *J*=9.90, 6.05, 4.10 Hz, 1 H).

### Intermediate 1-13A and 13B were synthesized according to the same method for Intermediate 1-12: ((cis)-6'-fluorotetrahydrospiro[cyclopropan-1,3'-pyrrolizin]-7a'(5'H)-yl)methanol and ((trans)-6'-fluorotetrahydrospiro[cyclopropan-1,3'-pyrrolizin]-7a'(5'H)-yl)methanol

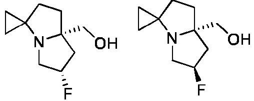

### Intermediate 1-13A

LC-MS: m/z 186 (M+H)⁺. ¹H NMR (400 MHz, CDCl₃) δ 5.26 - 5.02 (m, 1H), 3.48 (d, *J* = 10.8 Hz, 1H), 3.42 - 3.13 (m, 3H), 2.96 - 2.57 (m, 1H), 2.52 - 2.29 (m, 2H), 2.18 - 1.94 (m, 2H), 1.87 - 1.74 (m, 1H), 1.40 - 1.29 (m, 1H), 0.95 - 0.82 (m, 1H), 0.76 - 0.59 (m, 2H), 0.53 - 0.38 (m, 1H), 0.53 - 0.38 (m, 1H).

### Intermediate 1-13B

LC-MS: m/z 186 (M+H)⁺. ¹H NMR (400 MHz, CDCl₃) δ 5.30 - 5.06 (m, 1H), 3.57 - 3.41 (m, 2H), 3.33 (br dd, *J* = 12.4, 18.8 Hz, 1H), 3.07 - 2.86 (m, 1H), 2.82 - 2.42 (m, 1H), 2.40 - 2.27 (m, 1H), 2.25 - 2.12 (m, 1H), 2.11 - 1.93 (m, 2H), 1.73 (ddd, *J* = 2.0, 8.8, 12.5 Hz, 1H), 1.34 (ddd, *J* = 1.8, 7.8, 12.2 Hz, 1H), 0.92 - 0.81 (m, 1H), 0.77 - 0.57 (m, 2H), 0.44 (ddd, *J* = 3.8, 5.9, 9.6 Hz, 1H).

### Intermediate 1-14 was synthesized according to the same method for Intermediate 1-8 using different starting material: (7',7'-difluorodihydro-3'H-spiro[cyclopropan-1,2'-indolizinyl]-8a'(1'H)-yl)methanol.

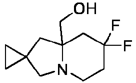

LC-MS: m/z 218 (M+H)⁺. ¹H NMR (400 MHz, CDCl₃) δ 3.66 (dd, *J*=10.88, 2.32 Hz, 1 H) 3.24 (d, *J*=10.88 Hz, 1 H) 2.97 (m, 4 H) 2.26 (d, *J*=11.62 Hz, 1 H) 1.95 (m, 6 H) 0.58 (m, 4 H).

### Intermediate 1-15 was synthesized according to the same method for Intermediate 1-8 using different starting material: (6',6'-difluorodihydro-3'H-spiro[cyclopropan-1,2'-indolizinyl]-8a'(1'H)-yl)methanol

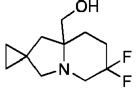

LC-MS: m/z 218 (M+H)⁺. ¹H NMR (400 MHz, CDCl₃) δ 3.68 (d, *J*=10.39 Hz, 1 H) 3.29 (m, 2 H) 2.96 (m, 4 H) 2.04 (m, 6 H) 0.55 (m, 4 H).

### Preparation of Intermediate 1-16A and 1-16B ((1S,6'R,7a'S)-2,2,6'-trifluorodihydro-1'H,3'H-spiro[cyclopropan-1,2'-pyrrolizin]-7a' (5'H)-yl)methanol and ((1R,6'R,7a'S)-2,2,6'-trifluorodihydro-1'H,3'H-spiro[cyclopropan-1,2'-pyrrolizin]-7a' (5'H)-yl)methanol

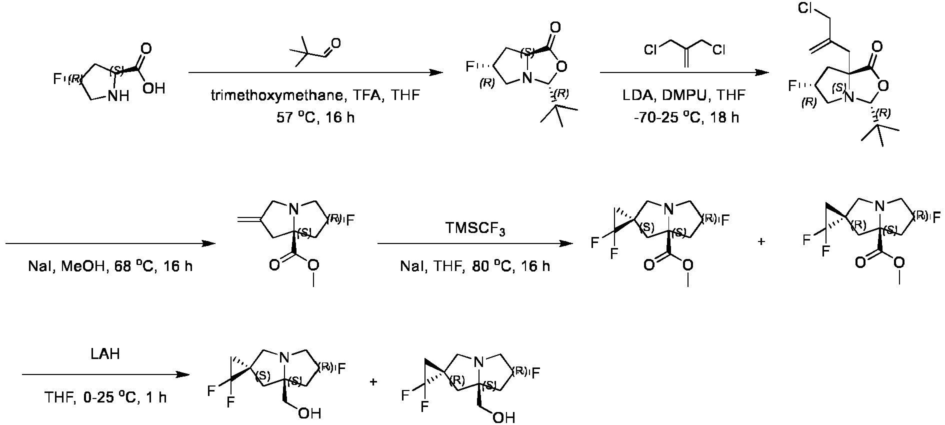

### Step 1: Preparation of (3R,6R,7aS)-3-(tert-butyl)-6-fluorotetrahydro-1H,3H-pyrrolo[1,2-c]oxazol-1-one

To a solution of (2S,4R)-4-fluoropyrrolidin-2-formic acid (13.3 g, 100 mmol, 1.00 eq) in THF (40 mL) was added 2,2-dimethylpropanal (17.3 g, 200 mmol, 22.1 mL, 2.00 eq), trimethoxymethane (31.9 g, 300 mmol, 32.9 mL, 3.00 eq) and TFA (1.14 g, 10.0 mmol, 744 µL, 0.100 eq). The mixture was reacted at 57 °C for 16 h and then concentrated under reduced pressure to afford the target product (22.0 g, crude), which was used directly in the next reaction without purification.

¹H NMR (400 MHz, CDCl₃) δ ppm 5.27 (m, 1 H) 4.52 (s, 1 H) 4.09 (t, *J*=8.14 Hz, 1 H) 3.56 (m, 1 H) 2.94 (m, 1 H) 2.54 (m, 1 H) 2.20 (m, 1 H) 0.96 (m, 9 H).

### Step 2: Preparation of (3R,6R,7aS)-3-(tert-butyl)-7a-(2-(chloromethyl)propenyl)-6-fluorotetrahydro-1H,3H-pyrrolo[1,2-c]oxazol-1-one

Under nitrogen protection, to a solution of (3R,6R,7aS)-3-(tert-butyl)-6-fluorotetrahydro-1H,3H-pyrrolo[1,2-c]oxazol-1-one (22.0 g, 109 mmol, 1.00 eq) in THF (220 mL) was added LDA (2.00 M, 82.0 mL, 1.50 eq) and DMPU (28.0 g, 219 mmol, 26.3 mL, 2.00 eq) dropwise at -70 °C. The mixture was reacted at -70 °C for 2 h, and then 3-chloro-2-(chloromethyl)prop-1-ene (17.8 g, 142 mmol, 16.5 mL, 1.30 eq) was added. The resulting mixture was reacted at 25 °C for 16 h, then quenched by adding saturated NH₄Cl aqueous solution (250 mL) dropwise at 0°C, and then extracted with EtOAc (250 mL *2). The combined organic phase was washed with saturated salt water (250 mL), then dried over anhydrous MgSO₄, and then filtered. The filtrate was concentrated under reduced pressure, and the residue was separated by silica gel column chromatography to obtain the target product (16.7 g, 57.6 mmol, 52.7% yield).

¹H NMR (400 MHz, CDCl₃) δ 5.37 (m, 1 H) 5.33 (s, 1 H) 5.24 (m, 1 H) 5.17 (s, 1 H) 4.21 (m, 3 H) 3.43 (m, 1 H) 3.23 (m, 1 H) 2.72 (s, 2 H) 2.37 (m, 1 H) 2.12 (m, 1 H) 0.91 (s, 9 H).

### Step 3: Preparation of methyl (2R,7aS)-2-fluoro-6-methylen tetrahydro-1H-pyrrolizin-7a(5H)-formate

To a solution of (3R,6R,7aS)-3-(tert-butyl)-7a-(2-(chloromethyl)propenyl)-6-fluorotetrahydro-1H,3H-pyrr olo[1,2-c]oxazol-1-one (11.2 g, 38.7 mmol, 1.00 eq) in MeOH (56 mL) was added NaI (6.95 g, 46.4 mmol, 1.20 eq). The mixture was reacted at 68 °C for 16 h, and then filtered. The filtrate was concentrated under reduced pressure. The residue was pulped with acetone (12 mL) at 25 °C for 30 min and then filtered. The solid was added with ACN (70 mL) and K₂CO₃ (12 g), and then reacted at 25 °C for 3 h and filtered. The filtrate was concentrated under reduced pressure. The residue was added with THF (70 mL) and filtered, and the filtrate was concentrated under reduced pressure to obtain the target product (3.50 g, 17.6 mmol, 45.5% yield), which was used directly in the next reaction without purification.

¹H NMR (400 MHz, CDCl₃) δ 5.29 (m, 1 H) 4.97 (dt, *J*=11.44, 1.87 Hz, 2 H) 3.82 (br d, *J*=13.86 Hz, 1 H) 3.72 (m, 3 H) 3.59 (br d, *J*=13.86 Hz, 1 H) 3.26 (m, 2 H) 3.04 (br d, *J*=16.07 Hz, 1 H) 2.81 (br d, *J*=16.07 Hz, 1 H) 2.56 (m, 1 H) 2.30 (m, 1 H).

### Step 4: Preparation of methyl (1S,6'R,7a'S)-2,2,6'-trifluorodihydro-1'H,3'H-spiro[cyclopropan-1,2'-pyrrolizin]-7a'( 5'H)-formate and methyl (1R,6'R,7a'S)-2,2,6'-trifluorodihydro-1'H,3'H-spiro[cyclopropan-1,2'-pyrrolizin]-7a'( 5'H)-formate

Under nitrogen protection, to a solution of methyl (2R, 7aS)-2-fluoro-6-methylentetrahydro-1H-pyrrolizin-7a(5H)-formate (2.00 g, 10.0 mmol, 1.00 *eq*) in THF (20 mL) was added NaI (752 mg, 5.02 mmol, 0.500 *eq*) and TMSCF₃ (3.57 g, 25.1 mmol, 2.50 *eq*)*.* The reaction solution was reacted at 80 °C for 16 h, and then concentrated under reduced pressure. The residue was subjected to silica gel column chromatography (ISCO^{®}; 20 g SepaFlash^{®} Silica Flash Column, eluent: 10.3% THF/PE @ 60 mL/min) to obtain Isomer A (269 mg, 1.08 mmol, 10.8% yield) and Isomer B (372 mg, 1.49 mmol, 14.9% yield) in turn.

**Isomer A:** ¹H NMR (400 MHz, CDCl₃) δ 5.33 (m, 1 H) 3.78 (s, 3 H) 3.49 (d, *J*=9.90 Hz, 1 H) 3.27 (m, 3 H) 2.45 (m, 4 H) 1.37 (m, 2 H).

**Isomer B:** ¹H NMR (400 MHz, CDCl₃) δ 5.26 (m, 1 H) 3.78 (m, 3 H) 3.31 (m, 4 H) 2.54 (m, 2 H) 2.35 (m, 2 H) 1.36 (m, 2 H).

### Step 5: Preparation of Intermediate 1-16A and 1-16B ((1S,6'R,7a'S)-2,2,6'-trifluorodihydro-1'H,3'H-spiro[cyclopropan-1,2'-pyrrolizin]-7a' (5'H)-yl)methanol and ((1R,6'R,7a'S)-2,2,6'-trifluorodihydro-1'H,3'H-spiro[cyclopropan-1,2'-pyrrolizin]-7a' (5'H)-yl)methanol

Under nitrogen protection, to a soluution of Isomer A obtained in the previous step in THF (3 mL) was added LAH (81.9 mg, 2.16 mmol, 2.00 eq) at 0 °C. The mixture was reacted at 25 °C for 1 h, and then added with H₂O (0.03 mL), NaOH aqueous solution (15%wt, 0.03 mL) and H₂O (0.09 mL) sequentially at 0 °C. The resulting mixture was dried over anhydrous magnesium sulfate and then filtered, and the filtrate was concentrated under reduced pressure to obtain the target product Intermediate **1-16A** (193 mg, 871 µmol, 80.7% yield), which was used directly in the next reaction without further purification.

LC-MS: m/z 222 (M+H)⁺. ¹H NMR (400 MHz, CDCl₃) δ 5.34 (br s, 1 H) 5.21 (br d, *J*=2.64 Hz, 1 H) 3.24 (m, 5 H) 2.77 (br s, 1 H) 2.19 (m, 3 H) 1.88 (d, *J*=12.98 Hz, 1 H) 1.33 (m, 2 H).

Intermediate 1-16B was synthesized according to the same method using the isomer B obtained in the previous step.

LC-MS: m/z 222 (M+H)⁺. ¹H NMR (400 MHz, CDCl₃) δ 5.28 (m, 1 H) 5.14 (m, 1 H) 3.27 (m, 5 H) 2.80 (m, 1 H) 2.20 (m, 3 H) 1.82 (br dd, *J*=13.31, 4.73 Hz, 1 H) 1.30 (m, 2 H).

**The following compounds were synthesized according to the same method for Intermediate 1-16A and 1-16B using different starting materials:**

### Intermediate 1-16C and 1-16D: ((1S,6'R,7a'R)-2,2,6'-trifluorodihydro-1'H,3'H-spiro[cyclopropan-1,2'-pyrrolizin]-7a' (5'H)-yl)methanol and ((1R,6'R,7a'R)-2,2,6'-trifluorodihydro-1'H,3'H-spiro[cyclopropan-1,2'-pyrrolizin]-7a '(5'H)-yl)methanol

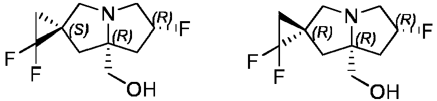

### Intermediate 1-16C

LC-MS: m/z 222 (M+H)⁺. ¹H NMR (400 MHz, CDCl₃) δ 5.31 (m, 1 H) 5.17 (m, 1 H) 3.56 (m, 1 H) 3.43 (m, 2 H) 3.21 (dd, *J*=12.53, 7.52 Hz, 1 H) 2.78 (d, *J*=12.59 Hz, 2 H) 2.10 (m, 4 H) 1.29 (s, 2 H).

### Intermediate 1-16D

LC-MS: m/z 222 (M+H)⁺. ¹H NMR (400 MHz, CDCl₃) δ5.38 (br t, *J*=4.95 Hz, 1 H) 5.24 (m, 1 H) 3.75 (m, 1 H) 3.42 (m, 2 H) 3.06 (m, 1 H) 2.68 (m, 2 H) 2.26 (m, 3 H) 1.82 (m, 1 H) 1.34 (m, 2 H).

### Intermediate 1-16E and 1-16F: ((1S,6'S,7a'S)-2,2,6'-trifluorodihydro-1'H,3'H-spiro[cyclopropan-1,2'-pyrrolizin]-7a'( 5'H)-yl)methanol and ((1R,6'S,7a'S)-2,2,6'-trifluorodihydro-1'H,3'H-spiro[cyclopropan-1,2'-pyrrolizin]-7a' (5'H)-yl)methanol

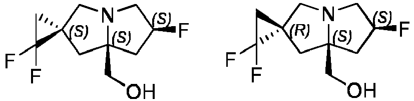

### Intermediate 1-16E

LC-MS: m/z 222 (M+H)⁺. ¹H NMR (400 MHz, CDCl₃) δ 5.31 (m, 1 H) 5.18 (t, *J*=4.29 Hz, 1 H) 3.57 (d, *J=*11.00 Hz, 1 H) 3.45 (m, 2 H) 3.21 (dd, *J*=12.54, 7.48 Hz, 1 H) 2.83 (m, 2 H) 2.10 (m, 4 H) 1.34 (m, 2 H).

### Intermediate 1-16F

LC-MS: m/z 222 (M+H)⁺. ¹H NMR (400 MHz, CDCl₃) δ 5.36 (m, 1 H) 5.22 (br d, *J*=4.40 Hz, 1 H) 3.74 (m, 1 H) 3.42 (m, 2 H) 3.03 (m, 1 H) 2.72 (m, 2 H) 2.35 (m, 1 H) 2.13 (m, 2 H) 1.78 (dd, *J*=13.53, 6.05 Hz, 1 H) 1.33 (m, 2 H).

### Intermediate 1-16G and 1-16H: ((1S,6'S,7a'R)-2,2,6'-trifluorodihydro-1'H,3'H-spiro[cyclopropan-1,2'-pyrrolizin]-7a' (5'H)-yl)methanol and ((1R,6'S,7a'R)-2,2,6'-trifluorodihydro-1'H,3'H-spiro[cyclopropan-1,2'-pyrrolizin]-7a' (5'H)-yl)methanol

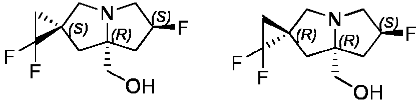

### Intermediate 1-16G

LC-MS: m/z 222 (M+H)⁺. ¹H NMR (400 MHz, CDCl₃) δ 5.35 (br s, 1 H) 5.18 (m, 1 H) 3.73 (m, 1 H) 3.28 (m, 5 H) 2.25 (m, 3 H) 1.87 (m, 1 H) 1.32 (m, 2 H).

### Intermediate 1-16H

LC-MS: m/z 222 (M+H)⁺. ¹H NMR (400 MHz, CDCl₃) δ 5.05 (m, 1 H) 3.48 (m, 1 H) 3.06 (m, 5 H) 2.03 (m, 3 H) 1.62 (m, 1 H) 1.08 (m, 2 H).

### Preparation of Intermediate 1-17 (1-methyltetrahydro-1H-spiro[cyclopenta[b]pyrrol-2,1'-cyclopropan]-3a(3H)-yl)meth anol

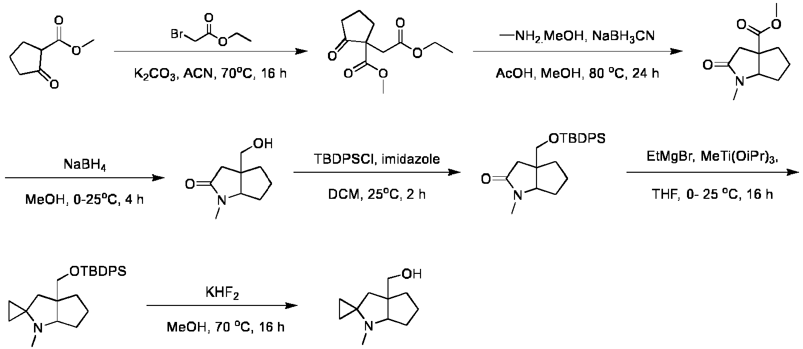

### Step 1: Preparation of methyl 1-(2-ethoxy-2-oxoethyl)-2-oxocyclopropan-1-formate

To a solution of methyl 2-oxocyclopentan-1-formate (20.0 g, 141 mmol, 17.5 mL, 1.00 eq) and ethyl 2-bromoacetate (25.9 g, 15.5 mmol, 17.1 mL, 1.10 eq) in ACN (200 mL) was added K₂CO₃ (29.2 g, 211 mmol, 1.50 eq). The reaction solution was reacted at 70°C for 16 h, and then filtered. The filtrate was concentrated under reduced pressure to afford the target product (32.0 g), which was used directly in the next reaction without further purification.

¹H NMR (400 MHz, CDCl₃) δ 4.16 - 4.06 (m, 2H), 3.71 (s, 3H), 3.03 - 2.76 (m, 2H), 2.65 - 2.53 (m, 1H), 2.51 - 2.35 (m, 1H), 2.51 - 2.35 (m, 1H), 2.17 - 1.95 (m, 1H), 2.17 - 1.95 (m, 2H), 1.26 - 1.20 (m, 1H), 1.26 - 1.20 (m, 1H), 1.24 (t, *J* = 7.2 Hz, 1H).

### Step 2: Preparation of methyl 1-methyl-2-oxohexahydrocyclopenta[b]pyrrol-3a(1H)-formate

To a solution of methyl 1-(2-ethoxy-2-oxoethyl)-2-oxocyclopropan-1-formate (5.00 g, 21.9 mmol, 1.00 eq) in MeOH (50 mL) was added methylamine in methanol (2.0 M, 16.4 mL, 1.50 eq), AcOH (1.32 g, 21.9 mmol, 1.25 mL, 1.00 eq) and NaBH₃CN (1.79 g, 28.5 mmol, 1.30 eq). The reaction solution was reacted at 80 °C for 24 h, and then concentrated under reduced pressure. The residue was washed with 3.0 M HCl (50 mL) and extracted with DCM (500 mL × 3). The combined organic phase was dried over anhydrous magnesium sulfate and then filtered. The filtrate was concentrated under reduced pressure, and the residue was separated by silica gel column chromatography to obtain the target product (700 mg, 3.02 mmol, 13.8% yield).

¹H NMR (400 MHz, CDCl₃) δ 4.15 (d, J = 5.6 Hz, 1H), 3.76 - 3.74 (m, 3H), 3.10 (d, *J* = 17.9 Hz, 1H), 2.81 (s, 3H), 2.39 (d, *J* = 17.9 Hz, 1H), 2.23 - 2.11 (m, 1H), 1.91 - 1.73 (m, 4H), 1.70 - 1.57 (m, 1H).

### Step 3: Preparation of 3a-(hydroxymethyl)-1-methylhexahydrocyclopenta[b]pyrrol-2(1H)-one

To a solution of methyl 1-methyl-2-oxohexahydrocyclopenta[b]pyrrol-3a(1H)-formate (650 mg, 3.30 mmol, 1.00 eq) in MeOH (10 mL) was added a solution of NaBH₄ (277 mg, 3.29 mmol, 2.20 eq) in MeOH (10 mL) at 0°C. The reaction solution was reacted at 25 °C for 4 h, then quenched by adding 2mL of 1M HCl, and then concentrated under reduced pressure. The residue was diluted with DCM (200mL), then dried over anhydrous magnesium sulfate and then filtered. The filtrate was concentrated under reduced pressure to obtain the target product (390 mg), which was used directly in the next reaction without further purification.

LC-MS: m/z 170 (M+H)⁺.

### Step 4: Preparation of 3a-(((tert-butylbiphenylsilyl)oxo)methyl)-1-methylhexahydrocyclopenta[b]pyrrol-2(1 H)-one

To a solution of 3a-(hydroxymethyl)-1-methylhexahydrocyclopenta[b]pyrrol-2(1H)-one (360 mg, 2.13 mmol, 1.00 eq) in DCM (5 mL) was added TBDPSCl (702 mg, 2.55 mmol, 1.20 eq) and imidazole (290 mg, 4.25 mmol, 2.00 eq) at room temperature. The reaction solution was reacted at room temperature for 2 h, and then concentrated under reduced pressure. The residue was separated by silica gel column chromatography to obtain the target product (540 mg, 1.19 mmol, 56.04% yield).

¹H NMR (400 MHz, CDCl₃) δ 7.70 - 7.59 (m, 4H), 7.52 - 7.37 (m, 6H), 3.64 - 3.46 (m, 2H), 2.80 (s, 3H), 2.61 (d, *J* = 17.5 Hz, 1H), 2.25 (d, *J* = 17.5 Hz, 1H), 2.08 - 1.84 (m, 1H), 1.83 - 1.75 (m, 1H), 1.74 - 1.65 (m, 2H), 1.65 - 1.51 (m, 3H), 1.08 (s, 9H).

### Step 5: Preparation of 3a-(((tert-butylbiphenylsilyl)oxo)methyl)-1-methylhexahydro-1H-spiro[cyclopenta[b] pyrrol-2,1'-cyclopropane]

Under nitrogen protection, to a solution of 3a-(((tert-butyldiphenylsilyl)oxo)methyl)-1-methylhexahydrocyclopenta[b]pyrrol-2(1H)-o ne (500 mg, 1.23 mmol, 1.00 eq) in THF (10 mL) was added MeTi(OiPr)₃ (1.0 M, 3.07 mL, 2.50 eq) and EtMgBr (3.0 M, 2.45 mL, 6.00 eq) at 0 °C. The reaction solution was reacted at 25°C for 16 h, then dilueted by adding water (10 mL), and then extracted with EtOAc (100 mL*2). The combined organic phase was washed with saturated salt water (50 mL), then dried over anhydrous sodium sulfate, and filtered. The filtrate was concentrated under reduced pressure, and the residue was separated by silica gel column chromatography to obtain the target product (190 mg, 385 µmol, 31.4% yield).

¹H NMR (400 MHz, CDCl₃) δ 7.68 (br t, *J* = 5.7 Hz, 4H), 7.54 - 7.35 (m, 6H), 3.73 - 3.42 (m, 2H), 2.77 - 2.50 (m, 1H), 2.12 - 1.39 (m, 11H), 1.09 (s, 9H), 0.96 - -0.27 (m, 1H), 0.96 - -0.31 (m, 1H).

### Step 6: Preparation of (1-methyltetrahydro-1H-spiro[cyclopenta[b]pyrrol-2,1'-cyclopropane]-3a(3H)-yl)met hanol

To a solution of 3a-(((tert-butyldiphenylsilyl)oxo)methyl)-1-methylhexahydro-1H-spiro[cyclopenta[b]pyrr ol-2,1'-cyclopropane] (190 mg, 453 µmol, 1.00 eq) in MeOH (5 mL) was added KHF₂ (707 mg, 9.05 mmol, 298 µL, 20.0 eq). The reaction solution was reacted at 70 °C for 16 h, and then concentrated under reduced pressure. The residue was separated by silica gel column chromatography to obtain the target product (72.6 mg, 360 µmol, 79.6% yield).

¹H NMR (400 MHz, CDCl₃) δ 3.70 - 3.44 (m, 3H), 3.70 - 3.44 (m, 1H), 2.97 - 2.70 (m, 2H), 2.21 - 1.94 (m, 3H), 1.87 (br d, *J* = 13.4 Hz, 3H), 1.73 - 1.50 (m, 3H), 1.37 (t, *J* = 7.6 Hz, 1H), 1.11 - 1.00 (m, 1H), 0.99 - 0.73 (m, 1H), 0.58 - 0.13 (m, 1H).

### Preparation of Intermediate 2-1 ((2-fluoro-6-(methoxymethoxy)-8-(4,4,5,5-tetramethyl-1,3,2-dioxaborolan-2-yl)napht halen-1-yl)ethynyl)triisopropylsilane

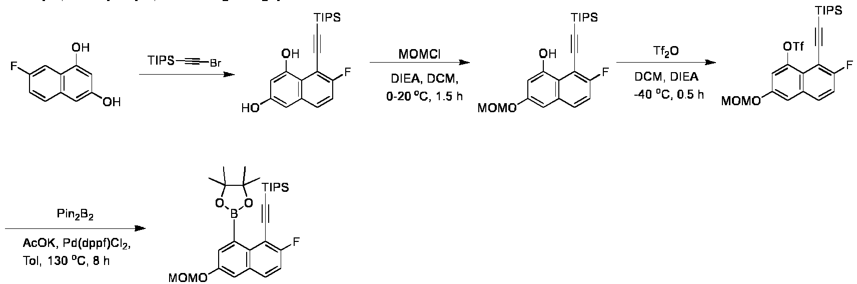

### Step 1: Preparation of 7-fluoro-8-((triisopropylsilyl)ethynyl)naphthalen-1,3-diol

Under nitrogen protection, to a solution of 7-fluoronaphthalen-1,3-diol (5 g, 28.1 mmol, 1 eq), (bromoacetylene)triisopropylsilane (7.33 g, 28.1 mmol, 1 eq), and potassium acetate (5.51 g, 56.1 mmol, 2 eq) in dioxane (120 mL) was added ruthenium dichloride 1-isopropyl-4-methyl-benzene (1.20 g, 1.96 mmol, 0.07 eq) at room temperature. The reaction solution was reacted at 110°C for 16 h and then filtered. The filter cake was washed with EtOAc (200 mL*2). The combined organic phase was concentrated under reduced pressure. The residue was separated by silica gel column chromatography to obtain the target product (8 g, 22.3 mmol, 79.5% yield).

¹H NMR (400 MHz, CDCl₃) δ 9.19 (m, 1 H) 7.60 (dd, *J*=9.17, 5.62 Hz, 1 H) 7.18 (m, 1 H) 6.73 (m, 2 H) 5.29 (br s, 1 H) 1.22 (m, 21 H)

### Step 2: Preparation of 7-fluoro-3-(methoxymethoxy)-8-((triisopropylsilyl)ethynyl)naphthalen-1-ol

To a solution of 7-fluoro-8-((triisopropylsilyl)ethynyl)naphthalen-1,3-diol (7 g, 19.5 mmol, 1 eq) and DIEA (5.05 g, 39.0 mmol, 6.80 mL, 2 eq) in DCM (100 mL) was added MOMCl (1.66 g, 20.6 mmol, 1.57 mL, 1.06 eq) at 0°C. The resulting reaction solution was reacted at 25 °C for 1.5 h, then quenched with H₂O (200 mL), then adjusted to pH 6-7 with 1N HCl, and then extracted with DCM (80.0 mL *2). The combined organic phase was washed with saturated salt water (200 mL), then dried over anhydrous sodium sulfate, and filtered. The filtrate was concentrated under reduced pressure, and the residue was separated by silica gel column chromatography to obtain the target product (4.2 g, 10.4 mmol, 53.4% yield).

¹H NMR (400 MHz, DMSO-*d*₆) δ 8.94 - 9.20 (m, 1 H) 8.93 - 9.13 (m, 1 H) 7.58 (dd, *J*=9.07, 5.69 Hz, 1 H) 7.10 (t, *J*=8.82 Hz, 1 H) 6.89 (d, J=2.50 Hz, 1 H) 6.73 (d, *J=*1.88 Hz, 1 H) 5.12 - 5.24 (m, 2 H) 3.43 (s, 3 H) 1.09 - 1.13 (m, 21 H)

### Step 3: Preparation of 7-fluoro-3-(methoxymethoxy)-8-((triisopropylsilyl)ethynyl)naphthalen-1-yltrifluoro methanesulfonate

To a solution of 7-fluoro-3-(methoxymethoxy)-8-((triisopropylsilyl)ethynyl)naphthalen-1-ol (4 g, 9.94 mmol, 1 *eq*) in DCM (40 mL) was added DIEA (3.85 g, 29.8 mmol, 5.19 mL, 3 *eq*) and Tf₂O (4.21 g, 14.9 mmol, 2.46 mL, 1.5 *eq*) at -40°C. The resulting reaction solution was reacted at -40 °C for 0.5 h, then diluted with H₂O (20 mL), and then extracted with DCM (50 mL*2). The combined organic phase was washed with saturated salt water (100 mL), then dried over anhydrous sodium sulfate, and filtered. The filtrate was concentrated under reduced pressure, and the residue was separated by silica gel column chromatography to obtain the target product as yellow oil (5 g, 8.88 mmol, 89.4% yield, 95% purity).

¹H NMR (400 MHz, CDCl₃) δ 7.72 (dd, J=9.03, 5.27 Hz, 1 H) 7.44 (d, J=2.26 Hz, 1 H) 7.36 - 7.39 (m, 1 H) 7.31 - 7.35 (m, 1 H) 5.20 - 5.37 (m, 2 H) 3.46 - 3.63 (m, 3 H) 1.17 - 1.33 (m, 17 H)

### Step 4: Preparation of ((2-fluoro-6-(methoxymethoxy)-8-(4,4,5,5-tetramethyl-1,3,2-dioxaborolan-2-yl)napht halen-1-yl)ethynyl)triisopropylsilane

Under nitrogen protection, to a solution of 7-fluoro-3-(methoxymethoxy)-8-((triisopropylsilyl)ethynyl)naphthalen-1-yl trifluoromethanesulfonate (5 g, 9.35 mmol, 1 eq), 4,4,5,5-tetramethyl-2-(4,4,5,5-tetramethyl-1,3,2-dioxaborolan-2-yl)-1,3,2-dioxaborolane (4.75 g, 18.70 mmol, 2 eq) and AcOK (2.75 g, 28.06 mmol, 3 eq) in toluene (100 mL) was added Pd(dppf)Cl₂ (684 mg, 935 umol, 0.1 eq). The resulting reaction solution was reacted at 130°C for 8 h, and then filtered. The filtrate was concentrated under reduced pressure, and the residue was separated by silica gel column chromatography to obtain the target product (3.5 g, 6.49 mmol, 69.3% yield).

¹H NMR (400 MHz, CDCl₃) δ 7.68 (dd, *J*=8.93, 5.62 Hz, 1 H) 7.52 (d, *J*=2.45 Hz, 1 H) 7.39 (d, *J*=2.57 Hz, 1 H) 7.24 (t, *J*=8.80 Hz, 1 H) 5.18 - 5.39 (m, 2 H) 3.43 - 3.58 (m, 3 H) 1.45 (s, 12 H) 1.15 - 1.21 (m, 20 H)

### Preparation of Intermediate 3-1 tert-butyl (1R,5S)-3-(2,7-dichloro-8-fluoropyridino[4,3-d]pyrimidin-4-yl)-3,8-diazabicyclo[3.2.1 ]octan-8-formate

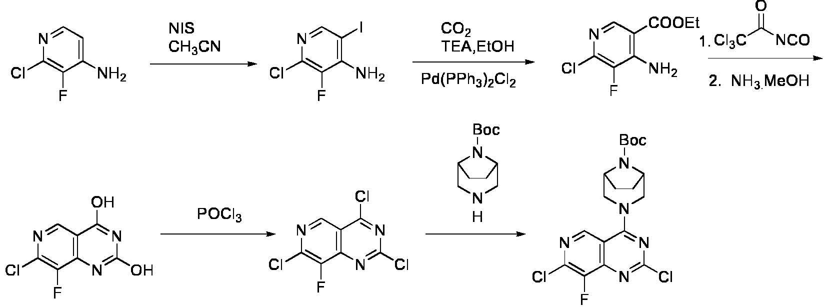

### Step 1: Preparation of 2-chloro-3-fluoro-5-iodopyridin-4-amine

To a solution of 2-chloro-3-fluoropyridin-4-amine (10 g, 68.5 mmol) and NIS (18.5 g, 82.2 mmol, 1.2 eq) in acetonitrile (50 mL) was added p-toluenesulfonic acid monohydrate (0.65 g, 3.43 mmol, 0.05 eq). The reaction solution was reacted at 70 °C for 16h, and then diluted with water (30 mL) and EtOAc (200 mL). The separated organic phase was washed with S. aq. Na₂CO₃, S. aq. Na₂SO₃ and saturated salt water sequentially, dried over anhydrous sodium sulfate and then filtered. The filtrate was concentrated under reduced pressure to obtain the target product (16.6 g, 61 mmol, yield: 89%), which was used directly in the next reaction without purification.

LC-MS: m/z 273 (M+H)⁺.

### Step 2: Preparation of ethyl 4-amino-6-chloro-5-fluoro nicotinate

Under nitrogen atmosphere, to a solution of 2-chloro-3-fluoro-5-iodopyridin-4-amine (8.2 g, 30 mmol, 1.0 eq) in EtOH (150 mL) was added Pd(PPh₃)₂Cl₂ (2.1 g, 3 mmol, 0.1 eq) and TEA (11.1 g, 0.11 mmol, 3.6 eq). The resulting reaction solution was reacted at 80 °C for 15 h under CO₂ atmosphere and then filtered. The filtrate was concentrated under reduced pressure to 70-80% of its original volume and then filtered again. The filter cakes were collected and combined and then dried in vacuum to obtain the target product (quantitative yield), which was used directly in the next reaction without purification.

LC-MS: m/z 219 (M+H)⁺.

### Step 3: Preparation of 7-chloro-8-fluoropyridino[4,3-d]pyrimidin-2,4-diol

To a solution of the above obtained ethyl 4-amino-6-chloro-5-fluoronicotinate (657 mg, 3 mmol) in THF (7 mL) was added trichloroacetyl isocyanate (673 mg, 3.6 mmol, 1.2 eq) at 0 °C. The reaction solution was reacted at RT for 30min, and then concentrated under reduced pressure. The residue was added with MeOH (15 mL), and cooled to 0°C, followed by the addition of NH₃ in methanol (7M in MeOH, 15 mL, 105 mmol). The resulting reaction solution was reacted at RT for 16 h, and then filtered. The filter cake was washed with methanol and then dried in vacuum to obtain the target product (quantitative yield), which was used directly in the next reaction without purification.

LC-MS: m/z 216 (M+H)⁺.

### Step 4: Preparation of 2,4,7-trichloro-8-fluoropyridino[4,3-d]pyrimidine

### A solution of 7-chloro-8-fluoropyridino[4,3-d]pyrimidin-2,4-diol (500 mg, 2.4 mmol) and DIPEA (1.55 g, 12 mmol, 5.0 eq) in POCl₃ (5 mL) was reacted at 100°C for 1 hour and then concentrated under reduced pressure to afford the target compound (quantitative yield), which was used directly in the next reaction without purification.

LC-MS: m/z 252 (M+H)⁺.

### Step 5: Preparation of tert-butyl (1R,5S)-3-(2,7-dichloro-8-fluoropyridino[4,3-d]pyrimidin-4-yl)-3,8-diazabicyclo[3.2.1 ]octane-8-formate

To a solution of (2,4,7-trichloro-8-fluoropyridino[4,3-d]pyrimidine obtained above and DIPEA (2 g, 15.5 mmol) in DCM (2 mL) was added tert-butyl 3,8-diazabicyclo[3.2.1]octan-8-formate (500 mg, 2.4 mmol) at -40 °C . The resulting reaction solution was reacted at the same temperature for 0.5 h, then diluted with water (2 mL), and then extracted with DCM (2 × 2 mL). The organic phase was separated and then washed with saturated salt water, dried over anhydrous sodium sulfate and then filtered. The filtrate was concentrated under reduced pressure to obtain the target product (400 mg, crude yield: 38.9%), which was used directly in the next reaction without purification.

LC-MS: m/z 428 (M+H)⁺.

### Preparation of Intermediate 4-1 tert-butyl (1R,5S)-3-(8-fluoro-7-(7-fluoro-3-(methoxymethoxy)-8-((triisopropylsilyl)ethynyl)nap hthalen-1-yl)-2-(2,2,2-trifluoroethoxy)pyridino[4,3-d]pyrimidin-4-yl)-3,8-diazabicycl o[3.2.1]octan-8-formate

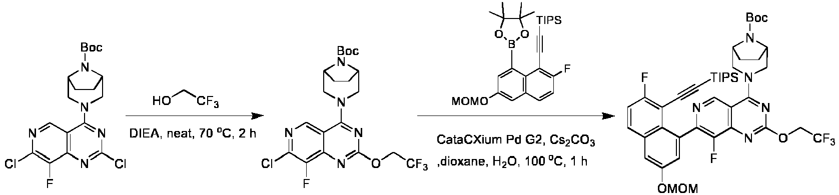

### Step 1: Preparation of tert-butyl (1R,5S)-3-(7-chloro-8-fluoro-2-(2,2,2-trifluoroethoxy)pyridino[4,3-d]pyrimidin-4-yl)-3,8-diazabicyclo[3.2.1]octan-8-formate

Tert-butyl (1R,5S)-3-(2,7-dichloro-8-fluoropyridino[4,3-d]pyrimidin-4-yl)-3,8-diazabicyclo[3.2.1]oc tan-8-formate (8.00 g, 18.7 mmol, 1.00 eq) and 2,2,2-trifluoroethanol (56.1 g, 560 mmol, 40.3 mL, 30.0 eq) were added with DIEA (7.24 g, 56.0 mmol, 9.76 mL, 3.00 eq). The resulting reaction solution was reacted at 70°C for 2 h, and then concentrated under reduced pressure. The residue was diluted with H₂O (100mL) and then extracted with EtOAc (100mL*3). The combined organic phase was washed with saturated salt water (100 mL), then dried over anhydrous magnesium sulfate, and filtered. The filtrate was concentrated under reduced pressure to obtain the target product (9 g, 18.3 mmol, 97.9% yield).

LC-MS: m/z 492 (M+H)⁺. ¹H NMR (400 MHz, *DMSO-d*₆) δ 8.71 (s, 1H), 4.82 (q, *J*=8.4 Hz, 2H), 4.44 (br d, *J*=12.0 Hz, 2H), 4.31 (br s, 2H), 3.63 (br s, 2H), 1.95 - 1.83 (m, 2H), 1.68 - 1.58 (m, 2H), 1.45 (s, 9H)

### Step 2: Preparation of tert-butyl (1R,5S)-3-(8-fluoro-7-(7-fluoro-3-(methoxymethoxy)-8-((triisopropylsilyl)ethynyl)nap hthalen-1-yl)-2-(2,2,2-trifluoroethoxy)pyridino[4,3-d]pyrimidin-4-yl)-3,8-diazabicycl o[3.2.1]octan-8-formate

Under nitrogen atmosphere, to a mixed solution of tert-butyl (1R,5S)-3-(7-chloro-8-fluoro-2-(2,2,2-trifluoroethoxy)pyridino[4,3-d]pyrimidin-4-yl)-3,8-diazabicyclo[3.2.1]octan-8-formate (9.00 g, 18.3 mmol, 1.00 *eq*), ((2-fluoro-6-(methoxymethoxy)-8-(4,4,5,5-tetramethyl-1,3,2-dioxaborolan-2-yl)naphthale n-1-yl)ethynyl)triisopropylsilane (9.85 g, 19.2 mmol, 1.05 *eq*) and Cs₂CO₃ (11.9 g, 36.6 mmol, 2.00 *eq*) in H₂O (20.0 mL) and dioxane (100 mL) was added CataCXium Pd G2 (611 mg, 915 umol, 0.05 eq). The resulting reaction solution was reacted at 100 °C for 1 h, then quenched with H₂O (50 mL), and then extracted with EtOAc (100 mL*3). The combined organic phase was dried over anhydrous magnesium sulfate and then filtered. The filtrate was concentrated under reduced pressure, and the residue was separated by silica gel column chromatography to obtain the target product (14.8 g, 17.6 mmol, 96.10% yield).

LC-MS: m/z 842 (M+H)⁺. ¹H NMR (400MHz, DMSO-*d₆*) δ 9.25 (s, 1H), 8.11 (dd, *J* = 5.9, 9.2 Hz, 1H), 7.75 (d, *J* = 2.5 Hz, 1H), 7.57 (t, *J* = 8.9 Hz, 1H), 7.35 (d, *J* = 2.4 Hz, 1H), 5.76 (s, 1H), 5.46 - 5.27 (m, 2H), 5.21 - 4.91 (m, 2H), 4.81 (d, *J* = 12.4 Hz, 1H), 4.38 - 4.17 (m, 3H), 3.81 (d, *J =* 12.0 Hz, 1H), 3.60 - 3.52 (m, 1H), 3.43 (s, 3H), 1.63 (d, *J =* 7.4 Hz, 1H), 1.46 (s, 9H), 0.80 (t, *J* = 7.8 Hz, 20H), 0.57 - 0.38 (m, 3H).

**The following compounds were synthesized according to the synthesis method for Intermediate 4-1 using different starting materials:**

### Intermediate 4-2 tert-butyl (1R,5S)-3-(7-(6-(bis(4-methoxybenzyl)amino)-4-methyl-3-(trifluoromethyl)pyridin-2-yl)-8-fluoro-2-(2,2,2-trifluoroethoxy)pyridino[4,3-d]pyrimidin-4-yl)-3,8-diazabicyclo[ 3.2.1]octan-8-formate

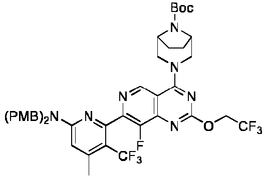

LC-MS: m/z 872 (M+H)⁺. ¹H NMR (400MHz, DMSO-*d₆*) δ 9.15 (s, 1H), 7.17 (br d, *J=* 8.4 Hz, 4H), 6.88 (br d, *J=* 8.4 Hz, 4H), 6.82 (s, 1H), 5.08 (q, *J=* 8.9 Hz, 2H), 4.69 (br s, 4H), 4.55 (br d, *J=* 12.1 Hz, 2H), 4.27 (br s, 2H), 3.73 (s, 6H), 3.70 - 3.62 (m, 2H), 2.39 (br s, 3H), 1.85 - 1.76 (m, 2H), 1.68 (br d, *J=* 7.5 Hz, 2H), 1.47 (s, 9H).

### Intermediate 4-3 tert-butyl (1R,5S)-3-(7-(2-((tert-butoxycarbonyl)amino)-7-fluorobenzo[d]thiazol-4-yl)-8-fluoro-2-(2,2,2-trifluoroethoxy)pyridino[4,3-d]pyrimidin-4-yl)-3,8-diazabicyclo[3.2.1]octan-8-formate

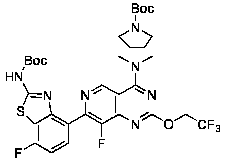

LC-MS: m/z 724 (M+H)⁺.

### Intermediate 4-4 tert-butyl (1R,5S)-3-(7-(2-((tert-butoxycarbonyl)amino)-3-cyano-7-fluorobenzo[b]thiophen-4-yl )-8-fluoro-2-(2,2,2-trifluoroethoxy)pyridino[4,3-d]pyrimidin-4-yl)-3,8-diazabicyclo[3. 2.1]octan-8-formate

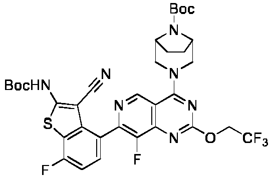

LC-MS: m/z 748 (M+H)⁺. ¹H NMR (400MHz, DMSO-*d₆*) δ 11.79 (s, 1H), 9.21 (s, 1H), 7.61 (dd, *J =* 5.3, 8.4 Hz, 1H), 7.42 (t, *J* = 8.8 Hz, 1H), 5.11 (q, *J* = 9.1 Hz, 2H), 4.57 (br d, *J* = 12.5 Hz, 2H), 4.28 (br s, 2H), 3.78 - 3.67 (m, 2H), 1.82 (br d, *J* = 4.8 Hz, 2H), 1.61 (br d, *J =* 7.5 Hz, 2H), 1.52 (s, 9H), 1.48 (s, 9H).

### Intermediate 4-5 tert-butyl (1R,SS)-3-(8-fluoro-7-(6-fluoro-5-methyl-1-(tetrahydro-2H-pyran-2-yl)-1H-indazol-4-yl)-2-(2,2,2-trifluoroethoxy)pyridino[4,3-d]pyrimidin-4-yl)-3,8-diazabicyclo[3.2.1]oct an-8-formate

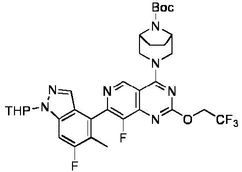

LC-MS: m/z 690 (M+H)⁺. ¹H NMR (400MHz, DMSO-*d₆*) δ 9.29 (s, 1 H) 7.75 - 7.81 (m, 2 H) 5.86 (dd, *J*=9.68, 1.98 Hz, 1 H) 5.12 (q, *J*=9.17 Hz, 2 H) 4.51 - 4.72 (m, 2 H) 4.29 (br s, 2 H) 3.91 (br d, *J*=11.88 Hz, 1 H) 3.76 - 3.84 (m, 1 H) 3.62 - 3.75 (m, 2 H) 2.32 - 2.47 (m, 1 H) 2.16 - 2.24 (m, 1 H) 2.19 (s, 2 H) 1.94 - 2.08 (m, 2 H) 1.83 (br d, *J*=3.30 Hz, 2 H) 1.70 (br d, *J*=7.48 Hz, 3 H) 1.59 (br s, 2 H) 1.48 (s, 9 H).

### Intermediate 4-6 tert-butyl (1R,5S)-3-(8-fluoro-7-(7-fluoro-8-((triisopropylsilyl)ethynyl)naphth-1-yl)-2-(2,2,2-trif luoroethoxy)pyridino[4,3-d]pyrimidin-4-yl)-3,8-diazabicyclo[3.2.1]octan-8-formate

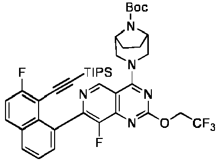

LC-MS: m/z 782 (M+H)⁺. ¹H NMR (400MHz, DMSO-*d₆*) δ 9.26 (s, 1 H) 8.24 (m, 2 H) 7.66 (m, 3 H) 5.08 (m, 2 H) 4.83 (br d, *J*=12.59 Hz, 1 H) 4.34 (m, 3 H) 3.83 (br d, *J*=11.86 Hz, 1 H) 3.41 (m, 1 H) 1.91 (br d, *J*=8.44 Hz, 2 H) 1.82 (m, 1 H) 1.65 (m, 1 H) 1.48 (s, 9 H) 0.82 (m, 18 H) 0.50 (m, 3 H).

### Intermediate 4-7 tert-butyl (1R,SS)-3-(7-(8-ethyl-7-fluoro-3-(methoxymethoxy)naphth-1-yl)-8-fluoro-2-(2,2,2-trifl uoroethoxy)pyridino[4,3-d]pyrimidin-4-yl)-3,8-diazabicyclo[3.2.1]octan-8-formate

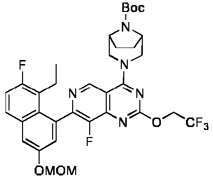

LC-MS: m/z 690 (M+H)⁺. ¹H NMR (400MHz, CDCl₃) δ 9.08 (s, 1 H) 7.71 (dd, *J =* 9.01, 5.88 Hz, 1 H) 7.55 (d, *J =* 2.63 Hz, 1 H) 7.28 (s, 1 H) 7.23 (d, *J =* 2.50 Hz, 1 H) 5.31 (d, *J* = 2.13 Hz, 2 H) 4.94 (q, *J* = 8.38 Hz, 2 H) 4.54 - 4.67 (m, 2 H) 4.44 (br s, 2 H) 3.60 - 3.90 (m, 2 H) 3.53 (s, 3 H) 2.51 (br dd, *J* = 13.95, 7.07 Hz, 1 H) 2.23 (ddd, *J* = 14.32, 7.32, 2.75 Hz, 1 H) 2.00 - 2.06 (m, 2 H) 1.81 (br d, *J =* 8.38 Hz, 2 H) 1.26 (s, 9 H) 0.85 (t, *J =* 7.44 Hz, 3 H).

### Intermediate 4-8 tert-butyl 3-(8-fluoro-7-(7-fluoro-3-(methoxymethoxy)-8-((triisopropylsilyl)ethynyl)naphthyl-1-yl)-2-(2,2,2-trifluoroethoxy)pyridino[4,3-d]pyrimidin-4-yl)-3,9-diazabicyclo[4.2.1]non an-9-formate

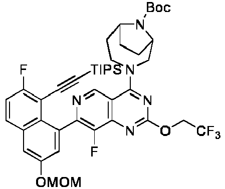

LC-MS: m/z 856 (M+H)⁺. ¹H NMR (400MHz, DMSO-*d₆*) δ 9.15 - 9.32 (m, 1 H) 8.05 - 8.19 (m, 1 H) 7.76 (br d, *J* = 2.32 Hz, 1 H) 7.50 - 7.64 (m, 1 H) 7.14 - 7.46 (m, 1 H) 5.28 - 5.43 (m, 2 H) 4.94 - 5.20 (m, 2 H) 4.08 - 4.51 (m, 4 H) 3.49 - 3.80 (m, 1 H) 3.39 - 3.48 (m, 3 H) 3.29 - 3.34 (m, 3 H) 2.01 - 2.21 (m, 2 H) 1.50 - 1.77 (m, 2 H) 1.44 (s, 3 H) 1.20 (s, 3 H) 0.94 - 1.06 (m, 3 H) 0.81 (t, *J =* 6.48 Hz, 18 H) 0.40 - 0.61 (m, 3 H).

### Intermediate 4-9 tert-butyl (1-(8-fluoro-7-(7-fluoro-3-(methoxymethoxy)-8-((triisopropylsilyl)ethynyl)naphthyl-1 -yl)-2-(2,2,2-trifluoroethoxy)pyridino[4,3-d]pyrimidin-4-yl)-3-methylpiperidin-3-yl)c arbamate

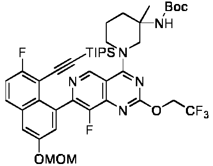

LC-MS: m/z 842 (M+H)⁺. ¹H NMR (400MHz, DMSO-*d₆*) δ 9.01 - 9.27 (m, 1 H) 8.12 (dd, *J* = 9.17, 5.87 Hz, 1 H) 7.73 - 7.80 (m, 1 H) 7.58 (t, *J* = 8.93 Hz, 1 H) 7.35 - 7.41 (m, 1 H) 7.12 - 7.20 (m, 1 H) 6.41 - 6.65 (m, 1 H) 5.32 - 5.42 (m, 2 H) 5.08 - 5.19 (m, 1 H) 4.92 - 5.05 (m, 1 H) 4.40 (br d, *J* = 12.35 Hz, 1 H) 3.63 - 3.74 (m, 1 H) 3.44 (s, 3 H) 3.35 - 3.41 (m, 1 H) 3.28 - 3.34 (m, 2 H) 2.01 - 2.14 (m, 1 H) 1.84 - 1.95 (m, 1 H) 1.50 - 1.83 (m, 2 H) 1.30 - 1.41 (m, 3 H) 1.21 - 1.27 (m, 2 H) 1.12 (s, 6 H) 0.72 - 0.88 (m, 18 H) 0.34 - 0.58 (m, 3 H)

### Intermediate 4-10 tert-butyl (1R,5S)-3-(7-(3-((bis-tert-butoxycarbonyl)amino)-2-fluoro-5-methyl-6-(trifluorometh yl)phenyl)-8-fluoro-2-(2,2,2-trifluoroethoxy)pyridino[4,3-d]pyrimidin-4-yl)-3,8-diaza bicyclo[3.2.1]octan-8-formate

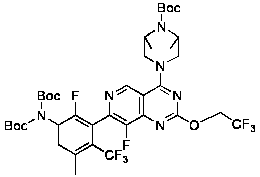

LC-MS: m/z 849 (M+H)⁺. ¹H NMR (400MHz, DMSO-*d₆*) δ 9.24 (s, 1 H) 7.82 (d, *J*=9.26 Hz, 1 H) 5.11 (q, *J*=9.01 Hz, 2 H) 4.57 (br d, *J*=12.38 Hz, 2 H) 4.29 (br s, 2 H) 3.69 (br d, *J*=12.26 Hz, 2 H) 2.28 (br s, 3 H) 1.82 (br d, *J*=4.13 Hz, 2 H) 1.66 (br d, *J*=7.50 Hz, 2 H) 1.47 (s, 9 H) 1.38 (s, 18 H).

### Intermediate 4-11 tert-butyl (1R,5S)-3-(7-(2-((bis-tert-butoxycarbonyl)amino)-3,5-dichloro-6-fluorophenyl)-8-fluo ro-2-(2,2,2-trifluoroethoxy)pyridino[4,3-d]pyrimidin-4-yl)-3,8-diazabicyclo[3.2.1]octa n-8-formate

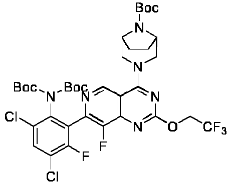

LC-MS: m/z 835 (M+H)⁺. ¹H NMR (400MHz, CDCl₃) δ 9.00 (s, 1 H) 7.68 (d, *J* = 7.09 Hz, 1 H) 4.81 - 5.01 (m, 2 H) 4.64 - 4.77 (m, 1 H) 4.34 - 4.49 (m, 3 H) 3.59 - 3.89 (m, 2 H) 2.02 (br d, *J =* 1.47 Hz, 2 H) 1.79 - 1.86 (m, 4 H) 1.78 - 1.86 (m, 1 H) 1.53 - 1.59 (m, 10 H) 1.43 - 1.51 (m, 9 H) 1.16 - 1.27 (m, 8 H).

### Intermediate 4-12 tert-butyl (1R,5S)-3-(7-(5-((bis-tert-butoxycarbonyl)amino)-3-chloro-2-(trifluoromethyl)phenyl )-8-fluoro-2-(2,2,2-trifluoroethoxy)pyridino[4,3-d]pyrimidin-4-yl)-3,8-diazabicyclo[3. 2.1]octan-8-formate

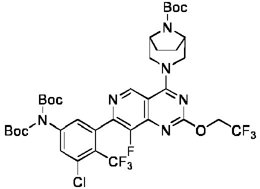

LC-MS: m/z 851 (M+H)⁺. ¹H NMR (400MHz, DMSO-*d₆*) *δ* 9.06 (br s, 1 H) 7.52 (s, 1 H) 7.20 (br s, 1 H) 4.92 (br d, *J*=7.46 Hz, 2 H) 4.44 (br s, 4 H) 3.74 (br s, 2 H) 2.02 (br s, 2 H) 1.78 (br s, 2 H) 1.55 (s, 9 H) 1.48 (s, 18 H).

### Intermediate 4-13 tert-butyl (1R,5S)-3-(7-(3-(bis(4-methoxybenzyl)amino)-2-fluoro-5-methyl-6-(trifluoromethyl)p henyl)-8-methoxy-2-(2,2,2-trifluoroethoxy)pyridino[4,3-d]pyrimidin-4-yl)-3,8-diazabi cyclo[3.2.1]octan-8-formate

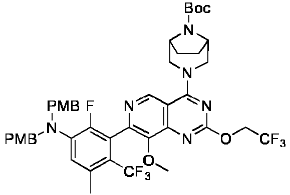

LC-MS: m/z 901 (M+H)⁺. ¹H NMR (400MHz, DMSO-*d₆*) *δ* 8.99 - 9.10 (m, 1 H) 7.26 - 7.45 (m, 1 H) 7.02 - 7.23 (m, 4 H) 6.75 - 6.96 (m, 4 H) 4.98 - 5.21 (m, 2 H) 4.47 - 4.55 (m, 2 H) 4.21 - 4.29 (m, 2 H) 4.04 - 4.13 (m, 4 H) 3.81 - 3.88 (m, 3 H) 3.71 - 3.78 (m, 6 H) 3.60 - 3.69 (m, 2 H) 2.00 - 2.12 (m, 3 H) 1.77 - 1.87 (m, 2 H) 1.62 - 1.71 (m, 2 H) 1.41 - 1.51 (m, 9 H).

### Intermediate 4-14 tert-butyl (1R,5S)-3-(7-(5-(bis(tert-butyloxycarbonyl)amino)-3-chloro-2-(trifluoromethyl)pheny l)-8-yloxy-2-(2,2,2-trifluoroethoxy)pyridino[4,3-d]pyrimidin-4-yl)-3,8-diazabicyclo[3. 2.1]octan-8-formate

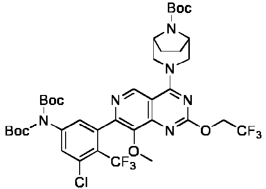

LC-MS: m/z 863 (M+H)⁺. ¹H NMR (400MHz, DMSO-*d₆*) δ 9.06 (s, 1H), 7.88 (s, 1H), 7.37 - 7.27 (m, 1H), 5.09 (br d, *J =* 9.0 Hz, 2H), 4.65 - 4.39 (m, 2H), 4.28 (br s, 2H), 3.95 - 3.87 (m, 3H), 3.76 - 3.53 (m, 2H), 1.82 (br s, 2H), 1.74 - 1.57 (m, 2H), 1.47 (s, 9H), 1.42 - 1.39 (m, 18H)

### Intermediate 4-15 tert-butyl (1R,5S)-3-(7-(3-acetylamino-5-chloro-2-fluoro-6-(trifluoromethyl)phenyl)-8-fluoro-2-(2,2,2-trifluoroethoxy)pyridino[4,3-d]pyrimidin-4-yl)-3,8-diazabicyclo[3.2.1]octan-8-f ormate

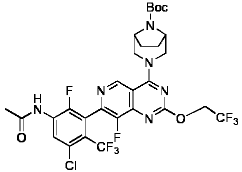

LC-MS: m/z 711 (M+H)⁺. ¹H NMR (400MHz, DMSO-*d₆*) δ 10.37 (s, 1H), 9.24 (s, 1H), 8.68 (br d, *J* = 6.9 Hz, 1H), 5.10 (q, *J* = 9.0 Hz, 2H), 4.56 (br d, *J* = 8.6 Hz, 2H), 4.29 (br s, 2H), 3.69 (br t, *J=* 12.2 Hz, 2H), 2.19 (s, 3H), 1.82 (br s, 2H), 1.71 (br d, *J =* 9.6 Hz, 2H), 1.47 (s, 9H).

### Intermediate 4-16 tert-butyl (1R,5S)-3-(7-(3-acetylamino-2,5-dichloro-6-(trifluoromethyl)phenyl)-8-fluoro-2-(2,2,2 -trifluoroethoxy)pyridino[4,3-d]pyrimidin-4-yl)-3,8-diazabicyclo[3.2.1]octan-8-forma te

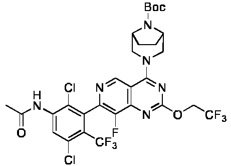

LC-MS: m/z 727 (M+H)⁺. ¹H NMR (400MHz, DMSO-*d₆*) *δ* 9.99 (s, 1 H) 9.25 (s, 1 H) 8.51 (s, 1 H) 4.98 - 5.20 (m, 2 H) 4.54 (br d, *J*=11.66 Hz, 2 H) 4.29 (br s, 2 H) 3.68 (br d, *J*=7.48 Hz, 2 H) 2.21 (s, 3 H) 1.82 (br d, *J*=3.30 Hz, 2 H) 1.70 (br d, *J*=7.92 Hz, 2 H) 1.46 (s, 9 H).

### Intermediate 4-17 tert-butyl (1R,5S)-3-(7-(3-acetylamino-2-chloro-5-fluoro-6-(trifluoromethyl)phenyl)-8-fluoro-2-(2,2,2-trifluoroethoxy)pyridino[4,3-d]pyrimidin-4-yl)-3,8-diazabicyclo[3.2. 1]octan-8-formate

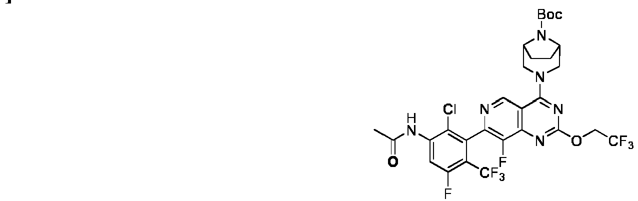

LC-MS: m/z 711 (M+H)⁺. ¹H NMR (400MHz, DMSO-*d₆*) *δ* 9.98 (s, 1 H) 9.26 (s, 1 H) 8.34 (d, *J*=13.63 Hz, 1 H) 5.11 (q, *J*=8.84 Hz, 2 H) 4.48 - 4.62 (m, 2 H) 4.22 - 4.36 (m, 2 H) 3.59 - 3.79 (m, 1 H) 3.69 (br t, *J*=12.82 Hz, 1 H) 2.18 - 2.26 (m, 3 H) 1.79 - 1.88 (m, 2 H) 1.71 (br d, *J*=7.75 Hz, 2 H) 1.42 - 1.51 (m, 9 H).

### Intermediate 4-18 tert-butyl (1R,5S)-3-(7-(3-amino-2,6-dichloro-5-fluorophenyl)-8-fluoro-2-(2,2,2-trifluoroethoxy)pyridino[4,3-d]pyrimidin-4-yl)-3,8-diazabicyclo[3.2. 1]octan-8-formate

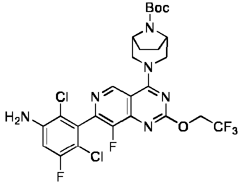

LC-MS: m/z 635 (M+H)⁺.

### Intermediate 4-19 tert-butyl (1R,5S)-3-(7-(5-acetylamino-3-chloro-2-(trifluoromethyl)phenyl)-8-fluoro-2-(2,2,2-trifluoroethoxy)pyridino[4,3-d]pyrimidin-4-yl)-3,8-diazabicyclo[3.2. 1]octan-8-formate

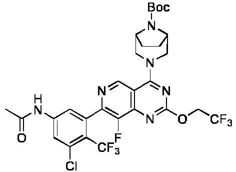

LC-MS: m/z 693 (M+H)⁺. ¹H NMR (400MHz, DMSO-*d₆*) δ 10.60 (s, 1H), 9.19 (s, 1H), 8.09 - 7.97 (m, 1H), 7.73 (s, 1H), 5.10 (q, *J* = 9.1 Hz, 2H), 4.56 (br s, 2H), 4.28 (br s, 2H), 3.76 - 3.63 (m, 2H), 2.11 (s, 3H), 1.82 (br s, 2H), 1.73 - 1.63 (m, 2H), 1.47 (s, 9H).

### Intermediate 4-20 tert-butyl (1R,5S)-3-(7-(3-chloro-5-(methylsulfonamide)-2-(trifluoromethyl)phenyl)-8-fluoro-2-(2,2,2-trifluoroethoxy)pyridino[4,3-d]pyrimidin-4-yl)-3,8-diazabicyclo[3.2. 1]octan-8-formate

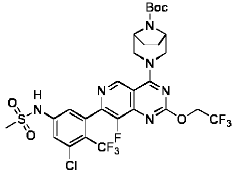

LC-MS: m/z 729 (M+H)⁺. ¹H NMR (400MHz, DMSO-*d₆*) *δ* 10.81 (s, 1 H), 9.20 (s, 1 H), 7.58 (d, *J*=1.8 Hz, 1 H), 7.22 (d, *J*=1.9 Hz, 1 H), 5.10 (q, *J*=9.0 Hz, 2 H), 4.56 (br s, 2 H), 4.28 (br s, 2 H), 3.69 (br d, *J*=12.5 Hz, 2 H), 3.23 (s, 3 H), 1.81 (br s, 2 H), 1.68 (br d, *J*=7.3 Hz, 2 H), 1.47 (s, 9 H).

### Intermediate 4-21 tert-butyl (1R,5S)-3-(7-(5-(bis-tert-butyloxyformylamino)-3-chloro-4-fluoro-2-(trifluoromethyl) phenyl)-8-fluoro-2-(2,2,2-trifluoroethoxy)pyridino[4,3-d]pyrimidin-4-yl)-3,8-diazabicyclo[3.2. 1]octan-8-formate

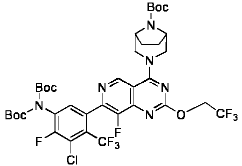

LC-MS: m/z 869 (M+H)⁺. ¹H NMR (400MHz, CDCl₃) *δ* 9.03 (br s, 1 H), 7.26 - 7.21 (m, 1 H), 4.90 (br d, *J =* 6.8 Hz, 2 H), 4.66 - 4.34 (m, 4 H), 3.85 - 3.59 (m, 2 H), 2.00 (br s, 2 H), 1.76 (br s, 2 H), 1.52 (s, 9 H), 1.45 (s, 18 H)

### Intermediate 4-22 tert-butyl (1R,5S)-3-(7-(5-(bis(4-methoxybenzyl)amino)-3-methyl-4-fluoro-2-(trifluoromethyl)p henyl)-8-fluoro-2-(2,2,2-trifluoroethoxy)pyridino[4,3-d]pyrimidin-4-yl)-3,8-diazabicyclo[3.2. 1]octan-8-formate

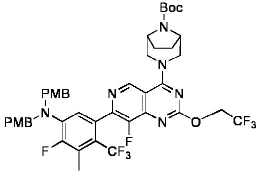

LC-MS: m/z 889 (M+H)⁺. ¹H NMR (400MHz, DMSO-*d₆*) *δ* 8.93 - 8.86 (m, 1 H), 7.10 - 7.02 (m, 4 H), 6.85 - 6.71 (m, 4 H), 6.66 - 6.53 (m, 1 H), 4.94 - 4.76 (m, 2 H), 4.57 - 4.29 (m, 4 H), 4.26 (s, 4 H), 3.72 (s, 6 H), 3.68 - 3.55 (m, 2 H), 2.47 - 2.32 (m, 3 H), 1.95 - 1.87 (m, 2 H), 1.68 (br d, *J =* 7.9 Hz, 2 H), 1.45 (s, 9 H).

### Intermediate 4-23 tert-butyl (1R,5S)-3-(7-(5-amino-3-fluoro-2-(trifluoromethyl)phenyl)-8-fluoro-2-(2,2,2-trifluoroethoxy)pyridino[4,3-d]pyrimidin-4-yl)-3,8-diazabicyclo[3.2. 1]octan-8-formate

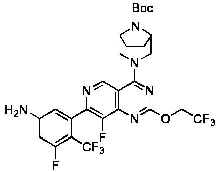

LC-MS: m/z 635 (M+H)⁺.

### Intermediate 4-24 tert-butyl (1R,5S)-3-(7-(5-amino-3-cyano-2-(trifluoromethyl)phenyl)-8-fluoro-2-(2,2,2-trifluoroethoxy)pyridino[4,3-d]pyrimidin-4-yl)-3,8-diazabicyclo[3.2. 1]octan-8-formate

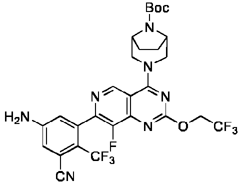

LC-MS: m/z 642 (M+H)⁺. ¹H NMR (400MHz, CDCl₃) *δ* 9.39 - 8.96 (m, 1H), 7.41 (br s, 1H), 6.91 - 6.73 (m, 1H), 6.62 (br s, 2H), 5.24 - 4.96 (m, 2H), 4.54 (br s, 2H), 4.28 (br s, 2H), 3.68 (br d, *J =* 10.4 Hz, 2H), 1.81 (br s, 2H), 1.68 (br s, 2H), 1.55 - 1.36 (m, 9H).

### Intermediate 4-25 tert-butyl (1R,5S)-3-(7-(5-amino-3-chloro-2-fluorophenyl)-8-fluoro-2-(2,2,2-trifluoroethoxy)pyridino[4,3-d]pyrimidin-4-yl)-3,8-diazabicyclo[3.2. 1]octan-8-formate

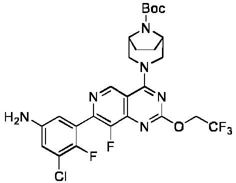

LC-MS: m/z 601 (M+H)⁺. ¹H NMR (400MHz, DMSO-*d₆*) *δ* 9.20 (s, 1H), 6.82 (m, 2H), 5.50 (s, 2H), 5.11 (q, J = 9.0 Hz, 2H), 4.57 (d, *J* = 12.5 Hz, 2H), 4.28 (s, 2H), 3.68 (d, *J =* 12.3 Hz, 2H), 1.80 (s, 2H), 1.68 (d, *J =* 7.3 Hz, 2H), 1.44 (s, 9H).

### Intermediate 4-26 tert-butyl (1R,5S)-3-(7-(5-amino-3-chloro-2-cyanophenyl)-8-fluoro-2-(2,2,2-trifluoroethoxy)pyridino[4,3-d]pyrimidin-4-yl)-3,8-diazabicyclo[3.2. 1]octan-8-formate

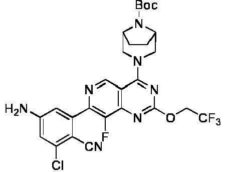

LC-MS: m/z 608 (M+H)⁺. ¹H NMR (400MHz, DMSO-*d₆*) *δ* 9.21 (s, 1H), 6.87 (d, *J* = 2.1 Hz, 1H), 6.80 (d, *J* = 1.8 Hz, 1H), 5.10 (q, *J* = 9.0 Hz, 2H), 4.56 (d, *J* = 12.1 Hz, 2H), 4.28 (s, 2H), 3.68 (d, *J =* 12.2 Hz, 2H), 1.81 (s, 2H), 1.68 (d, *J =* 7.4 Hz, 2H), 1.47 (s, 9H).

### Intermediate 4-27 tert-butyl (1R,5S)-3-(7-(5-amino-2-chloro-3-(trifluoromethyl)phenyl)-8-fluoro-2-(2,2,2-trifluoroethoxy)pyridino[4,3-d]pyrimidin-4-yl)-3,8-diazabicyclo[3.2. 1]octan-8-formate

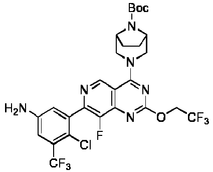

LC-MS: m/z 651 (M+H)⁺.

### Intermediate 4-28 tert-butyl (1R,5S)-3-(7-(5-amino-2-cyano-3-(trifluoromethyl)phenyl)-8-fluoro-2-(2,2,2-trifluoroethoxy)pyridino[4,3-d]pyrimidin-4-yl)-3,8-diazabicyclo[3.2. 1]octan-8-formate

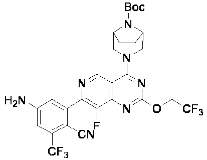

LC-MS: m/z 642 (M+H)⁺.

### Intermediate 4-29 tert-butyl (1R,5S)-3-(7-(5-amino-2-chloro-3-cyanophenyl)-8-fluoro-2-(2,2,2-trifluoroethoxy)pyridino[4,3-d]pyrimidin-4-yl)-3,8-diazabicyclo[3.2. 1]octan-8-formate

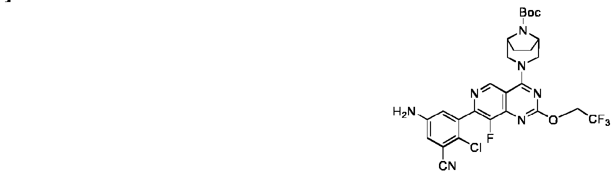

LC-MS: m/z 608 (M+H)⁺. ¹H NMR (400MHz, DMSO-*d₆*) *δ* 9.21 (s,1H), 7.14 (d,*J* = 2.7 Hz,1H), 7.02 (d, *J* = 2.7 Hz, 1H), 5.10 (dt, *J =* 9.1, 7.0 Hz,2H), 4.56 (s, 2H), 4.29 (s, 2H), 3.68 (d, *J =* 12.2 Hz,2H), 1.81 (s, 2H), 1.69 (d, *J =* 7.5 Hz, 2H), 1.48 (s, 11H).

### Intermediate 4-30 tert-butyl (1R,5S)-3-(7-(5-amino-2,3-dichlorophenyl)-8-fluoro-2-(2,2,2-trifluoroethoxy)pyridino[4,3-d]pyrimidin-4-yl)-3,8-diazabicyclo[3.2. 1]octan-8-formate

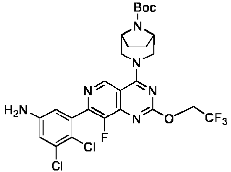

LC-MS: m/z 617 (M+H)⁺.

### Intermediate 4-31 tert-butyl (1R,5S)-3-(7-(3-amino-6-chloro-2-cyanophenyl)-8-fluoro-2-(2,2,2-trifluoroethoxy)pyridino[4,3-d]pyrimidin-4-yl)-3,8-diazabicyclo[3.2. 1]octan-8-formate

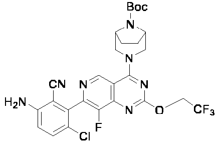

LC-MS: m/z 608 (M+H)⁺. ¹H NMR (400MHz, DMSO-*d₆*) *δ* 9.27 (s, 1H), 7.53 (d, *J* = 9.1 Hz, 1H), 6.97 (d, *J* = 9.1 Hz, 1H), 6.46 (s, 2H), 5.10 (q, *J* = 9.0 Hz, 2H), 4.64 (d, *J* = 12.3 Hz, 1H), 4.47 (d, *J* = 11.9 Hz, 1H), 4.28 (s, 2H), 3.76 (d, *J* = 11.4 Hz, 1H), 3.59 (s, 1H), 1.83 - 1.64 (m, 4H), 1.47 (s, 8H).

### Intermediate 4-32 tert-butyl (1R,5S)-3-(7-(5-(benzyloxy)-3-chloro-2-(trifluoromethyl)phenyl)-8-fluoro-2-(2,2,2-trifluoroethoxy)pyridino[4,3-d]pyrimidin-4-yl)-3,8-diazabicyclo[3.2. 1]octan-8-formate

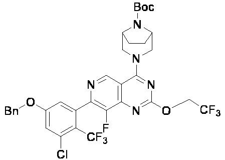

LC-MS: m/z 742 (M+H)⁺.

### Intermediate 4-33 tert-butyl (1R,5S)-3-(7-(5-amino-2-(trifluoromethyl)pyridin-3-yl)-8-fluoro-2-(2,2,2-trifluoroethoxy)pyridino[4,3-d]pyrimidin-4-yl)-3,8-diazabicyclo[3.2. 1]octan-8-formate

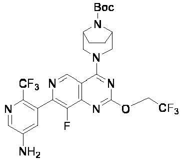

LC-MS: m/z 618 (M+H)⁺. ¹H NMR (400MHz, DMSO-*d₆*) *δ* 9.18 (s, 1 H) 8.12 (d, *J*=2.20 Hz, 1 H) 6.87 (s, 1 H) 6.32 (br d, *J*=4.18 Hz, 2 H) 5.09 (q, *J*=9.17 Hz, 2 H) 4.55 (br d, *J*=12.10 Hz, 2 H) 4.27 (br s, 2 H) 3.67 (br d, *J*=11.44 Hz, 2 H) 1.93 (br s, 2 H) 1.80 (br s, 2 H) 1.35 (s, 9 H).

### Intermediate 4-34 tert-butyl (1R,5S)-3-(7-(5-amino-4-(trifluoromethyl)pyridin-3-yl)-8-fluoro-2-(2,2,2-trifluoroethoxy)pyridino[4,3-d]pyrimidin-4-yl)-3,8-diazabicyclo[3.2. 1]octan-8-formate

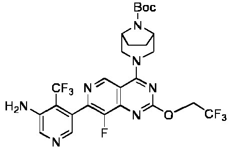

LC-MS: m/z 618 (M+H)⁺. ¹H NMR (400MHz, DMSO-*d₆*) *δ* 9.18 (s, 1 H), 8.38 (s, 1 H), 7.73 (s, 1 H), 6.26 (s, 2 H), 5.09 (q, *J*=9.0 Hz, 2 H), 4.55 (br d, *J*=11.6 Hz, 2 H), 4.28 (br s, 2 H), 3.67 (br d, *J*=10.3 Hz, 2 H), 1.82 (br s, 2 H), 1.67 (br d, *J*=7.5 Hz, 2 H), 1.47 (s, 9 H).

### Intermediate 4-35 tert-butyl (1R,5S)-3-(7-(5-amino-4-methyl-2-(trifluoromethyl)pyridin-3-yl)-8-fluoro-2-(2,2,2-trifluoroethoxy)pyridino [4,3-d] pyrimidin-4-yl)-3,8-diazabicyclo [3.2. 1]octan-8-formate

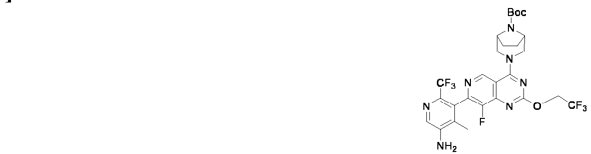

LC-MS: m/z 632 (M+H)⁺. ¹H NMR (400MHz, DMSO-*d₆*) *δ* 9.23 (s, 1 H) 8.12 (s, 1 H) 6.02 - 6.16 (m, 2 H) 5.00 - 5.21 (m, 2 H) 4.55 (br t, *J* = 14.42 Hz, 2 H) 4.30 (br s, 2 H) 3.60 - 3.80 (m, 2 H) 2.40 - 2.48 (m, 3 H) 1.83 (br d, *J=* 1.76 Hz, 2 H) 1.70 (br d, *J =* 7.92 Hz, 2 H) 1.47 (s, 9 H).

### Intermediate 4-36 tert-butyl (1R,5S)-3-(7-(6-(bis(4-methoxybenzyl)amino)-2-methyl-3-(trifluoromethyl)pyridin-4-yl)-8-fluoro-2-(2,2,2-trifluoroethoxy)pyridino[4,3-d]pyrimidin-4-yl)-3,8-diazabicyclo[3.2. 1]octan-8-formate

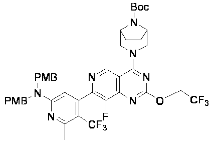

LC-MS: m/z 872 (M+H)⁺. ¹H NMR (400MHz, DMSO-*d₆*) *δ* 9.12 (s, 1H), 7.26 - 7.11 (m, 4H), 6.89 (d, *J* = 8.4 Hz, 4H), 6.50 (s, 1H), 5.07 (q, *J* = 9.0 Hz, 2H), 4.85 - 4.68 (m, 4H), 4.59 - 4.43 (m, 2H), 4.32 - 4.21 (m, 2H), 3.73 (s, 6H), 3.70 - 3.60 (m, 2H), 2.60 (s, 3H), 1.85 - 1.76 (m, 2H), 1.69 - 1.60 (m, 2H), 1.46 (s, 9H).

### Intermediate 4-37 tert-butyl (1R,SS)-3-(8-fluoro-7-(1-(tetrahydro-2H-pyran-2-yl)-5-(trifluoromethyl)-1H-indazol-4-yl)-2-(2,2,2-trifluoroethoxy)pyridino[4,3-d]pyrimidin-4-yl)-3,8-diazabicyclo[3.2.1]o ctan-8-formate

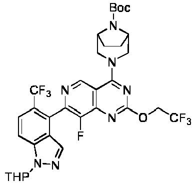

LC-MS: m/z 726 (M+H)⁺. ¹H NMR (400MHz, DMSO-*d₆*) *δ* 9.27 (d, *J =* 5.0 Hz, 1H), 8.12 (d, *J* = 8.9 Hz, 1H), 7.91 (d, *J* = 8.2 Hz, 2H), 6.09-5.93 (m, 1H), 5.21-5.02 (m, 2H), 4.59 (t, *J* = 12.0 Hz, 2H), 4.45-4.18 (m, 2H), 3.92 (d, *J* = 7.8 Hz, 1H), 3.85-3.54 (m, 3H), 2.38 (dd, *J* = 22.2, 12.9 Hz, 1H), 2.03 (d, *J* = 6.9 Hz, 2H), 1.94-1.66 (m, 5H), 1.61 (s, 2H), 1.47 (s, 9H).

### Example 1 Preparation of 4-(4-((1R,5S)-3,8-diazabicyclo[3.2.1]octan-3-yl)-8-fluoro-2-((3-(methoxymethyl)tetrah ydro-1H-pyrrolizin-7a(5H)-yl)methoxy)pyridino[4,3-d]pyrimidin-7-yl)-5-ethynyl-6-fl uoronaphthalen-2-ol

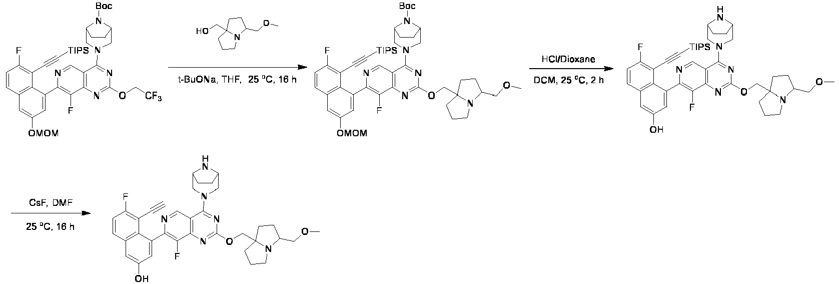

### Step 1: Preparation of Tert-butyl (1R,5S)-3-(8-fluoro-7-(7-fluoro-3-(methoxymethoxy)-8-((triisopropylsilyl)ethynyl)nap hthalen-1-yl)-2-((3-(methoxymethyl)tetrahydro-1H-pyrrolizin-7a(5H)-yl)methoxy)py ridino[4,3-d]pyrimidin-4-yl)-3,8-diazabicyclo[3.2.1]octan-8-formate

To a solution of (3-(methoxymethyl)tetrahydro-1H-pyrrolizin-7a(5H)-yl)methanol (1.01 g, 5.45 mmol, 4.59 *eq)* and tert-butyl (1R,5S)-3-(8-fluoro-7-(7-fluoro-3-(methoxymethoxy)-8-((triisopropylsilyl)ethynyl)naphth alen-1-yl)-2-(2,2,2-trifluoroethoxy)pyridino[4,3-d]pyrimidin-4-yl)-3,8-diazabicyclo[3.2.1] octan-8-formate (1.00 g, 1.19 mmol, 1.00 *eq*) in THF (20 mL) was added t-BuONa (913 mg, 9.50 mmol, 8.00 *eq*)*.* The reaction solution was reacted at 25°C for 16 h, then quenched with H₂O (10 mL), and then extracted with EtOAc (30 mL*3). The combined organic phase was washed with saturated salt water (100 mL), then dried over anhydrous magnesium sulfate, and filtered. The filtrate was concentrated under reduced pressure, and the residue was separated by silica gel column chromatography to obtain the target product (723 mg, 257 umol, 21.7% yield, 33.0 % purity), which was used directly in the next reaction without purification.

### Step 2: Preparation of 4-(4-((1R,5S)-3,8--diazabicyclo[3.2.1]octan-3-yl)-8-fluoro-2-((3-(methoxymethyl)tetra hydro-1H-pyrrolizin-7a(5H)-yl)methoxymethyl) pyridino[4,3-d] pyrimidin-7-yl)-6-fluoro-5-((triisopropylsilyl)ethynyl)naphthalen-2-ol

To a solution of tert-butyl (1R,5S)-3-(8-fluoro-7-(7-fluoro-3-(methoxymethoxy)-8-((triisopropylsilyl)ethynyl)naphth alen-1-yl)-2-((3-(methoxymethyl)tetrahydro-1H-pyrrolizin-7a(5H)-yl)methoxy)pyridino[4 ,3-d]pyrimidin-4-yl)-3,8-diazabicyclo[3.2.1]octan-8-formate (670 mg, 238 µmol, 33.0 % purity, 1.00 *eq*) in DCM (6 mL) was added HCl/dioxane (4.00 M, 945 µL, 15.8 *eq*)*.* The reaction solution was reacted at 25°C for 2 h and then concentrated under reduced pressure to afford the target product (430 mg, crude), which was used directly in the next reaction without purification.

### Step 3: Preparation of 4-(4-((1R,SS)-3,8-diazabicyclo[3.2.1]octan-3-yl)-8-fluoro-2-((3-(methoxymethyl)tetrah ydro-1H-pyrrolizin-7a(5H)-yl)methoxy)pyridino[4,3-d]pyrimidin-7-yl)-5-ethynyl-6-fl uoronaphthalen-2-ol

To a solution of 4-(4-((1R,SS)-3,8-diazabicyclo[3.2.1] octan-3-yl)-8-fluoro-2-((3-(methoxymethyl)tetrahydro-1H-pyrrolizin-7a(5H)-yl)methoxy methyl) pyridino[4,3-d] pyrimidin-7-yl)-6-fluoro-5-((triisopropylsilyl)ethynyl)naphthalen-2-ol (400 mg, 163 µmol, 32.0% purity, 1.00 *eq*) in DMF (6 mL) was added CsF (497 mg, 3.27 mmol, 121 uL, 20.0 *eq*)*.* The reaction solution was reacted at 25°C for 16 h, and then filtered. The filtrate was concentrated under reduced pressure, and the residue was separated by prep-HPLC to obtain the target product (63.0 mg).

LC-MS: m/z 627 (M+H)⁺.

**Example 1 Isomer 1** (11.3 mg, 17.6 umol, 10.8%yield) and **Example 1 Isomer 2** (19.2 mg, 29.9 umol, 18.3% yield) were obtained by chiral SFC separation.

### Example 1 Isomer 1

LC-MS: m/z 627 (M+H)⁺. ¹H NMR (400 MHz, DMSO-*d₆*) *δ* 10.17 (br s, 1 H) 9.03 (s, 1 H) 7.98 (dd, *J*=9.16, 5.90 Hz, 1 H) 7.47 (t, J=8.91 Hz, 1 H) 7.39 (d, *J*=2.51 Hz, 1 H) 7.18 (d, *J*=2.26 Hz, 1 H) 4.48 (br d, *J*=11.80 Hz, 1 H) 4.31 (br d, *J*=12.30 Hz, 1 H) 3.95 - 4.08 (m, 2 H) 3.94 (s, 1 H) 3.64 (br d, *J*=12.05 Hz, 1 H) 3.57 (br s, 3 H) 3.17 - 3.28 (m, 5 H) 2.79 - 2.94 (m, 2 H) 2.65 - 2.70 (m, 1 H) 1.90 - 2.06 (m, 2 H) 1.78 (br d, *J*=4.52 Hz, 3 H) 1.51 - 1.69 (m, 8 H).

### Example 1 Isomer 2

LC-MS: m/z 627 (M+H)⁺. ¹H NMR (400 MHz, DMSO-*d₆*) *δ*10.17 (br s, 1 H) 9.04 (s, 1 H) 7.98 (dd, *J*=9.16, 5.90 Hz, 1 H) 7.47 (t, *J*=9.03 Hz, 1 H) 7.40 (d, *J*=2.51 Hz, 1 H) 7.18 (d, *J*=2.26 Hz, 1 H) 4.49 (br d, *J*=12.05 Hz, 1 H) 4.32 (br d, *J*=12.30 Hz, 1 H) 3.96 - 4.10 (m, 2 H) 3.94 (s, 1 H) 3.65 (br d, *J*=14.31 Hz, 1 H) 3.56 - 3.62 (br s, 3 H) 3.17 - 3.28 (m, 5 H) 2.80 - 2.95 (m, 2 H) 2.68 (br d, *J*=1.76 Hz, 1 H) 1.90 - 2.03 (m, 2 H) 1.78 (br s, 3 H) 1.51 - 1.72 (m, 8 H).

### Example 1 Isomer 1 and Example 1 Isomer 2 were separated again by SFC to obtain Example 1 Isomer 1A, Example 1 Isomer 1B, Example 1 Isomer 2A and Example 1 Isomer 2B: 4-(4-((1R,5S)-3,8-diazabicyclo[3.2.1]octan-3-yl)-8-fluoro-2-((3S,7aR)-(3-(methoxymet hyl)tetrahydro-1H-pyrrolizin-7a(5H)-yl)methoxy)pyridino[4,3-d]pyrimidin-7-yl)-5-et hynyl-6-fluoronaphthalen-2-ol, 4-(4-((1R,5S)-3,8-diazabicyclo[3.2.1]octan-3-yl)-8-fluoro-2-((3R,7aS)-(3-(methoxymet hyl)tetrahydro-1H-pyrrolizin-7a(5H)-yl)methoxy)pyridino[4,3-d]pyrimidin-7-yl)-5-et hynyl-6-fluoronaphthalen-2-ol, 4-(4-((1R,5S)-3,8-diazabicyclo[3.2.1]octan-3-yl)-8-fluoro-2-((3R,7aR)-(3-(methoxymet hyl)tetrahydro-1H-pyrrolizin-7a(5H)-yl)methoxy)pyridino[4,3-d]pyrimidin-7-yl)-5-et hynyl-6-fluoronaphthalen-2-ol, 4-(4-((1R,SS)-3,8-diazabicyclo[3.2.1]octan-3-yl)-8-fluoro-2-((3S,7aS)-(3-(methoxymet hyl)tetrahydro-1H-pyrrolizin-7a(SH)-yl)methoxy)pyridino [4,3-d] pyrimidin-7-yl)-5-et hynyl-6-fluoronaphthalen-2-ol

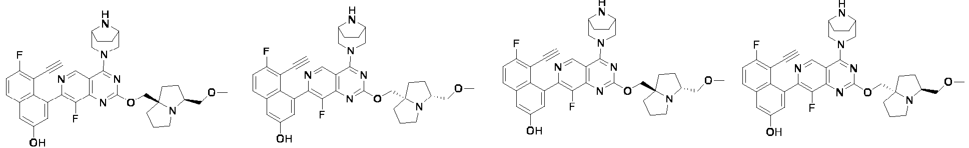

### Example 1 Isomer 1A

LC-MS: m/z 627 (M+H)⁺. ¹H NMR (400 MHz, DMSO-*d*₆) *δ* 10.14 ( s, 1 H), 9.03 (s, 1 H), 7.98 (dd, *J*=9.20, 5.92 Hz, 1 H), 7.46 (t, *J*=9.00 Hz, 1 H), 7.39 (d, *J*=2.64 Hz, 1 H), 7.17 (d, *J*=2.56 Hz, 1 H), 4.47 (d, *J*=12.44 Hz, 1 H), 4.30 (d, *J*=12.32 Hz, 1 H), 3.97 - 4.07 (m, 2 H), 3.92 (s, 1 H), 3.56-3.66 (m, 4 H), 3.17 - 3.27 (m, 5 H), 2.79 - 2.92 (m, 2 H), 2.63 - 2.69 (m, 1 H) , 1.51 - 2.03 (m, 13 H).

### Example 1 Isomer 1B

LC-MS: m/z 627 (M+H)⁺. ¹H NMR (400 MHz, DMSO-*d₆*) *δ* 10.14 ( s, 1 H) , 9.03 (s, 1 H), 7.98 (dd, *J*=9.24, 5.92 Hz, 1 H), 7.46 (t, *J*=9.00 Hz, 1 H), 7.39 (d, *J*=2.56 Hz, 1 H), 7.17 (d, *J*=2.60 Hz, 1 H), 4.47 (d, *J*=12.32Hz, 1 H), 4.30 (d, *J*=12.24 Hz, 1 H), 3.97 - 4.07 (m, 2 H), 3.92 (s, 1 H), 3.56-3.66 (m, 4 H), 3.17 - 3.27 (m, 5 H), 2.80 - 2.92 (m, 2 H), 2.63 - 2.68 (m, 1 H), 1.51 - 2.04 (m, 13 H).

### Example 1 Isomer 2A

LC-MS: m/z 627 (M+H)⁺. ¹H NMR (400 MHz, DMSO-*d₆*) *δ* 10.15(s, 1 H), 9.03(s, 1 H), 7.99-7.95 (m, 1H), 7.46(t, *J* = 8 Hz, 1H), 7.39(s, 1 H), 7.18(s, 1H), 4.47 (d, *J* = 8Hz, 1H), 4.30(d, *J* = 8Hz, 1H), 4.11(d, *J* = 4Hz, 1H), 4.01(d, *J* = 4Hz, 1H),3.93(s, 1H), 3.63 (d, *J* = 8 Hz, 1H), 3.57-3.53 (m, 3H), 3.44-3.40 (m, 1H),3.25 (s, 3H), 3.19 (s, 2H), 2.72-2.64(m, 2H), 2.05-1.97(m, 2H), 1.71-1.56(m, 9H), 1.52-1.44(m, 1H).

### Example 1 Isomer 2B

LC-MS: m/z 627 (M+H)⁺. ¹H NMR (400 MHz, DMSO-*d₆*) *δ*10.15(s, 1 H), 9.04(s, 1 H), 7.96 (m, 1H), 7.46 (t, *J* = 8 Hz, 1H), 7.39(m, 1 H), 7.18 (m, 1H), 4.65-4.56 (m, 1H), 4.48-4.39(m, 1H), 4.30(d, *J* = 8Hz, 1H), 4.15-4.06(m, 1H),4.02(t, *J* = 4 Hz,1H), 3.93(s,1H),3.85-3.75(m,1H), 3.63(d, *J* = 4 Hz,1H),3.58-3.54 (m, 3H), 3.44-3.40 (m, 2H),3.25 (s, 3H), 3.18 (s, 2H), 2.72-2.66(m, 2H),2.07-2.02(m, 3H),1.71-1.57(m, 7H), 1.51-1.44 (m,1H).

**The following examples were synthesized according to the method of Example 1 using different starting materials:**

### Example 2 Preparation of 4-(4-((1R,5S)-3,8-diazabicyclo[3.2.1]octan-3-yl)-8-fluoro-2-((3-((ethoxy)methyl)tetrah ydro-1H-pyrrolizin-7a(5H)-yl)methoxy)pyridino[4,3-d]pyrimidin-7-yl)-5-ethynyl-6-fl uoronaphthalen-2-ol

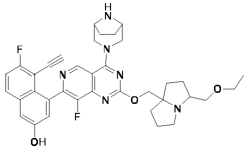

LCMS: m/z 641 (M+H)⁺.

### Example 3 Preparation of 4-(4-((1R,5S)-3,8-diazabicyclo[3.2.1]octan-3-yl)-8-fluoro-2-((3-((isopropyloxy)methyl) tetrahydro-1H-pyrrolizin-7a(5H)-yl)methoxy)pyridino [4,3-d] pyrimidin-7-yl)-5-ethyn yl-6-fluoronaphthalen-2-ol

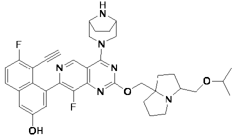

LCMS: m/z 655 (M+H)⁺.

### Example 4 Preparation of 4-(4-((1R,5S)-3,8-diazabicyclo[3.2.1]octan-3-yl)-8-fluoro-2-((3-((cyclopropylmethylox y)methyl)tetrahydro-1H-pyrrolizin-7a(5H)-yl)methoxy)pyridino[4,3-d]pyrimidin-7-yl )-5-ethynyl-6-fluoronaphthalen-2-ol

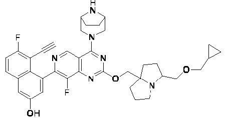

LCMS: m/z 667 (M+H)⁺.

### Example 5 4-(4-((1R,5S)-3,8-diazabicyclo[3.2.1]octan-3-yl)-2-((3-(cyclopropoxymethyl) tetrahydro-1H-pyrrolizin-7a(5H)-yl)methoxy)-8-fluoropyridino[4,3-d]pyrimidin-7-yl)-5-et hynyl-6-fluoronaphthalen-2-ol

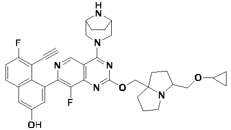

LCMS: m/z 653 (M+H)⁺.

### Example 6 4-(4-((1R,5S)-3,8-diazabicyclo [3.2.1] octan-3-yl)-8-fluoro-2-(((2R)-2-fluoro-5-(methoxymet hyl)tetrahydro-1H-pyrrolizin-7a(5H)-yl)methoxy)pyridino[4,3-d]pyrimidin-7-yl)-5-ethyny l-6-fluoronaphthalen-2-ol

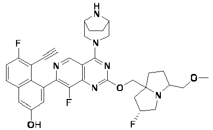

LCMS: m/z 645 (M+H)⁺.

Isomer 6A and isomer 6B were obtained by chiral separation: ***cis*-4-(4-((1R,5S)-3,8-diazabicyclo[3.2.1]octan-3-yl)-8-fluoro-2-(((2R)-2-fluoro-5-(methoxy methyl)tetrahydro-1H-pyrrolizin-7a(5H)-yl)methoxy)pyridino[4,3-d]pyrimidin-7-yl)-5-eth ynyl-6-fluoronaphthalen-2-ol and *trans*-4-(4-((1R,SS)-3,8-diazabicyclo [3.2.1] octan-3-yl)-8-fluoro-2-(((2R)-2-fluoro-5-(methox ymethyl)tetrahydro-1H-pyrrolizin-7a(5H)-yl)methoxy)pyridino[4,3-d]pyrimidin-7-yl)-5-et hynyl-6-fluoronaphthalen-2-ol**

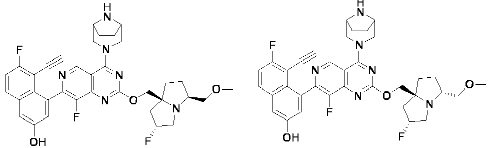

### Isomer 6A

LCMS: m/z 645 (M+H)⁺. ¹H NMR (400 MHz, DMSO-*d₆*) δ 10.17(s, 1 H), 9.03(s, 1 H), 7.97 (q, *J* = 8 Hz, 1H), 7.47 (t, *J* = 8 Hz, 1H), 7.39(s, 1 H), 7.18 (d, *J* = 4Hz, 1H), 5.47-5.32(m, 1H), 4.50 (d, *J =* 8 Hz, 1H), 4.31 (d, *J =* 8 Hz, 1H),4.07 (s, 2H), 3.96 (s, 1H), 3.66(d, *J =* 12 Hz, 1H),3.56(s, 3H), 3.28-3.21(m, 6H), 3.00-2.85 (m,3H), 2.36-2.24(m, 1H), 2.087-1.92 (m,3H), 1.86-1.82(m, 1H), 1.73-1.58 (m,6H).

### Isomer 6B

LCMS: m/z 645 (M+H)⁺. ¹H NMR (400 MHz, DMSO-*d₆*) δ 10.14 ( s, 1 H), 9.03 (s, 1 H) , 7.98 (dd, J=5.92 Hz, 9.20 Hz, 1 H), 7.46 (t, J=9.00 Hz, 1 H), 7.38 (d, J=2.52Hz, 1 H), 7.17 (d, J=2.48 Hz, 1 H), 5.34 (m, 0.5 H), 5.20 (m, 0.5 H), 4.47 (m, 1 H), 4.29(m, 1 H), 4.18 (m, 1 H), 4.10 (m, 1 H), 3.94 (d, J=5.28Hz, 1 H), 3.63 (m, 1 H), 3.48 (m, 5 H), 3.26 (s, 3 H), 3.00 (m, 2 H), 2.29 (m, 1 H), 1.72 (m, 9 H).

### Example 7 4-(4-((1R,SS)-3,8-diazabicyclo [3.2.1] octan-3-yl)-2-(((2R)-5-(cyclopropoxymethyl)-2-fluorot etrahydro-1H-pyrrolizin-7a(5H)-yl)methoxy)-8-fluoropyridino[4,3-d]pyrimidin-7-yl)-5-eth ynyl-6-fluoronaphthalen-2-ol

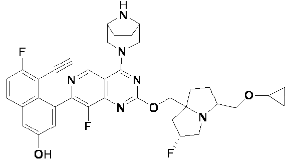

LCMS: m/z 671 (M+H) +.

### Example 8A and Example 8B cis4-(4-((1R,5S)-3,8-diazabicyclo [3.2.1] octan-3-yl)-8-fluoro-2-((3-(trifluoromethoxymethyl) tetrahydro-1H-pyrrolizin-7a(5H)-yl)methoxy)pyridino[4,3-d]pyrimidin-7-yl)-5-ethynyl-6-f luoronaphthalen-2-ol and trans4-(4-((1R,5S)-3,8-diazabicyclo [3.2.1] octan-3-yl)-8-fluoro-2-((3-(trifluoromethoxymeth yl)tetrahydro-1H-pyrrolizin-7a(5H)-yl)methoxy)pyridino[4,3-d]pyrimidin-7-yl)-5-ethynyl-6-fluoronaphthalen-2-ol

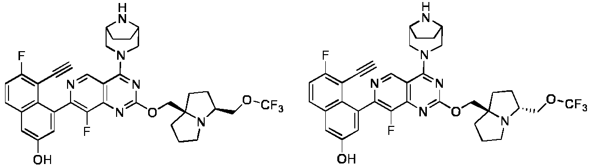

### Example 8A

LCMS: m/z 681 (M+H)⁺.

### Example 8B

LCMS: m/z 681 (M+H)⁺.

### Example 9 4-(4-(1R,5S)-3,8-diazabicyclo[3.2.1]octan-3-yl)-2-(dihydro-1'H,3'H-spiro[1,2'-pyrrolizin]-7 a'(5'H)-yl)methoxy)-8-fluoropyridino[4,3-d]pyrimidin-7-yl)-5-ethynyl-6-fluoro-naphthalen -2-ol

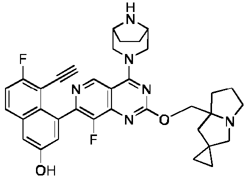

LCMS: m/z 609 (M+H)⁺. ¹H NMR (400 MHz, DMSO-*d₆*) δ10.15(s, 1 H), 9.03(m, 1 H), 7.97 (t, *J =* 8 Hz, 1H), 7.46(t, *J* = 8 Hz, 1H), 7.39(d, *J =* 4 Hz, 1H),7.18-7.17 (m,1H), 4.47(t, *J =* 8Hz, 1H), 4.34-4.21 (m, 2H), 4.08-4.04(m, 1H ), 3.94(d, *J* = 4 Hz, 1H),3.63(t, *J* = 8 Hz, 1H), 3.55 (s, 3H), 2.99-2.94 (m, 1H), 2.74-2.61 (m, 3H), 2.03-1.85 (m, 3H), 1.83-1.76 (m, 3H), 1.71-1.66(m, 4H), 0.49-0.45(m, 4H).

### Example 10 4-(4-((1R,5S)-3,8-diazabicyclo[3.2.1]octan-3-yl)-8-fluoro-2-(((6'R)-6'-fluorodihydro-1'H,3' H-spiro[cyclopropan-1,2'-pyrrolizin]-7a'(5'H)-yl)methoxy)pyridino[4,3-d]pyrimidin-7-yl)-5-ethynyl-6-fluoronaphthalen-2-ol

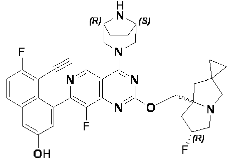

LCMS: m/z 627 (M+H)⁺.

### Example 10 was subjected to chiral resolution to obtain isomer 10A and 10B: 4-(4-((1R,5S)-3,8-diazabicyclo [3.2.1] octan-3-yl)-8-fluoro-2-(((6'R,7a' S)-6'-fluorodihydro-1' H,3'H-spiro[cyclopropan-1,2'-pyrrolizin]-7a'(5'H)-yl)methoxy)pyridino[4,3-d]pyrimidin-7 -yl)-5-ethynyl-6-fluoronaphthalen-2-ol and 4-(4-((1R,5S)-3,8-diazabicyclo [3.2.1] octan-3-yl)-8-fluoro-2-(((6'R,7a'R)-6'-fluorodihydro-1 'H,3'H-spiro[cyclopropan-1,2'-pyrrolizin]-7a'(5'H)-yl)methoxy)pyridino[4,3-d]pyrimidin-7-yl)-5-ethynyl-6-fluoronaphthalen-2-ol

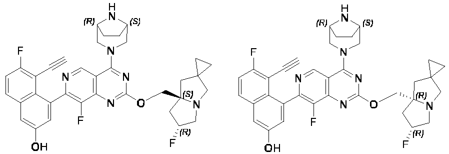

### Isomer 10A:

LCMS: m/z 627 (M+H)⁺. ¹H NMR (400MHz, DMSO-*d₆*) δ 10.18 ( s, 1 H), 9.06 (s, 1 H), 7.99 (dd, *J*=5.92, 9.24 Hz, 1 H), 7.48 (t, *J*=9.00 Hz, 1 H), 7.40 (d, *J*=2.56Hz, 1 H), 7.19 (m, 1 H), 5.41 (m, 0.5 H), 5.28 (m, 0.5 H), 4.49 (m, 1 H), 4.35 (m, 2 H), 4.09 (d, *J*=10.24 Hz,1 H), 3.95 (m,1 H), 3.58 (m, 4 H), 3.27(m, 2H), 3.11 (m, 3 H), 2.70 (m, 1 H), 2.16 (m, 3 H), 1.68 (m, 4 H), 0.51 (m, 4H).

### Isomer 10B:

LCMS: m/z 627 (M+H)⁺. ¹H NMR (400MHz, DMSO-*d*₆) δ 9.02 (s, 1 H), 7.99 (dd, *J*=5.96, 9.16 Hz, 1 H), 7.48 (t, *J*=9.04 Hz, 1 H), 7.40 (d, *J*=2.52Hz, 1 H), 7.16 (m, 1 H), 5.48 (m, 0.5 H), 5.35 (m, 0.5 H), 4.48 (m, 1 H), 4.30 (m, 2 H), 4.12(m, 1 H), 3.93 (s, 1 H), 3.58 (m, 4 H), 3.26 (m, 2 H), 3.01 (m, 0.5 H), 2.89 (m, 0.5 H), 2.80(d, *J*=9.92 Hz,1 H), 2.62 (d, *J*=9.92 Hz,1 H), 2.32 (m, 1 H), 2.03(m, 2H), 1.82 (m, 3 H), 1.65 (m, 3 H), 0.48 (m, 4 H).

### Example 11 4-(4-((1R,SS)-3,8-diazabicyclo [3.2.1] octan-3-yl)-8-fluoro-2-((tetrahydrospiro [cyclopropan-1,3'-pyrrolizin]-7a'(5'H)-yl)methoxy)pyridino[4,3d]pyrimidin-7-yl)-5-ethynyl-6-fluoronap hthalen-2-ol

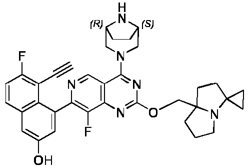

LCMS: m/z 609 (M+H)⁺. ¹H NMR (400MHz, DMSO-*d*₆) δ 9.03 (m, 1 H), 7.95 (dd, *J*=6.00, 9.28Hz, 1 H), 7.45(t, *J*=9.00Hz, 1 H), 7.37(d, *J*=2.56Hz, 1 H), 7.18 (m, 1 H), 4.49 (m,2 H), 4.32 (m, 1 H),4.14 (m, 2 H), 3.92 (m, 1 H), 3.76 (m, 1 H), 3.62 (m, 1 H), 3.58(m, 1H), 2.90 (m, 1 H), 2.63 (m, 1 H), 2.04(m, 7H), 1.63 (m, 6 H), 1.24 (m, 1 H), 0.75 (m, 1 H), 0.58 (m, 1 H), 0.43 (m, 1 H), 0.33 (m, 1 H).

### Example 12A and 12B were synthesized using Intermediate 1-13A and 1-13B as materials respectively: 4-(4-((1R,5S)-3,8-diazabicyclo [3.2.1] octan-3-yl)-8-fluoro-2-(((trans)-6'-fluorotetrahydrospi ro[cyclopropan-1,3'-pyrrolizin]-7a'(5'H)-yl)methoxy)pyridino[4,3-d]pyrimidin-7-yl)-5-eth ynyl-6-fluoronaphthalen-2-ol and 4-(4-((1R,5S)-3,8-diazabicyclo[3.2.1]octan-3-yl)-8-fluoro-2-(((cis)-6'-fluorotetrahydrospiro[ cyclopropan-1,3'-pyrrolizin]-7a'(5'H)-yl)methoxy)pyridino[4,3-d]pyrimidin-7-yl)-5-ethyny l-6-fluoronaphthalen-2-ol

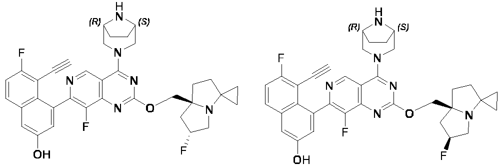

### Example 12A

LCMS: m/z 627 (M+H)⁺. ¹H NMR (400MHz, DMSO-*d*₆) δ 10.15(s,1 H), 9.04(s,1 H), 7.99-7.95(m,1 H), 7.46 (t, *J =* 8 Hz,1H), 7.39(d, *J =* 4 Hz 1H) , 7.18(d, *J =* 4 Hz 1H), 5.25(d, *J* = 4 Hz,1H), 4.49(d, J=12 Hz,1H), 4.32(d, *J* =12 Hz,1H), 4.25-4.17(m, 2H), 3.94(s,1H), 3.65(d, *J =* 12 Hz,1H), 3.57(d, *J* =8 Hz, 3H), 3.26-3.14(m, 1H), 3.05-2.96(m, 1H), 2.39-2.22(m, 2H), 2.15-2.05 (m, 1H), 2.00-1.95(m,2H), 1.65(s,4H), 1.34-1.23(m, 1H), 0.85-0.75 (m,1H), 0.61-0.55(m, 1H), 0.45-0.37(m, 2H).

### Example 12B

LCMS: m/z 627 (M+H)⁺. ¹H NMR (400MHz, DMSO-*d*₆) δ 10.19(s,1 H), 9.03(s,1 H), 7.99-7.95(m,1 H), 7.46 (t, *J =* 8 Hz,1H), 7.39(d, *J =* 4 Hz 1H) , 7.18(d, *J =* 4 Hz 1H), 5.34(d, *J* = 4 Hz,1H), 4.50 (d, *J* =12 Hz,1H), 4.31-4.27(m,1H), 4.27-4.16(m, 2H), 3.95(d, *J* = 4 Hz,1H), 3.66(d, *J* = 12 Hz,1H), 3.57(d, *J* =12 Hz, 3H), 3.23-3.15(m, 1H), 2.99-2.85(m, 1H), 2.40-2.31(m, 1H), 2.17-2.09 (m, 1H), 2.05-1.86(m,3H), 1.65(s,4H), 1.34-1.23(m, 2H), 0.81-0.76 (m,1H), 0.64-0.58(m, 1H), 0.52-0.46(m, 1H), 0.38-0.33(m, 1H).

### Example 13 4-(4-((1R,SS)-3,8-diazabicyclo [3.2.1] octan-3-yl)-2-((7',7'-difluorotetrahydro-3'-H-spiro [cyc lopropan-1,2'-pyrrolizin]-8a'(1'H)-yl)methoxy)-8-fluoropyridino[4,3d]pyrimidin-7-yl)-5-et hynyl-6-fluoronaphthalen-2-ol trifluoroacetate

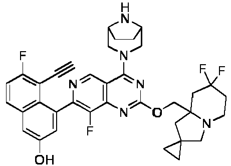

LCMS: m/z 659 (M+H)⁺. ¹H NMR (400MHz, DMSO-*d*₆) δ 10.25 (br, 1 H), 9.42 (m, 1 H), 9.15 (m, 2 H), 8.00 (dd, *J*=6.00,9.20Hz,1 H), 7.47(t, *J*=9.20Hz, 1 H), 7.42(d, *J*=2.40Hz, 1 H), 7.19 (m, 1 H), 4.63 (m, 5 H), 4.23 (s, 2 H), 3.91(m, 4 H), 3.52 (m, 1 H), 3.39 (m,4 H), 3.52 (m, 1 H), 3.39 (m, 1H), 2.53 (m, 1 H), 1.97 (m, 5 H), 0.74 (m, 4 H).

### Example 14 4-(4-((1R,5S)-3,8-diazabicyclo [3.2.1] octan-3-yl)-2-((6',6'-difluorotetrahydro-3'-H-spiro [cyc lopropan-1,2'-pyrrolizin]-8a'(1'H)-yl)methoxy)-8-fluoropyridino[4,3d]pyrimidin-7-yl)-5-et hynyl-6-fluoronaphthalen-2-ol

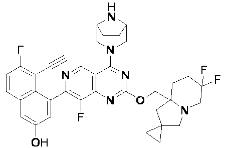

LCMS: m/z 645 (M+H)⁺. ¹H NMR (400MHz, DMSO-*d*₆) δ 10.15 (s, 1 H), 9.05 (s, 1 H), 7.97 (dd, *J*=5.92, 9.24Hz,1 H), 7.46(t, *J*=9.00Hz, 1 H), 7.39(d, *J*=2.60Hz, 1 H), 7.18 (s, 1 H), 4.64(dd, *J*=2.92, 10.88Hz,1 H), 4.47 (d, *J*=12.64Hz, 1 H), 4.35 (m, 2 H), 3.93 (m, 1 H), 3.60(m, 4 H), 3.10 (m, 4 H), 2.81 (dd, *J*=3.96,8.68Hz,1 H), 2.84 (m,1 H), 2.02 (m, 1 H), 1.91 (m, 3H), 1.68 (m, 5H), 0.52 (m, 4 H).

### Examples 15A and 15B were synthesized according to the method of Example 1 using Intermediate 1-16A and 1-16B: 4-(4-((1R,5S)-3,8-diazabicyclo[3.2.1]octan-3-yl)-8-fluoro-2-(((1S,6'R,7a'S)-2,2,6'-triflu orodihydro-1'H,3'H-spiro[cyclopropan-1,2'-pyrrolizin]-7a'(5'H)-yl)methoxy)pyridino[4,3-d]pyrimidin-7-yl)-5-ethynyl-6-fluoronaphthalen-2-ol and 4-(4-((1R,5S)-3,8-diazabicyclo [3.2.1] octan-3-yl)-8-fluoro-2-(((1R,6'R,7a' S)-2,2,6'-trifluorod ihydro-1'H,3'H-spiro[cyclopropan-1,2'-pyrrolizin]-7a'(5'H)-yl)methoxy)pyridino[4,3-d]py rimidin-7-yl)-5-ethynyl-6-fluoronaphthalen-2-ol

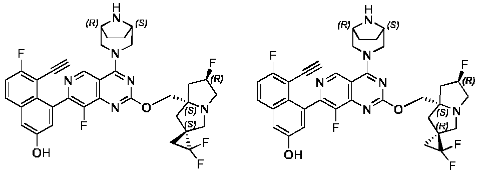

### Example 15A

LCMS: m/z 663 (M+H)⁺. ¹H NMR (400MHz, DMSO-*d*₆) δ 10.16 (s, 1 H), 9.05 (s, 1 H), 7.98 (dd, *J*=5.92, 9.16Hz, 1 H),7.47(t, *J*=9.04Hz, 1 H), 7.40(d, J=2.64Hz, 1 H), 7.18 (m, 1 H), 5.43 (m, 0.5 H), 5.29(m, 0.5 H), 4.51 (t, *J*=12.16Hz, 1 H), 4.31 (m, 1 H), 4.05(m, 2 H), 3.94 (d, *J*=4.16Hz, 1 H), 3.61 (m, 4H), 3.12 (m, 4 H), 2.22 (m, 5 H), 1.69(s, 4H), 1.54 (m, 2 H).

### Example 15B

LCMS: m/z 663 (M+H)⁺. ¹H NMR (400MHz, DMSO-*d*₆) δ 10.17(s, 1 H), 9.06 (s, 1 H),7.98 (dd, *J*=5.92, 9.12Hz, 1 H), 7.47(t, *J*=9.00Hz, 1 H), 7.40(d, *J*=2.68Hz, 1 H), 7.18 (m, 1 H), 5.35 (m, 0.5 H), 521(m, 0.5 H), 4.48 (m, 1 H), 4.31 (m, 2 H), 4.18(m, 1 H), 3.94 (s, 1 H), 3.61 (m, 4H), 3.21 (m, 2 H), 3.01 (m, 2 H), 2.16 (m, 5 H), 1.69(s, 4H), 1.54 (m, 2H).

### Examples 15C and 15D were synthesized according to the method of Example 1 using Intermediate 1-16C and 1-16D: 4-(4-((1R,5S)-3,8-diazabicyclo [3.2.1] octan-3-yl)-8-fluoro-2-(((1S,6'R,7a'R)-2,2,6'-triflu orodihydro-1'H,3'H-spiro[cyclopropan-1,2'-pyrrolizin]-7a'(5'H)-yl)methoxy)pyridino[4,3-d]pyrimidin-7-yl)-5-ethynyl-6-fluoronaphthalen-2-ol and 4-(4-((1R,5S)-3,8-diazabicyclo [3.2.1] octan-3-yl)-8-fluoro-2-(((1R,6'R,7a'R)-2,2,6'-trifluoro dihydro-1'H,3'H-spiro[cyclopropan-1,2'-pyrrolizin]-7a'(5'H)-yl)methoxy)pyridino[4,3-d]p yrimidin-7-yl)-5-ethynyl-6-fluoronaphthalen-2-ol

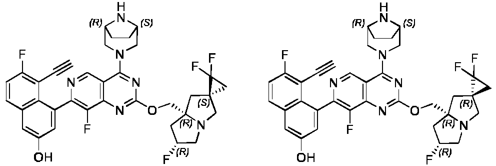

### Example 15C

LCMS: m/z 663 (M+H)⁺. ¹H NMR (400MHz, DMSO-*d*₆) δ 10.20(s, 1 H), 9.05 (s, 1 H),7.98 (dd, *J*=5.92, 9.28Hz, 1 H), 7.48(t, *J*=8.96Hz, 1 H), 7.40(d, *J*=2.52Hz, 1 H), 7.19 (d, *J*=2.52Hz, 1 H), 5.45 (m, 0.5 H), 5.32(m, 0.5 H), 4.48 (d, *J*=11.56Hz, 1 H), 4.30 (m, 3 H), 3.96 (d, *J*=2.88Hz, 1 H), 3.59 (m, 4H), 3.31 (m, 2 H), 3.01 (m, 2 H), 2.16 (m, 5 H), 1.69(s, 4H), 1.54 (m, 2 H) .

### Example 15D

LCMS: m/z 663 (M+H)⁺. ¹H NMR (400MHz, DMSO-*d*₆) δ 10.16(s, 1 H), 9.04 (s, 1 H), 7.98 (dd, *J*=6.00, 9.28Hz,1 H), 7.48(t, *J*=9.04Hz, 1 H), 7.40(d, *J*=2.44Hz, 1 H), 7.19 (m, 1 H), 5.51 (m, 0.5 H), 5.37(m, 0.5 H), 4.50 (m, 1 H), 4.30 (m, 1 H), 4.16 (m, 1 H), 3.98 (m, 2H), 3.61 (m, 4 H), 3.28 (m, 1 H), 3.07 (m, 1 H), 2.79 (m, 1 H), 2.21 (m, 5 H), 1.59(s, 6H).

### Examples 15E and 15F were synthesized according to the method of Example 1 using Intermediate 1-16E and 1-16F: 4-(4-((1R,5S)-3,8-diazabicyclo[3.2.1]octan-3-yl)-8-fluoro-2-(((1S,6'S,7a'S)-2,2,6'-triflu orodihydro-1'H,3'H-spiro[cyclopropan-1,2'-pyrrolizin]-7a'(5'H)-yl)methoxy)pyridino[4,3-d]pyrimidin-7-yl)-5-ethynyl-6-fluoronaphthalen-2-ol and 4-(4-((1R,5S)-3,8-diazabicyclo [3.2.1] octan-3-yl)-8-fluoro-2-(((1R,6' S,7a' S)-2,2,6'-trifluorod ihydro-1'H,3'H-spiro[cyclopropan-1,2'-pyrrolizin]-7a'(5'H)-yl)methoxy)pyridino[4,3-d]py rimidin-7-yl)-5-ethynyl-6-fluoronaphthalen-2-ol

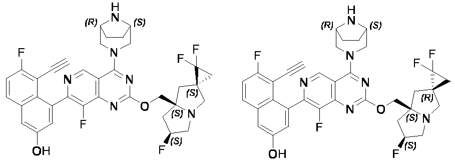

### Example 15E

LCMS: m/z 663 (M+H)⁺. ¹H NMR (400MHz, DMSO-*d*₆) δ 10.15 (s, 1 H), 9.05 (s, 1 H), 7.97 (dd, *J*=5.92, 9.16Hz,1 H), 7.46(t, *J*=9.00Hz, 1 H), 7.39(d, *J*=2.52Hz, 1 H), 7.17 (d, *J*=2.52Hz,1 H), 5.44 (m, 0.5 H), 5.30(m, 0.5 H), 4.48 (d, *J*=11.72Hz, 1 H), 4.30 (m, 3H), 3.93 (d, J=2.84Hz, 1 H), 3.61 (m, 4H), 3.16 (m, 2 H), 2.77 (m, 2 H) , 2.14 (m, 5 H) , 1.61(s, 6H) .

### Example 15F

LCMS: m/z 663 (M+H)⁺. ¹H NMR (400MHz, DMSO-*d*₆) δ 10.16(s, 1 H), 9.04 (s, 1 H), 7.97 (dd, *J*=5.92, 9.16Hz,1 H), 7.46(t, *J*=8.96Hz, 1 H), 7.39(d, *J*=2.60Hz, 1 H), 7.18 (m, 1 H), 5.50 (m, 0.5 H), 5.36(m, 0.5 H), 4.50 (m, 1 H), 4.29 (m, 1 H), 4.14(m, 1 H), 4.01(m, 1 H), 3.94 (s, 1 H), 3.60 (m, 4H), 3.28 (m, 1 H), 3.03 (m, 2 H), 2.78(m, 1 H), 2.16 (m, 5 H) , 1.65(s, 6H).

### Examples 15G and 15H were synthesized according to the method of Example 1 using Intermediate 1-16G and 1-16H: 4-(4-((1R,5S)-3,8-diazabicyclo[3.2.1]octan-3-yl)-8-fluoro-2-(((1S,6'S,7a'R)-2,2,6'-triflu orodihydro-1'H,3'H-spiro[cyclopropan-1,2'-pyrrolizin]-7a'(5'H)-yl)methoxy)pyridino[4,3-d]pyrimidin-7-yl)-5-ethynyl-6-fluoronaphthalen-2-ol and 4-(4-((1R,5S)-3,8-diazabicyclo [3.2.1] octan-3-yl)-8-fluoro-2-(((1R,6' S,7a'R)-2,2,6'-trifluorod ihydro-1'H,3'H-spiro[cyclopropan-1,2'-pyrrolizin]-7a'(5'H)-yl)methoxy)pyridino[4,3-d]py rimidin-7-yl)-5-ethynyl-6-fluoronaphthalen-2-ol

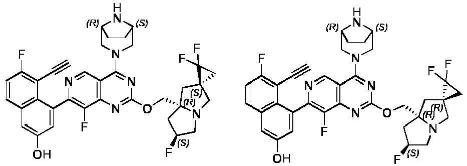

### Example 15G

LCMS: m/z 663 (M+H)⁺. ¹H NMR (400MHz, DMSO-*d*₆) δ 10.17(s, 1 H), 9.04 (s, 1 H), 7.97 (dd, *J*=5.96, 9.20Hz,1 H), 7.46(t, *J*=9.00Hz, 1 H), 7.39(d, *J*=2.56Hz, 1 H), 7.17 (m, 1 H), 5.42 (m, 0.5 H), 5.28(m, 0.5 H), 4.49 (m, 1 H), 4.29 (m, 1H), 4.04 (m, 2H), 3.93 (m, 1 H), 3.62 (m, 4H), 3.09 (m, 4 H), 2.11 (m, 5 H) , 1.56(s, 6H).

### Example 15H

LCMS: m/z 663 (M+H)⁺. ¹H NMR (400MHz, DMSO-*d*₆) δ 9.01 (s, 1 H), 7.66 (m, 1 H), 7.21(m, 1H), 7.01(m, 2 H), 5.34 (m, 0.5 H), 5.20(m, 0.5 H), 4.28 (m, 4 H), 3.75 (m, 1H), 3.58 (m, 3 H), 3.17 (m, 3 H), 2.99 (m, 2 H), 2.19 (m, 5 H), 1.56(s, 6H) .

**The following examples were synthesized according to the method of Example 1 using Intermediate 1-11A and 1-11B as the starting materials:**

### Example 16A and 16B 4-(4-((1R,5S)-3,8-diazabicyclo[3.2.1]octan-3-yl)-2-(((cis)-2,2-difluorodihydro-1'H,3'H-spiro [cyclopropan-1,2'-pyrrolizin]-7a'(5'H)-yl)methoxy)-8-fluoropyridino[4,3-d]pyrimidin-7-yl) -5-ethynyl-6-fluoronaphthalen-2-ol and 4-(4-((1R,5S)-3,8-diazabicyclo[3.2.1]octan-3-yl)-2-(((trans)-2,2-difluorodihydro-1'H,3'H-sp iro[cyclopropan-1,2'-pyrrolizin]-7a'(5'H)-yl)methoxy)-8-fluoropyridino[4,3-d]pyrimidin-7 -yl)-5-ethynyl-6-fluoronaphthalen-2-ol

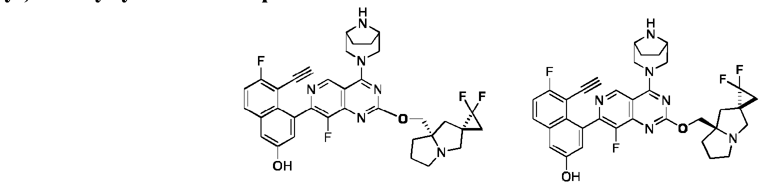

### Example 16A 4-(4-((1R,5S)-3,8-diazabicyclo[3.2.1]octan-3-yl)-2-(((cis)-2,2-difluorodihydro-1'H,3'H-spiro [cyclopropan-1,2'-pyrrolizin]-7a'(5'H)-yl)methoxy)-8-fluoropyridino[4,3-d]pyrimidin-7-yl) -5-ethynyl-6-fluoronaphthalen-2-ol

LCMS: m/z 645 (M+H)⁺. ¹H NMR (400 MHz, DMSO-d₆) δ10.14(s, 1 H), 9.03(s, 1 H), 7.99-7.95 (m, 1H), 7.46(t, *J* = 8 Hz, 1H), 7.39(s,1H), 7.18(t, *J* = 4 Hz, 1H), 4.49(t, *J* = 12Hz, 1H), 4.31(t, *J =* 12Hz, 1H), 4.09(d, *J*=12Hz,1H), 3.98(d, *J*=4Hz,1H), 3.95-3.93(m, 1H), 3.65(t, *J* = 8Hz, 1H), 3.59-3.56 (m, 3H), 3.06(d, *J* = 12 Hz, 1H),3.00-2.95(m, 1H), 2.80-2.76 (m, 1H), 2.71-2.65 (m, 1H), 2.14-2.10 (m, 1H), 1.96-1.87 (m, 3H), 1.84-1.75(m, 2H), 1.66(s, 4H), 1.56-1.51(m,2H).

### Example 16B 4-(4-((1R,5S)-3,8-diazabicyclo[3.2.1]octan-3-yl)-2-(((trans)-2,2-difluorodihydro-1'H,3'H-sp iro[cyclopropan-1,2'-pyrrolizin]-7a'(5'H)-yl)methoxy)-8-fluoropyridino[4,3-d]pyrimidin-7 -yl)-5-ethynyl-6-fluoronaphthalen-2-ol

LCMS: m/z 645 (M+H)⁺. ¹H NMR (400 MHz, DMSO-d₆) δ10.17(s, 1 H), 9.04(s, 1 H), 7.99-7.95 (m, 1H), 7.46(t, *J* = 8 Hz, 1H), 7.39(s,1H),7.18(d, *J* = 4 Hz, 1H), 4.49(t, *J* = 12Hz, 1H), 4.33(d, *J* = 12Hz, 1H), 4.22-4.15(m, 2H), 3.92(s,1H), 3.95-3.93(m, 1H), 3.65(d, *J* = 12Hz, 1H), 3.58-3.56 (m, 3H), 3.12-3.08(m, 1H), 3.04-3.00(m, 1H), 2.73 (d, *J* = 12Hz,1H), 2.56-2.54 (m, 1H), 2.10-2.05 (m, 1H), 2.01-1.97 (m, 1H), 1.92 (d, *J =* 16Hz,1H), 1.84-1.75(m, 2H), 1.66-1.44(m, 7H).

**Example 16B** was separated by chiral HPLC(SFC-150 (Waters), column: IG 20*250mm, 10um (Daicel), column tmeperature: 35 °C, mobile phase: CO₂/EtOH[0.5%NH₃ (7M in MeOH)] = 120/40) to obtain isomers **Example 16B-1** and Example **16B-2.**

### Example 16B-1 4-(4-((1R,5S)-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-5-ethynyl-6-fluoronaphthalen-2-ol

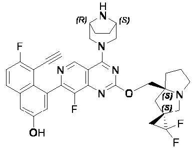

RT: 15.079 min. LCMS: m/z 645 (M+H)⁺. ¹H NMR (400 MHz, DMSO-*d*₆) δ 9.04(s, 1 H), 7.93-7.89(m,1 H), 7.41(d, *J* = 8Hz, 1H), 7.32(s,1H), 7.17(s,1H),4.49(d, *J* = 12Hz, 1H), 4.35(d, *J* = 12Hz, 1H), 4.20-4.16 (m, 2H), 3.89(s, 1H), 3.64-3.55 (m, 5H), 3.13-3.09 (m, 1 H), 3.05-3.00 (m, 1 H),2.74(d, *J* = 12Hz,1H), 2.57-2.55 (m,1H), 2.11-2.06(m, 1H), 2.02-1.98 (m, 1H), 1.92(d, *J =* 12Hz, 1H), 1.84-1.75(m,3H), 1.66-1.47(m,8H).

### Example 16B-2 4-(4-((1R,5S)-3,8-diazabicyclo[3.2.1]octan-3-yl)-2-(((1R,7a'R)-2,2-difluorodihydro-1'H,3'H -spiro[cyclopropan-1,2'-pyrrolizin]-7a'(5'H)-yl)methoxy)-8-fluoropyridino[4,3-d]pyrimidi n-7-yl)-5-ethynyl-6-fluoronaphthalen-2-ol

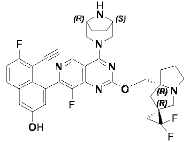

RT: 20.572 min. LCMS: m/z 645 (M+H)⁺. ¹H NMR (400 MHz, DMSO-*d*₆) δ 9.04(s, 1 H), 7.95-7.99(m,1 H), 7.46(t, *J* = 8Hz, 1H), 7.39(s,1H), 7.18(s,1H),4.49(d, *J* = 12Hz, 1H), 4.35(d, *J* = 12Hz, 1H), 4.20-4.18 (m, 2H), 392(s, 1H),3.65-3.56 (m, 5H), 3.13-3.09 (m, 1 H), 3.05-3.00 (m, 1 H),2.74(d, *J* = 12Hz,1H), 2.57-2.55 (m,1H), 2.11-2.06(m, 1H), 2.02-1.98 (m, 1H), 1.92(d, *J =* 12Hz, 1H),1.84-1.74(m,3H), 1.67-1.45(m,8H).

**The following examples were synthesized according to the method of Example 1 using Intermediate 1-11A as the starting material:**

### Example 17: 4-(4-(1R,5S)-3,8-diazabicyclo[3.2.1]octan-3-yl)-2-(((trans)-2,2-difluorodihydro-1'H,3'H-spi ro[cyclopropan-1,2'-pyrrolizin]-7a'(5'H)-yl)methoxy)-8-fluoropyridino[4,3-d]pyrimidin-7-yl)-5-ethyl-6-fluoronaphthalen-2-ol

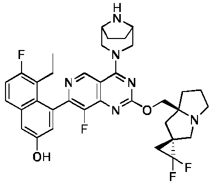

LCMS: m/z 649 (M+H)⁺. ¹H NMR (400 MHz, DMSO- *d*₆) δ10.01(s, 1 H), 9.10(s, 1 H),7.77(q, *J* = 4Hz,1 H), 7.38(d, *J* = 8Hz, 1H), 7.34(d, *J =* 4Hz, 1H), 7.03(d, *J=* 4Hz, 1H), 4.44 (d, *J =* 12Hz, 2H), 4.26-4.17(m, 2 H),3.62 (t, J=12Hz, 2H), 3.55(s, 2 H), 3.13(q, *J =* 8Hz,1H), 3.04-2.99 (m,1H), 2.74(d, *J* = 12Hz, 1H), 2.57-2.55 (m, 1H), 2.39-2.34 (m, 1H), 2.15 (t, *J* =8Hz, 1H), 2.12-2.06 (m, 1H), 2.02-1.98 (m, 1H), 1.92(d, *J* = 12Hz, 1H),1.80-1.73(m,2H ), 1.63-1.44(m,7H), 7.38(t, *J* = 8Hz, 3H).

Two isomers were obtained by chiral HPLC separation:

### Example 17A: 4-(4-(1R,5S)-3,8-diazabicyclo[3.2.1]octan-3-yl)-2-(((1S,7a'S)-2,2-difluorodihydro-1'H,3'H-s piro[cyclopropan-1,2'-pyrrolizin]-7a'(5'H)-yl)methoxy)-8-fluoropyridino[4,3-d]pyrimidin-7-yl)-5-ethyl-6-fluoronaphthalen-2-ol

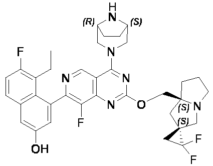

RT: 14.093 min (U3000 (ThermoFisher); column: CHIRALPAK IF-3 4.6mm*150mm, 3um; column temperature: 40 °C; mobile phase: n-hexane:ethanol-diethylamine=85/15-0.1%). LCMS: m/z 649 (M+H)⁺. ¹H NMR (400 MHz, DMSO-*d₆*) δ 9.94(s, 1 H), 9.11(s, 1 H),7.77(q, *J* = 4Hz,1 H), 7.38(d, *J* = 8Hz, 1H), 7.34(s, 1H), 7.04(d, *J =* 4Hz, 1H), 4.44 (d, *J* = 12Hz, 2H), 4.26-4.17(m, 2 H),3.65-3.56 (m, 4H), 3.13(q, *J* = 8Hz,1H), 3.04-2.99 (m,1H), 2.74(d, *J* = 12Hz, 1H), 2.57-2.54 (m, 1H), 2.39-2.34 (m, 1H), 2.17-2.00 (m, 2H), 2.00-1.98 (m, 1H), 1.92(d, *J* = 12Hz, 1H),1.82-1.44(m,10H ), 0.73(t, *J* = 8Hz, 3H).

### Example 17B: 4-(4-(1R,5S)-3,8-diazabicyclo[3.2.1]octan-3-yl)-2-(((1R,7a'R)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-5-ethyl-6-fluoronaphthalen-2-ol

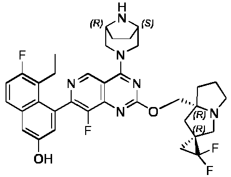

RT: 14.840 min (U3000 (ThermoFisher); column: CHIRALPAK IF-3 4.6mm*150mm, 3um; column temperature: 40 °C; mobile phase: n-hexane:ethanol-diethylamine=85/15-0.1%). LCMS: m/z 649 (M+H)⁺. 1H NMR (400 MHz, DMSO-*d₆*) δ 9.96(s, 1 H), 9.11(s, 1 H),7.77(q, *J* = 4Hz,1 H), 7.38(d, *J* = 8Hz, 1H), 7.34(s, 1H), 7.03(d, *J* = 4Hz, 1H), 4.45(d, *J =* 16Hz, 2H), 4.26-4.17(m, 2 H),3.65-3.55 (m, 4H), 3.13(q, *J* = 8Hz,1H), 3.04-2.99 (m,1H), 2.74(d, *J* = 12Hz, 1H), 2.57-2.54 (m, 1H), 2.39-2.34 (m, 1H), 2.17-2.00 (m, 2H), 2.00-1.98 (m, 1H), 1.92(d, *J* = 12Hz, 1H),1.80-1.44(m,10H ), 0.73(t, *J* = 8Hz, 3H).

### Example 18: 6-(4-((1R,5S)-3,8-diazabicyclo[3.2.1]octan-3-yl)-2-(((trans)-2,2-difluorodihydro-1'H,3'H-sp iro[cyclopropan-1,2'-pyrrolizin]-7a'(5'H)-yl)methoxy)-8-fluoropyridino[4,3d]pyrimidin-7-yl)-4-methyl-5-(trifluoromethyl)pyridin-2-amine

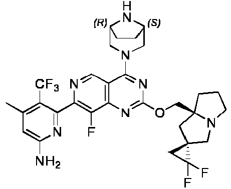

LCMS: m/z 635 (M+H)⁺. ¹H NMR (400MHz, DMSO-*d*₆) δ 9.01 (s, 1 H), 6.78 (s, 1 H), 4.36 (d, *J*=9.20Hz, 1 H), 4.17 (m, 2 H), 3.55 (m, 4 H), 3.09 (m, 1 H), 2.99 (m, 1 H), 2.70(m, 2H), 2.54 (m, 1 H), 2.36 (m, 3 H), 2.05 (m, 1 H), 1.97 (m, 1 H), 1.99(m, 1 H), 1.79 (m, 2H), 1.56 (m, 7 H).

### Example 19: 6-(4-((1R,5S)-3,8-diazabicyclo[3.2.1]octan-3-yl)-2-(((trans)-2,2-difluorodihydro-1'H,3'H-sp iro[cyclopropan-1,2'-pyrrolizin]-7a'(5'H)-yl)methoxy)-8-fluoropyridino[4,3d]pyrimidin-7-yl)-4-methyl-5-(pentafluoroethyl)pyridin-2-amine

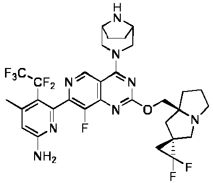

LCMS: m/z 685 (M+H)⁺.

**The following examples were synthesized according to the method of Example 1 using Intermediate 1-11A-1 as the starting material:**

### Example 20: 4-(2-(((1S,7a'S)-2,2-difluorodihydro-1'H,3'H-spiro[cyclopropan-1,2'-pyrrolizin]-7a'(5'H)-y l)methoxy)-8-fluoro-4-(1-methyl-3,8-diazabicyclo[3.2.1]octan-3-yl)pyridino[4,3-d]pyrimidi n-7-yl)-5-ethynyl-6-fluoronaphthol-2-ol

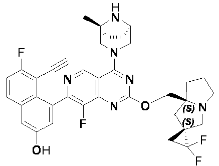

LCMS: m/z 659 (M+H)⁺. ¹H NMR (400MHz, DMSO-*d*₆) δ 10.20 (br, 1 H), 9.06 (s, 1 H), 7.98 (dd, *J*=5.88Hz, *J*=9.20Hz,1 H), 7.48(t, *J*=9.00Hz, 1 H), 7.40(d, *J*=2.64Hz, 1 H), 7.19 (s, 1 H), 4.30 (m, 4H), 3.94 (s, 1H), 3.58(m, 2H), 3.11 (m, 1 H), 3.02 (m,1 H), 2.71 (d, *J*=11.96Hz, 1 H), 2.55 (m, 1H), 2.06 (m, 2 H), 1.71 (m, 10 H), 1.25 (m, 5H).

Isomer 20A and 20B were obtained by chiral separation (SFC-150 (Waters); column: OD 20*250mm, 10um (Daicel)):

### 4-(2-(((1S,7a'S)-2,2-difluorodihydro-1'H,3'H-spiro[cyclopropan-1,2'-pyrrolizin]-7a'(5'H)-y l)methoxy)-8-fluoro-4-((1S,5R)-1-methyl-3,8-diazabicyclo[3.2.1]octan-3-yl)pyridino[4,3-d] pyrimidin-7-yl)-5-ethynyl-6-fluoronaphthol-2-ol and 4-(2-(((1S,7a'S)-2,2-difluorodihydro-1'H,3'H-spiro[cyclopropan-1,2'-pyrrolizin]-7a'(5'H)-y l)methoxy)-8-fluoro-4-((1R,5S)-1-methyl-3,8-diazabicyclo[3.2.1]octan-3-yl)pyridino[4,3-d] pyrimidin-7-yl)-5-ethynyl-6-fluoronaphthol-2-ol

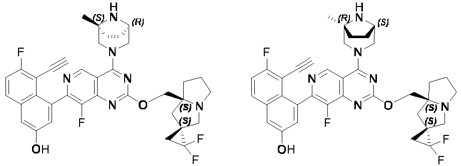

### Isomer 20A

RT: 0.905min. LCMS: m/z 659 (M+H)⁺. ¹H NMR (400MHz, DMSO-*d*₆) δ 10.18(s, 1 H), 9.07(s, 1 H),8.01-7.97(m,1 H), 7.48(t, *J* = 8Hz, 1H), 7.40(d, *J =* 4Hz, 1H), 7.19(s, 1H), 4.44 (t, *J=* 12Hz, 1H), 4.35-4.15(m, 3H),3.94 (s,1H), 3.60-3.57(m, 2 H), 3.38(s,1H), 3.13-3.09 (m,1H), 3.05-3.00(m,1H), 2.74 (d, *J* =12Hz,1H), 2.57-2.55 (m, 1H), 2.11-1.99 (m, 2H), 1.93(d, *J =* 16Hz, 1H),1.84-1.46(m,9H), 1.38-1.32(m,1H), 1.26(s, 3H).

### Isomer 20B

RT: 1.419 min. LCMS: m/z 659 (M+H)⁺. ¹H NMR (400MHz, DMSO-*d*₆) δ 10.20(s, 1 H), 9.07(s, 1 H), 8.01-7.97(m,1 H), 7.48(t, *J* = 8Hz, 1H), 7.40(d, *J* = 4Hz, 1H), 7.20(s, 1H), 4.49-4.35(m,2H), 4.31(d, *J* = 12Hz, 1H), 4.20(s, 2H), 3.95(s, 1H), 3.64-3.56(m, 2 H), 3.43(d, *J* =16Hz, 1H), 3.13-3.09(m, 1H), 3.04-3.00(m, 1H), 2.74 (d, *J* =12Hz, 1H), 2.57-2.55(m, 1H), 2.11-2.07(m, 1H), 2.02-1.99(m, 1H), 1.82-1.35(m, 10H), 1.29-1.24(m, 3H).

### Example 21: (1S,7a'S)-7a'-(((4-(1R,5S)-3,8-diazabicyclo[3.2.1]octan-3-yl)-7-(8-ethynyl-7-fluoronaphthol -1-yl)-8-fluoropyridino[4,3-d]pyrimidin-2-yl)oxy)methyl)-2,2-difluorodihydro-1'H,3'H-spi ro[cyclopropan-1,2'-pyrrolizin]

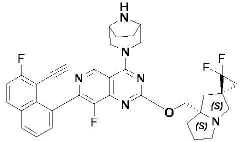

LC-MS: m/z 609 (M+H)⁺. ¹H NMR (400 MHz, DMSO-*d*₆) δ 9.07(s, 1 H), 8.25(d, *J* = 8Hz, 1H), 8.22(d, *J* = 8Hz, 1H), 7.79-7.60(m,3H), 4.51(d, *J =* 12Hz, 1H), 4.38(d, *J* = 12Hz, 1H), 4.24-4.19(m, 2 H), 4.02(s, 1H), 3.67-3.62(m, 4H), 3.14-3.09(m,1H), 3.05-3.00 (m,1H), 2.74(d, *J* = 12Hz, 1H), 2.57-2.55(m, 1H), 2.11-2.06 (m, 1H), 2.02-1.90(m, 2H), 1.82-1.45(m, 9H).

### Example 22: (1S,7a'S)-7a'-(((4-((1R,5S)-3,8-diazabicyclo[3.2.1]octan-3-yl)-8-fluoro-7-(6-fluoro-5-methyl -1H-indazol-4-yl)pyridino[4,3-d]pyrimidin-2-yl)oxy)methyl)-2,2-difluorodihydro-1'H,3'H-spiro[cyclopropan-1,2'-pyrrolizin]

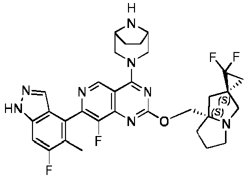

LC-MS: m/z 609 (M+H)⁺. ¹H NMR (400 MHz, DMSO-*d₆*) δ 13.21(s, 1 H), 9.20(s, 1 H),7.74(s,1 H), 7.50(d, *J* = 8Hz, 1H), 4.47(s,2H), 4.25-4.19(m, 2 H), 3.64-3.58 (m, 3H), 3.14(q, *J* = 7Hz, *J* = 5Hz,1H), 3.05-3.00 (m,1H), 2.74(d, *J* = 12Hz, 1H), 2.57-2.55 (m, 4H), 2.19(s, 2 H), 2.12-1.45 (m, 8H).

### Example 23: 4-(4-((1R,5S)-3,8-diazabicyclo[3.2.1]octan-3-yl)-2-(((1S,7a'S)-2,2-difluorodihydro-1'H ,3'H-spiro[cyclopropan-1,2'-pyrrolizin]-7a'(5'H)-yl)methoxy)-8-fluoropyridino[4,3-d] pyrimidin-7-yl)-7-fluorobenzo[d]thiazol-2-amine

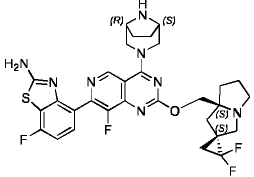

LC-MS: m/z 627 (M+H)⁺. ¹H NMR (400 MHz, DMSO-*d₆*) δ 9.08 (s, 1 H), 7.91 (s, 1 H), 7.42(dd, *J*=5.80,8.48Hz, 1 H), 7.07(t, *J*=8.88Hz, 1 H), 4.38 (d, *J*=12.12Hz,2 H), 4.19 (m,2 H), 3.57 (m, 4H), 3.09 (m, 1 H), 3.01 (m, 1 H), 2.70 (d, *J*=11.84Hz,1 H), 2.54 (m, 1 H), 2.08 (m, 1 H), 1.98(m, 1H), 1.88 (d, *J*=13.32Hz,1 H), 1.63 (m, 9 H).

### Example 24: 4-(4-((1R,5S)-diazabicyclo[3.2.1]octan-3-yl)-2-(((1S,7a'S)-2,2-difluorodihydro-1'H,3'H-spir o[cyclopropan-1,2'-pyrrolizin]-7a'(5'H)-yl)methoxy)-8-fluoropyridino[4,3-d]pyrimidin-7-y l)-2-amino-7-fluorobenzo[b]thiophen-3-carbonitrile

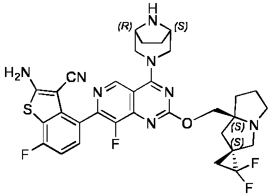

LC-MS: m/z 651 (M+H)⁺. ¹H NMR (400MHz, DMSO-*d*₆) δ 9.07(s, 1 H), 8.10(s, 2H), 7.44-7.40 (m, 1H), 7.15(t, *J =* 8 Hz, 1H), 4.43(d, *J =* 12Hz, 2H),4.23-4.17(m, 2H), 3.63(d, *J*=12Hz,2H), 3.53(s,2H), 3.13-3.09 (m, 1H), 3.05-3.00(m,1H), 2.74 (d, *J* = 12Hz, 1H), 2.57-2.55 (m, 1H), 2.12-2.07 (m, 1H), 2.03-1.97 (m, 1H),1.92-1.89(m, 2H), 1.86-1.71(m, 2H), 1.64-1.44(m, 7H).

### Example 25: 2-(4-((1R,5S)-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-4,6-dichloro-3-fluoroaniline

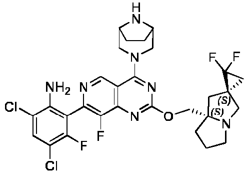

LC-MS: m/z 638 (M+H)⁺. ¹H NMR (400MHz, DMSO-*d*₆) δ 10.18 (s, 1 H), 9.59 (s, 1 H), 9.36 (s, 1 H), 9.25 (s, 1 H), 7.71 (d, *J*=7.56Hz, 1 H), 5.76(s, 2 H), 4.67(m ,4 H), 4.21(m, 2 H),3.64(m, 2 H), 3.13 (m, 1 H), 2.47 (m, 1 H), 2.28(m,4 H), 1.88 (m,9 H).

### Example 26: 3-(4-((1R,5S-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-2-fluoro-5-methyl-4-(trifluoromethyl)aniline

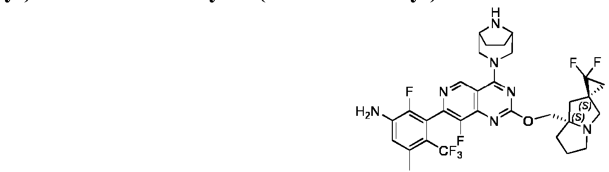

LC-MS: m/z 652 (M+H)⁺. ¹H NMR (400MHz, DMSO-*d*₆) δ 9.09 (s, 1 H),7.31 (d, *J*=11.76Hz, 1 H), 5.86(s, 1 H), 4.42(d, *J*=11.40Hz, 2 H), 4.19(d, *J*=3.24Hz, 2 H), 3.61 (m, 4 H), 3.11 (m, 1 H), 3.02(m,1 H), 2.71 (d, *J*=11.84Hz, 1 H), 2.55 (m, 1 H), 2.17(s, 3 H), 2.09 (m,1 H), 2.00 (m,1 H), 1.88 (d, *J*=13.32Hz, 1 H), 1.64 (m, 10 H).

### Example 27: 3-(4-((1R,5S-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-5-chloro-4-(trifluoromethyl)aniline

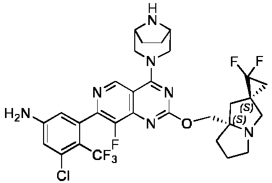

LC-MS: m/z 654 (M+H)⁺. ¹H NMR (400MHz, DMSO-*d*₆) δ 9.06 (s, 1 H), 6.89 (s, 1 H), 6.48(s, 1 H), 6.34(s, 1 H), 4.39(d, *J*=12.08Hz, 2 H), 4.20(m, 2 H), 3.59 (m, 4 H), 3.10 (m, 1 H), 3.02(m,1 H), 2.71 (d, *J*=11.88Hz, 1 H), 2.56 (m, 1 H), 2.07 (m,1 H), 1.99 (m,1 H), 1.88 (d, *J*=13.32Hz, 1 H), 1.62 (m, 10 H).

### Example 28: 4-(4-(3,9-diazabicyclo[4.2.1]nonan-3-yl)-2-(((1S,7a'S)-2,2-difluorodihydro-1'H,3'H-spiro[c yclopropan-1,2'-pyrrolizin]-7a'(5'H)-yl)methoxy)-8-fluoropyridino[4,3-d]pyrimidin-7-yl)-5 -ethynyl-6-fluoronaphthalen-2-ol

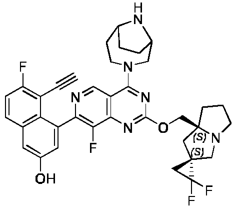

LC-MS: m/z 659 (M+H)⁺. ¹H NMR (400 MHz, DMSO-*d₆*) 610.20(s, 1 H), 9.09(d, *J* = 16Hz, 1 H), 8.00-7.95 (m, 1H), 7.47(t, *J* = 8 Hz, 1H), 7.40(s, 1H),7.22-7.19(m, 1H), 4.71(d, *J*=12Hz,1H), 4.42(d, *J*=12Hz,1H),4.25-4.15(m, 2H), 4.09-3.99(m,2H), 3.92-3.82(m,2H), 3.73-3.67(m,2H), 3.13-3.08(m, 1H), 3.04-3.00(m, 1H), 2.74 (d, *J* = 12Hz, 1H), 2.57-2.55 (m, 1H), 2.11-1.95 (m, 3H),1.92-1.89(m, 3H), 1.84-1.72(m,3H),1.65-1.45(m,5H).

### Example 29: 4-(4-(3-amino-3-methylpiperidin-1-yl)-2-(((1S,7a'S)-2,2-difluorodihydro-1'H,3'H-spiro[cyc lopropan-1,2'-pyrrolizin]-7a'(5'H)-yl)methoxy)-8-fluoropyridino[4,3-d]pyrimidin-7-yl)-5-e thynyl-6-fluoronaphthalen-2-ol

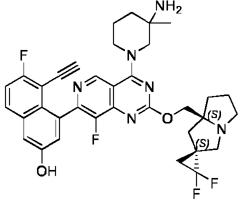

LC-MS: m/z 647 (M+H)⁺. ¹H NMR (400 MHz, DMSO-*d₆*) δ10.16(s, 2 H), 9.25(d, *J =* 56Hz, 1 H), 7.99-7.96 (m, 1H), 7.46(t, *J* = 8 Hz, 1H), 7.40(d, *J* = 4Hz, 1H),7.22-7.20(m, 1H),4.24-4.16(m, 2H), 4.10(m,1H), 4.03-4.00(d, *J*=12Hz,1H), 3.95 (s, 1H), 3.89(d, *J* = 12Hz, 1H),3.73-3.65 (m, 1H), 3.60-3.47(m, 2H),3.13-2.99(m, 2H), 2.73 (d, *J* = 12Hz, 1H), 2.56-2.54 (m, 1H), 2.10-2.05 (m, 1H), 2.02-1.97 (m, 2H),1.92(s, 1H), 1.89(s, 1H), 1.83-1.74(m,3H),1.64-1.44(m, 5H),1.10(d, *J =* 4Hz,3H).

### Example 30 3-(4-((1R,5S-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-methoxypyridino[4,3-d]pyrimi din-7-yl)-2-fluoro-5-methyl-4-(trifluoromethyl)aniline

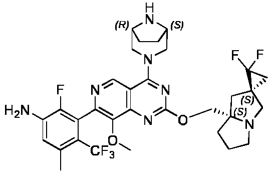

LC-MS: m/z 664 (M+H)⁺. ¹H NMR (400 MHz, DMSO-*d₆*) δ 8.96 (s, 1 H) , 7.18 (d, J=11.68Hz, 1H), 5.67(s, 2H), 4.35(d, J=12.40Hz, 2 H), 4.20(m, 2H), 3.85(s, 3 H), 3.53 (m, 4 H), 3.11 (m, 1 H), 3.02(m,1 H), 2.71 (m, 1 H), 1.73 (m, 17 H).

### Example 31: 3-(4-((1R,5S)-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-methoxypyridino[4,3-d]pyrimi din-7-yl)-5-chloro-4-(trifluoromethyl)aniline

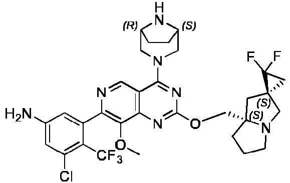

LC-MS: m/z 666 (M+H)⁺. ¹H NMR (400 MHz, DMSO-*d₆*) δ 8.92 (s, 1 H) , 6.82 (d, *J*=2.24Hz, 1H), 6.36 (d, *J*=2.28Hz, 1H), 6.19(s, 2H), 4.36(m, 2H), 4.20(m, 2H), 3.89(s, 3 H), 3.57 (m, 4 H), 3.09 (m, 1 H), 3.00(m,1 H), 2.70 (m, 1 H), 2.55 (m, 1 H), 1.64 (m, 13 H).

### Example 32: 4-(4-((1R,5S)-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-2-amino-6-fluorobenzo[b]thiophen-3-carbonitrile

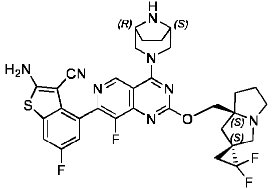

LC-MS: m/z 651 (M+H)⁺. ¹H NMR (400 MHz, DMSO-*d₆*) δ δ 9.07(s,1 H), 7.82-7.80 (m, 2H), 7.28-7.25 (m, 1H), 4.42(d, *J =* 12 Hz,2H), 4.22-4.16(m, 2H), 3.62(d, *J =* 12 Hz,2H), 3.51(s,2H), 3.12-3.08(m, 1H), 3.04-2.99(m, 1H), 2.73(d, *J* = 12 Hz, 1H), 2.56-2.54 (m, 1H), 2.11-2.06(m,1H), 2.02-1.96(m, 1H), 1.91-1.71(m, 4H), 1.63-1.45(m, 7H).

### Example 33: (1S,7a'S)-7a'-(((4-((1R,5S)-3,8-diazabicyclo[3.2.1]octan-3-yl)-8-fluoro-7-(5-(trifluoromethy 1)-1H-indazol-4-yl)pyridino[4,3-d]pyrimidin-2-yl)oxy)methyl)-2,2-bifluorotetrahydro-1'H, 3'H-spiro[cyclopropan-1,2'-pyrrolizin]

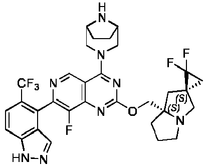

LC-MS: m/z 645 (M+H)⁺. ¹H NMR (400 MHz, DMSO-*d₆*) δ 13.71 (s, 1 H), 9.17 (s, 1 H), 7.86 (m, 3 H), 4.46(m, 2H), 4.21(m, 2H), 3.63 (m, 4 H), 3.10 (m, 1 H), 3.02(m,1 H), 2.71 (m, 1 H), 2.55 (m, 1 H), 2.08 (m,1 H), 2.00 (m,1 H), 1.89 (m, 1 H), 1.67 (m, 10 H).

### Example 34: 3-(4-((1R,5S-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-5-chloro-2-fluoro-4-(trifluoromethyl)aniline

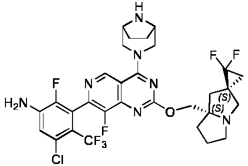

LC-MS: m/z 672 (M+H)⁺. ¹H NMR (400 MHz, DMSO-*d₆*) δ 9.11(s, 1 H), 7.09(d, *J*=7.92Hz, 1 H), 6.48(s,2 H), 4.39(m, 2 H), 4.20(m, 2 H), 3.61 (m, 4 H), 3.10 (m, 1 H), 3.00(m,1 H), 2.70 (m, 1 H), 2.54 (m, 1 H), 2.06 (m,1 H), 1.99 (m,1 H), 1.88 (m, 1 H), 1.64 (m, 10 H).

### Example 35: 3-(4-((1R,5S-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-2,5-dichloro-4-(trifluoromethyl)aniline

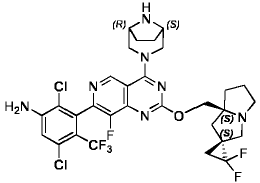

LC-MS: m/z 688 (M+H)⁺. ¹H NMR (400 MHz, DMSO-*d₆*) δ 9.12 (s, 1 H), 7.15(s, 1 H), 6.64(s,2 H), 4.39(m, 2 H), 4.20(m, 2 H), 3.59 (m, 4 H), 3.10 (m, 1 H), 3.00(m,1 H), 2.70 (m, 1 H), 2.54 (m, 1 H), 2.06 (m,1 H), 1.97 (m,1 H), 1.88 (m, 1 H), 1.64 (m, 10 H).

### Example 36: 3-(4-((1R,5S-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-2-chloro-5-fluoro-4-(trifluoromethyl)aniline

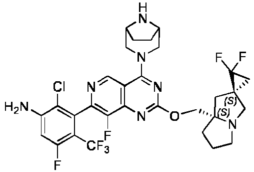

LC-MS: m/z 672 (M+H)⁺. ¹H NMR (400 MHz, DMSO-*d₆*) δ 9.13 (s, 1 H), 6.86 (d, *J*=13.68Hz, 1 H), 6.68(s,2 H), 4.38(d, *J*=12.32Hz, 2 H), 4.19(m, 2 H), 3.58 (m, 4 H), 3.10 (m, 1 H), 3.00(m,1 H), 2.71 (m, 1 H), 2.53 (m, 1 H), 2.07 (m,1 H), 2.00 (m,1 H), 1.89 (m, 1 H), 1.65 (m, 10 H).

### Example 37: 3-(4-((1R,5S)-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-2,4-dichloro-5-fluoroaniline

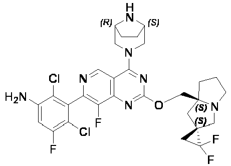

LC-MS: m/z 638 (M+H)⁺. ¹H NMR (400 MHz, DMSO-*d₆*) δ 9.18(s, 1 H), 6.94(d, *J* = 12 Hz,1H), 6.05 (s, 2H), 4.49(d, *J =* 12 Hz, 2H), 4.25-4.18(m, 2H), 3.78(s, 2H), 3.71 (d, *J =* 16 Hz, 2H), 3.12-3.08(m, 1H), 3.04-2.99(m, 1H), 2.74(d, *J* = 12 Hz, 1H), 2.57-2.55 (m, 1H), 2.10-2.05(m, 1H), 2.02-1.95(m, 1H), 1.92 (d, *J=* 16 Hz, 1H), 1.84-1.70(m, 6H), 1.65-1.44(m, 4H).

### Example 38: N-(3-(4-((1R,5S)-3,8-diazabicyclo[3.2.1]octan-3-yl)-2-(((1S,7a'S)-2,2-difluorodihydro-1'H,3 'H-spiro[cyclopropan-1,2'-pyrrolizin]-7a'(5'H)-yl)methoxy)-8-fluoropyridino[4,3-d]pyrimi din-7-yl)-5-chloro-4-(trifluoromethyl)phenyl)acetamide

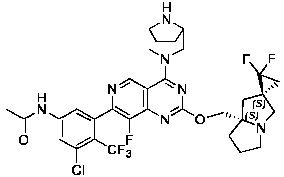

LC-MS: m/z 696 (M+H)⁺. ¹H NMR (400 MHz, DMSO-*d₆*) δ10.50(s, 1H), 9.09(s,1 H), 8.03 (s, 1H), 7.70 (s, 1H), 4.43-4.40(m, 2H), 4.22-4.15(m, 2H), 3.58(m, 4H), 3.12-3.07(m, 1H), 3.03-2.98(m, 1H), 2.73(d, *J* = 12 Hz, 1H), 2.55-2.53 (m, 1H), 2.10(s,3H), 2.08-1.95(m, 2H), 1.91-1.70(m, 3H),1.63-1.44(m, 8H).

### Example 39: N-(3-(4-((1R,5S)-3,8-diazabicyclo[3.2.1]octan-3-yl)-2-(((1S,7a'S)-2,2-difluorodihydro-1'H,3 'H-spiro[cyclopropan-1,2'-pyrrolizin]-7a'(5'H)-yl)methoxy)-8-fluoropyridino[4,3-d]pyrimi din-7-yl)-5-chloro-4-(trifluoromethyl)phenyl)methanesulfonamide

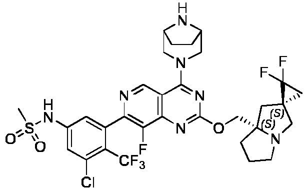

LC-MS: m/z 732 (M+H)⁺. ¹H NMR (400 MHz, DMSO-*d₆*) δ 9.12(s, 1 H), 8.03 (s, 1H),7.33 (s, 1H), 6.99 (s, 1H), 4.51(t, *J =* 12 Hz,2H), 4.25-4.19(m, 2H), 3.85(s,2H), 3.75-3.65(m,2H), 3.14-3.09(m, 1H), 3.05-3.01(m, 1H), 2.99(s,3H), 2.75(d, *J =* 12 Hz, 1H), 2.58-2.55 (m, 1H), 2.11-2.05(m,1H), 2.03-1.97(m, 1H), 1.92 (d, *J =* 12 Hz,1H), 1.83-1.78(m, 6H), 1.65-1.45(m, 4H).

### Example 40: 3-(4-((1R,5S)-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl))-5-chloro-4-(trifluoromethyl)phenol

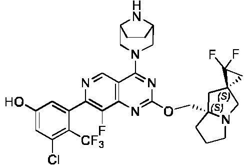

LC-MS: m/z 655 (M+H)⁺. ¹H NMR (400 MHz, DMSO-*d₆*) δ 9.08 (s, 1 H), 7.20 (d, *J*=2.44Hz, 1H), 6.80 (d, *J*=2.40Hz, 1H), 4.40(d, *J*=12.04Hz, 2H), 4.20(m, 2 H), 3.59(m, 4 H), 3.10 (m, 1 H), 3.01(m,1 H), 2.71 (m, 1 H), 2.55 (m, 1 H), 2.07 (m, 1 H), 1.99 (m, 1 H), 1.87 (m, 1 H), 1.64 (m, 10 H).

### Example 41: 5-(4-((1R,5S)-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-3-chloro-2-fluoro-4-(trifluoromethyl)aniline

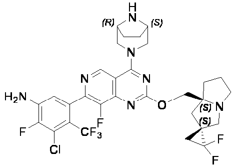

LC-MS: m/z 672 (M+H)⁺. ¹H NMR (400 MHz, DMSO-*d₆*) δ 9.05 (s, 1 H) , 6.69 (d, *J*=8.28Hz, 1 H), 6.49(s,2 H), 4.38(d, *J*=12.44Hz, 2 H), 4.18(m, 2 H), 3.57 (m, 4 H), 3.09 (m, 1 H), 3.00(m,1 H), 2.70 (m, 1 H), 2.53 (m, 1 H), 2.06 (m,1 H), 1.99 (m,1 H), 1.87 (m, 1 H), 1.61 (m, 10 H).

### Example 42: 5-(4-((1R,5S)-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-2-fluoro-3-methyl-4-(trifluoromethyl)aniline

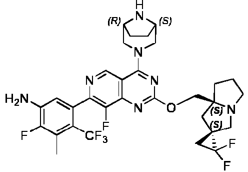

LC-MS: m/z 652 (M+H)⁺. ¹H NMR (400 MHz, DMSO-*d₆*) δ 9.04(s,1 H), 6.57 (d, *J =* 8 Hz,1H),6.02 (s, 2H), 4.43(d, *J* = 12 Hz,2H), 4.21-4.15(m, 2H), 3.62-3.59(m, 4H), 3.12-3.07(m, 1H), 3.03-2.98(m, 1H), 2.73(d, *J* = 12 Hz, 1H), 2.56-2.54 (m, 1H), 2.34 (s, 3H), 2.09-2.04(m,1H), 2.01-1.95(m, 1H), 1.91 (d, *J =* 16 Hz,1H), 1.83-1.70(m, 3H), 1.65-1.44(m, 7H).

### Example 43: 3-(4-((1R,5S-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-5-amino-2-(trifluoromethyl)benzonitrile

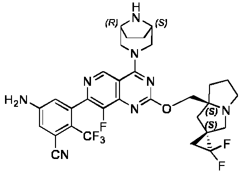

LC-MS: m/z 645 (M+H)⁺. ¹H NMR (400 MHz, DMSO-*d₆*) δ 9.07 (s, 1 H),7.22 (d, *J*=2.40Hz, 1 H), 6.83 (d, *J*=2.32Hz, 1 H), 6.61(s,2 H), 4.39(m, 2 H), 4.20(m, 2 H), 3.58 (m, 4 H), 3.10 (m, 1 H), 3.00(m,1 H), 2.71 (m, 1 H), 2.55 (m, 1 H), 2.07 (m,1 H), 2.00 (m,1 H), 1.88 (m, 1H), 1.64 (m, 10 H).

### Example 44: 3-(4-((1R,5S-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-5-fluoro-4-(trifluoromethyl)aniline

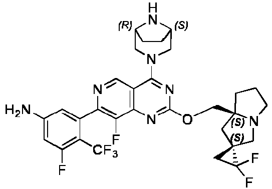

LC-MS: m/z 638 (M+H)⁺. ¹H NMR (400 MHz, DMSO-*d₆*) δ 9.06(s,1 H), 6.60 (d, *J =* 16 Hz,1H), 6.39 (d, *J =* 4 Hz,3H), 4.42(d, *J =* 12 Hz,2H), 4.22-4.16(m, 2H), 3.62(s,1H), 3.59(s, 3H), 3.13-3.08(m, 1H), 3.04-2.99(m, 1H), 2.74(d, *J* = 12 Hz, 1H), 2.57-2.54 (m, 1H), 2.10-2.05(m,1H), 2.02-1.95(m, 1H), 1.92 (d, *J =* 16 Hz,1H), 1.83-1.71(m, 3H), 1.63-1.44(m, 7H).

### Example 45: 3-(4-((1R,5S-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-4-chloro-5-(trifluoromethyl)aniline

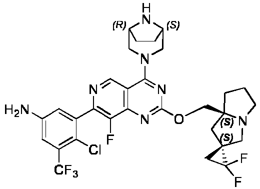

LC-MS: m/z 654 (M+H)⁺. ¹H NMR (400 MHz, DMSO-*d₆*) δ 9.11(s,1 H), 7.17 (d, *J =* 4 Hz,1H), 6.91 (d, *J* = 4 Hz,1H),5.95 (s, 2H), 4.44(d, *J* = 12 Hz,2H), 4.24-4.18(m, 2H), 3.62(s, 1H), 3.59(s, 3H), 3.13-3.08(m, 1H), 3.04-3.00(m, 1H), 2.74(d, *J* = 12 Hz, 1H), 2.58-2.54 (m, 1H), 2.11-2.05(m,1H), 2.02-1.96(m, 1H), 1.92 (d, *J* = 12 Hz,1H), 1.84-1.73(m, 3H), 1.67-1.45(m, 7H).

### Example 46: 3-(4-((1R,5S-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-5-chloro-4-fluoroaniline

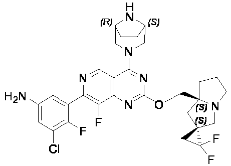

LC-MS: m/z 604 (M+H)⁺. ¹H NMR (400 MHz, DMSO-*d₆*) δ 9.10(s,1 H), 6.85-6.82 (m, 1H), 6.78-6.75 (m, 1H), 5.47 (s, 2H), 4.42(d, *J* = 12 Hz,2H), 4.23-4.17(m, 2H), 3.60(d, *J* = 12 Hz,2H), 3.53(s,2H), 3.13-3.08(m, 1H), 3.04-3.00(m, 1H), 2.74(d, *J =* 12 Hz, 1H), 2.57-2.55 (m, 1H), 2.11-2.06(m,1H), 2.02-1.97(m, 1H), 1.92 (d, J*=*12 Hz,1H), 1.85-1.71(m, 3H), 1.64-1.45(m, 7H).

### Example 47: 3-(4-((1R,5S-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-4,5-dichloroaniline

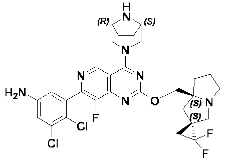

LC-MS: m/z 620 (M+H)⁺. ¹H NMR (400 MHz, DMSO-*d₆*) δ 9.08(s, 1H), 6.89(d, *J*=2.60Hz, 2 H), 6.65(d, *J*=2.68Hz, 2 H), 5.74 (s, 2 H), 4.38(d, *J*=11.84Hz, 2 H), 4.18(m, 2 H), 3.54 (m, 4 H), 3.10 (m, 1 H), 3.00(m,1 H), 2.70 (m, 1 H), 2.53 (m, 1 H), 2.06 (m,1 H), 1.98 (m,1 H), 1.87 (m, 1 H), 1.58 (m, 10 H).

### Example 48: 3-(4-((1R,5S-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-5-amino-2-chlorobenzonitrile

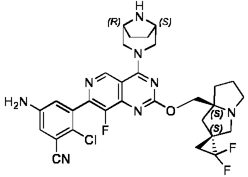

LC-MS: m/z 611 (M+H)⁺. ¹H NMR (400 MHz, DMSO-*d₆*) δ 9.11 (s, 1 H), 7.13 (d, *J*=2.72Hz, 1H), 7.01 (d, *J*=2.76Hz, 1H), 6.01(s, 2 H), 4.40(d, *J*=12.00Hz, 2H), 4.20(m, 2 H), 3.60(m, 4 H), 3.10 (m, 1 H), 3.01(m,1 H), 2.70 (m, 1 H), 2.54 (m, 1 H), 2.09 (m, 1 H), 1.99 (m, 1 H), 1.88 (m, 1 H), 1.61 (m, 10 H).

### Example 49: 2-(4-((1R,5S)-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-4-amino-6-chlorobenzonitrile

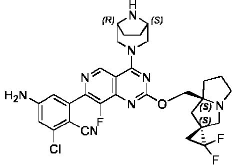

LC-MS: m/z 611 (M+H)⁺. ¹H NMR (400 MHz, DMSO-*d₆*) δ 9.11(s, 1 H), 6.86 (d, *J* = 4 Hz, 1H), 6.79 (d, *J =* 4 Hz,1H), 6.71 (s, 2H), 4.42(d, *J =* 12 Hz,2H), 4.23-4.17(m, 2H), 3.62(d, *J* = 12 Hz,1H), 3.55(s, 3H), 3.12-3.07(m, 1H), 3.03-2.99(m, 1H), 2.73(d, *J* = 12 Hz, 1H), 2.56-2.54 (m, 1H), 2.10-2.05(m,1H), 2.02-1.96(m, 1H), 1.91 (d, *J =* 12 Hz,1H), 1.83-1.70(m, 3H), 1.65-1.44(m, 7H).

### Example 50: 2-(4-((1R,5S)-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-4-amino-6-(trifluoromethyl)benzonitrile

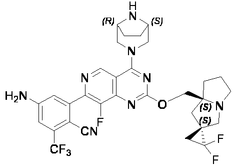

LC-MS: m/z 645 (M+H)⁺. ¹H NMR (400 MHz, DMSO-*d₆*) δ 9.14(s,1 H), 7.17 (d, *J* = 4 Hz,1H), 7.02 (d, *J* = 4 Hz,1H), 6.95 (s, 2H), 4.45(d, *J* = 12 Hz,2H), 4.25-4.19(m, 2H), 3.65(s,1H), 3.62(s, 3H), 3.13-3.09(m, 1H), 3.05-3.00(m, 1H), 2.74(d, *J* = 12 Hz, 1H), 2.57-2.55 (m, 1H), 2.13-2.05(m,1H), 2.03-1.97(m, 1H), 1.92 (d, *J* = 12 Hz,1H), 1.83-1.76(m, 3H), 1.65-1.45(m, 7H).

### Example 51: 2-(4-((1R,5S)-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-6-amino-3-chlorobenzonitrile

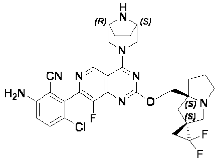

LC-MS: m/z 611 (M+H)⁺. ¹H NMR (400 MHz, DMSO-*d₆*) δ 9.17 (s, 1 H),7.51 (d, *J*=9.04Hz, 1 H), 6.95 (d, *J*=9.04Hz, 1 H), 6.45(s,2 H), 4.50(m, 1 H), 4.34(m, 1 H), 4.20(m, 2 H), 3.64 (m, 4 H), 3.09 (m, 1 H), 3.01(m,1 H), 2.70 (m, 1 H), 2.56 (m, 1 H), 2.07 (m,1 H), 1.99 (m,1 H), 1.91 (m, 1 H), 1.67 (m, 10 H).

### Example 52: 5-(4-((1R,5S)-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-6-(trifluoromethyl)pyridin-3-amine

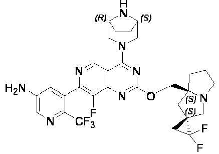

LC-MS: m/z 621 (M+H)⁺. ¹H NMR (400 MHz, DMSO-*d₆*) δ 9.12(s,1 H), 8.13 (d, *J* = 4 Hz,1H), 6.97 (d, *J* = 4 Hz,1H),6.31 (s, 2H), 4.51(d, *J* = 12 Hz,2H), 4.25-4.19(m, 2H), 3.83(s, 2H), 3.73(d, *J =* 12 Hz, 2H), 3.13-3.09(m, 1H), 3.04-3.00(m, 1H), 2.75(d, *J =* 12 Hz, 1H), 2.58-2.55 (m, 1H), 2.11-2.06(m,1H), 2.02-1.96(m, 1H), 1.93 (d, *J =* 12 Hz,1H), 1.84-1.74(m, 3H), 1.65-1.45(m, 7H).

### Example 53: 5-(4-((1R,5S)-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-4-(trifluoromethyl)pyridin-3-amine

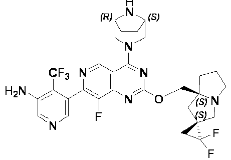

LC-MS: m/z 621 (M+H)⁺. ¹H NMR (400 MHz, DMSO-*d₆*) δ 9.09 (s, 1 H), 8.38 (s, 1 H), 7.14 (s, 1 H), 6.24 (s, 2 H), 4.42(d, *J*=12.24Hz, 2H), 4.19(m, 2 H), 3.62(m, 4 H), 3.10 (m, 1 H), 3.01(m,1 H), 2.71 (m, 1 H), 2.55 (m, 1 H), 2.07 (m, 1 H), 1.98 (m, 1 H), 1.89 (m, 1 H), 1.64 (m, 10 H).

### Example 54: 5-(4-((1R,5S)-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-4-methyl-6-(trifluoromethyl)pyridin-3-amine

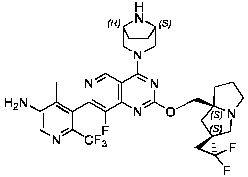

LC-MS: m/z 635 (M+H)⁺. ¹H NMR (400 MHz, DMSO-*d₆*) δ 9.12(s, 1 H), 8.10(s, 1 H), 6.06 (s,2H), 4.41(d, *J =* 12 Hz,2H), 4.23-4.15(m, 2H), 3.63(d, *J =* 12 Hz,1H), 3.57(s, 3H), 3.12-3.07(m, 1H), 3.03-2.98(m, 1H), 2.73(d, *J* = 12 Hz, 1H), 2.56-2.54 (m, 1H), 2.09-2.05(m,1H), 2.01-1.95(m, 1H), 1.91 (d, *J* = 12 Hz,1H), 1.83-1.76(m, 6H), 1.64-1.43(m, 7H).

### Example 55: 4-(4-((1R,5S)-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-6-methyl-5-(trifluoromethyl)pyridin-2-amine

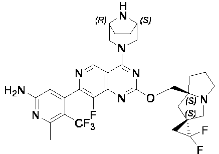

LC-MS: m/z 635 (M+H)⁺. ¹H NMR (400 MHz, DMSO-*d₆*) δ 9.10 (s, 1 H) ,6.69 (s, 2 H), 6.26(s,1 H), 4.51(d, J=13.00Hz, 2 H), 4.21(m, 2 H), 3.94 (m, 2 H), 3.79(d, J=13.16Hz, 2 H), 3.10 (m, 1 H), 3.03(m,1 H), 2.72 (m, 1 H), 2.54 (m, 1 H), 2.49 (m, 3 H), 2.08 (m,1 H), 1.99 (m,1 H), 1.89 (m, 1 H), 1.65 (m, 10 H).

### Example 56: 4-(4-((1R,5S)-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy)-8-fluoropyridino[4,3-d]pyrimidin -7-yl)-2-chloro-3-(trifluoromethyl)aniline

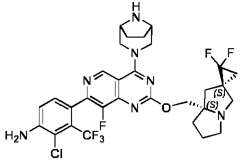

LC-MS: m/z 654 (M+H)⁺. ¹H NMR (400 MHz, DMSO-*d₆*) δ 9.12(s,1 H), 7.47 (d, *J* = 8Hz,1H), 7.14 (d, *J* = 8 Hz,1H),6.09 (s, 2H), 4.48(d, *J* = 12 Hz,2H), 4.24-4.19(m, 2H), 3.69-3.65(m, 4H), 3.13-3.08(m, 1H), 3.04-2.99(m, 1H), 2.74(d, *J* = 12 Hz, 1H), 2.57-2.54(m,1H), 2.12-2.05(m,1H), 2.01-1.97(m, 1H), 1.92 (d, *J* = 16 Hz,1H), 1.83-1.44(m, 10H).

**The following examples were synthesized according to the method of Example 1 using Intermediate D-1-11A-1 as the starting material:**

### Example D01: 4-(4-((1R,5S)-3,8-diazabicyclo[3.2.1]octan-3-yl)-2-(((1S,7a'S)-2,2-difluorodihydro-1'H ,3'H-spiro[cyclopropan-1,2'-pyrrolizin]-7a'(5'H)-yl-5',5'-d2)methoxy-d2)-8-fluoropyr idino[4,3-d]pyrimidin-7-yl)-5-ethynyl-6-fluoronaphthalen-2-ol

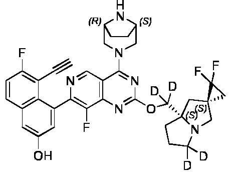

LC-MS: m/z 649 (M+H)⁺. ¹H NMR (400MHz, DMSO-*d*₆) δ 10.15 (s, 1H), 9.05 (s, 1H) , 7.98 (dd,*J*=5.92, 9.24Hz,1H), 7.47 (t,*J*=9.04Hz, 1H), 7.40 (d,*J*=2.60Hz, 1H), 7.18 (d,*J*=2.64Hz, 1 H), 4.47 (d,*J*=12.36Hz, 1 H), 4.31(m, 1 H), 3.94 (s, 1 H), 3.63(m,4H), 3.29 (m, 1 H), 3.10(m, 1 H), 2.71 (d,J=11.72Hz, 1 H), 2.07 (m, 1 H), 1.99 (m, 1 H) , 1.89 (m, 1 H), 1.67 (m, 1 H).

**The following examples were synthesized according to the method of Example 1 using Intermediate D-1-11A-2 as the starting material:**

### Example D02: 4-(4-((1R,5S)-3,8-diazabicyclo[3.2.1]octan-3-yl)-2-(((1R,7a'R)-2,2-difluorodihydro-1' H,3'H-spiro[cyclopropan-1,2'-pyrrolizin]-7a'(5'H)-yl-5',5'-d2)methoxy-d2)-8-fluorop yridino[4,3-d]pyrimidin-7-yl)-5-ethynyl-6-fluoronaphthalen-2-ol

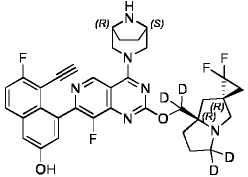

LC-MS: m/z 649 (M+H)⁺. ¹H NMR (400MHz, DMSO-*d*₆) δ 10.15 (s, 1 H), 9.05 (s, 1 H) , 7.98 (dd,*J*=5.96, 9.20Hz,1 H), 7.47 (t,*J*=9.04Hz, 1 H), 7.40 (d,*J*=2.60Hz, 1 H), 7.18 (d,*J*=2.56Hz, 1 H), 4.47 (d,*J*=12.20Hz, 1 H), 4.32(m, 1 H), 3.94 (s, 1 H), 3.63(m,4H), 3.29 (m, 1 H), 3.10(m, 1 H), 2.71 (d,*J*=11.72Hz, 1 H), 2.07 (m, 1 H), 1.99 (m, 1 H) , 1.89 (m, 1H), 1.67 (m, 1H).

**The following examples were synthesized according to the method of Example 1 using Intermediate D-1-11A-1 as the starting material: Example D03: 3-(4-((1R,5S-3,8-diazabicyclo[3.2.1]octan-3-yl)-2-(((1S,7a'S)-2,2-difluorodihydro-1'H,3'H-spiro[cyclopropan-1,2'-pyrrolizin]-7a'(5'H)-yl)methoxy-d2)-8-fluoropyridino[4,3-d]pyrimi din-7-yl)-5-chloro-4-(trifluoromethyl)aniline**

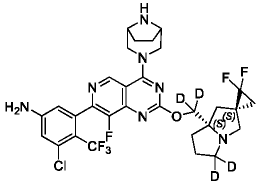

LC-MS: m/z 658 (M+H)⁺. ¹H NMR (400MHz, DMSO-*d*₆) δ 9.07(s,1 H), 6.90 (d, *J =* 4 Hz,1H), 6.48 (d, *J* = 4 Hz,1H),6.34 (s, 2H), 4.46(d, *J* = 12 Hz,2H), 3.69-3.63(m, 4H), 3.13-3.08(m, 1H), 2.74(d, *J* = 12 Hz, 1H), 2.10-2.05(m,1H), 2.02-1.95(m, 1H), 1.92 (d, *J =* 16 Hz,1H), 1.84-1.73(m, 3H), 1.69-1.45(m, 7H).

**The following examples were synthesized according to the method of Example 1 using Intermediate 1-17 as the starting material:**

### Example E01: 4-(4-((1R,5S)-3,8-diazabicyclo[3.2.1]octan-3-yl)-8-fluoro-2-((1-methyltetrahydro-1H-spiro[ cyclopenta[b]pyrrol-2,1'-cyclopropan]-3a(3H)-yl)methoxy)pyridino[4,3-d]pyrimidin-7-yl)-5-ethynyl-6-fluoronaphthalen-2-ol

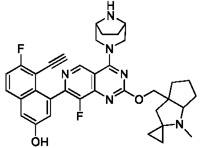

LC-MS: m/z 623 (M+H)⁺.

### Example P01: Preparation of ((1R,5S)-3-(2-(((1S,7a'S)-2,2-difluorodihydro-1'H,3'H-spiro[cyclopropan-1,2'-pyrrolizin]-7 a'(5'H)-yl)methoxy)-7-(8-ethynyl-7-fluoro-3-hydroxynaphthalen-1-yl)-8-fluoropyridino[4, 3-d]pyrimidin-4-yl)-3,8-diazabicyclo[3.2.1]octan-8-yl)methylisobutyrate trifluoroacetate

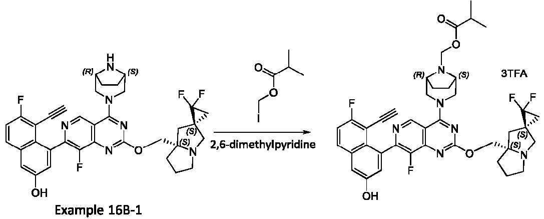

**Example 16B-1** (100mg, 0.155mmol) was dissolved in THF(5mL), and then added with 2,6-dimethylpyridine(83mg, 0.775mmol) and iodomethyl isobutyrate (189mg, 0.775mmol) sequentially. After addition, the mixture was stirred at room temperature for 16 h, then added with acetonitrile to dissolve and then separated by preparative HPLC to obtain the target product (28mg).

LC-MS: m/z 745 (M+H)⁺. ¹H NMR (400MHz, DMSO-*d*₆) δ 10.28 (s, 1 H), 9.45(d, J=9.64Hz, 1 H), 9.19 (m, 2 H), 8.00 (dd, *J*=5.96,9.20Hz,1 H), 7.49(t, *J*=9.04Hz, 1 H), 7.43(d, *J*=2.52Hz, 1 H), 7.18 (d, *J*=2.60Hz, 1 H), 5.59 (m, 2 H), 4.95 (s, 2 H), 4.72 (m, 1 H), 4.56 (m, 1 H), 4.25 (s, 2 H), 4. 12(m, 1 H), 3.88 (m, 5 H), 3.73 (m,2 H), 2.70 (m, 2 H), 2.40 (m, 2H), 2.22 (m, 3 H), 1.98 (m, 7 H), 1.15 (m, 6H).

**The following compounds were synthesized according to the same synthesis method of example P01:**

### Example P02: isopropyl (1R,5S)-3-(2-(((1S,7a'S)-2,2-difluorodihydro-1'H,3'H-spiro[cyclopropan-1,2'-pyrrolizin]-7 a'(5'H)-yl)methoxy)-7-(8-ethynyl-7-fluoro-3-hydroxylnaphthalen-1-yl)-8-fluoropyridino[4, 3-d]pyrimidin-4-yl)-3,8-diazabicyclo[3.2.1]octan-8-formate

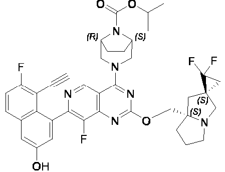

LC-MS: m/z 731 (M+H)⁺. ¹H NMR (400MHz, DMSO-*d*₆) δ 9.07 (s, 1 H), 7.97 (dd, *J*=5.96, 9.24Hz, 1 H),7.46(t, *J*=9.04Hz, 1 H), 7.39(d, *J*=2.56Hz, 1 H), 7.18 (d, *J*=2.48Hz, 1 H), 4.87 (m, 1 H), 4.56 (m, 1 H), 4.41 (m, 3 H), 4.20 (m, 2 H), 3.92 (s, 1H), 3.64(m, 2 H), 3.10 (m, 1 H), 3.01 (m,1 H), 2.70 (d, J=11.84Hz, 1 H),2.54 (m, 1H), 2.06 (m, 1 H), 1.99 (m, 1 H), 1.81 (m, 7 H), 1.61 (m, 3 H), 1.24 (d, J=6.48Hz, 6H).

### Example P03: ((1R,5S)-3-(2-(((1S,7a'S)-2,2-difluorodihydro-1'H,3'H-spiro[cyclopropan-1,2'-pyrrolizin]-7 a'(5'H)-yl)methoxy)-7-(8-ethynyl-7-fluoro-3-hydroxynaphthalen-1-yl)-8-fluoropyridino[4, 3-d]pyrimidin-4-yl)-3,8-diazabicyclo[3.2.1]octan-8-yl)methylisopropyl carbonate

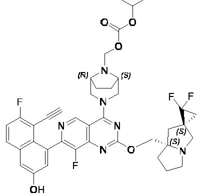

LC-MS: m/z 761 (M+H)⁺. ¹H NMR (400 MHz, DMSO-*d₆*) δ10.28(s, 1 H), 9.57(s, 1 H), 9.29(s, 1 H), 9.19(s, 1 H), 8.01(t, *J* = 8Hz,1 H), 7.52-7.44(m, 2H), 7.19(s, 1 H), 5.65-5.56(m, 2H), 4.95(s, 2H),4.87-4.74(m,2H), 4.60 (d, *J =* 12Hz, 1H), 4.25(s, 2H), 4.18 (d, *J* = 12Hz, 1H),3.93-3.86(m, 5H), 3.77-3.74 (m, 2H), 2.70-2.66(m, 2 H), 2.60(s,1H), 2.43-2.34(m,2H), 2.25-2.18 (m,3H), 1.99-1.90(m, 6H), 1.25-1.24 (m, 6H).

### Example P04 and Example P05: Preparation of 1-(isobutyryloxy)ethyl(1R,5S)-3-(2-(((1S,7a'S)-2,2-difluorodihydro-1'H,3'H-spiro[cyclopro pan-1,2'-pyrrolizin]-7a'(5'H)-yl)methoxy)-7-(8-ethynyl-7-fluoro-3-(((1-(isobutyryloxy)etho xy)carbonyl)oxy)naphthalen-1-yl)-8-fluoropyridino[4,3-d]pyrimidin-4-yl)-3,8-diazabicyclo [3.2.1]octan-8-formate and 1-(isobutyryloxy)ethyl(1R,5S)-3-(2-(((1S,7a'S)-2,2-difluorodihydro-1'H,3'H-spiro[cyclopro pan-1,2'-pyrrolizin]-7a'(5'H)-yl)methoxy)-7-(8-ethynyl-7-fluoro-3-hydroxynaphthalen-1-yl )-8-fluoropyridino[4,3-d]pyrimidin-4-yl)-3,8-diazabicyclo[3.2.1]octan-8-formate

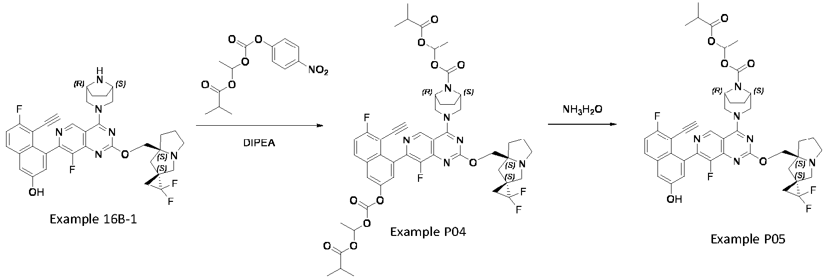

### Step 1: Preparation of 1-(isobutyryloxy)ethyl(1R,5S)-3-(2-(((1S,7a'S)-2,2-difluorodihydro-1'H,3'H-spiro[cyclopro pan-1,2'-pyrrolizin]-7a'(5'H)-yl)methoxy)-7-(8-ethynyl-7-fluoro-3-(((1-(isobutyryloxy)etho xy)carbonyl)oxy)naphthalen-1-yl)-8-fluoropyridino[4,3-d]pyrimidin-4-yl)-3,8-diazabicyclo [3.2.1]octan-8-formate

Example 16B-1 (100mg, 0.155mmol) was dissolved in DCM(4mL), and then added with DIPEA (100mg, 0.775mmol), and ethyl 1-(((4-nitrophenoxy)carbonyl)oxy)isobutyrate(184mg, 0.62mmol). After addition, the mixture was stirred at room temperature for 16 h. After the reaction was completed, the reaction solution was concentrated to dryness, added with acetonitrile to dissolve, and then separated by preparative HPLC to obtain the target product (65 mg).

LC-MS: m/z 961 (M+H)⁺. ¹H NMR (400MHz, DMSO-*d*₆) δ 9.08 (s, 1 H), 8.25 (dd, *J*=5.92Hz, 9.28Hz,1 H), 8.15 (d, *J*=2.52Hz, 1 H), 7.68(t, *J*=9.00Hz, 1 H), 7.62(m, 1 H), 6.75 (m, 2 H), 4.46 (m, 4 H), 4.20 (m, 2 H), 4.08 (s, 1 H), 3.68(m, 2 H), 3.09 (m,1 H), 3.00 (m,1 H), 2.07 (d, *J*=11.84Hz, 1 H), 2.61 (m, 3 H), 2.09 (m, 1H), 1.99 (m, 1 H), 1.85 (m, 7 H), 1.57 (m, 9H), 1.09 (m, 12H).

### Step 1: Preparation of 1-(isobutyryloxy)ethyl(1R,5S)-3-(2-(((1S,7a'S)-2,2-difluorodihydro-1'H,3'H-spiro[cyclopro pan-1,2'-pyrrolizin]-7a'(5'H)-yl)methoxy)-7-(8-ethynyl-7-fluoro-3-hydroxynaphthalen-1-yl )-8-fluoropyridino[4,3-d]pyrimidin-4-yl)-3,8-diazabicyclo[3.2.1]octan-8-formate

Example P04 (105mg, 0.109mmol) was dissolved in acetonitrile (4mL), and then added with ammonia water (10 drops). After addition, the mixture was stirred at room temperature for 2 h. After the reaction was completed, the reaction solution was concentrated to dryness, added with acetonitrile to dissolve, and then separated by preparative HPLC to obtain the target product (65 mg).

LC-MS: m/z 803 (M+H)⁺. ¹H NMR (400MHz, DMSO-*d*₆) : 10.16 (s, 1 H), 9.06 (s, 1 H), 7.98 (dd, *J*=5.92, 9.24Hz,1 H), 7.47(t, *J*=9.00Hz, 1 H), 7.40(d, *J*=2.60Hz, 1 H), 7.18(d, *J*=2.52Hz, 1 H), 6.74 (m, 1 H), 4.59 (m, 1 H), 4.39 (m,3 H), 4.22 (m,2 H), 3.93 (s, 1 H), 3.67(m, 2H), 3.11 (m,2 H), 2.71 (d, *J*=11.84Hz, 1 H), 2.55 (m, 1 H), 1.98 (m, 10H), 1.56 (m, 6H), 1.08 (d, *J*=7.16Hz,6 H).

**The following compounds were synthesized according to the same synthesis method of example P05:**

### Example P06: 1-(isobutyryloxy)ethyl (1R,5S)-3-(7-(5-amino-3-chloro-2-(trifluoromethyl)phenyl)-2-(((1S,7a'S)-2,2-difluoro dihydro-1'H,3'H-spiro[cyclopropan-1,2'-pyrrolizin]-7a'(5'H)-yl)methoxy)-8-fluoropy ridino[4,3-d]pyrimidin-4-yl)-3,8-diazabicyclo[3.2.1]octan-8-formate

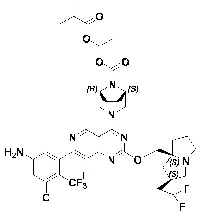

LC-MS: m/z 812 (M+H)⁺. ¹H NMR (400MHz, DMSO-*d*₆) : 9.07 (s, 1 H) , 6.89(d, J=2.20Hz, 1 H), 6.73 (dd, J=5.32Hz, J=10.76Hz,1 H), 6.47(d, J=2.28Hz, 1 H), 6.35 (s, 2 H) , 4.53 (m, 2 H), 4.36 (m, 2H), 4.23 (m, 2 H), 3.67 (m,2 H), 3.10(m,1 H), 3.01 (m, 1 H), 2.71 (m, 1H), 2.55 (m, 1 H), 2.09 (m, 1 H), 1.98 (m, 1 H), 1.65 (m, 14H).

### Biological test examples

The following biological test examples further described and explained the present invention, but these examples were not intended to limit the scope of the present invention.

### KRAS^{G12D} binding inhibition assay

### Experimental Procedure

TR-FRET technology was used to detect the binding ability of the compound to KRAS^{G12D} protein. The biotin-labeled GDP-loaded KRAS^{G12D} recombinant human protein was co-incubated with Cy5-labeled tracer, europium-labeled streptavidin, and the compound (with a final concentration of 2% DMSO) in a buffer (HEPES (pH 7.5), MgCl₂, Tween-20, and DTT). After incubation at 22°C for 60 minutes, the reaction activity was detected by the dual-wavelength technology of EnVision multifunctional microplate reader, and the protein binding capacity (POC) was calculated using the ratiometric emission factor.

100 POC means that there is no compound; 0 POC means that the control compound completely inhibits the binding of the tracer to KRAS^{G12D} at this concentration. The POC value is fitted using a four-parameter logistic model curve, and IC₅₀ represents the 50 POC concentration value.

The results show that the example compounds of the present invention show good inhibitory activity against KRAS^{G12D}.

### ERK phosphorylation inhibition assay

### Experimental Procedure

KRAS^{G12D} mutant cells (such as GP2D, AGS, etc.) were seeded in a 384-well plate and cultured overnight in a 37°C, 5% CO₂ incubator.

200 nL of diluted compound was added with Echo 500 to a final DMSO concentration of 0.5%, and cultured in a 37°C, 5% CO2 incubator for 1 hour. Then hEGF was added for acting for 10 minutes.

The culture medium was discarded, and then cell fixative was added to fix the cells.

The cells were washed once with PBS, and then incubated with cold 100% methanol.

Methanol was removed and the cells were washed with PBS.

PBS was removed, and each well was added with Li-Cor blocking buffer, and blocked at room temperature for 1 hr.

The blocking solution was removed, and primary antibody mixture was added to each well, and incubated at room temperature at 4°C overnight.

The primary antibody mixture was removed, and the cells were washed by adding PBST for 3 times.

Secondary antibody mixture was added and incubated at room temperature in the dark for 45 min.

The secondary antibody mixture was removed, and the cells were washed by adding PBST for 3 times. Finally, PBST was suctionied out, and the plate was centrifuged upside down at 1000 rpm for 1 min.

The plate was read by Odyssey CLx. The test results were shown in Tables 1 and 2 below.

The structure of the reference compound MRTX1133 is as follows:

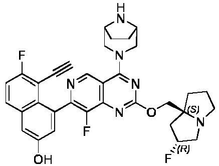

**Table 1 Results of GP2D ERK phosphorylation inhibition experiment**

| **Example** | **IC₅₀ (nM)** |
|---|---|
| **Example 1 Isomer 1A** | 2.6 |
| **Example 1 Isomer 1B** | 2.1 |

**Table 2 Results of AGS ERK phosphorylation inhibition experiment**

| **Example** | **IC₅₀ (nM)** |
|---|---|
| **Example 1 Isomer 1A** | 9.67 |
| **Example 1 Isomer 1B** | 3.76 |
| **Example 1 Isomer 2A** | 19 |
| **Example 1 Isomer 2B** | 139 |
| **Example 9** | 8.2 |
| **Example 16A** | 10.6 |
| **Example 16B** | 3.34 |

The results showed that the example compounds of the present invention showed good inhibitory activity on ERK phosphorylation in KRAS^{G12D} mutant cells.

### Inhibitory effect of compounds on proliferation of KRAS G12D mutant cells

### Experimental Procedure

### 1. Cell Culture

(a) Resuscitating cells in T75 cell culture flask:
(b) When the cell confluence reaches 80-90%, subculturing the cells.

### 2. Cell Proliferation Assay

### Experimental Procedure

The diluted test compound was added to a 384-well cell culture plate with a nanoliter pipetting system, and duplicate wells were set up. An equal volume of culture medium was added to the positive control group; and an equal volume of DMSO was added to the negative control group, then centrifugation was conducted at 1000 rpm for 1 min at room temperature.

Cells were inoculated into a) 384-well culture plate, with an equal volume of cells added to the negative control group, and only an equal volume of culture medium added to the positive control group. The plate was centrifuged at 1000 rpm for 1 min at room temperature, and the final DMSO concentration of the compound was 0.5% at last. The plate was placed in a 37°C, 5% CO₂ constant temperature incubator for 7 days.

20 µL/well of CellTiter-Glo^{®} 3D to b) was added to 384-well cell culture plate, and the plate was shook at 320 rpm for 20 min in the dark, and incubated at room temperature in the dark for 2 hrs.

The luminescence value was read using an Envision multi-functional microplate reader.

### 3. Data Analysis

The inhibition rate (IR) of the test compound was calculated using the following formula: IR (%) = (1- (RLU compound - RLU blank control) / (RLU meidum control - RLU blank control))* 100%. The inhibition rates of different concentrations of compounds were calculated in Excel, and then the inhibition curves were plotted and the related parameters (including the minimum inhibition rate, maximum inhibition rate and IC₅₀) were calculated using GraphPad Prism software. The experimental results are shown in Table 3.

**Table 3 Cell proliferation inhibition activity of the example compounds of the present invention**

| | GP2D | AsPC-1 | AGS |
|---|---|---|---|
| | IC₅₀ (nM) | IC₅₀ (nM) | IC₅₀ (nM) |
| **MRTX1133** | 1.28 | 6.77 | 5.15 |
| **Example 10A** | 1.07 | 23.5 | / |
| **Example 10B** | 0.51 | 17.3 | / |
| **Example 11** | 3.01 | 38.2 | / |
| **Example 12A** | 3.07 | 24.8 | / |
| **Example 12B** | 29.58 | 390 | / |
| **Example 13** | 42.8 | 209.5 | / |
| **Example 14** | > 100 | 921 | / |
| **Example 15A** | 13.1 | 108.3 | / |
| **Example 15B** | 7.35 | 50.8 | / |
| **Example 15C** | 12.08 | 60.61 | / |
| **Example 15D** | 44.47 | 177.35 | / |
| **Example 15E** | 32.19 | 314 | / |
| **Example 15F** | >100 | >1000 | / |
| **Example 15G** | 39.15 | 329.1 | / |
| **Example 15H** | 9.30 | 226.3 | / |
| **Example 16B-1** | 0.20 | 1.24 | 1.37 |
| **Example 16B-2** | 1.55 | 9.22 | / |
| **Example 17A** | 0.26 | 1.07 | / |
| **Example 17B** | 1.15 | 7.84 | / |
| **Example 18** | 9.83 | 307.1 | / |
| **Example 20A** | 0.44 | 4.11 | / |
| **Example 20B** | 0.58 | 4.86 | / |
| **Example 21** | 4.92 | 19.1 | / |
| **Example 22** | > 100 | > 1000 | / |
| **Example 23** | > 100 | > 1000 | / |
| **Example 24** | 2.5 | 62 | / |
| **Example 25** | > 100 | > 1000 | / |
| **Example 27** | 0.38 | 4.14 | 5.24 |
| **Example 28** | 19.82 | 188.4 | / |
| **Example 29** | 17.67 | 180.4 | / |
| **Example 30** | > 100 | > 1000 | / |
| **Example 31** | 24.31 | 258.52 | / |
| **Example 32** | 21 | 483 | / |
| **Example 33** | > 100 | > 1000 | / |
| **Example 34** | 1.03 | 11.07 | 21.49 |
| **Example 35** | 4.89 | 45.67 | 79.74 |
| **Example 36** | 20.43 | 166.73 | 297.93 |
| **Example 37** | > 100 | > 1000 | >1000 |
| **Example 38** | 14.10 | 142.27 | / |
| **Example 39** | 38.26 | 521.32 | / |
| **Example 40** | 13.97 | 51.58 | / |
| **Example 41** | 1.41 | 14.24 | 16.56 |
| **Example 42** | 0.68 | 8.83 | 13.86 |
| **Example 43** | 4.17 | 114.92 | / |
| **Example 44** | 1.60 | 23.32 | / |
| **Example 45** | 60.05 | 896 | / |
| **Example 46** | > 100 | > 1000 | / |
| **Example 47** | 4.54 | 62.29 | / |
| **Example 48** | 66 | 779 | / |
| **Example 49** | 24.38 | >1000 | / |
| **Example 50** | > 100 | > 1000 | / |
| **Example 51** | > 100 | > 1000 | > 1000 |
| **Example 52** | > 100 | > 1000 | / |
| **Example 53** | 60.05 | 896 | / |
| **Example 54** | > 100 | > 1000 | > 1000 |
| **Example 55** | 6.50 | 56.69 | / |

### Pharmacokinetic test evaluation

Male ICR mice, weighing 22-24 g, were fasted overnight and orally administered with 30 mg/kg of a solution of the compound of the present invention or the control compound [10% DMSO + 60% PEG400 + 30% aqueous solution). Blood was collected at 0.25, 0.5, 1.0, 2.0, 4.0, 6.0, 8.0, 12.0 and 24 h after administration of the compound of the present invention respectively, and the concentration of the compound of the present invention or the control compound in plasma was determined by LC/MS/MS.

**Table 4 Main pharmacokinetic parameters in plasma**

| Compound No. | *T*ₘₐₓ | *C*ₘₐₓ | *AUC*_{0*-*t} | *AUC*_{0-∞} | *t*_{1/2} |
|---|---|---|---|---|---|
| | (h) | (ng/mL) | (ng·h/mL) | (ng·h/mL) | (h) |
| **Example 24** | 0.5 | 461 | 1252 | 1266 | 4.74 |
| **Example 26** | 0.25 | 462 | 3384 | 3770 | 6.97 |
| **Example 27** | 0.25 | 953 | 5089 | 5654 | 9.32 |
| *MRTX1133* | NA | BLOQ | BLOQ | BLOQ | NA |

| | | | | | |
|---|---|---|---|---|---|
| BLOQ: below the minimum detection limit; NA: not obtained | | | | | |

From the test results, it can be seen that the compound of the present invention has good oral pharmacokinetic properties.

### Pharmacokinetic test evaluation

Male SD rats, weighing about 220g, were fasted overnight and orally administered with 30 mg/kg of the solution of the compound of the present invention or the control compound [10% DMSO + 60% PEG400 + 30% aqueous solution). Blood was collected at 0.25, 0.5, 1.0, 2.0, 4.0, 6.0, 8.0, 12.0 and 24 h after administration of the compound of the present invention respectively, and the concentration of the compound of the present invention or the control compound in plasma was determined by LC/MS/MS.

It can be seen from the test results that the compound of the present invention has good pharmacokinetic properties.

### Pharmacodynamic evaluation of antitumor activity (AsPC-1 CDX tumor model)

100 uL of 5×10⁶ AsPC-1 tumor cell suspension was subcutaneously inoculated into the right posterior abdomen of the nude mice. The health status of the mice was monitored daily, and the measurement began when the tumor grew to a palpable size. The tumor volume was calculated using the formula: 0.5×L×W², wherein L and W represent the length and width of the tumor, respectively. When the tumor grew to ~200 mm³, the mice were randomly divided into groups. The mice were intraperitoneally injected or orally administered with the corresponding dose of the compound every day, and their general condition was monitored at the same time. The tumor was measured 3 times a week, and the body weight was measured twice a week.

The test results show that the compounds of the present invention have good anti-tumor effect.

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

## Claims

1. A compound of formula (A0), or a stereoisomer, a tautomer, a crystal form, a pharmaceutically acceptable salt, a hydrate, a solvate, or a prodrug thereof: 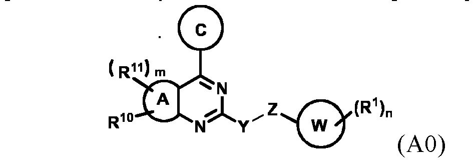
ring C is a group selected from the group consisting of: 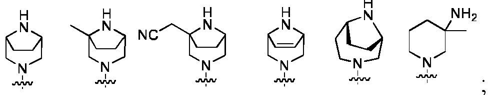
Y is selected from bond, O, NH, and N(C₁-C₃alkyl);
Z is substituted or unsubstituted C₁-C₆ alkylene; wherein said substituted refers to being substituted with one or more R;
W is selected from substituted or unsubstituted C₃-C₁₄cycloalkyl, and substituted or unsubstituted 4-14 membered saturated or unsaturated heterocyclyl; wherein said substituted refers to being substituted with one or more R; 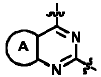
is a group selected from the group consisting of: 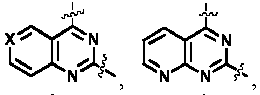 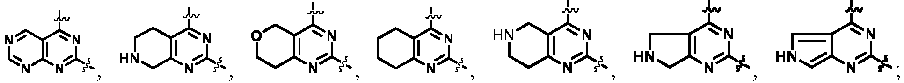 wherein X is selected from N, CH, CD, CF, and C(CN);
R¹ is selected from -L₁-Q-L₂-L₃;
wherein:
L₁ is selected from substituted or unsubstituted C₁-C₆alkylene; wherein said substituted refers to being substituted with one or more R;
Q is selected from O, S, SO₂, NH, and N(C₁-C₃alkyl);
L₂ is selected from null, and substituted or unsubstituted C₁-C₆alkylene; wherein said substituted refers to being substituted with one or more R;
L₃ is a substituted or unsubstituted group selected from the group consisting of: -C₁-C₆alkyl, -C₃-C₆cycloalkyl, -C₄-C₆heterocyclyl, -C₁-C₆alkylene(C₃-C₆cycloalkyl), -C₁-C₆alkylene(C₄-C₆heterocyclyl), -C₁-C₆alkylene(C₁-C₆alkoxy), -C₁-C₆alkylene(C₃-C₆cycloalkoxy), and -C₁-C₆alkylene(C₄-C₆heterocyclyloxy); wherein said substituted refers to being substituted with one or more R;
n is an integer of 0, 1, 2, 3, 4, 5 or 6; provided that when W is a monocyclic or bicyclic ring, n is not 0;
R¹⁰ is a substituted or unsubstituted group selected from the group consisting of: C₆-C₁₄ aryl, and 5-14 membered heteroaryl; wherein said substituted refers to being substituted with one or more Ra;
R¹¹ is each independently a substituted or unsubstituted group selected from the group consisting of: H, deuterium, halogen, cyano, ester group, amino, amido, sulfonyl , ureido, C₁-C₆alkyl, C₁-C₆deuterated alkyl, C₁-C₆haloalkyl, C₃-C₆cycloalkyl, 4-6 membered heterocyclyl, C₁-C₆alkoxy, C₃-C₆cycloalkoxy, and 4-6 membered heterocyclyloxy; wherein said substituted refers to being substituted with one or more R;
Ra is each independently a substituted or unsubstituted group selected from the group consisting of: H, D, halogen, CN, NH₂, OH, CONH₂, NHCO(C₁-C₆alkyl), NHCO(C₃-C₆cycloalkyl), SONH₂, SO₂NH₂, NHSO₂(C₁-C₆alkyl), NHSO₂(C₃-C₆cycloalkyl), C₁-C₆alkyl, C₁-C₆deuterated alkyl, C₁-C₆haloalkyl, C₁-C₆alkoxy, C₂-C₆alkenyl, C₂-C₆alkynyl, C₃-C₂ocycloalkyl, 4-20 membered heterocyclyl, C₃-C₂₀cycloalkoxy, and 4-20 membered heterocyclyloxy; wherein said substituted refers to being substituted with one or more R;
m is an integer of 0, 1, 2, 3, 4, 5 or 6;
each R is the same or different, and is each independently selected from deuterium, C₁-C₁₈ alkyl, deuterated C₁-C₁₈ alkyl, halogenated C₁-C₁₈ alkyl, (C₃-C₁₈cycloalkyl)C₁-C₁₈ alkyl, (4-20 membered heterocyclyl)C₁-C₁₈ alkyl, (C₁-C₁₈salkoxy)C₁-C₁₈ alkyl, (C₃-C₁₈cycloalkoxy)C₁-C₁₈ alkyl, (4-20 membered heterocyclyloxy)C₁-C₁₈ alkyl, ethenyl, ethynyl, (C₁-C₆alkyl)ethenyl, deuterated (C₁-C₆alkyl)ethenyl, halogenated (C₁-C₆alkyl)ethenyl, (C₁-C₆alkyl)ethynyl, deuterated (C₁-C₆alkyl)ethynyl, halogenated (C₁-C₆alkyl)ethynyl, (C₃-C₁₄cycloalkyl)ethynyl, (4-14 membered heterocyclyl)ethynyl, C₁-C₁₈salkoxy, deuterated C₁-C₁₈salkoxy, halogenated C₁-C₁₈salkoxy, 4-20 membered heterocyclylC(O), C₃-C₂ocycloalkyl, 4-20 membered heterocyclyl, C₆-C₁₄aryl, 5-14membered heteroaryl, halogen, nitro, hydroxy, oxo, cyano, ester group, amino, amido, sulfonamido, sulfonyl, and ureido.

2. The compound of claim 1, or the stereoisomer, the tautomer, the crystal form, the pharmaceutically acceptable salt, the hydrate, the solvate, or the prodrug thereof, wherein the compound has a structure of formula (A): 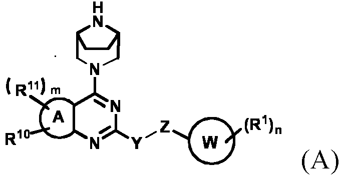
Y is selected from bond, O, NH, and N(C₁-C₃alkyl);
Z is substituted or unsubstituted C₁-C₆ alkylene; wherein said substituted refers to being substituted with one or more R;
W is selected from substituted or unsubstituted C₃-C₁₄cycloalkyl, and substituted or unsubstituted 4-14 membered saturated or unsaturated heterocyclyl; wherein said substituted refers to being substituted with one or more R; 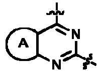
is a group selected from the group consisting of: 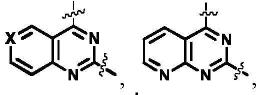 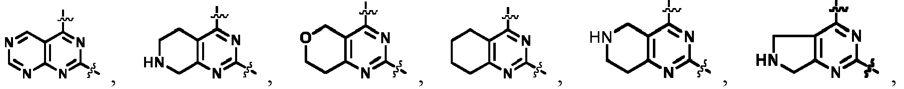 and 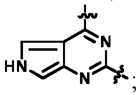
wherein X is selected from N, CH, CD, CF, and C(CN);
R¹ is selected from -L₁-Q-L₂-L₃;
wherein:
L₁ is selected from substituted or unsubstituted C₁-C₆alkylene; wherein said substituted refers to being substituted with one or more R;
Q is selected from O, S, SO₂, NH, and N(C₁-C₃alkyl);
L₂ is selected from null, and substituted or unsubstituted C₁-C₆alkylene; wherein said substituted refers to being substituted with one or more R;
L₃ is a substituted or unsubstituted group selected from the group consisting of: -C₁-C₆alkyl, -C₃-C₆cycloalkyl, -C₄-C₆heterocyclyl, -C₁-C₆alkylene(C₃-C₆cycloalkyl), -C₁-C₆alkylene(C₄-C₆heterocyclyl), -C₁-C₆alkylene(C₁-C₆alkoxy), -C₁-C₆alkylene(C₃-C₆cycloalkoxy), and -C₁-C₆alkylene(C₄-C₆heterocyclyloxy); wherein said substituted refers to being substituted with one or more R;
n is an integer of 0, 1, 2, 3, 4, 5 or 6; provided that when W is a monocyclic or bicyclic ring, n is not 0;
R¹⁰ is a substituted or unsubstituted group selected from the group consisting of: C₆-C₁₄ aryl, and 5-14 membered heteroaryl; wherein said substituted refers to being substituted with one or more Ra;
R¹¹ is each independently a substituted or unsubstituted group selected from the group consisting of: H, deuterium, halogen, cyano, ester group, amino, amido, sulfonyl , ureido, C₁-C₆alkyl, C₁-C₆deuterated alkyl, C₁-C₆haloalkyl, C₃-C₆cycloalkyl, 4-6 membered heterocyclyl, C₁-C₆alkoxy, C₃-C₆cycloalkoxy, and 4-6 membered heterocyclyloxy;
Ra is each independently a substituted or unsubstituted group selected from the group consisting of: H, D, halogen, CN, NH₂, OH, CONH₂, NHCO(C₁-C₆alkyl), NHCO(C₃-C₆cycloalkyl), SONH₂, SO₂NH₂, NHSO₂(C₁-C₆alkyl), NHSO₂(C₃-C₆cycloalkyl), C₁-C₆alkyl, C₁-C₆deuterated alkyl, C₁-C₆haloalkyl, C₁-C₆alkoxy, C₂-C₆alkenyl, C₂-C₆alkynyl, C₃-C₂ocycloalkyl, 4-20 membered heterocyclyl, C₃-C₂₀cycloalkoxy, and 4-20 membered heterocyclyloxy; wherein said substituted refers to being substituted with one or more R;
m is an integer of 0, 1, 2, 3, 4, 5 or 6;
each R is the same or different, and is each independently selected from deuterium, C₁-C₁₈ alkyl, deuterated C₁-C₁₈ alkyl, halogenated C₁-C₁₈ alkyl, (C₃-C₁₈cycloalkyl)C₁-C₁₈ alkyl, (4-20 membered heterocyclyl)C₁-C₁₈ alkyl, (C₁-C₁₈salkoxy)C₁-C₁₈ alkyl, (C₃-C₁₈cycloalkoxy)C₁-C₁₈ alkyl, (4-20 membered heterocyclyloxy)C₁-C₁₈ alkyl, ethenyl, ethynyl, (C₁-C₆alkyl)ethenyl, deuterated (C₁-C₆alkyl)ethenyl, halogenated (C₁-C₆alkyl)ethenyl, (C₁-C₆alkyl)ethynyl, deuterated (C₁-C₆alkyl)ethynyl, halogenated (C₁-C₆alkyl)ethynyl, (C₃-C₁₄cycloalkyl)ethynyl, (4-14 membered heterocyclyl)ethynyl, C₁-C₁₈salkoxy, deuterated C₁-C₁₈salkoxy, halogenated C₁-C₁₈salkoxy, 4-20 membered heterocyclylC(O), C₃-C₂ocycloalkyl, 4-20 membered heterocyclyl, C₆-C₁₄aryl, 5-14 membered heteroaryl, halogen, nitro, hydroxy, oxo, cyano, ester group, amino, amido, sulfonamido, sulfonyl, and ureido.

3. The compound of any one of claims 1-2, or the stereoisomer, the tautomer, the crystal form, the pharmaceutically acceptable salt, the hydrate, the solvate, or the prodrug thereof, wherein the compound has a structure of formula (IA) or formula (IB): 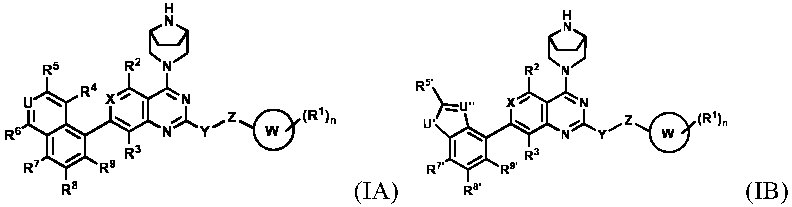
wherein,
U is selected from N, CH, CD, and CF;
U' is selected from O, and S;
U" is selected from N, and C(CN);
X is selected from N, CH, CD, CF, and C(CN);
Y is selected from bond, O, NH, and N(C₁-C₃alkyl);
Z is substituted or unsubstituted C₁-C₆ alkylene; wherein said substituted refers to being substituted with one or more R;
W is selected from substituted or unsubstituted C₃-C₁₄cycloalkyl, and substituted or unsubstituted 4-14 membered saturated or unsaturated heterocyclyl; wherein said substituted refers to being substituted with one or more R;
R¹ is selected from -L₁-Q-L₂-L₃; wherein:
L₁ is selected from substituted or unsubstituted C₁-C₆alkylene; wherein said substituted refers to being substituted with one or more R;
Q is selected from O, S, SO₂, NH, and N(C₁-C₃alkyl);
L₂ is selected from null, and substituted or unsubstituted C₁-C₆alkylene; wherein said substituted refers to being substituted with one or more R;
L₃ is a substituted or unsubstituted group selected from the group consisting of: -C₁-C₆alkyl, -C₃-C₆cycloalkyl, -C₄-C₆heterocyclyl, -C₁-C₆alkylene(C₃-C₆cycloalkyl), -C₁-C₆alkylene(C₄-C₆heterocyclyl), -C₁-C₆alkylene(C₁-C₆alkoxy), -C₁-C₆alkylene(C₃-C₆cycloalkoxy), and -C₁-C₆alkylene(C₄-C₆heterocyclyloxy); wherein said substituted refers to being substituted with one or more R;
n is an integer of 1, 2, 3, 4, 5 or 6;
R² and R³ are the same or different, and are each independently a substituted or unsubstituted group selected from the group consisting of: H, D, halogen, CN, C₁-C₆alkyl, C₁-C₆deuterated alkyl, C₁-C₆haloalkyl, and C₁-C₆alkoxy; wherein said substituted refers to being substituted with one or more R;
R⁴ is a substituted or unsubstituted group selected from the group consisting of: halogen, C₁-C₆alkyl, C₂-C₆alkenyl, and C₂-C₆alkynyl; wherein said substituted refers to being substituted with one or more R;
R⁸ is selected from OH, SONH₂, and NHSO₂CH₃;
R⁵, R⁶, R⁷, R⁹, R^{7'} , R^{8'}, and R^{9'} are the same or different, and are each independently a substituted or unsubstituted group selected from the group consisting of: H, D, halogen, CN, C₁-C₆alkyl, C₁-C₆deuterated alkyl, C₁-C₆haloalkyl, and C₁-C₆alkoxy; wherein said substituted refers to being substituted with one or more R;
R^{5'} is selected from H, D, halogen, CN, NH₂, OH, CONH₂, NHCO(C₁-C₆alkyl), NHCO(C₃-C₆cycloalkyl), SONH₂, SO₂NH₂, NHSO₂(C₁-C₆alkyl), NHSO₂(C₃-C₆cycloalkyl), C₁-C₆ alkyl, C₁-C₆deuterated alkyl, C₁-C₆haloalkyl, and C₁-C₆alkoxy;
each R is the same or different, and is each independently selected from deuterium, C₁-C₁₈ alkyl, deuterated C₁-C₁₈ alkyl, halogenated C₁-C₁₈ alkyl, (C₃-C₁₈cycloalkyl)C₁-C₁₈ alkyl, (4-20 membered heterocyclyl)C₁-C₁₈ alkyl, (C₁-C₁₈salkoxy)C₁-C₁₈ alkyl, (C₃-C₁₈cycloalkoxy)C₁-C₁₈ alkyl, (4-20 membered heterocyclyloxy)C₁-C₁₈ alkyl, ethenyl, ethynyl, (C₁-C₆alkyl)ethenyl, deuterated (C₁-C₆alkyl)ethenyl, halogenated (C₁-C₆alkyl)ethenyl, (C₁-C₆alkyl)ethynyl, deuterated (C₁-C₆alkyl)ethynyl, halogenated (C₁-C₆alkyl)ethynyl, (C₃-C₁₄cycloalkyl)ethynyl, (4-14 membered heterocyclyl)ethynyl, C₁-C₁₈salkoxy, deuterated C₁-C₁₈salkoxy, halogenated C₁-C₁₈salkoxy, C₃-C₂₀cycloalkyl, 4-20 membered heterocyclyl, C₆-C₁₄aryl, 5-14 membered heteroaryl, halogen, nitro, hydroxy, oxo, cyano, ester group, amino, amido, sulfonamido, sulfonyl, and ureido.

4. The compound of any one of claims 1-3, or the stereoisomer, the tautomer, the crystal form, the pharmaceutically acceptable salt, the hydrate, the solvate, or the prodrug thereof, wherein the compound has a structure of formula (IIA) or formula (IIB): 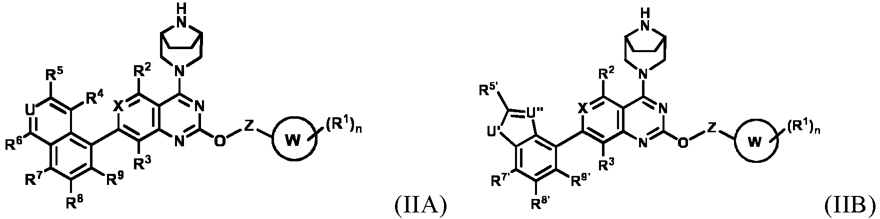 wherein R¹, R², R³, R⁴, R⁵, R⁶, R⁷, R⁸, R⁹, R^{5'}, R^{7'}, R^{8'}, R^{9'}, U, U', U", X, Z, W and n are defined as in claim 3.

5. The compound of any one of claims 1-4, or the stereoisomer, the tautomer, the crystal form, the pharmaceutically acceptable salt, the hydrate, the solvate, or the prodrug thereof, wherein the compound has a structure of formula (VA) or formula (VB): 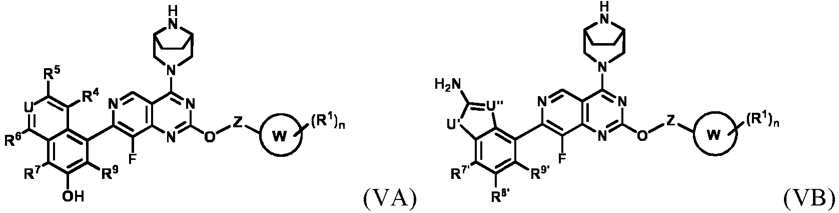 wherein R¹, R⁴, R⁵, R⁶, R⁷, R⁹, R^{7'}, R^{8'}, R^{9'}, U, U', U", Z, W and n are defined as in claim 3.

6. The compound of any one of claims 1-5, or the stereoisomer, the tautomer, the crystal form, the pharmaceutically acceptable salt, the hydrate, the solvate, or the prodrug thereof, wherein the compound has a structure of formula (VIA) or formula (VIB): 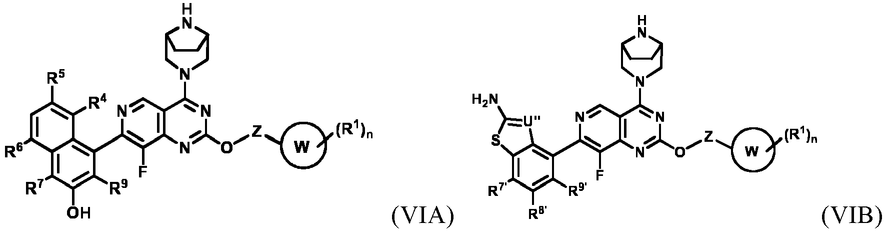 wherein R¹, R⁴, R⁵, R⁶, R⁷, R⁹, R^{1'}, R^{8'}, R^{9'}, U", Z, W and n are defined as in claim 3.

7. The compound of any one of claims 1-6, or the stereoisomer, the tautomer, the crystal form, the pharmaceutically acceptable salt, the hydrate, the solvate, or the prodrug thereof, wherein the compound has a structure of formula (VIIA) or formula (VIIB): 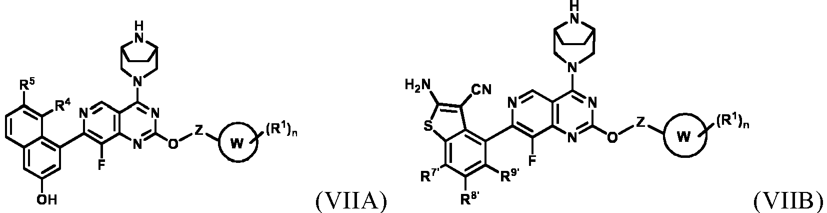 wherein R¹, R⁴, R⁵, R^{7'} , R^{8'}, R^{9'}, Z, W and n are defined as in claim 3.

8. The compound of any one of claims 1-7, or the stereoisomer, the tautomer, the crystal form, the pharmaceutically acceptable salt, the hydrate, the solvate, or the prodrug thereof, wherein the compound has a structure of formula (VIIIA) or formula (VIIIB): 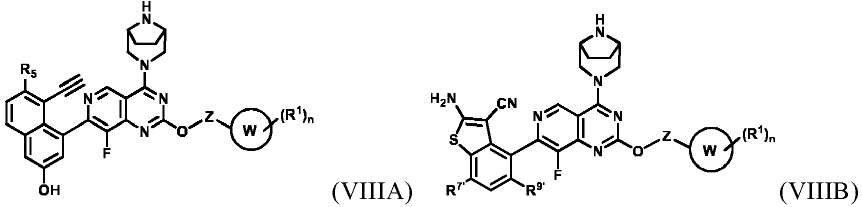 wherein R¹, R⁴, R⁵, R^{7'} , R^{9'}, Z, W and n are defined as in claim 3.

9. The compound of claim 1, or the stereoisomer, the tautomer, the crystal form, the pharmaceutically acceptable salt, the hydrate, the solvate, or the prodrug thereof, wherein the compound has a structure of formula (IXA) or formula (IXB): 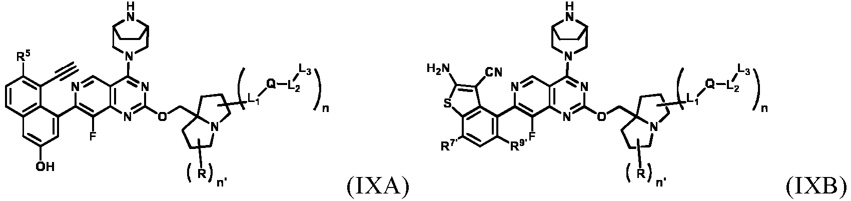
wherein n' is an integer of 0, 1, 2, 3, 4, 5, or 6;
R⁵, R, R⁷, R⁹, R^{7'} , R^{9'}, L₁, Q, L₂, L₃ and n are defined as in claim 3.

10. The compound of claim 1, or the stereoisomer, the tautomer, the crystal form, the pharmaceutically acceptable salt, the hydrate, the solvate, or the prodrug thereof, wherein the compound has a structure of formula (X): 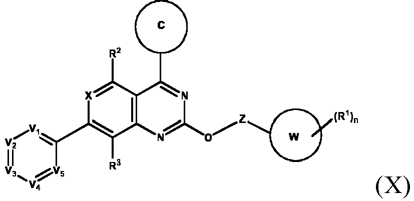 wherein,
V₁, V₂, V₃, V₄, and V₅ are each independently selected from N, and CRᵥ; Rᵥ is the same or different, and is each independently selected from H, C₁-C₃ alkyl, C₃-C₆cycloalkyl, 4-6 membered heterocyclyl, C₁-C₃alkoxy, C₃-C₆cycloalkoxy, 4-6 membered heterocyclyloxy, halogenated C₁-C₃ alkyl, halogenated C₃-C₆cycloalkyl, halogenated 4-6membered heterocyclyl, (HO)-C₁-C₃ alkyl, (HO)-C₃-C₆cycloalkyl, (NH₂)-C₁-C₃ alkyl, (NH₂)-C₃-C₆cycloalkyl, halogen, CN, -C≡CH, OH, NH₂, CONH₂, NHCO(C₁-C₆alkyl), NHCO(C₃-C₆cycloalkyl), SO₂NH₂, NHSO₂(C₁-C₆alkyl), and NHSO₂(C₃-C₆cycloalkyl), wherein the heterocyclyl is optionally substituted with one or more oxo (=O);
R² and R³ are the same or different, and are each independently a substituted or unsubstituted group selected from the group consisting of: H, D, halogen, CN, C ₁-C₆alkyl, C₁-C₆deuterated alkyl, C₁-C₆haloalkyl, and C₁-C₆alkoxy; wherein said substituted refers to being substituted with one or more R;
ring C, R, R¹, X, Z, W and n are defined as in claim 1;
preferably, the compound has a structure of formula (XI): 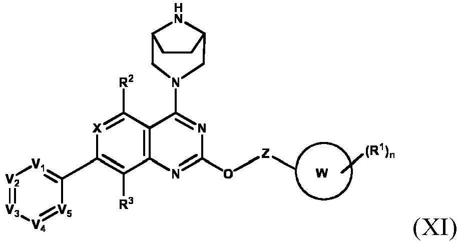
V₁, V₂, V₃, V₄, and V₅ are each independently selected from N, and CRᵥ; each Rᵥ is the same or different, and is each independently selected from H, C₁-C₃ alkyl, C₃-C₆cycloalkyl, 4-6 membered heterocyclyl, C₁-C₃alkoxy, C₃-C₆cycloalkoxy, 4-6 membered heterocyclyloxy, halogenated C₁-C₃ alkyl, halogenated C₃-C₆cycloalkyl, halogenated 4-6 membered heterocyclyl, (HO)-C₁-C₃ alkyl, (HO)-C₃-C₆cycloalkyl, (NH₂)-C₁-C₃ alkyl, (NH₂)-C₃-C₆cycloalkyl, halogen, CN, -C≡CH, OH, NH₂, CONH₂, NHCO(C₁-C₆alkyl), NHCO(C₃-C₆cycloalkyl), SO₂NH₂, NHSO₂(C₁-C₆alkyl), and NHSO₂(C₃-C₆cycloalkyl), wherein the heterocyclyl is optionally substituted with one or more oxo (=O);
R² and R³ are the same or different, and are each independently a substituted or unsubstituted group selected from the group consisting of: H, D, halogen, CN, C ₁-C₆alkyl, C₁-C₆deuterated alkyl, C₁-C₆haloalkyl, and C₁-C₆alkoxy; wherein said substituted refers to being substituted with one or more R;
wherein R, R¹, X, Z, W and n are defined as in claim 1;
preferably, the compound has a structure of formula (XII): 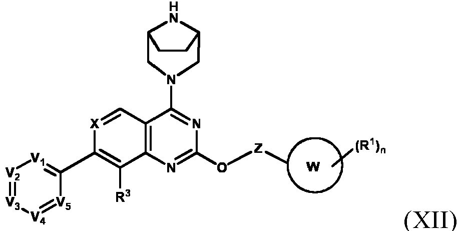
V₁, V₂, V₃, V₄, and V₅ are each independently selected from N, and CRᵥ; Rᵥ is the same or different, and is each independently selected from H, C₁-C₃ alkyl, C₃-C₆cycloalkyl, 4-6 membered heterocyclyl, C₁-C₃alkoxy, C₃-C₆cycloalkoxy, 4-6 membered heterocyclyloxy, halogenated C₁-C₃ alkyl, halogenated C₃-C₆cycloalkyl, halogenated 4-6 membered heterocyclyl, (HO)-C₁-C₃ alkyl, (HO)-C₃-C₆cycloalkyl, (NH₂)-C₁-C₃ alkyl, (NH₂)-C₃-C₆cycloalkyl, halogen, CN, -C≡CH, OH, NH₂, CONH₂, NHCO(C₁-C₆alkyl), NHCO(C₃-C₆cycloalkyl), SO₂NH₂, NHSO₂(C₁-C₆alkyl), and NHSO₂(C₃-C₆cycloalkyl), wherein the heterocyclyl is optionally substituted with one or more oxo (=O);
R³ is a substituted or unsubstituted group selected from the group consisting of: H, D, halogen, CN, C₁-C₆alkyl, C₁-C₆deuterated alkyl, C₁-C₆haloalkyl, and C₁-C₆alkoxy; wherein said substituted refers to being substituted with one or more R;
wherein R, R¹, X, Z, W and n are defined as in claim 1;
preferably, the compound has a structure of formula (XIII): 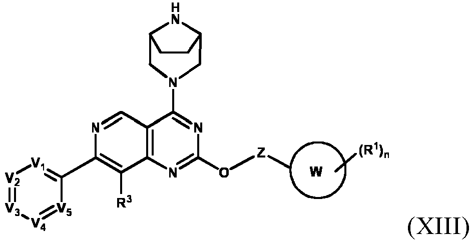
V₁, V₂, V₃, V₄, and V₅ are each independently selected from N, and CRᵥ; Rᵥ is the same or different, and is each independently selected from H, C₁-C₃ alkyl, C₃-C₆cycloalkyl, 4-6 membered heterocyclyl, C₁-C₃alkoxy, C₃-C₆cycloalkoxy, 4-6 membered heterocyclyloxy, halogenated C₁-C₃ alkyl, halogenated C₃-C₆cycloalkyl, halogenated 4-6membered heterocyclyl, (HO)-C₁-C₃ alkyl, (HO)-C₃-C₆cycloalkyl, (NH₂)-C₁-C₃ alkyl, (NH₂)-C₃-C₆cycloalkyl, halogen, CN, -C≡CH, OH, NH₂, CONH₂, NHCO(C₁-C₆alkyl), NHCO(C₃-C₆cycloalkyl), SO₂NH₂, NHSO₂(C₁-C₆alkyl), and NHSO₂(C₃-C₆cycloalkyl), wherein the heterocyclyl is optionally substituted with one or more oxo (=O);
R³ is a substituted or unsubstituted group selected from the group consisting of: H, D, halogen, CN, C₁-C₆alkyl, C₁-C₆deuterated alkyl, C₁-C₆haloalkyl, and C₁-C₆alkoxy; wherein said substituted refers to being substituted with one or more R;
wherein R, R¹, Z, W and n are defined as in claim 1.

11. The compound of claim 1, or the stereoisomer, the tautomer, the crystal form, the pharmaceutically acceptable salt, the hydrate, the solvate, or the prodrug thereof, wherein R¹⁰ is 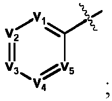 preferably, R¹⁰ is 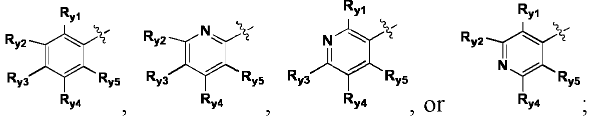 wherein Rᵥ₁, Rᵥ₂, Rᵥ₃, Rᵥ₄ and Rᵥ₅ are each independently selected from H, C₁-C₃ alkyl, C₃-C₆cycloalkyl, 4-6 membered heterocyclyl, C₁-C₃alkoxy, C₃-C₆cycloalkoxy, 4-6 membered heterocyclyloxy, halogenated C₁-C₃alkyl(such as CF₃, CF₂CF₃), halogenated C₃-C₆cycloalkyl, halogenated 4-6 membered heterocyclyl, (HO)-C₁-C₃ alkyl, (HO)-C₃-C₆cycloalkyl, (NH₂)-C₁-C₃ alkyl, (NH₂)-C₃-C₆cycloalkyl, halogen, CN, -C≡CH, OH, NH₂, CONH₂, NHCO(C₁-C₆alkyl), NHCO(C₃-C₆cycloalkyl, SO₂NH₂, NHSO₂(C₁-C₆alkyl), NHSO₂(C₃-C₆cycloalkyl) H, C₁-C₃ alkyl, C₃-C₆cycloalkyl, 4-6 membered heterocyclyl, C₁-C₃alkoxy, C₃-C₆cycloalkoxy, 4-6 membered heterocyclyloxy, halogenated C₁-C₃ alkyl, halogenated C₃-C₆cycloalkyl, halogenated 4-6 membered heterocyclyl, (HO)-C₁-C₃ alkyl, (HO)-C₃-C₆cycloalkyl, (NH₂)-C₁-C₃ alkyl, (NH₂)-C₃-C₆cycloalkyl, halogen, CN, -C≡CH, OH, NH₂, CONH₂, NHCO(C₁-C₆alkyl), NHCO(C₃-C₆cycloalkyl), SO₂NH₂, NHSO₂(C₁-C₆alkyl), and NHSO₂(C₃-C₆cycloalkyl); the heterocyclyl is optionally substituted with one or more oxo (=O).

12. The compound of any one of claims 1-10, or the stereoisomer, the tautomer, the crystal form, the pharmaceutically acceptable salt, the hydrate, the solvate, or the prodrug thereof, wherein R¹⁰ is a substituted or unsubstituted group selected from the group consisting of: phenyl, naphthyl, 5-6 membered monocyclic heteroaryl (such as pyridyl), 9-10 membered bicyclic heteroaryl(such as indazolyl, benzothiazole, benzothiophene, and benzofuran), wherein said substituted is to be substituted with one or more groups selected from the group consisting of: halogen, hydroxy, cyano, NH₂, C₁-C₆ alkyl, halogenated C₁-C₆ alkyl, C₁-C₆alkoxy, halogenated C₁-C₆alkoxy, C₃-C₆cycloalkyl, 4-6 membered heterocyclyl, C₂-C₆alkenyl, and C₂-C₆alkynyl; preferably, R¹⁰ is selected from:

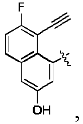

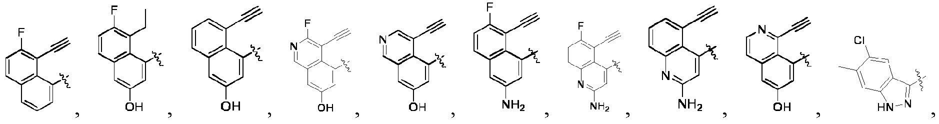

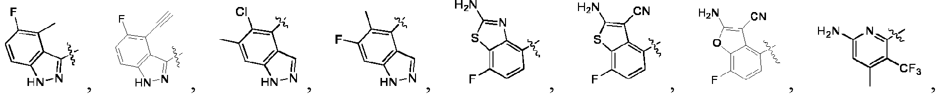

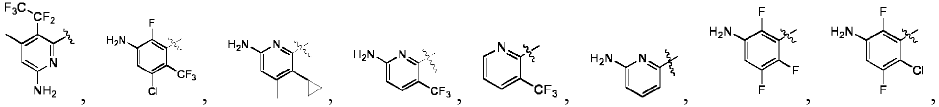

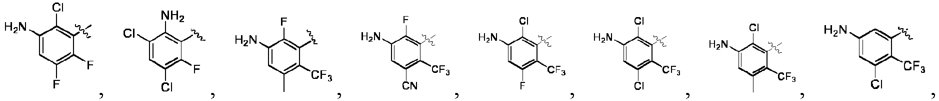

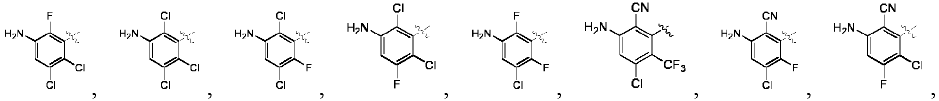

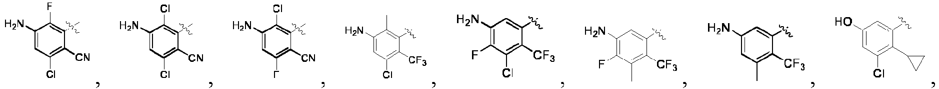

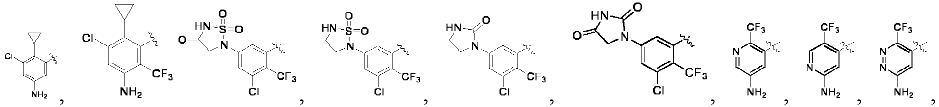

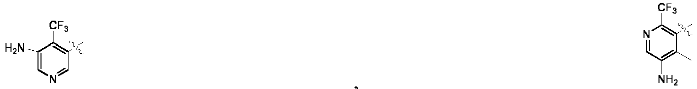

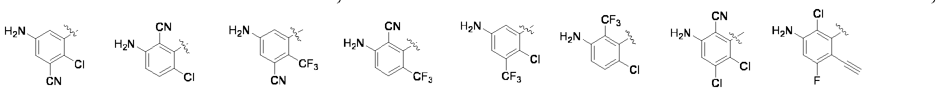

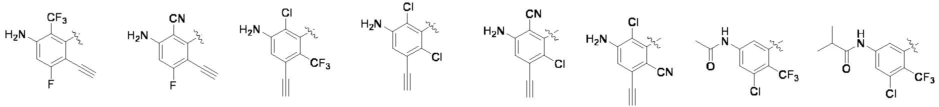

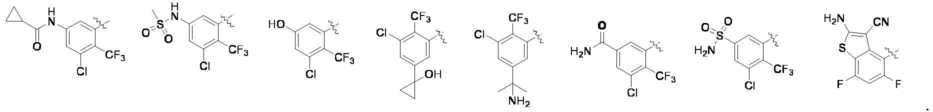

13. The compound of any one of claims 1-12, or the stereoisomer, the tautomer, the crystal form, the pharmaceutically acceptable salt, the hydrate, the solvate, or the prodrug thereof, wherein W is selected from: 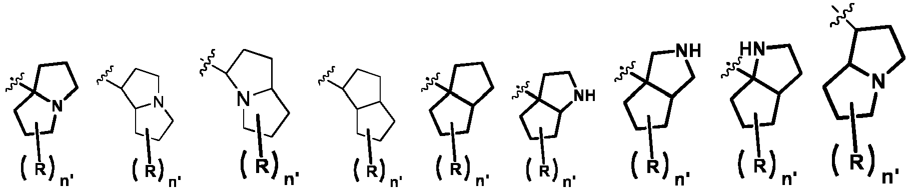 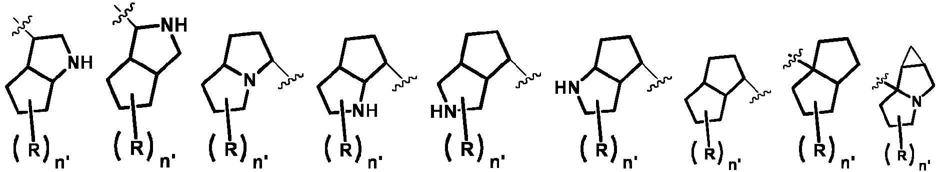 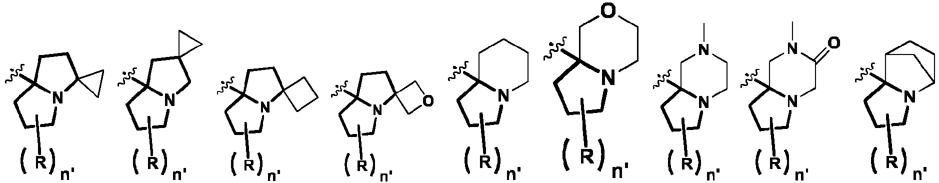 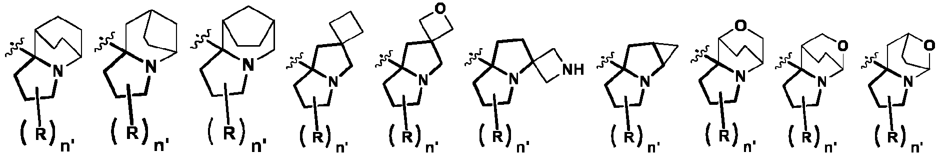 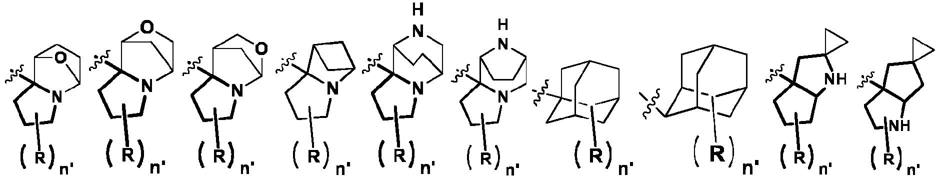 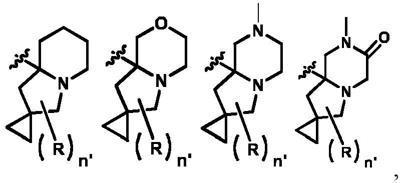 wherein n' is an integer of 0, 1, 2, 3, 4, 5, or 6; preferably, when W is selected from: 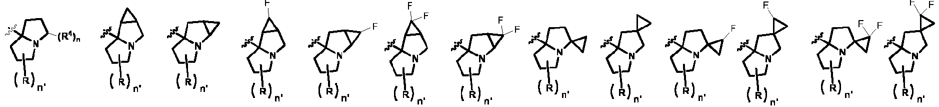 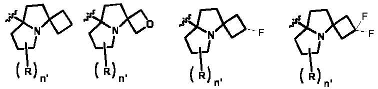 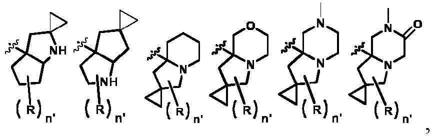 n' may be 0, 1 or 2, R is defined as in claim 1, R may be substituted on any ring of the polycyclic ring (such as bridged ring or spiro ring).

14. The compound of any one of claims 1-13, or the stereoisomer, the tautomer, the crystal form, the pharmaceutically acceptable salt, the hydrate, the solvate, or the prodrug thereof, wherein the compound is selected from the group consisting of: 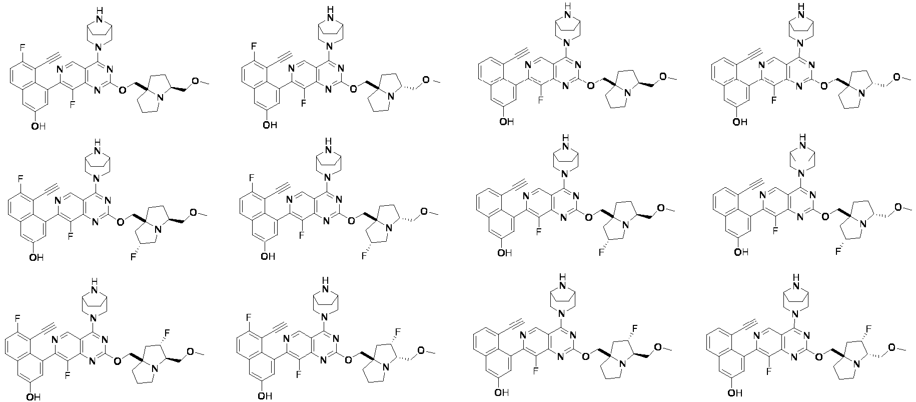 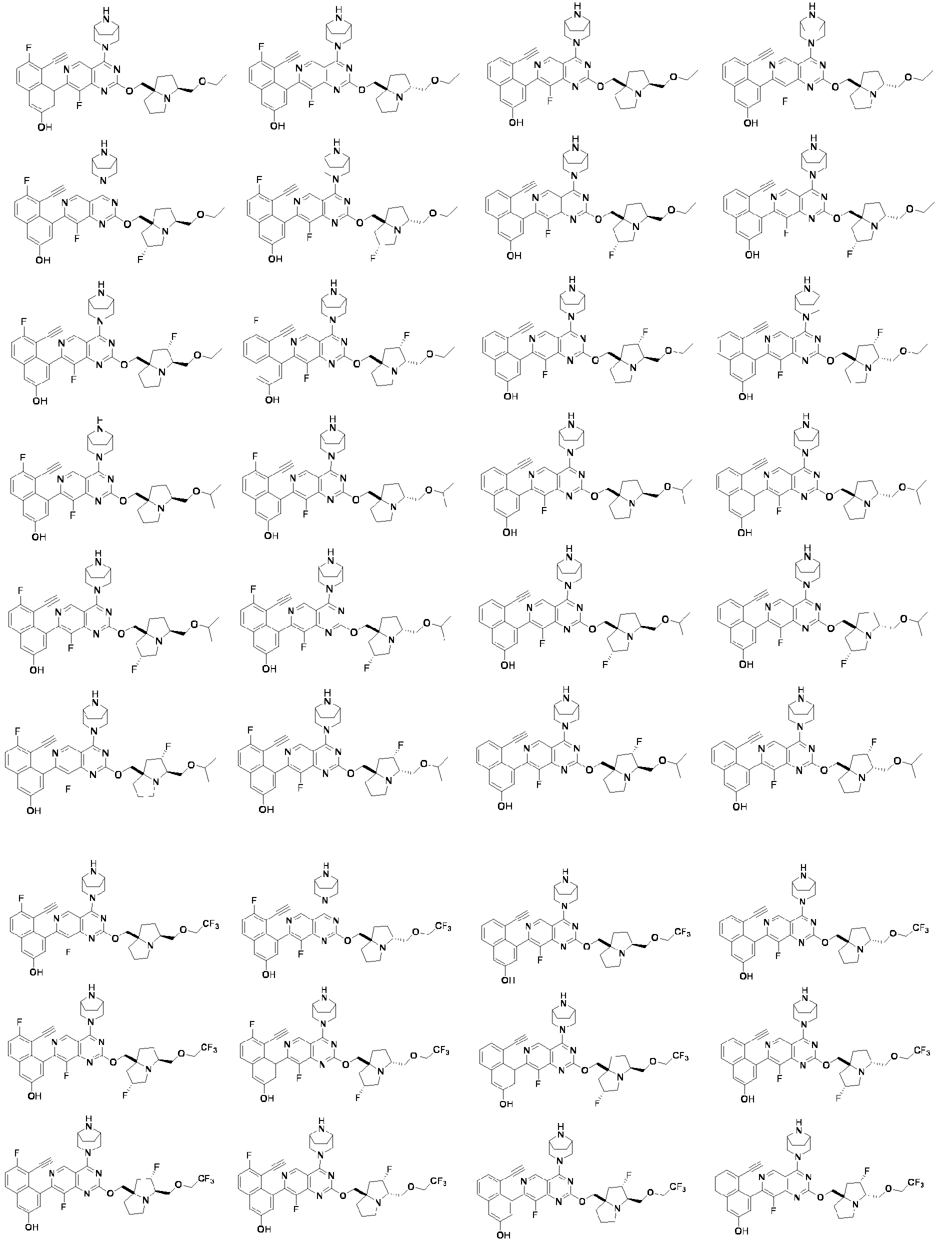 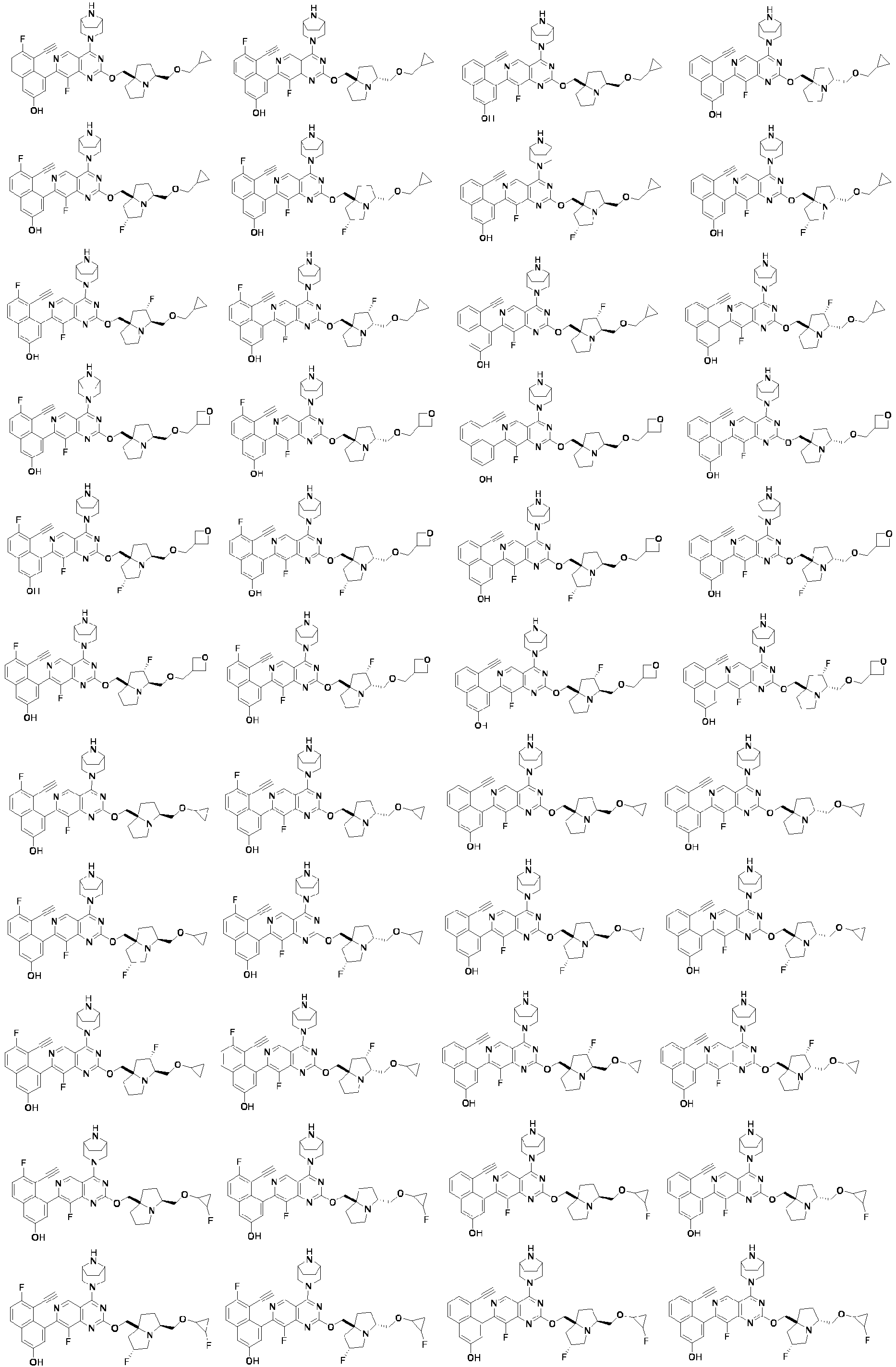 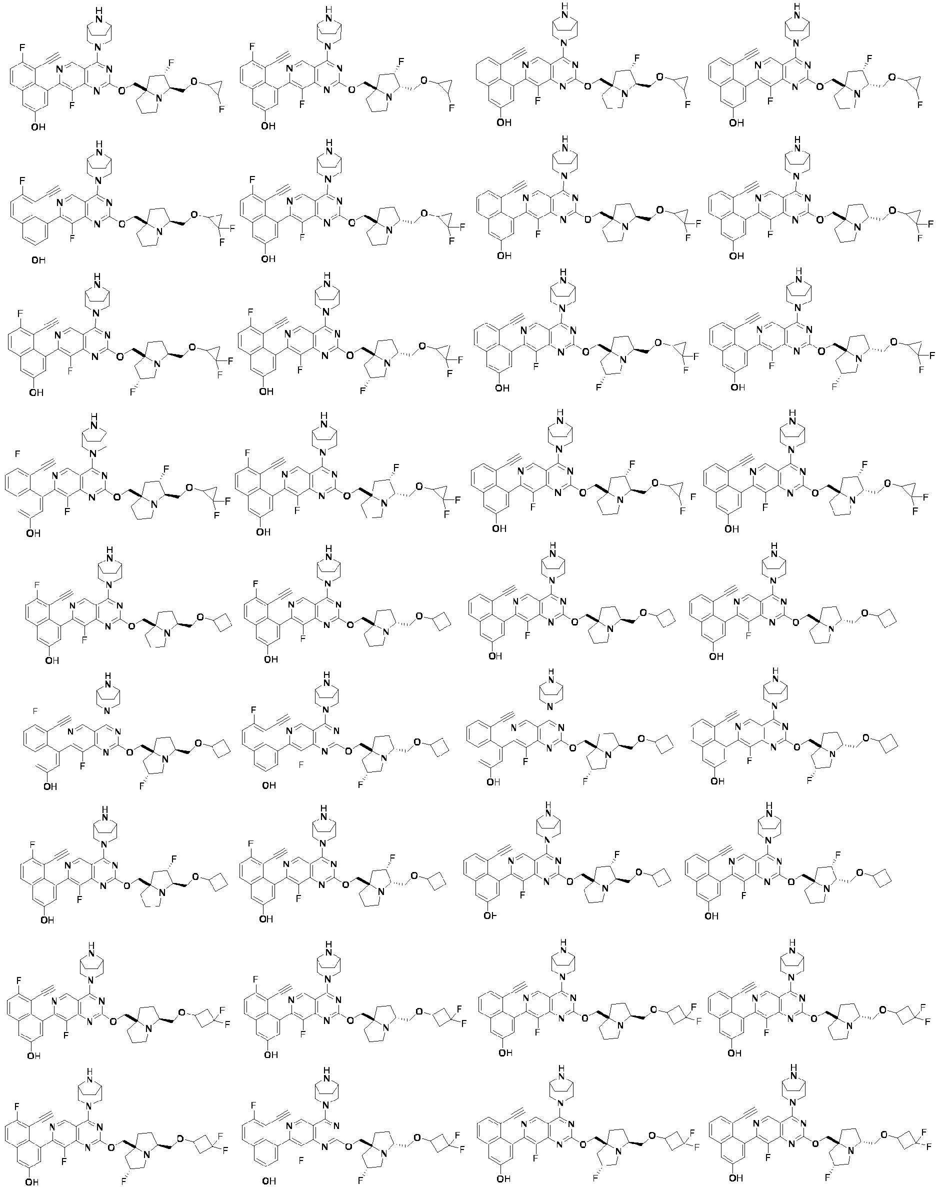 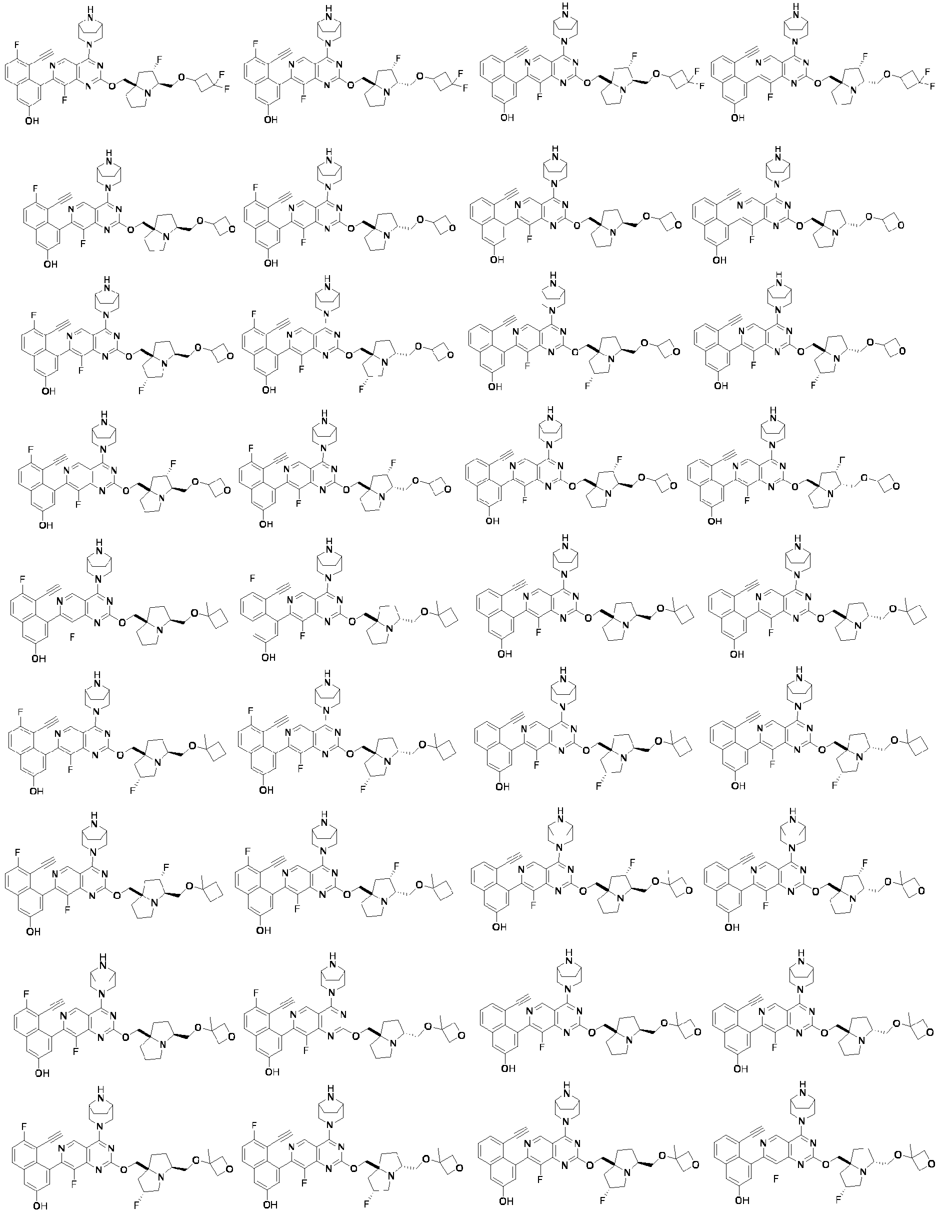 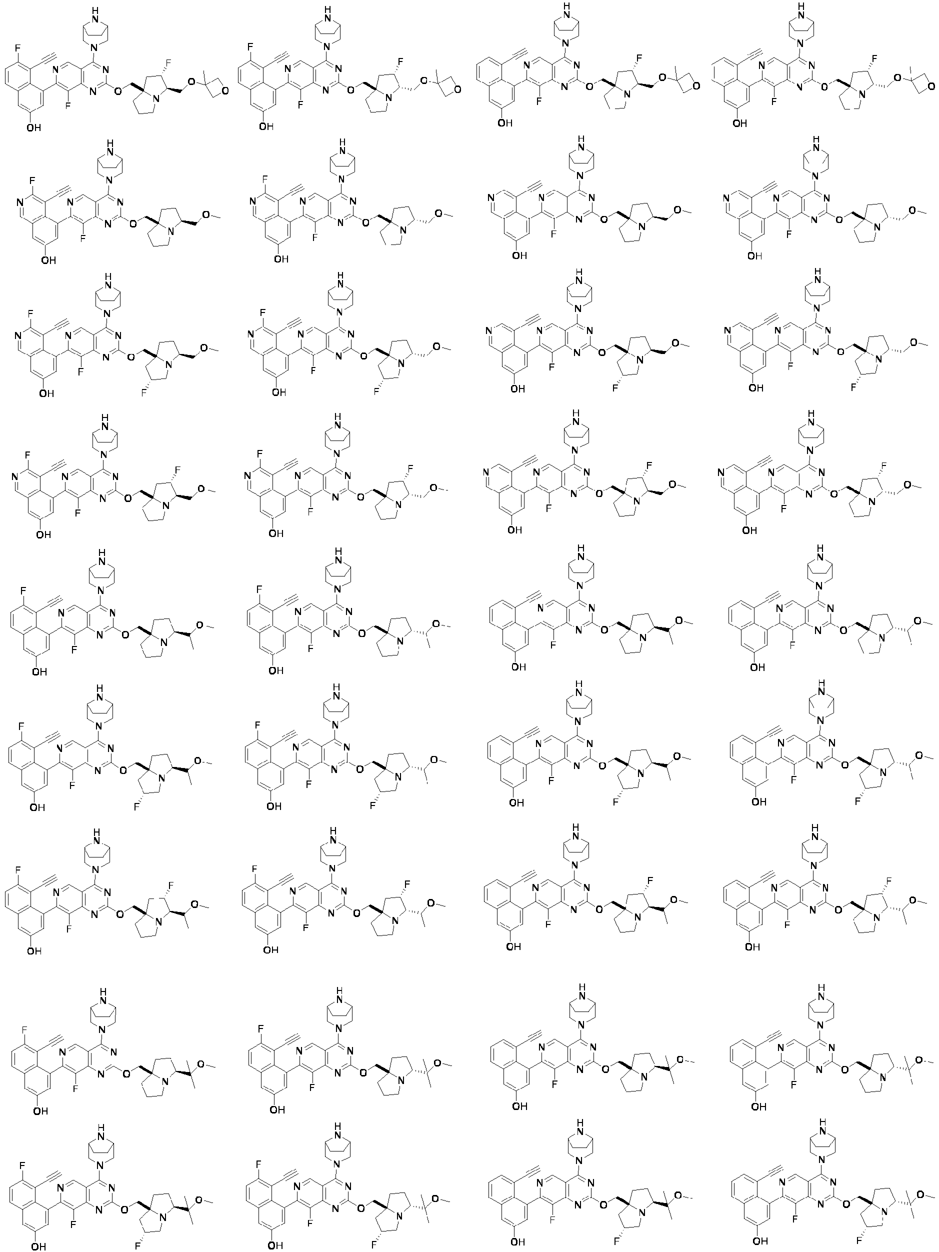 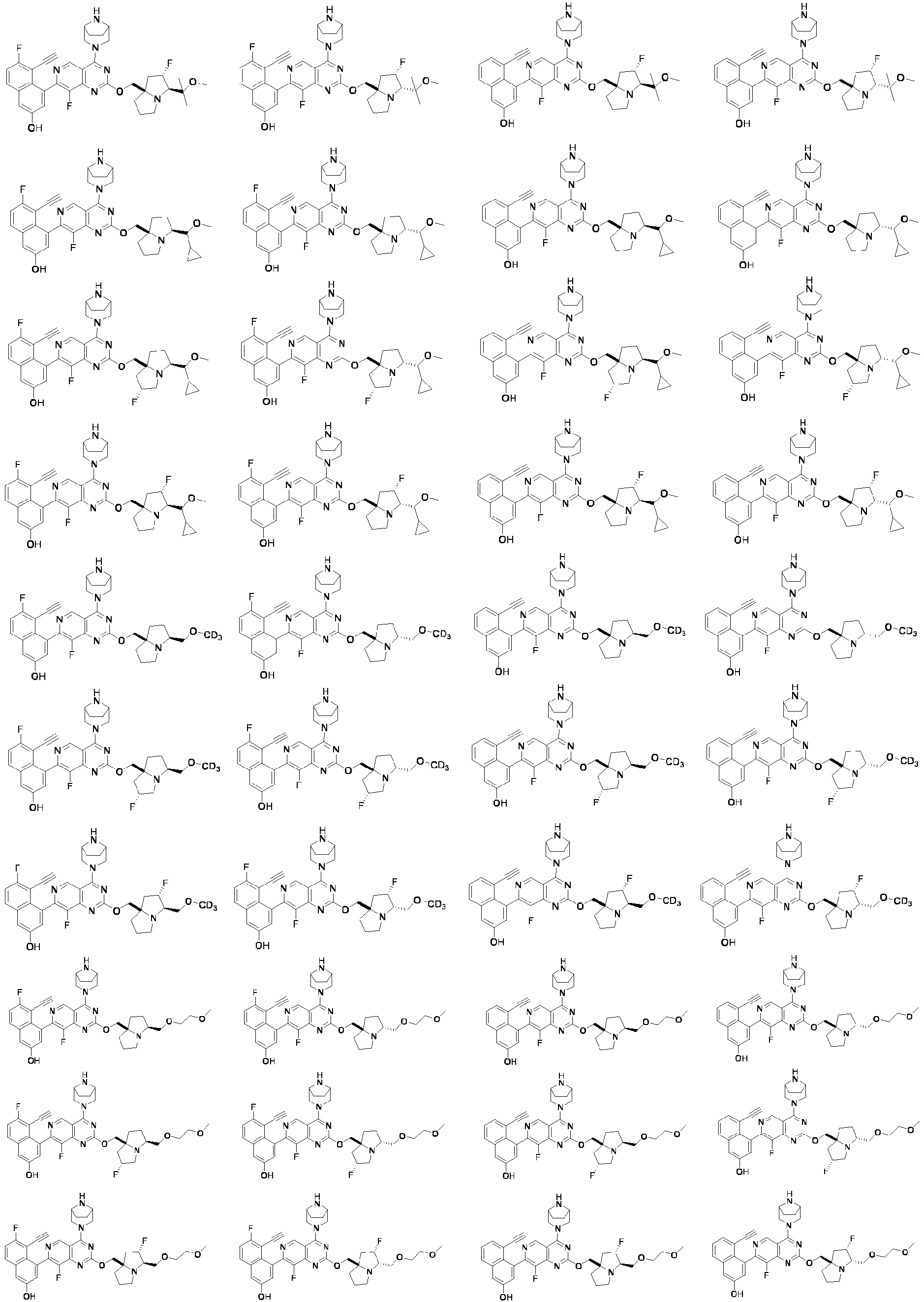 or is selected from 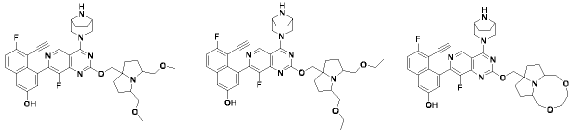 or is selected from 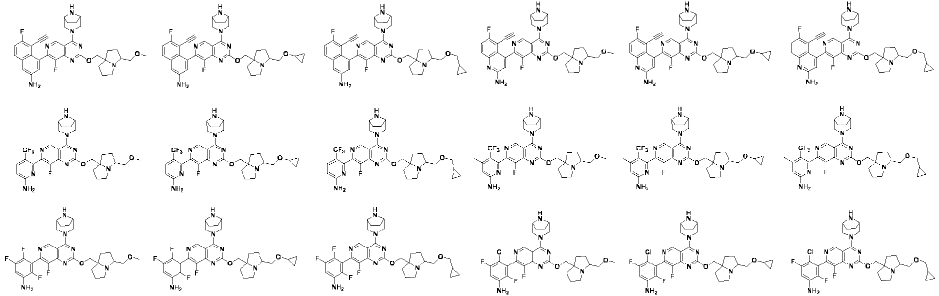 or is selected from 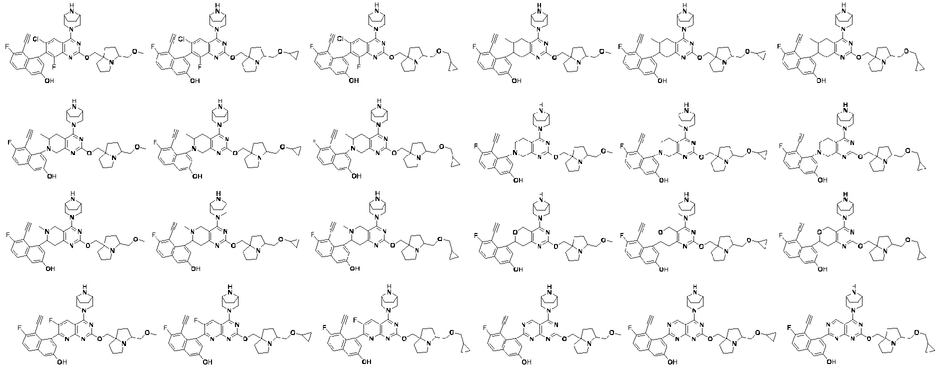 or is selected from 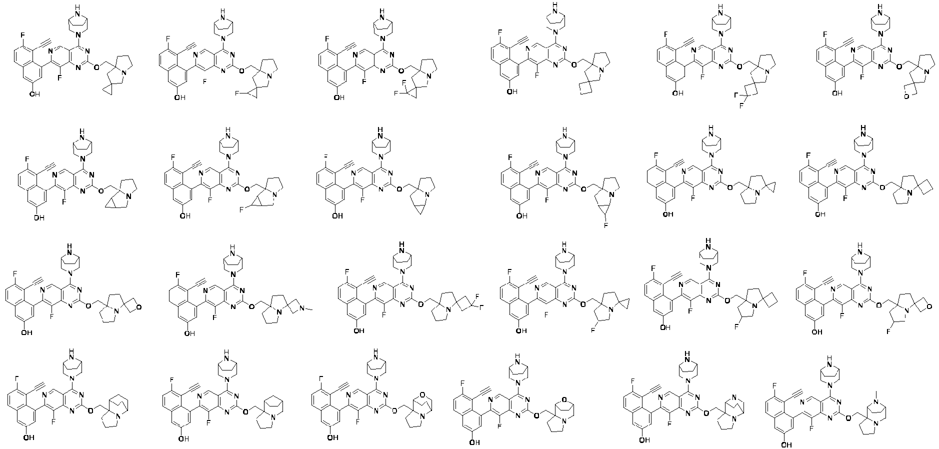 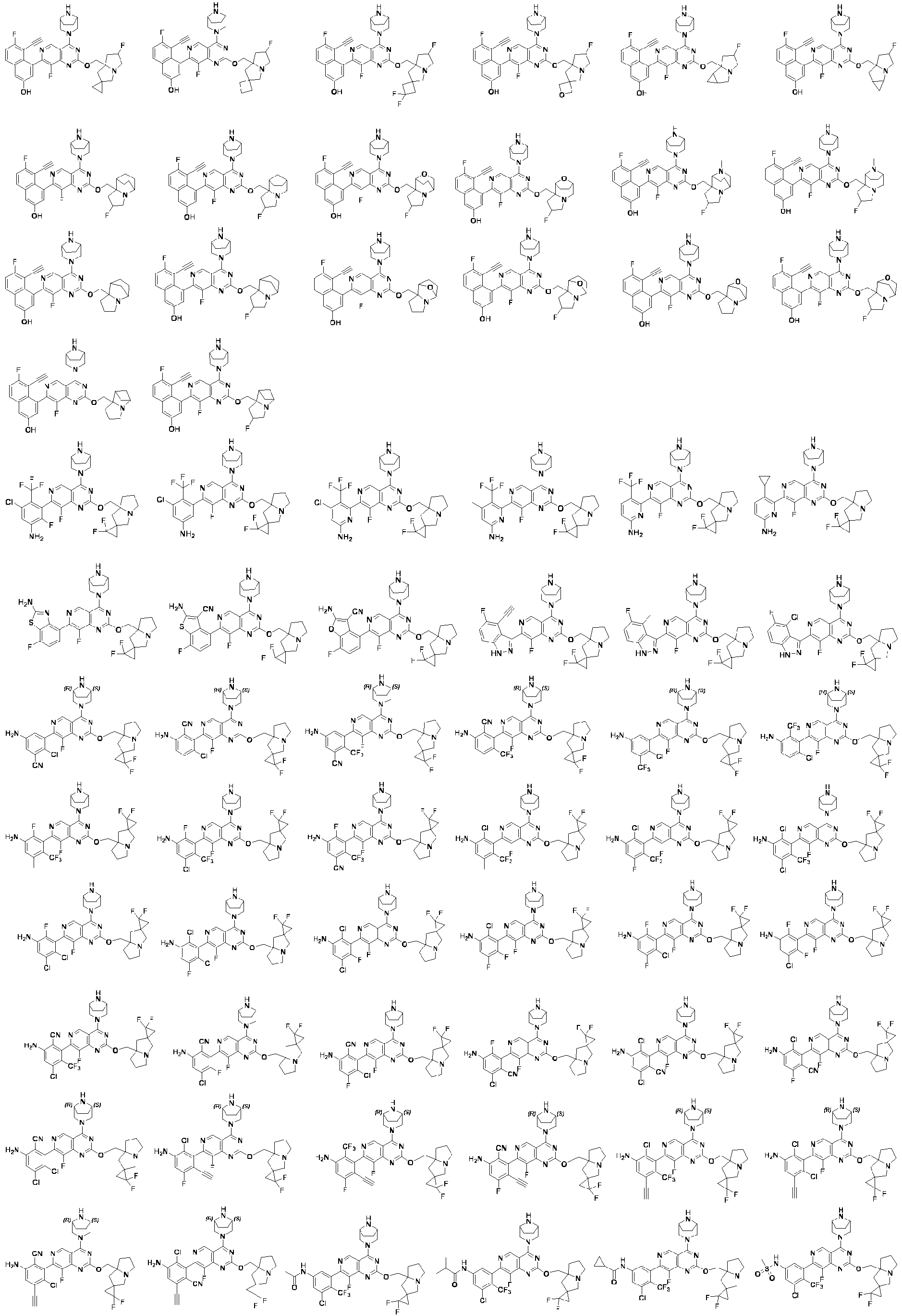 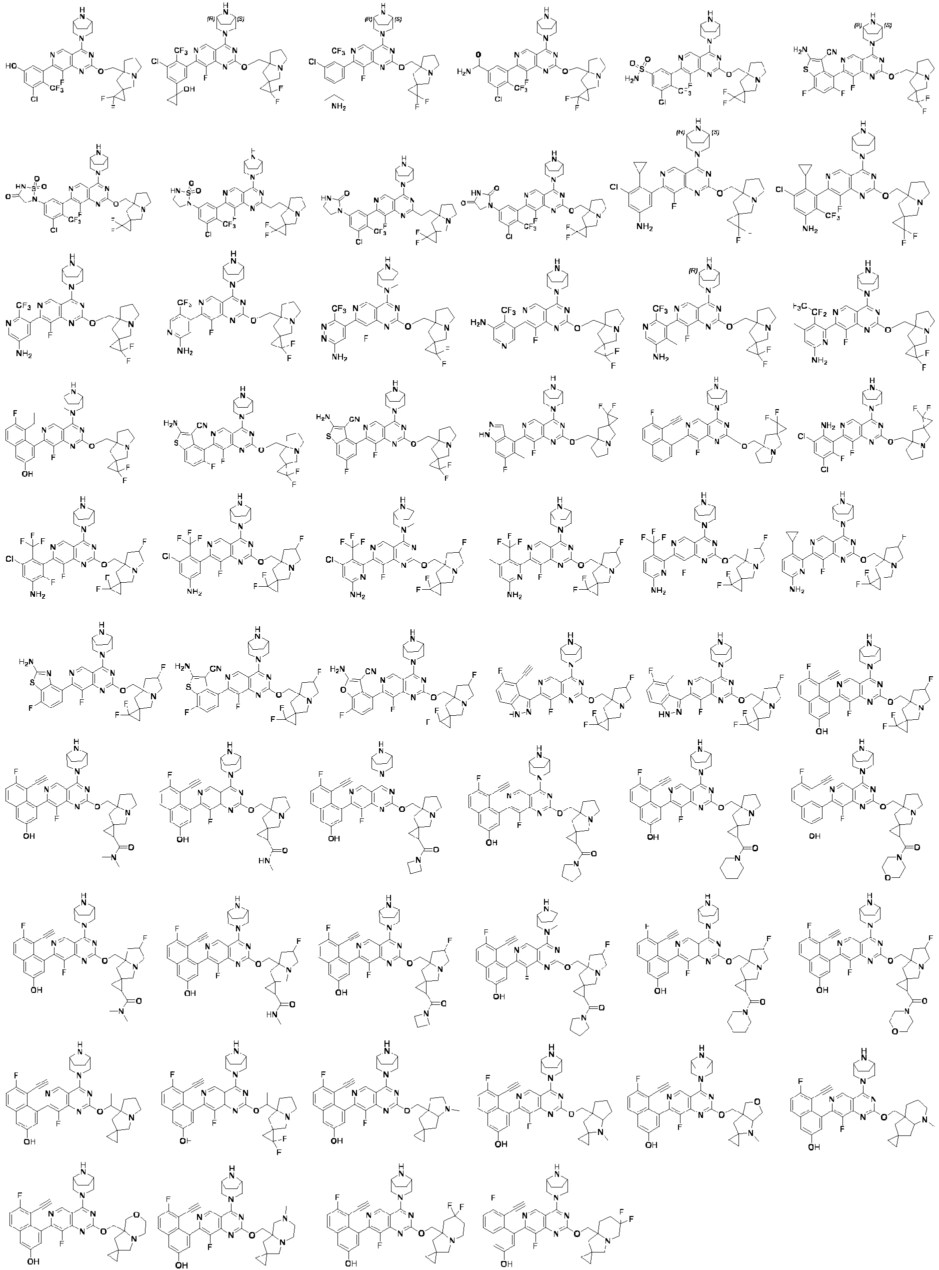 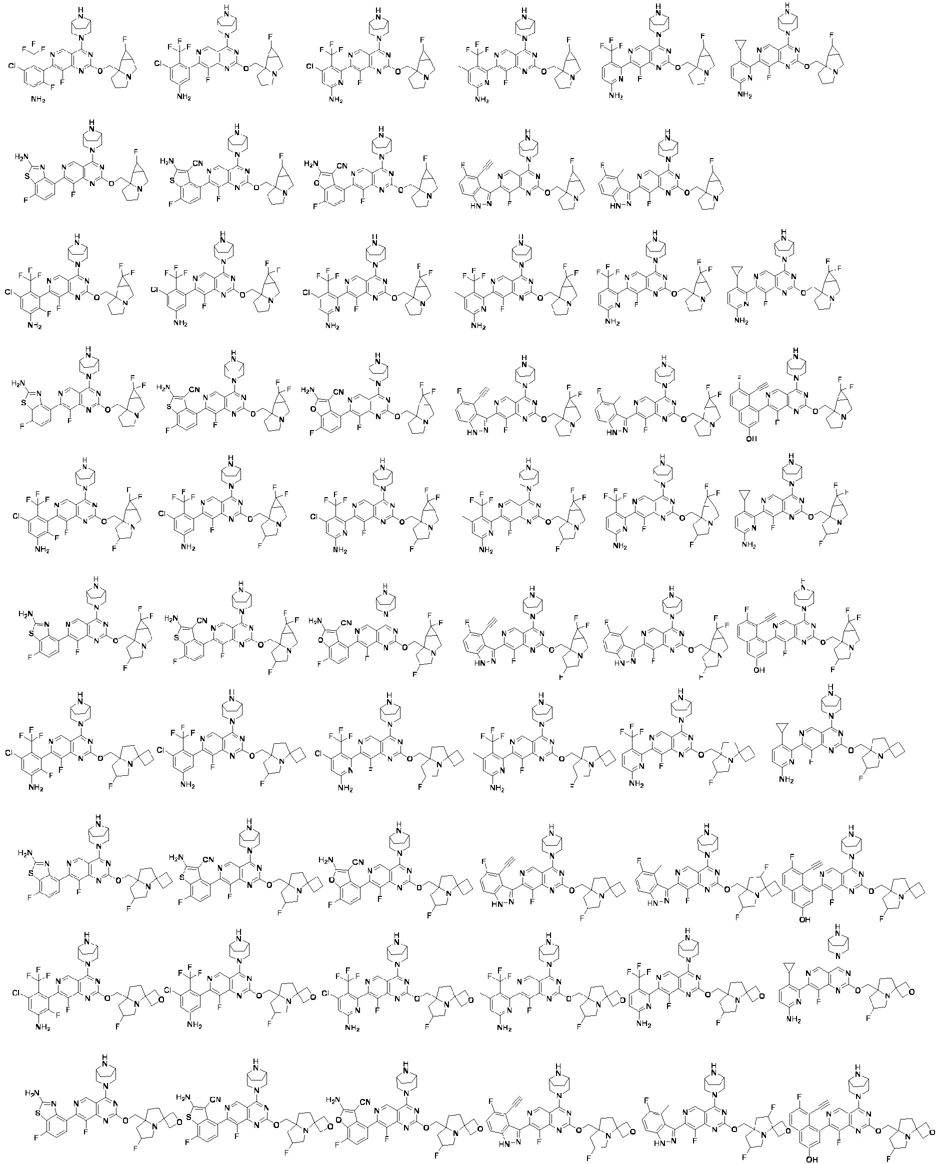 or is selected from: 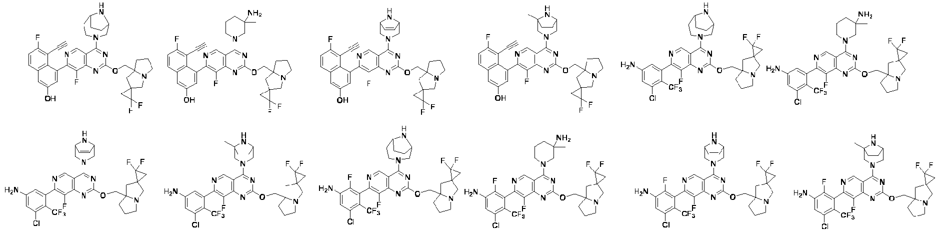 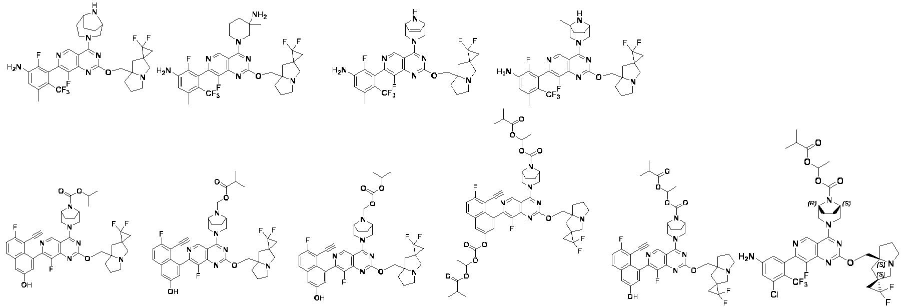 or is selected from: 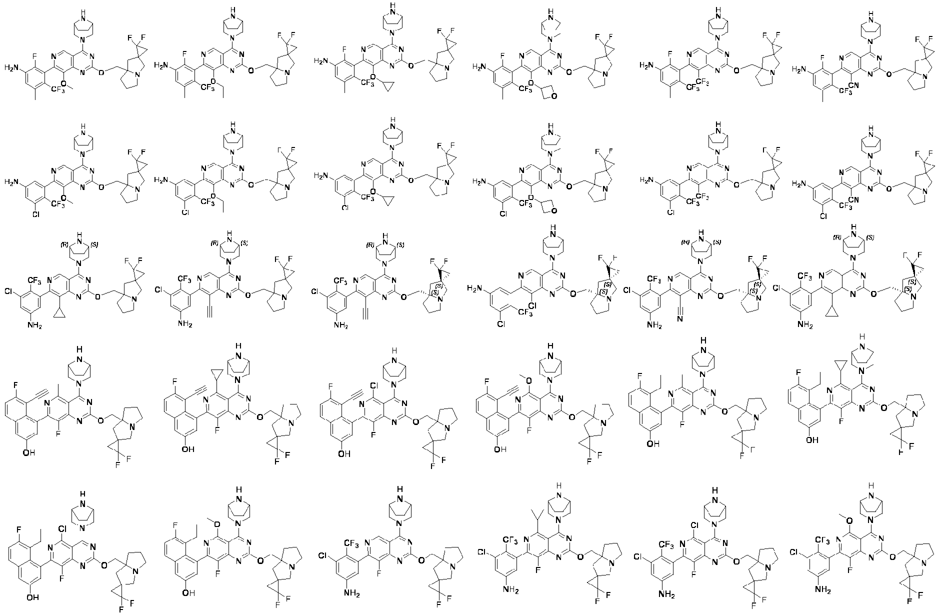 or is selected from:

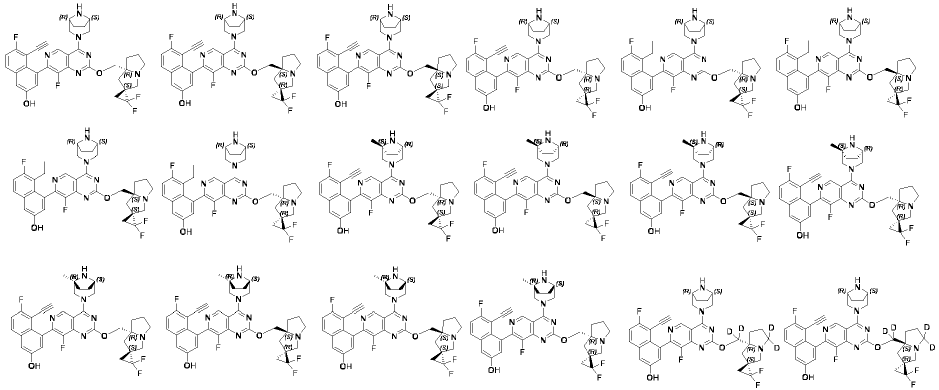

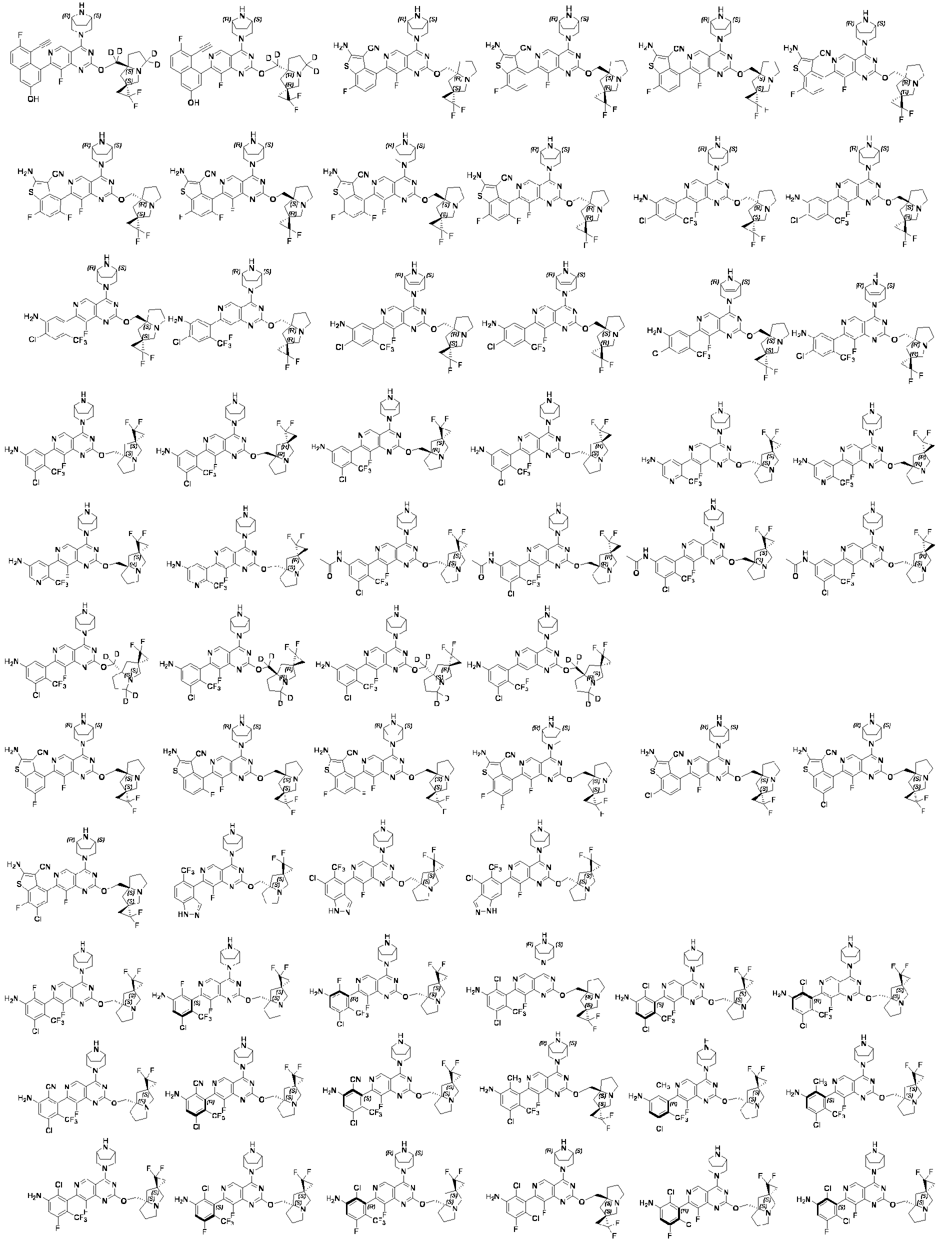

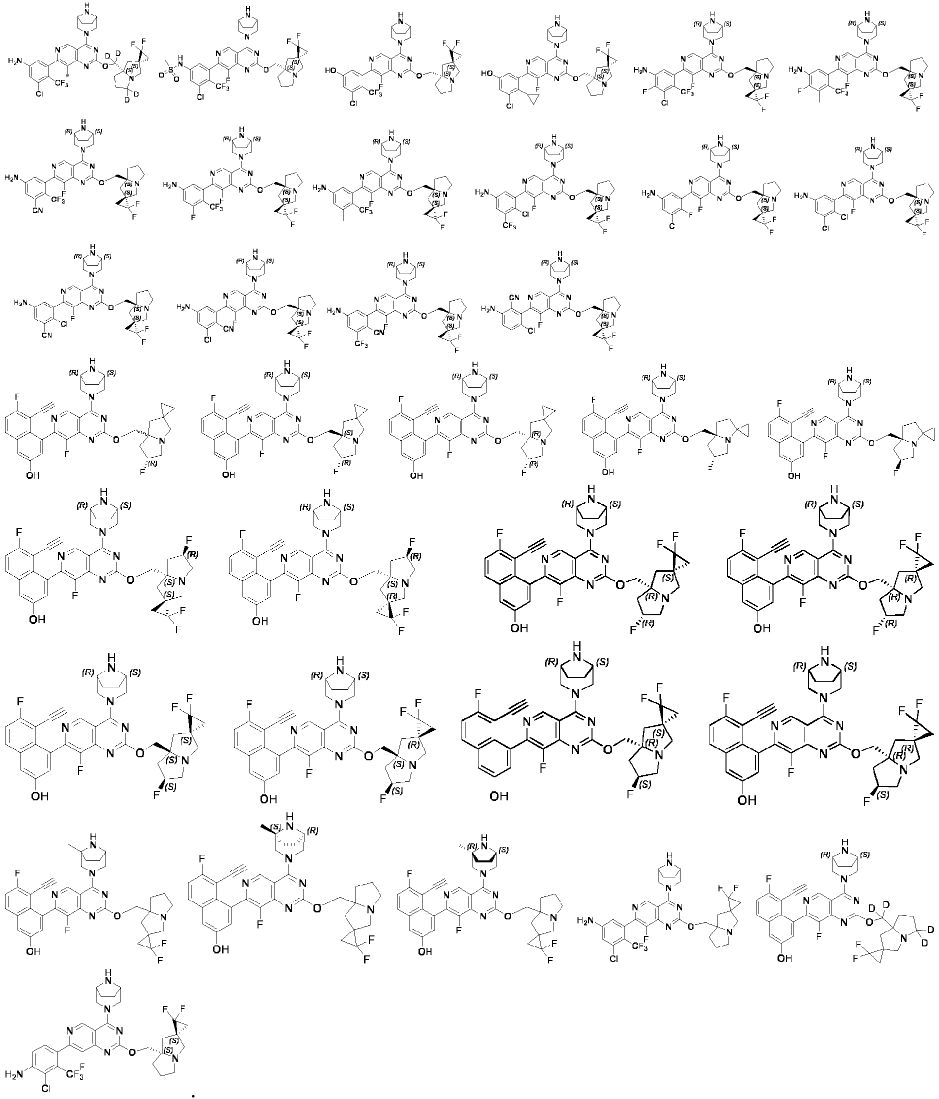

15. A pharmaceutical composition, wherein comprising one or more of the compound of any one of claims 1-14, or the stereoisomer, the tautomer, the crystal form, the pharmaceutically acceptable salt, the hydrate, the solvate, or the prodrug thereof; and pharmaceutically acceptable carriers.

16. A use of the compound of any one of claims 1 -14, or the stereoisomer, the tautomer, the crystal form, the pharmaceutically acceptable salt, the hydrate, the solvate, or the prodrug thereof, or the pharmaceutical composition of claim 15, wherein in preparation of a drug for preventing and/or treating a disease associated with the activity or expression of KRAS^{G12D}.
